# EUROPÄISCHE PATENTSCHRIFT

(11) **EP 2 137 188 B1**
(45) Veröffentlichungstag und Bekanntmachung des Hinweises auf die Patenterteilung: **06.06.2012**
(21) Anmeldenummer: 08716201.2
(22) Anmeldetag: 04.03.2008
(51) Int. Cl.: C07D 487/04, A61K 31/70, A61P 35/00

(54) **SUBSTITUIERTE IMIDAZO- UND TRIAZOLOPYRIMIDINE**
SUBSTITUTED IMIDAZOPYRIMIDINES AND TRIAZOLOPYRIMIDINES
IMIDAZOPYRIMIDINES ET TRIAZOLOPYRIMIDINES SUBSTITUÉES

(30) Priorität: 16.03.2007 DE 102007012645
(43) Veröffentlichungstag der Anmeldung: 30.12.2009
(73) Patentinhaber: Bayer Pharma Aktiengesellschaft, 13353 Berlin (DE)
(72) Erfinder: SIEGEL, Stephan, 10779 Berlin (DE); WILMEN, Andreas, 50676 Köln (DE); RÖHRIG, Susanne, 40724 Hilden (DE); SVENSTRUP, Niels, 42553 Velbert (DE); GNOTH, Mark, Jean, 40822 Mettmann (DE); HEITMEIER, Stefan, 42489 Wülfrath (DE); RESTER, Ulrich, 42115 Wuppertal (DE); ZUBOV, Dmitry, 42857 Remscheid (DE); STAYLE, Jochen, 42115 Wuppertal (DE); SPERZEL, Michael, 58566 Kierspe (DE)
(86) Internationale Anmeldenummer: PCT/EP2008/001682
(87) Internationale Veröffentlichungsnummer: WO 2008/113469

(56) Entgegenhaltungen:
- EP-A- 0 641 564
- WO-A-03/014137

## Beschreibung

Die Erfindung betrifft substituierte Imidazo- und Triazolopyrimidine und Verfahren zu ihrer Herstellung sowie ihre Verwendung zur Herstellung von Arzneimitteln zur Behandlung und/oder Prophylaxe von Krankheiten, insbesondere von hämatologischen Erkrankungen, vorzugsweise von Leukopenien und Neutropenien.

Glykogen Synthase Kinase 3 (GSK3) gehört zur Familie der Serine/Threonin-Kinasen. Spezifische Substrate sind unter anderem Zytoskelettproteine und Transkriptionsfaktoren. Zwei Isoformen, GSK3α und GSK3β, wurden bisher identifiziert (Woodgett JR., Trends Biochem. Sci. (1991), 16(5), 177-81). Beide Isoformen sind in vornehmlich ruhenden, nicht proliferierenden Zellen konstitutiv aktiv.

GSK3β kommt eine zentrale Bedeutung innerhalb des Wnt/Wingless-Signaltransduktionsweges zu. Dieser stellt einer der wichtigsten, evolutionärkonservierten Signalsysteme dar. Wnt-Signale kontrollieren sehr frühe musterbildende Prozesse während der Embryogenese, sie induzieren Mesodermbildung und viele Organe, und sie steuern die Proliferation und Differenzierung von Stammzellen (Wodarz A., Nusse R., Annu. Rev. Cell Dev. Biol. (1998), 14, 59-88; Kirstetter et al., Nat Immunol. (2006), 7(10), 1048-56). Der Wnt-Signalweg ist intrazellulär aufgegliedert, wodurch unterschiedlichste Prozesse gesteuert werden können. Innerhalb der Wnt-Kaskade ist die Glykogen Synthase Kinase 3 Bestandteil eines Multiproteinkomplexes, zu dem u.a. das Strukturmoleküle Axin, das Tumorsuppressor-Protein APC sowie der Transkriptionskofaktor β-Catenin gehören. β-Catenin ist dabei das wichtigste Substrat der GSK3β. Die Konsequenz dieser GSK3β-vermittelten Phophorylierung ist der proteasomale Abbau von β-Catenin. Inhibition der GSK3-Aktivität führt zu einer Akkumulation des β-Catenins in der Zelle mit einer sich anschließenden Translokation in den Zellkern. Dort fungiert β-Catenin als ein Kofaktor in Transkriptionskomplexe und damit für die Expression definierter Zielgene mit verantwortlich.

Strahlen- oder Chemotherapien gehören zu den Standardansätzen bei der Krebsbekämpfung. Beide Therapieformen sind im Bezug auf ihre Zielzellen unspezifisch, d.h. es werden nicht nur Tumorsondern auch nicht-transformierte, proliferierende Zellen getroffen. Zu diesen nichttransformierten, proliferienden Zellen gehören auch hämatopoetische Vorläuferzellen, die sich u.a. zu neutrophilen Granulozyten entwickeln. Eine signifikante Verringerung der Anzahl an Neutrophilen wird als Neutropenie bezeichnet. Eine durch Chemo- oder Strahlentherapie induzierte Neutropenie resultiert klinisch in einer erhöhten Infektanfälligkeit. Bei ausgeprägter Neutropenie erhöht sich die Morbidität und unter Umständen auch die Mortalität einer Therapie (O'Brien et al., British Journal of Cancer (2006), 95, 1632 - 1636).

Inhibition der GSK3-Aktivität führt zu einer gesteigerten Proliferations- und Differenzierungsrate hämatopoetischer Stammzellen und kann dementsprechend zur therapeutischen Intervention hinsichtlich einer therapieinduzierten Neutropenie genutzt werden.

W02006/044687 beschreibt die Verwendung von Imidazopyrimidinylaminen als Kinase Inhibitoren zur Behandlung von Krebs und WO01/083485 offenbart Imidazo- und Triazolopyrimidine unter anderem zur Behandlung von Asthma und Krebs. WO2005/044793 offenbart unter anderem die Verwendung von Imidazopyrimidinylaminen als CRF (corticotropin releasing factor) Rezeptor Antagonisten zur Behandlung von Depressionen.

Eine Aufgabe der vorliegenden Erfindung ist daher die Bereitstellung neuer Verbindungen als GSK3β-Inhibitoren zur Behandlung von hämatologischen Erkrankungen, vorzugsweise von Neutropenie bei Menschen und Tieren.

Gegenstand der Erfindung sind Verbindungen der Formel 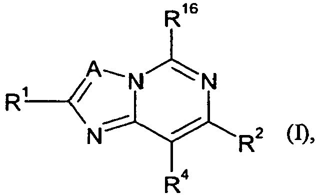 in welcher
- A: für N oder CR¹⁵ steht, wobei R¹⁵ für Wasserstoff, Brom oder Chlor steht,
- R¹: für Wasserstoff, Hydroxy, Amino, Hydroxycarbonyl, Aminocarbonyl, Trifluormethyl, Trifluormethoxy, Cyano, C₁-C₄-Alkyl, C₁-C₄-Alkoxy, C₁-C₆-Alkylamino, C₁-C₄-Alkylcarbonyl, C₁-C₄-Alkoxycarbonyl, C₁-C₆-Alkylaminocarbonyl, Dihydroxypropylaminocarbonyl, Dihydroxybutylaminocarbonyl, Dihydroxypentylaminocarbonyl, C₁-C₄-Alkylcarbonylamino, C₁-C₄-Alkylsulfonylamino, 5- oder 6-gliedriges Heterocyclyl-carbonyl, -CH₂R¹³ oder -CH₂CH₂R¹⁴ steht, wobei Alkoxy, Alkylamino, Alkylcarbonyl, Alkoxycarbonyl, Alkylaminocarbonyl, Alkylcarbonylamino und Alkylsulfonylamino substituiert sein können mit einem Substituenten, wobei der Substituent ausgewählt wird aus der Gruppe bestehend aus Hydroxy, Amino, Hydroxycarbonyl, Aminocarbonyl, C₁-C₄-Alkoxy, C₁-C₄-Alkylamino, C₁-C₄-Alkoxycarbonyl, C₁-C₄-Alkylaminocarbonyl, C₁-C₄-Alkylcarbonylamino und 5- oder 6-gliedriges Heterocyclyl, worin Heterocyclyl substituiert sein kann mit 1 bis 3 Substituenten, wobei die Substituenten unabhängig voneinander ausgewählt werden aus der Gruppe bestehend aus Halogen, Hydroxy, Oxo, C₁-C₄-Alkyl, C₁-C₄-Alkoxy, C₁-C₄-Alkylamino, C₁-C₄-Alkylearbonyl, C₁-C₄-Alkoxycarbonyl und C₁-C₄-Alkylaminocarbonyl, und wobei Heterocyclylcarbonyl substituiert sein kann mit 1 bis 3 Substituenten, wobei die Substituenten unabhängig voneinander ausgewählt werden aus der Gruppe bestehend aus Halogen, Hydroxy, Oxo, C₁-C₄-Alkyl, C₁-C₄-Alkoxy, C₁-C₄-Alkylamino, C₁-C₄-Alkylcarbonyl, C₁-C₄-Alkoxycarbonyl und C₁-C₄-Alkylaminocarbonyl, und wobei
R¹³ für Hydroxy, Amino, Cyano, Hydroxycarbonyl, Aminocarbonyl, C₁-C₄-Alkoxy, C₁-C₄-Alkylamino, C₁-C₄-Alkoxycarbonyl, C₁-C₄-Alkylaminocarbonyl, C₁-C₄ Alkylcarbonylamino, 5- oder 6-gliedriges Heterocyclyl oder 5- oder 6-gliedriges Heteroaryl steht, worin Alkoxy, Alkylamino, Alkoxycarbonyl, Alkylaminocarbonyl und Alkylcarbonylamino substituiert sein können mit einem Substituenten, wobei der Substituent ausgewählt wird aus der Gruppe bestehend aus Hydroxy, Amino, Hydroxycarbonyl, Aminocarbonyl, C₁-C₄-Alkoxy, C₁-C₄-Alkylamino, C₁-C₄-Alkoxycarbonyl, C₁-C₄-Alkylaminocarbonyl und C₁-C₄-Alkylcarbonylamino, und worin Heterocyclyl und Heteroaryl substituiert sein können mit 1 bis 3 Substituenten, wobei die Substituenten unabhängig voneinander ausgewählt werden aus der Gruppe bestehend aus Halogen, Hydroxy, Oxo, C₁-C₄-Alkyl, C₁-C₄-Alkoxy, C₁-C₄-Alkylamino, C₁-C₄-Alkylearbonyl, C₁-C₄-Alkoxycarbonyl und C₁-C₄-Alkylaminocarbonyl, und wobei
R¹⁴ für Hydroxy, Amino, Cyano, Hydroxycarbonyl, Aminocarbonyl, C₁-C₄-Alkoxy, C₁-C₄-Alkylamino, C₁-C₄-Alkoxycarbonyl, C₁-C₄-Alkylaminocarbonyl, C₁-C₄-Alkylcarbonylamino, 5- oder 6-gliedriges Heterocyclyl oder 5- oder 6-gliedriges Heteroaryl steht, worin Alkoxy, Alkylamino, Alkoxycarbonyl, Alkylaminocarbonyl und Alkylcarbonylamino substituiert sein können mit einem Substituenten, wobei der Substituent ausgewählt wird aus der Gruppe bestehend aus Hydroxy, Amino, Hydroxycarbonyl, Aminocarbonyl, C₁-C₄-Alkoxy, C₁-C₄-Alkylamino, C₁-C₄-Alkoxycarbonyl, C₁-C₄-Alkylaminocarbonyl und C₁-C₄-Alkylcarbonylamino, und worin Heterocyclyl und Heteroaryl substituiert sein können mit 1 bis 3 Substituenten, wobei die Substituenten unabhängig voneinander ausgewählt werden aus der Gruppe bestehend aus Halogen, Hydroxy, Oxo, C₁-C₄-Alkyl, C₁-C₄-Alkoxy, C₁-C₄-Alkylamino, C₁-C₄-Alkylcarbonyl, C₁-C₄-Aloxycarbonylund C₁-C₄-Alkylaminocarbonyl,
- R²: für C₆-C₁₀-Aryl oder 5- bis 10-gliedriges Heteroaryl steht, wobei Aryl und Heteroaryl substituiert sein können mit 1 bis 3 Substituenten, wobei die Substituenten unabhängig voneinander ausgewählt werden aus der Gruppe bestehend aus Hydroxy, Hydroxymethyl, Amino, Halogen, Cyano, Trifluormethyl, Trifluormethoxy, Aminocarbonyl, C₁-C₄-Alkyl, C₁-C₄-Alkoxy, C₁-C₄-Alkoxymethyl, C₁-C₄-Alkylamino, C₁-C₄-Alkylaminomethyl, C₁-C₄-Alkylcarbonyl, C₁-C₄-Alkoxycarbonyl, C₁-C₄-Alkylaminocarbonyl, C₁-C₄-Alkylcarbonylamino, C₁-C₄-Alkylsulfonyl, C₁-C₄-Alkylsulfonylamino, C₁-C₄-Alkylaminosulfonyl, Phenyl, Benzyloxy, 5- oder 6-gliedrigem Heterocyclyl, 5- oder 6-gliedrigem Heterocyclylcarbonyl, 5- oder 6-gliedrigem Heterocyclylmethyl und 5- oder 6-gliedrigem Heteroaryl, worin Phenyl, Benzyloxy, Heterocyclyl, Heterocyclylcarbonyl, Heterocyclylmethyl und Heteroaryl substituiert sein können mit 1 bis 3 Substituenten, wobei die Substituenten unabhängig voneinander ausgewählt werden aus der Gruppe bestehend aus Halogen, Cyano, Trifluormethyl, Trifluormethoxy, Aminocarbonyl, C₁-C₄-Alkyl, C₁-C₄-Alkoxy, C₁-C₄-Alkylarnino, C₁-C₄-Alkylcarbonyl, C₁-C₄-Alkoxycarbonyl, C₁-C₄-Alkylaminocarbonyl und C₁-C₄-Alkylcarbonylamino, oder zwei der Substituenten am Aryl zusammen mit den Kohlenstoffatomen, an die sie gebunden sind, ein 1,3-Dioxolan oder 1,4-Dioxan bilden,
- R⁴: für Wasserstoff, Halogen, Cyano, Trifluormethyl, C₁-C₃-Alkyl, Methylthio oder Cyclopropyl steht,
- R¹⁶: für eine Gruppe der Formel 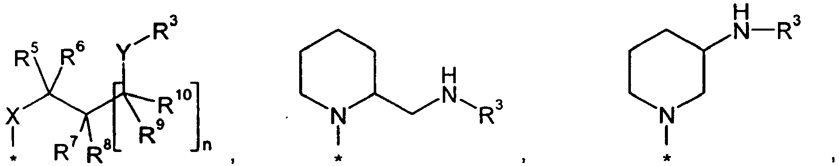 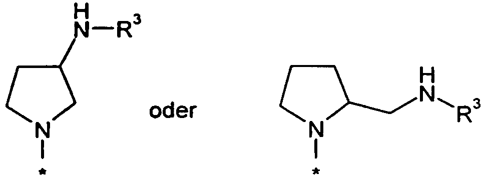 steht, wobei
* die Anknüpfstelle an den Heterocyclus bedeutet,
n für die Zahl 0 oder 1 steht,
X für NR¹¹, S oder O steht, wobei R¹¹ für Wasserstoff, C₁-C₃-Alkyl oder Cyclopropyl steht,
Y für NR¹², S oder O steht, wobei
R¹² für Wasserstoff, C₁-C₃-Alkyl oder Cyclopropyl steht,
- R³: für 2-Pyridyl, Pyrimid-2-yl, 2-Aminopyrimid-4-yl, 2-Cyclopropylaminopyrimid-4-yl, 2-Methylaminopyrimid-4-yl, 2-Ethylaminopyrimid-4-yl, 1,3-Thiazol-2-yl, 1,3-Thiazol-4-yl oder 1,3-Thiazol-5-yl steht, wobei 2-Pyridyl, Pyrimid-2-yl, 2-Aminopyrimid-4-yl, 1,3-Thiazol-2-yl, 1,3-Thiazol-4-yl und 1,3-Thiazol-5-yl substituiert sind mit 1 bis 3 Substituenten, wobei die Substituenten unabhängig voneinander ausgewählt werden aus der Gruppe bestehend aus Halogen, Cyano, Nitro, Amino, Trifluormethyl, Trifluormethoxy, Aminocarbonyl, Trifluormethylcarbonyl, C₁-C₄-Alkyl, C₁-C₄-Alkoxy, C₁-C₄-Alkylamino, C₁-C₄-Alkylearbonyl, C₁-C₄-Alkoxycarbonyl, C₁-C₄-Alkylaminocarbonyl und C₃-C₆-Cycloalkylcarbonyl, worin Alkyl, Alkoxy, Alkylamino, Alkylcarbonyl, Alkoxycarbonyl, Alkylaminocarbonyl und Cycloalkylcarbonyl substituiert sein können mit einem Substituenten, wobei der Substituent ausgewählt wird aus der Gruppe bestehend aus Halogen, Cyano, Hydroxy, Amino, Trifluormethyl und C₃-C₆-Cycloalkyl,
- oder R³: für eine Gruppe der Formel 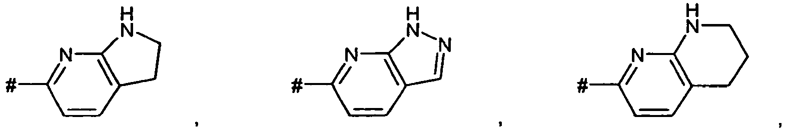 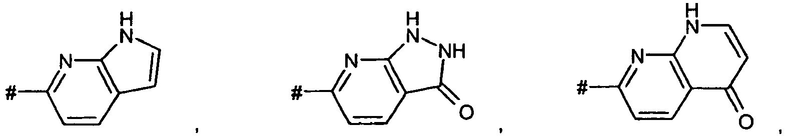 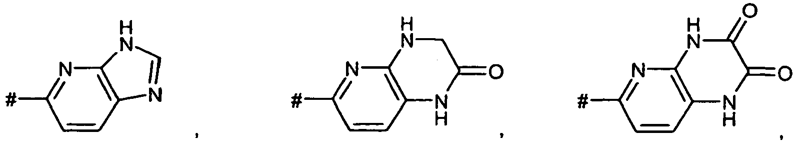 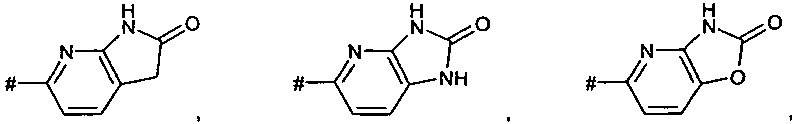 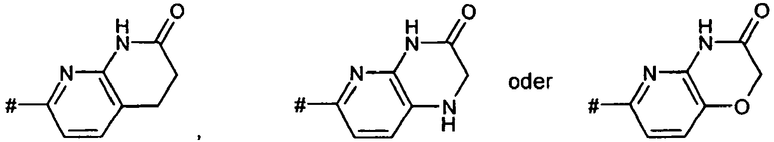 steht,
wobei # die Anknüpfstelle an Y bedeutet,
- R⁵: für Wasserstoff, C₁-C₃-Alkyl oder Cyclopropyl steht,
- R⁶: für Wasserstoff oder C₁-C₃-Alkyl steht,
- R⁷: für Wasserstoff, C₁-C₃-Alkyl oder Cyclopropyl steht,
- R⁸: für Wasserstoff oder C₁-C₃-Alkyl steht,
- R⁹: für Wasserstoff, C₁-C₃-Alkyl oder Cyclopropyl steht,
- R¹⁰: für Wasserstoff oder C₁-C₃-Alkyl steht,
und ihre Salze, ihre Solvate und die Solvate ihrer Salze.

Erfindungsgemäße Verbindungen sind die Verbindungen der Formel (I) und deren Salze, Solvate und Solvate der Salze, sowie die von Formel (I) umfassten, nachfolgend als Ausführungsbeispiel(e) genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung umfasst deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegenden Erfindung sämtliche tautomere Formen.

Als Salze sind im Rahmen der vorliegenden Erfindung physiologisch unbedenkliche Salze der erfmdungsgemäßen Verbindungen bevorzugt. Umfasst sind aber auch Salze, die für pharmazeutische Anwendungen selbst nicht geeignet sind aber beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, *N*-Methylmorpholin, Arginin, Lysin, Ethylendiamin, *N*-Methylpiperidin und Cholin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Außerdem umfasst die vorliegende Erfindung auch Prodrugs der erfindungsgemäßen Verbindungen. Der Begriff "Prodrugs" umfaßt Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Rahmen der vorliegenden Erfindung haben die Substituenten, soweit nicht anders spezifiziert, die folgende Bedeutung:

Alkyl per se und "Alk" und "Alkyl" in Alkoxy Alkylamino, Alkylcarbonyl, Alkoxycarbonyl Alkylaminocarbonyl, Alkylcarbonylamino, Alkylsulfonyl, Alkylsulfonylamino und Alkylaminosulfonyl stehen für einen linearen oder verzweigten Alkylrest mit 1 bis 6, bevorzugt 1 bis 4 Kohlenstoffatomen, beispielhaft und vorzugsweise für Methyl, Ethyl, n-Propyl, iso-Propyl, n-Butyl, tert-Butyl, n-Pentyl und n-Hexyl.

Alkoxy steht beispielhaft und vorzugsweise für Methoxy, Ethoxy, n-Propoxy, iso-Propoxy, n-Butoxy und tert-Butoxy.

Alkylamino steht für einen Alkylaminorest mit einem oder zwei (unabhängig voneinander gewählten) Alkylsubstituenten, beispielhaft und vorzugsweise für Methylamino, Ethylamino, n-Propylamino, iso-Propylamino, tert-Butylamino, *N,N*-Dimethylamino, *N,N*-Diethylamino, *N*-Ethyl-N-methylamino, *N*-Methyl-*N*-n-propylamino, *N*-iso-Propyl-*N*-n-propylamino und N-tert-Butyl-*N-*methylamino. C₁-C₄-Alkylamino steht beispielsweise für einen Monoalkylaminorest mit 1 bis 4 Kohlenstoffatomen oder für einen Dialkylaminorest mit jeweils 1 bis 4 Kohlenstoffatomen pro Alkylsubstituent.

Alkylcarbonyl steht beispielhaft und vorzugsweise für Methylcarbonyl, Ethylcarbonyl, n-Propylcarbonyl, iso-Propylcarbonyl, n-Butylcarbonyl und tert-Butylcarbonyl.

Alkoxycarbonyl steht beispielhaft und vorzugsweise für Methoxycarbonyl, Ethoxycarbonyl, n-Propoxycarbonyl, iso-Propoxycarbonyl, n-Butoxycarbonyl und tert-Butoxycarbonyl.

Alkylaminocarbonyl steht für einen Alkylaminocarbonylrest mit einem oder zwei (unabhängig voneinander gewählten) Alkylsubstituenten, beispielhaft und vorzugsweise für Methylaminocarbonyl, Ethylaminocarbonyl, n-Propylaminocarbonyl, iso-Propylaminocarbonyl, tert-Butylaminocarbonyl, *N,N*-Dimethylaminocarbonyl, *N,N*-Diethylaminocarbonyl, *N*-Ethyl-*N*-methylaminocarbonyl, *N-*Methyl-*N*-n-propylaminocarbonyl, *N*-iso-Propyl-*N*-n-propylaminocarbonyl und *N*-tert-Butyl-*N-*methylaminocarbonyl. C₁-C₄-Alkylaminocarbonyl steht beispielsweise für einen Monoalkylaminocarbonylrest mit 1 bis 4 Kohlenstoffatomen oder für einen Dialkylaminocarbonylrest mit jeweils 1 bis 4 Kohlenstoffatomen pro Alkylsubstituent.

Alkylcarbonylamino steht beispielhaft und vorzugsweise für Methylcarbonylamino, Ethylcarbonylamino, n-Propylcarbonylamino, iso-Propylcarbonylamino, n-Butylcarbonylamino und tert-Butylcarbonylamino.

Alkylsulfonyl steht beispielhaft und vorzugsweise für Methylsulfonyl, Ethylsulfonyl, n-Propylsulfonyl, iso-Propylsulfonyl, n-Butylsulfonyl und tert-Butylsulfonyl.

Alkylaminosulfonyl steht für einen Alkylaminosulfonylrest mit einem oder zwei (unabhängig voneinander gewählten) Alkylsubstituenten, beispielhaft und vorzugsweise für Methylaminosulfonyl, Ethylaminosulfonyl, n-Propylaminosulfonyl, iso-Propylaminosulfonyl, tert-Butylaminosulfonyl, *N,N-*Dimethylaminosulfonyl, *N,N*-Diethylaminosulfonyl, *N*-Ethyl-*N*-methylaminosulfonyl, *N*-Methyl-*N*-n-propylaminosulfonyl, *N*-iso-Propyl-*N*-n-propylaminosulfonyl und *N*-tert-Butyl-*N-*methylaminosulfonyl. C₁-C₄-Alkylaminosulfonyl steht beispielsweise für einen Monoalkylaminosulfonylrest mit 1 bis 4 Kohlenstoffatomen oder für einen Dialkylamino-sulfonylrest mit jeweils 1 bis 4 Kohlenstoffatomen pro Alkylsubstituent.

Alkylsulfonylamino steht beispielhaft und vorzugsweise für Methylsulfonylamino, Ethylsulfonylamino, n-Propylsulfonylamino, iso-Propylsulfonylamino, n-Butylsulfonylamino und tert-Butylsulfonylamino.

Cycloalkyl steht für eine monocyclische Cycloalkylgruppe mit in der Regel 3 bis 6 Kohlenstoffatomen, beispielhaft und vorzugsweise für Cycloalkyl seien genannt Cyclopropyl, Cyclobutyl, Cyclopentyl und Cyclohexyl.

Heterocyclyl steht für einen monocyclischen, heterocyclischen Rest mit 5 oder 6 Ringatomen und bis zu 3, vorzugsweise bis zu 2 Heteroatomen und/oder Heterogruppen aus der Reihe N, O, S, SO, SO₂, wobei ein Stickstoffatom auch ein N-Oxid bilden kann. Die Heterocyclyl-Reste können gesättigt oder teilweise ungesättigt sein. Bevorzugt sind 5- oder 6-gliedrige, monocyclische gesättigte Heterocyclylreste mit bis zu zwei Heteroatomen aus der Reihe O, N und S, beispielhaft und vorzugsweise für Pyrrolidin-2-yl, Pyrrolidin-3-yl, Pyrrolinyl, Tetrahydrofuranyl, Tetrahydrothienyl, Pyranyl, Piperidin-1-yl, Piperidin-2-yl, Piperidin-3-yl, Piperidin-4-yl, Thiopyranyl, Morpholin-1-yl, Morpholin-2-yl, Morpholin-3-yl, Piperazin-1-yl, Piperazin-2-yl.

Heteroaryl steht für einen aromatischen, mono- oder bicyclischen Rest mit in der Regel 5 bis 10, vorzugsweise 5 oder 6 Ringatomen und bis zu 5, vorzugsweise bis zu 4 Heteroatomen aus der Reihe S, O und N, wobei ein Stickstoffatom auch ein N-Oxid bilden kann, beispielhaft und vorzugsweise für Thienyl, Furyl, Pyrrolyl, Thiazolyl, Oxazolyl, Oxadiazolyl, Pyrazolyl, Imidazolyl, Triazolyl, Pyridyl, Pyrimidyl, Pyridazinyl, Pyrazinyl, Indolyl, Indazolyl, Benzofuranyl, Benzothiophenyl, Chinolinyl, Isochinolinyl, Benzoxazolyl, Benzimidazolyl.

Halogen steht für Fluor, Chlor, Brom und Jod, vorzugsweise für Fluor und Chlor.

In den Formeln der Gruppe, die für R³ stehen kann, steht der Endpunkt der Linie, neben der jeweils ein # steht, nicht für ein Kohlenstoffatom beziehungsweise eine CH₂-Gruppe sondern ist Bestandteil der Bindung zu dem Atom, an das R³ gebunden ist.

In den Formeln der Gruppe, die für R¹⁶ stehen kann, steht der Endpunkt der Linie, neben der jeweils ein * steht, nicht für ein Kohlenstoffatom beziehungsweise eine CH₂-Gruppe sondern ist Bestandteil der Bindung zu dem Atom, an das R¹⁶ gebunden ist.

Bevorzugt sind Verbindungen der Formel (I), in welcher
- A: für N oder CR¹⁵ steht, wobei R¹⁵ für Wasserstoff, Brom oder Chlor steht,
- R¹: für Wasserstoff, Hydroxy, Amin, Hydroxycarbonyl, Aminocarbonyl, Trifluormethyl, Trifluormethoxy, Cyano, C₁-C₄-Alkyl, C₁-C₄-Alkoxy, C₁-C₆-Alkylamino, C₁-C₄-Alkylcarbonyl, C₁-C₄-Alkoxycarbonyl, C₁-C₆-Alkylaminocarbonyl, Dihydroxypropylaminocarbonyl, Dihydroxybutylaminocarbonyl, Dihydroxypentylaminocarbonyl, C₁-C₄-Alkylcarbonylamino, C₁-C₄-Alkylsulfonylamino, 5- oder 6-gliedriges Heterocyclyl-carbonyl, -CH₂R¹³ oder -CH₂CH₂R¹⁴ steht, wobei Alkoxy, Alkylamino, Alkylcarbonyl, Alkoxycarbonyl, Alkylaminocarbonyl, Alkylcarbonylamino und Alkylsulfonylamino substituiert sein können mit einem Substituenten, wobei der Substituent ausgewählt wird aus der Gruppe bestehend aus Hydroxy, Amino, Hydroxycarbonyl, Aminocarbonyl, C₁-C₄-Alkoxy, C₁-C₄-Alkylamino, C₁-C₄-Alkoxycarbonyl, C₁-C₄-Alkylaminocarbonyl, C₁-C₄-Alkylcarbonylamino und 5- oder 6-gliedriges Heterocyclyl, worin Heterocyclyl substituiert sein kann mit 1 bis 3 Substituenten, wobei die Substituenten unabhängig voneinander ausgewählt werden aus der Gruppe bestehend aus Halogen, Hydroxy, Oxo, C₁-C₄-Alkyl, C₁-C₄-Alkoxy, C₁-C₄-Alkylamino, C₁-C₄-Alkylcarbonyl, C₁-C₄-Alkoxycarbonyl und C₁-C₄-Alkylaminocarbonyl, und wobei Heterocyclylcarbonyl substituiert sein kann mit 1 bis 3 Substituenten, wobei die Substituenten unabhängig voneinander ausgewählt werden aus der Gruppe bestehend aus Halogen, Hydroxy, Oxo, C₁-C₄-Alkyl, C₁-C₄-Alkoxy, C₁-C₄-Alkylamino, C₁-C₄-Alkylcarbonyl, C₁-C₄-Alkoxycarbonyl und C₁-C₄-Alkylaminocarbonyl, und wobei R¹³ für Hydroxy, Amino, Cyano, Hydroxycarbonyl, Aminocarbonyl, C₁-C₄-Alkoxy, C₁-C₄-Alkylamino, C₁-C₄-Alkoxycarbonyl, C₁-C₄-Alkylaminocarbonyl, C₁-C₄-Alkylcarbonylamino, 5- oder 6-gliedriges Heterocyclyl oder 5- oder 6-gliedriges Heteroaryl steht, worin Alkoxy, Alkylamino, Alkoxycarbonyl, Alkylaminocarbonyl und Alkylcarbonylamino substituiert sein können mit einem Substituenten, wobei der Substituent ausgewählt wird aus der Gruppe bestehend aus Hydroxy, Amino, Hydroxycarbonyl, Aminocarbonyl, C₁-C₄-Alkoxy, C₁-C₄-Alkylamino, C₁-C₄-Alkoxycarbonyl, C₁-C₄-Alkylaminocarbonyl und C₁-C₄-Alkylcarbonylamino, und worin Heterocyclyl und Heteroaryl substituiert sein können mit 1 bis 3 Substituenten, wobei die Substituenten unabhängig voneinander ausgewählt werden aus der Gruppe bestehend aus Halogen, Hydroxy, Oxo, C₁-C₄-Alkyl, C₁-C₄-Alkoxy, C₁-C₄-Alkylamino, C₁-C₄-Alkylcarbonyl, C₁-C₄-Alkoxycarbonyl und C₁-C₄-Alkylaminocarbonyl, und wobei
R¹⁴ für Hydroxy, Amino, Cyano, Hydroxycarbonyl, Aminocarbonyl, C₁-C₄-Alkoxy, C₁-C₄-Alkylamino, C₁-C₄-Alkoxycarbonyl, C₁-C₄-Alkylaminocarbonyl, C₁-C₄-Alkylcarbonylamino, 5- oder 6-gliedriges Heterocyclyl oder 5- oder 6-gliedriges Heteroaryl steht, worin Alkoxy, Alkylamino, Alkoxycarbonyl, Alkylaminocarbonyl und Alkylcarbonylamino substituiert sein können mit einem Substituenten, wobei der Substituent ausgewählt wird aus der Gruppe bestehend aus Hydroxy, Amino, Hydroxycarbonyl, Aminocarbonyl, C₁-C₄-Alkoxy, C₁-C₄-Alkylamino, C₁-C₄-Alkoxycarbonyl, C₁-C₄-Alkylaminocarbonyl und C₁-C₄-Alkylcarbonylamino, und worin Heterocyclyl und Heteroaryl substituiert sein können mit 1 bis 3 Substituenten, wobei die Substituenten unabhängig voneinander ausgewählt werden aus der Gruppe bestehend aus Halogen, Hydroxy, Oxo, C₁-C₄-Alkyl, C₁-C₄-Alkoxy, C₁-C₄-Alkylamino, C₁-C₄-Alkylearbonyl, C₁-C₄-Alkoxycarbonyl und C₁-C₄-Alkylaminocarbonyl,

- R²: für C₆-C₁₀-Aryl oder 5- bis 10-gliedriges Heteroaryl steht, wobei Aryl und Heteroaryl substituiert sein können mit 1 bis 3 Substituenten, wobei die Substituenten unabhängig voneinander ausgewählt werden aus der Gruppe bestehend aus Hydroxy, Hydroxymethyl, Amino, Halogen, Cyano, Trifluormethyl, Trifluormethoxy, Aminocarbonyl, C₁-C₄-Alkyl, C₁-C₄-Alkoxy, C₁-C₄-Alkoxymethyl, C₁-C₄-Alkylamino, C₁-C₄-Alkylaminomethyl, C₁-C₄-Alkylcarbonyl, C₁-C₄-Alkoxycarbonyl, C₁-C₄-Alkylaminocarbonyl, C₁-C₄-Alkylcarbonylamino, C₁-C₄-Alkylsulfonyl, C₁-C₄-Alkylsulfonylamino, C₁-C₄-Alkylaminosulfonyl, Phenyl, Benzyloxy, 5- oder 6-gliedrigem Heterocyclyl, 5- oder 6-gliedrigem Heterocyclylcarbonyl, 5- oder 6-gliedrigem Heterocyclylmethyl und 5- oder 6-gliedrigem Heteroaryl, worin Phenyl, Benzyloxy, Heterocyclyl, Heterocyclylcarbonyl, Heterocyclylmethyl und Heteroaryl substituiert sein können mit 1 bis 3 Substituenten, wobei die Substituenten unabhängig voneinander ausgewählt werden aus der Gruppe bestehend aus Halogen, Cyano, Trifluormethyl, Trifluormethoxy, Aminocarbonyl, C₁-C₄-Alkyl, C₁-C₄-Alkoxy, C₁-C₄-Alkylamino, C₁-C₄-Alkylcarbonyl, C₁-C₄-Alkoxycarbonyl, C₁-C₄-Alkylaminocarbonyl und C₁-C₄-Alkylcarbonylamino, oder zwei der Substituenten am Aryl zusammen mit den Kohlenstoffatomen, an die sie gebunden sind, ein 1,3-Dioxolan oder 1,4-Dioxan bilden,
- R⁴: für Wasserstoff, Halogen, Cyano, Trifluormethyl, C₁-C₃-Alkyl, Methylthio oder Cyclopropyl steht,
- R¹⁶: für eine Gruppe der Formel 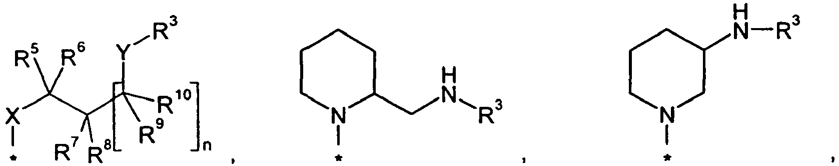 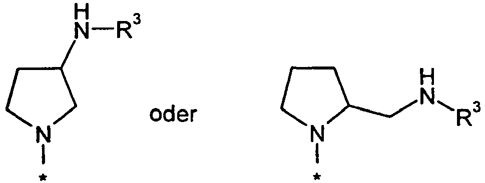
steht, wobei
* die Anknüpfstelle an den Heterocyclus bedeutet,
n für die Zahl 0 oder 1 steht,
X für NR¹¹, S oder O steht, wobei R¹¹ für Wasserstoff, C₁-C₃-Alkyl oder Cyclopropyl steht,
Y für NR¹², S oder O steht, wobei R¹² für Wasserstoff, C₁-C₃-Alkyl oder Cyclopropyl steht,
R³ für 2-Pyridyl, Pyrimid-2-yl, 2-Aminopyrimid-4-yl, 2-Cyclopropylaminopyrimid-4-yl, 2-Methylaminopyrimid-4-yl, 2-Ethylaminopyrimid-4-yl, 1,3-Thiazol-2-yl, 1,3-Thiazol-4-yl oder 1,3-Thiazol-5-yl steht, wobei 2-Pyridyl, Pyrimid-2-yl, 2-Aminopyrimid-4-yl, 1,3-Thiazol-2-yl, 1,3-Thiazol-4-yl und 1,3-Thiazol-5-yl substituiert sind mit 1 bis 3 Substituenten, wobei die Substituenten unabhängig voneinander ausgewählt werden aus der Gruppe bestehend aus Halogen, Cyano, Nitro, Amino, Trifluormethyl, Trifluormethoxy, Aminocarbonyl, Trifluormethylcarbonyl, C₁-C₄-Alkyl, C₁-C₄-Alkoxy, C₁-C₄- Alkylamino, C₁-C₄-Alkylcarbonyl, C₁-C₄-Alkoxycarbonyl, C₁-C₄-Alkylaminocarbonyl und C₃-C₆-Cycloalkylcarbonyl, worin Alkyl, Alkoxy, Alkylamino, Alkylcarbonyl, Alkoxycarbonyl, Alkylaminocarbonyl und Cycloalkylcarbonyl substituiert sein können mit einem Substituenten, wobei der Substituent ausgewählt wird aus der Gruppe bestehend aus Halogen, Cyano, Hydroxy, Amino, Trifluormethyl und C₃-C₆-Cycloalkyl, R⁵ für Wasserstoff, C₁-C₃-Alkyl oder Cyclopropyl steht, R⁶ für Wasserstoff oder C₁-C₃-Alkyl steht, R⁷ für Wasserstoff, C₁-C₃-Alkyl oder Cyclopropyl steht, R⁸ für Wasserstoff oder C₁-C₃-Alkyl steht, R⁹ für Wasserstoff, C₁-C₃-Alkyl oder Cyclopropyl steht, R¹⁰ für Wasserstoff oder C₁-C₃-Alkyl steht, und ihre Salze, ihre Solvate und die Solvate ihrer Salze.

Bevorzugt sind auch Verbindungen der Formel (I), in welcher
- A: für N oder CR¹⁵ steht, wobei R¹⁵ für Wasserstoff oder Chlor steht,
- R¹: für Wasserstoff, Hydroxycarbonyl, Aminocarbonyl, Trifluormethyl, Cyano, C₁-C₄-Alkyl, C₁-C₄-Alkoxy, C₁-C₆-Alkylamino, C₁-C₄-Alkoxycarbonyl, C₁-C₆-Alkylaminocarbonyl, Dihydroxypropylaminocarbonyl, Dihydroxybutylaminocarbonyl, Dihydroxypentylaminocarbonyl, C₁-C₄-Alkylcarbonylamino, C₁-C₄-Alkylsulfonylamino, 5- oder 6-gliedriges Heterocyclyl-carbonyl, -CH₂R¹³ oder -CH₂CH₂R¹⁴ steht, wobei Alkoxy, Alkylamino, Alkoxycarbonyl, Alkylaminocarbonyl, Alkylcarbonylamino und Alkylsulfonylamino substituiert sein können mit einem Substituenten, wobei der Substituent ausgewählt wird aus der Gruppe bestehend aus Hydroxy, Amino, Hydroxycarbonyl, Aminocarbonyl, C₁-C₄-Alkoxy, C₁-C₄-Alkylamino, C₁-C₄-Alkoxycarbonyl, C₁-C₄-Alkylaminocarbonyl, C₁-C₄-Alkylcarbonylamino und 5- oder 6- gliedriges Heterocyclyl, worin Heterocyclyl substituiert sein kann mit 1 bis 3 Substituenten, wobei die Substituenten unabhängig voneinander ausgewählt werden aus der Gruppe bestehend aus Halogen, Hydroxy, Oxo, C₁-C₄-Alkyl, C₁-C₄-Alkoxy und C₁-C₄-Alkylamino, und wobei Heterocyclylcarbonyl substituiert sein kann mit 1 bis 3 Substituenten, wobei die Substituenten unabhängig voneinander ausgewählt werden aus der Gruppe bestehend aus Halogen, Hydroxy, Oxo, C₁-C₄-Alkyl, C₁-C₄-Alkoxy und C₁-C₄-Alkylamino, und wobei
R¹³ für Hydroxy, Amino, Cyano, Hydroxycarbonyl, Aminocarbonyl, C₁-C₄-Alkoxy, C₁-C₄-Alkylamino, C₁-C₄-Alkoxycarbonyl, C₁-C₄-Alkylaminocarbonyl, C₁-C₄-Alkylcarbonylamino, 5- oder 6-gliedriges Heterocyclyl oder 5- oder 6-gliedriges Heteroaryl steht, worin Alkoxy, Alkylamino, Alkoxycarbonyl, Alkylaminocarbonyl und Alkylcarbonylamino substituiert sein können mit einem Substituenten, wobei der Substituent ausgewählt wird aus der Gruppe bestehend aus Hydroxy, Amino, Hydroxycarbonyl, Aminocarbonyl, C₁-C₄-Alkoxy, C₁-C₄-Alkylamino, C₁-C₄-Alkoxycarbonyl, C₁-C₄-Alkylaminocarbonyl und C₁-C₄-Alkylcarbonylamino, und worin Heterocyclyl und Heteroaryl substituiert sein können mit 1 bis 3 Substituenten, wobei die Substituenten unabhängig voneinander ausgewählt werden aus der Gruppe bestehend aus Halogen, Hydroxy, Oxo, C₁-C₄-Alkyl, C₁-C₄-Alkoxy und C₁-C₄-Alkylamino, und wobei
R¹⁴ für Hydroxy, Amino, Cyano, Hydroxycarbonyl, Aminocarbonyl, C₁-C₄-Alkoxy, C₁-C₄-Alkylamino, C₁-C₄-Alkoxycarbonyl, C₁-C₄-Alkylaminocarbonyl, C₁-C₄- Alkylcarbonylamino, 5- oder 6-gliedriges Heterocyclyl oder 5- oder 6-gliedriges Heteroaryl steht, worin Alkoxy, Alkylamino, Alkoxycarbonyl, Alkylaminocarbonyl und Alkylcarbonylamino substituiert sein können mit einem Substituenten, wobei der Substituent ausgewählt wird aus der Gruppe bestehend aus Hydroxy, Amino, Hydroxycarbonyl, Aminocarbonyl, C₁-C₄-Alkoxy, C₁-C₄-Alkylamino, C₁-C₄-Alkoxycarbonyl, C₁-C₄-Alkylaminocarbonyl und C₁-C₄-Alkylcarbonylamino, und worin Heterocyclyl und Heteroaryl substituiert sein können mit 1 bis 3 Substituenten, wobei die Substituenten unabhängig voneinander ausgewählt werden aus der Gruppe bestehend aus Halogen, Hydroxy, Oxo, C₁-C₄-Alkyl, C₁-C₄-Alkoxy und C₁-C₄-Alkylamino,
- R²: für C₆-C₁₀-Aryl, Thienyl, Furyl, Pyrrolyl, Thiazolyl, Oxazolyl, Oxadiazolyl, Pyrazolyl, Imidazolyl, Pyridyl, Pyrimidyl, Pyridazinyl, Pyrazinyl, Indolyl, Indazolyl, Chinolinyl, Benzofuranyl oder Benzoxazolyl steht, wobei Aryl, Thienyl, Furyl, Pyrrolyl, Thiazolyl, Oxazolyl, Oxadiazolyl, Pyrazolyl, Imidazolyl, Pyridyl, Pyrimidyl, Pyridazinyl, Pyrazinyl, Indolyl, Indazolyl, Chinolinyl, Benzofuranyl und Benzoxazolyl substituiert sein können mit 1 bis 3 Substituenten, wobei die Substituenten unabhängig voneinander ausgewählt werden aus der Gruppe bestehend aus Hydroxy, Hydroxymethyl, Amino, Halogen, Cyano, Trifluormethyl, Trifluormethoxy, Aminocarbonyl, C₁-C₄-Alkyl, C₁-C₄-Alkoxy, C₁-C₄-Alkoxymethyl, C₁-C₄-Alkylamino, C₁-C₄-Alkylaminomethyl, C₁-C₄-Alkylcarbonyl, C₁-C₄-Alkoxycarbonyl, C₁-C₄-Alkylaminocarbonyl, C₁-C₄-Alkylcarbonylamino, C₁-C₄-Alkylsulfonyl, C₁-C₄-Alkylsulfonylamino, C₁-C₄-Alkylaminosulfonyl, Phenyl, Benzyloxy, 5- oder 6-gliedrigem Heterocyclyl, 5- oder 6-gliedrigem Heterocyclylcarbonyl, 5- oder 6-gliedrigem Heterocyclylmethyl und 5- oder 6-gliedrigem Heteroaryl, worin Phenyl, Benzyloxy, Heterocyclyl, Heterocyclylcarbonyl, Heterocyclylmethyl und Heteroaryl substituiert sein können mit 1 bis 3 Substituenten, wobei die Substituenten unabhängig voneinander ausgewählt werden aus der Gruppe bestehend aus Halogen, Cyano, Trifluormethyl, Trifluormethoxy, Aminocarbonyl, C₁-C₄-Alkyl, C₁-C₄-Alkoxy, C₁-C₄-Alkylamino, C₁-C₄-Alkylcarbonyl, C₁-C₄-Alkoxycarbonyl, C₁-C₄-Alkylaminocarbonyl und C₁-C₄-Alkylcarbonylamino, oder zwei der Substituenten am Aryl zusammen mit den Kohlenstoffatomen, an die sie gebunden sind, ein 1,3-Dioxolan oder 1,4-Dioxan bilden,
- R⁴: für Wasserstoff, Halogen, Cyano, Trifluormethyl, C₁-C₃-Alkyl oder Cyclopropyl steht,
- R¹⁶: für eine Gruppe der Formel 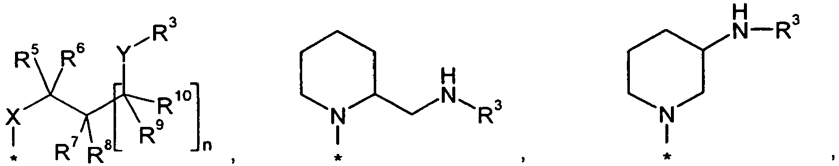 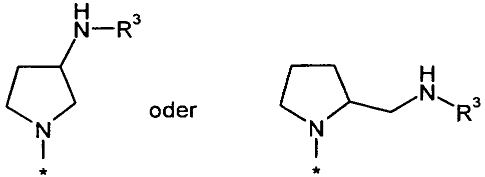 steht, wobei
* die Anknüpfstelle an den Heterocyclus bedeutet,
n für die Zahl 0 oder 1 steht,
X für NR¹¹, S oder O steht, wobei R¹¹ für Wasserstoff oder Methyl steht,
Y für NR¹², S oder O steht, wobei R¹² für Wasserstoff oder Methyl steht,
R³ für 2-Pyridyl, Pyrimid-2-yl, 2-Aminopyrimid-4-yl, 2-Cyclopropylaminopyrimid-4- yl, 2-Methylaminopyrimid-4-yl, 2-Ethylaminopyrimid-4-yl, 1,3-Thiazol-2-yl, 1,3-Thiazol-4-yl oder 1,3-Thiazol-5-yl steht, wobei 2-Pyridyl, Pyrimid-2-yl, 2-Aminopyrimid-4-yl, 1,3-Thiazol-2-yl, 1,3-Thiazol-4-yl und 1,3-Thiazol-S-yl substituiert sind mit 1 bis 3 Substituenten, wobei die Substituenten unabhängig voneinander ausgewählt werden aus der Gruppe bestehend aus Halogen, Cyano, Nitro, Amino, Trifluormethyl, Trifluormethoxy, Aminocarbonyl, Trifluormethylcarbonyl, C₁-C₄-Alkyl, C₁-C₄-Alkoxy, C₁-C₄-Alkylamino, C₁-C₄-Alkylcarbonyl, C₁-C₄-Alkoxycarbonyl, C₁-C₄-Alkylaminocarbonyl und C₃-C₆-Cycloalkylcarbonyl, worin Alkyl, Alkoxy, Alkylamino, Alkylcarbonyl, Alkoxycarbonyl, Alkylaminocarbonyl und Cycloalkylcarbonyl substituiert sein können mit einem Substituenten, wobei der Substituent ausgewählt wird aus der Gruppe bestehend aus Halogen, Cyano, Hydroxy, Amino, Trifluormethyl und C₃-C₆-Cycloalkyl,
R⁵ für Wasserstoff oder Methyl steht,
R⁶ für Wasserstoff oder Methyl steht,
R⁷ für Wasserstoff oder Methyl steht,
R⁸ für Wasserstoff oder Methyl steht,
R⁹ für Wasserstoffoder Methyl steht,
R¹⁰ für Wasserstoff oder Methyl steht,
und ihre Salze, ihre Solvate und die Solvate ihrer Salze.

Bevorzugt sind auch Verbindungen der Formel (I), in welcher
- A: für N oder CR¹⁵ steht, wobei R¹⁵ für Wasserstoff steht,
- R¹: für Wasserstoff, Trifluormethyl, Cyano, C₁-C₄-Alkyl, C₁-C₆-Alkylaminocarbonyl, Dihydroxypropylaminocarbonyl, Dihydroxybutylaminocarbonyl, Dihydroxypentylaminocarbonyl, Pyrrolidinylcarbonyl, Piperidinylcarbonyl, Morpholinylcarbonyl, Piperazinylcarbonyl, -CH₂R¹³ oder -CH₂CH₂R¹⁴ steht, wobei Alkylaminocarbonyl substituiert sein kann mit einem Substituenten, wobei der Substituent ausgewählt wird aus der Gruppe bestehend aus Hydroxy, Amino, Hydroxycarbonyl, Aminocarbonyl, C₁-C₄-Alkoxy, C₁-C₄-Alkylamino, Pyrrolidinyl, Piperidinyl, Morpholinyl und Piperazinyl, worin Pyrrolidinyl, Piperidinyl, Morpholinyl und Piperazinyl substituiert sein können mit 1 bis 3 Substituenten, wobei die Substituenten unabhängig voneinander ausgewählt werden aus der Gruppe bestehend aus Halogen, Hydroxy, Oxo, C₁-C₄-Alkyl, C₁-C₄-Alkoxy und C₁-C₄-Alkylamino, und wobei Pyrrolidinylcarbonyl, Piperidinylcarbonyl, Morpholinylcarbonyl und Piperazinyl-carbonyl substituiert sein können mit 1 bis 3 Substituenten, wobei die Substituenten unabhängig voneinander ausgewählt werden aus der Gruppe bestehend aus Halogen, Hydroxy, Oxo, C₁-C₄-Alkyl, C₁-C₄-Alkoxy und C₁-C₄-Alkylamino, und wobei
R¹³ für Hydroxycarbonyl, C₁-C₄-Alkoxy, C₁-C₄-Alkylamino, C₁-C₄-Alkylaminocarbonyl, Pyrrolidinyl, Piperidinyl, Morpholinyl, Piperazinyl, Pyrazolyl, Imidazolyl, Triazolyl oder Pyridyl steht, worin Alkoxy, Alkylamino und Alkylaminocarbonyl substituiert sein können mit einem Substituenten, wobei der Substituent ausgewählt wird aus der Gruppe bestehend aus Hydroxy, Amino, Hydroxycarbonyl, Aminocarbonyl, C₁-C₄-Alkoxy, C₁-C₄-Alkylamino, C₁-C₄-Alkoxycarbonyl, C₁-C₄-Alkylaminocarbonyl und C₁-C₄-Alkylcarbonylamino, und worin Pyrrolidinyl, Piperidinyl, Morpholinyl, Piperazinyl, Pyrazolyl, Imidazolyl, Triazolyl und Pyridyl substituiert sein können mit 1 bis 3 Substituenten, wobei die Substituenten unabhängig voneinander ausgewählt werden aus der Gruppe bestehend aus Halogen, Hydroxy, Oxo, C₁-C₄-Alkyl, C₁-C₄-Alkoxy und C₁-C₄-Alkylamino, und wobei
R¹⁴ für Hydroxycarbonyl, C₁-C₄-Alkoxy, C₁-C₄-Alkylamino, C₁-C₄-Alkylaminocarbonyl, Pyrrolidinyl, Piperidinyl, Morpholinyl, Piperazinyl, Pyrazolyl, Imidazolyl, Triazolyl oder Pyridyl steht, worin Alkoxy, Alkylamino und Alkylaminocarbonyl substituiert sein können mit einem Substituenten, wobei der Substituent ausgewählt wird aus der Gruppe bestehend aus Hydroxy, Amino, Hydroxycarbonyl, Aminocarbonyl, C₁-C₄-Alkoxy, C₁-C₄-Alkylamino, C₁-C₄-Alkoxycarbonyl, C₁-C₄-Alkylaminocarbonyl und C₁-C₄-Alkylcarbonylamino, und worin Pyrrolidinyl, Piperidinyl, Morpholinyl, Piperazinyl, Pyrazolyl, Imidazolyl, Triazolyl und Pyridyl substituiert sein können mit 1 bis 3 Substituenten, wobei die Substituenten unabhängig voneinander ausgewählt werden aus der Gruppe bestehend aus Halogen, Hydroxy, Oxo, C₁-C₄-Alkyl, C₁-C₄-Alkoxy und C₁-C₄-Alkylamino,
- R²: für Phenyl steht, wobei Phenyl substituiert sein kann mit 1 bis 3 Substituenten, wobei die Substituenten unabhängig voneinander ausgewählt werden aus der Gruppe bestehend aus Hydroxy, Halogen, Cyano, Trifluormethyl, Trifluormethoxy, Aminocarbonyl, C₁-C₄-Alkyl, C₁-C₄-Alkoxy, C₁-C₄-Alkoxymethyl, C₁-C₄-Alkylaminomethyl, C₁-C₄-Alkylcarbonyl, C₁-C₄-Alkoxycarbonyl, C₁-C₄-Alkylaminocarbonyl, C₁-C₄-Alkylcarbonylamino, C₁-C₄-Alkylsulfonyl, C₁-C₄-Alkylsulfonylamino, C₁-C₄-Alkylaminosulfonyl, Phenyl, Pyrrolidinyl, Piperidinyl, Morpholinyl, Piperazinyl, Pyrrolidinylmethyl, Piperidinylmethyl, Morpholinylmethyl und Piperazinylmethyl, worin Phenyl, Pyrrolidinyl, Piperidinyl, Morpholinyl, Piperazinyl, Pyrrolidinylmethyl, Piperidinylmethyl, Morpholinylmethyl und Piperazinylmethyl substituiert sein können mit 1 bis 3 Substituenten, wobei die Substituenten unabhängig voneinander ausgewählt werden aus der Gruppe bestehend aus Halogen, Cyano, Trifluormethyl, Trifluormethoxy und C₁-C₄-Alkyl,
- R⁴: für Wasserstoff oder Chlor steht,
- R¹⁶: für eine Gruppe der Formel 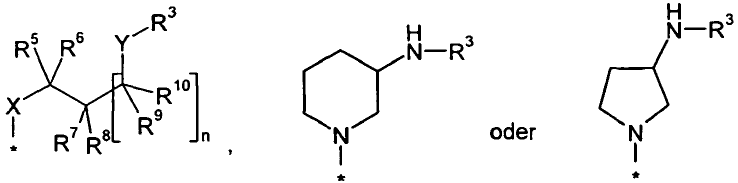 steht, wobei
* die Anknüpfstelle an den Heterocyclus bedeutet,
n für die Zahl 0 steht,
X für NR¹¹, S oder O steht, wobei R¹¹ für Wasserstoff oder Methyl steht,
Y für NR¹², S oder O steht, wobei R¹² für Wasserstoff oder Methyl steht,
R³ für 2-Pyridyl, Pyrimid-2-yl, 2-Aminopyrimid-4-yl, 2-Cyclopropylaminopyrimid-4-yl, 2-Methylaminopyrimid-4-yl, 2-Ethylaminopyrimid-4-yl, 1,3-Thiazol-2-yl, 1,3-Thiazol-4-yl oder 1,3-Thiazol-5-yl steht, wobei 2-Pyridyl, Pyrimid-2-yl, 2-Aminopyrimid-4-yl, 1,3-Thiazol-2-yl, 1,3-Thiazol-4-yl und 1,3-Thiazol-5-yl substituiert sind mit 1 bis 3 Substituenten, wobei die Substituenten unabhängig voneinander ausgewählt werden aus der Gruppe bestehend aus Halogen, Cyano, Nitro, Amino, Trifluormethyl, Trifluormethoxy, Aminocarbonyl, Trifluormethylcarbonyl, C₁-C₄-Alkylamino, C₁-C₄-Alkylcarbonyl, C₁-C₄-Alkoxycarbonyl, C₁-C₄-Alkylaminocarbonyl und C₃-C₆-Cycloalkylcarbonyl, worin Alkylamino, Alkylcarbonyl, Alkoxycarbonyl, Alkylaminocarbonyl und Cycloalkylcarbonyl substituiert sein können mit einem Substituenten, wobei der Substituent ausgewählt wird aus der Gruppe bestehend aus Halogen, Cyano, Hydroxy, Amino, Trifluormethyl und C₃-C₆-Cycloalkyl,
R⁵ für Wasserstoff oder Methyl steht,
R⁶ für Wasserstoff steht,
R⁷ für Wasserstoff oder Methyl steht,
R⁸ für Wasserstoff steht,
R⁹ für Wasserstoff steht,
R¹⁰ für Wasserstoff steht,
und ihre Salze, ihre Solvate und die Solvate ihrer Salze.

Bevorzugt sind auch Verbindungen der Formel (I), in welcher
- A: für N oder CR¹⁵ steht, wobei R¹⁵ für Wasserstoff steht,
- R¹: für Wasserstoff, Methyl oder -CH₂R¹³ steht, wobei R¹³ für Morpholinyl steht,
- R²: für Phenyl steht, wobei Phenyl substituiert sein kann mit 1 bis 3 Substituenten, wobei die Substituenten unabhängig voneinander ausgewählt werden aus der Gruppe bestehend aus Chlor, Fluor, Trifluormethyl, Trifluormethoxy und Methyl,
- R⁴: für Wasserstoff steht,
- R¹⁶: für eine Gruppe der Formel 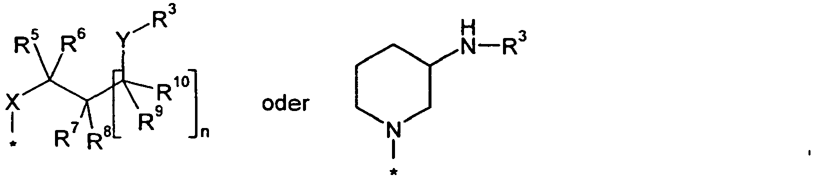 steht, wobei
* die Anknüpfstelle an den Heterocyclus bedeutet,
n für die Zahl 0 steht,
X für NR¹¹ oder O steht, wobei R¹¹ für Wasserstoff steht,
Y für NR¹² oder O steht, wobei R¹² für Wasserstoff steht,
R³ für 2-Pyridyl oder 1,3-Thiazol-2-yl steht, wobei 2-Pyridyl und 1,3-Thiazol-2-yl substituiert sind mit 1 bis 2 Substituenten, wobei die Substituenten unabhängig voneinander ausgewählt werden aus der Gruppe bestehend aus Cyano, Nitro, Amino, Trifluormethylcarbonyl und Methylcarbonyl,
R⁵ für Wasserstoff oder Methyl steht,
R⁶ für Wasserstoff steht,
R⁷ für Wasserstoff oder Methyl steht,
R⁸ für Wasserstoff steht,
R⁹ für Wasserstoff steht,
R¹⁰ für Wasserstoff steht,
und ihre Salze, ihre Solvate und die Solvate ihrer Salze.

Bevorzugt sind auch Verbindungen der Formel (I), in welcher
- A: für N oder CR¹⁵ steht, wobei R¹⁵ für Wasserstoff steht,
- R¹: für Wasserstoff, Methyl oder -CH₂R¹³ steht, wobei R¹³ für Morpholinyl steht,
- R²: für Phenyl steht, wobei Phenyl substituiert sein kann mit 1 bis 3 Substituenten, wobei die Substituenten unabhängig voneinander ausgewählt werden aus der Gruppe bestehend aus Chlor, Fluor, Trifluormethyl, Trifluormethoxy und Methyl,
- R⁴: für Wasserstoff steht,
- R¹⁶: für eine Gruppe der Formel 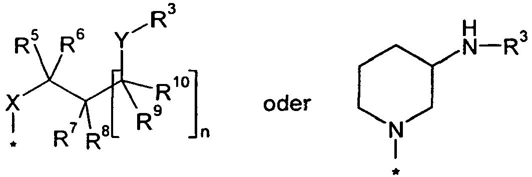 steht, wobei
* die Anknüpfstelle an den Heterocyclus bedeutet,
n für die Zahl 0 steht,
X für NR¹¹ steht, wobei R¹¹ für Wasserstoff steht,
Y für NR¹² steht, wobei R¹² für Wasserstoff steht,
R³ für 2-Pyridyl oder 1,3-Thiazol-2-yl steht, wobei 2-Pyridyl und 1,3-Thiazol-2-yl substituiert sind mit 1 bis 2 Substituenten, wobei die Substituenten unabhängig voneinander ausgewählt werden aus der Gruppe bestehend aus Cyano, Nitro, Amino, Trifluormethylcarbonyl und Methylcarbonyl,
R⁵ für Wasserstoff oder Methyl steht,
R⁶ für Wasserstoff steht,
R⁷ für Wasserstoff oder Methyl steht,
R⁸ für Wasserstoff steht,
R⁹ für Wasserstoff steht,
R¹⁰ für Wasserstoff steht,
und ihre Salze, ihre Solvate und die Solvate ihrer Salze.

Bevorzugt sind auch Verbindungen der Formel (I), in welcher
- A: für N oder CR¹⁵ steht, wobei R¹⁵ für Wasserstoff steht,
- R¹: für Wasserstoff, Methyl oder -CH₂R¹³ steht, wobei R¹³ für Morpholinyl steht,
- R²: für Phenyl steht, wobei Phenyl substituiert sein kann mit 1 bis 3 Substituenten, wobei die Substituenten unabhängig voneinander ausgewählt werden aus der Gruppe bestehend aus Chlor, Fluor, Trifluormethyl, Trifluormethoxy und Methyl,
- R⁴: für Wasserstoff steht,
- R¹⁶: für eine Gruppe der Formel 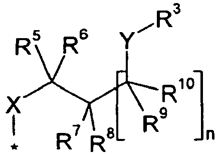 steht, wobei
* die Anknüpfstelle an den Heterocyclus bedeutet,
n für die Zahl 0 steht,
X für NR¹¹ steht, wobei R¹¹ für Wasserstoff steht,
Y für NR¹² steht, wobei R¹² für Wasserstoff steht,
R³ für 2-Pyridyl oder 1,3-Thiazol-2-yl steht, wobei 2-Pyridyl und 1,3-Thiazol-2-yl substituiert sind mit 1 bis 2 Substituenten, wobei die Substituenten unabhängig voneinander ausgewählt werden aus der Gruppe bestehend aus Cyano, Nitro, Amino, Trifluormethylcarbonyl und Methylcarbonyl,
R⁵ for Wasserstoff oder Methyl steht,
R⁶ for Wasserstoff steht,
R⁷ for Wasserstoff oder Methyl steht,
R⁸ für Wasserstoff steht,
R⁹ für Wasserstoff steht,
R¹⁰ für Wasserstoff steht,
und ihre Salze, ihre Solvate und die Solvate ihrer Salze.

Bevorzugt sind auch Verbindungen der Formel (I), in welcher
- A: für N oder CR¹⁵ steht, wobei R¹⁵ für Wasserstoff steht,
- R¹: für Wasserstoff, Methyl oder -CH₂R¹³ steht, wobei R¹³ für Morpholinyl steht,
- R²: für Phenyl steht, wobei Phenyl substituiert sein kann mit 1 bis 3 Substituenten, wobei die Substituenten unabhängig voneinander ausgewählt werden aus der Gruppe bestehend aus Chlor, Fluor, Trifluormethyl, Trifluormethoxy und Methyl,
- R⁴: für Wasserstoff steht, 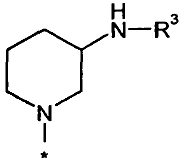
- R¹⁶: für eine Gruppe der Formel steht, wobei
* die Anknüpfstelle an den Heterocyclus bedeutet,
R³ für 2-Pyridyl oder 1,3-Thiazol-2-yl steht, wobei 2-Pyridyl und 1,3-Thiazol-2-yl substituiert sind mit 1 bis 2 Substituenten, wobei die Substituenten unabhängig voneinander ausgewählt werden aus der Gruppe bestehend aus Cyano, Nitro, Amino, Trifluormethylcarbonyl und Methylcarbonyl,
und ihre Salze, ihre Solvate und die Solvate ihrer Salze.

Bevorzugt sind auch Verbindungen der Formel (I), in welcher
- A: für N oder CR¹⁵ steht, wobei R¹⁵ für Wasserstoff steht,
- R¹: für C₁-C₆-Alkylaminocarbonyl, 1,3-Dihydroxyprop-2-ylaminocarbonyl, Pyrrolidinylcarbonyl, Piperidinylcarbonyl, Morpholinyl-carbonyl, Piperazinylcarbonyl, -CH₂R¹³ oder - CH₂CH₂R¹⁴ steht, wobei Alkylaminocarbonyl substituiert sein kann mit einem Substituenten, wobei der Substituent ausgewählt wird aus der Gruppe bestehend aus Hydroxy, Amino, Aminocarbonyl, C₁-C₄-Alkoxy, C₁-C₄-Alkylamino, Pyrrolidinyl, Piperidinyl, Morpholinyl und Piperazinyl, worin Pyrrolidinyl, Piperidinyl, Morpholinyl und Piperazinyl substituiert sein können mit 1 bis 3 Substituenten, wobei die Substituenten unabhängig voneinander ausgewählt werden aus der Gruppe bestehend aus Halogen, Hydroxy, Oxo, C₁-C₄-Alkyl, C₁-C₄-Alkoxy und C₁-C₄-Alkylamino, und wobei Pyrrolidinylcarbonyl, Piperidinylcarbonyl, Morpholinylcarbonyl und Piperazinyl-carbonyl substituiert sein können mit 1 bis 3 Substituenten, wobei die Substituenten unabhängig voneinander ausgewählt werden aus der Gruppe bestehend aus Halogen, Hydroxy, Oxo, C₁-C₄-Alkyl, C₁-C₄-Alkoxy und C₁-C₄-Alkylamino, und wobei
R¹³ für C₁-C₄-Alkoxy, C₁-C₄-Alkylamino, C₁-C₄-Alkylamino-carbonyl, Pyrrolidinyl, Piperidinyl, Morpholinyl, Piperazinyl, Pyrazolyl, Imidazolyl, Triazolyl oder Pyridyl steht, worin Alkoxy, Alkylamino und Alkylaminocarbonyl substituiert sein können mit einem Substituenten, wobei der Substituent ausgewählt wird aus der Gruppe bestehend aus Hydroxy, Amino, Aminocarbonyl, C₁-C₄-Alkoxy, C₁-C₄-Alkylamino, C₁-C₄-Alkoxycarbonyl, C₁-C₄-Alkylaminocarbonyl und C₁-C₄-Alkylcarbonylamino, und worin Pyrrolidinyl, Piperidinyl, Morpholinyl, Piperazinyl, Pyrazolyl, Imidazolyl, Triazolyl und Pyridyl substituiert sein können mit 1 bis 3 Substituenten, wobei die Substituenten unabhängig voneinander ausgewählt werden aus der Gruppe bestehend aus Halogen, Hydroxy, Oxo, C₁-C₄-Alkyl, C₁-C₄-Alkoxy und C₁-C₄-Alkylamino, und wobei
R¹⁴ für C₁-C₄-Alkoxy, C₁-C₄-Alkylamino, C₁-C₄-Alkylamino-carbonyl, Pyrrolidinyl, Piperidinyl, Morpholinyl, Piperazinyl, Pyrazolyl, Imidazolyl, Triazolyl oder Pyridyl steht, worin Alkoxy, Alkylamino und Alkylaminocarbonyl substituiert sein können mit einem Substituenten, wobei der Substituent ausgewählt wird aus der Gruppe bestehend aus Hydroxy, Amino, Aminocarbonyl, C₁-C₄-Alkoxy, C₁-C₄-Alkylamino, C₁-C₄-Alkoxycarbonyl, C₁-C₄-Alkylaminocarbonyl und C₁-C₄-Alkylcarbonylamino, und worin Pyrrolidinyl, Piperidinyl, Morpholinyl, Piperazinyl, Pyrazolyl, Imidazolyl, Triazolyl und Pyridyl substituiert sein können mit 1 bis 3 Substituenten, wobei die Substituenten unabhängig voneinander ausgewählt werden aus der Gruppe bestehend aus Halogen, Hydroxy, Oxo, C₁-C₄-Alkyl, C₁-C₄-Alkoxy und C₁-C₄-Alkylamino,
- R²: für Phenyl steht, wobei Phenyl substituiert sein kann mit 1 bis 3 Substituenten, wobei die Substituenten unabhängig voneinander ausgewählt werden aus der Gruppe bestehend aus Chlor, Fluor, Trifluormethyl, Trifluormethoxy und Methyl,
- R⁴: für Wasserstoff steht,
- R¹⁶: für eine Gruppe der Formel 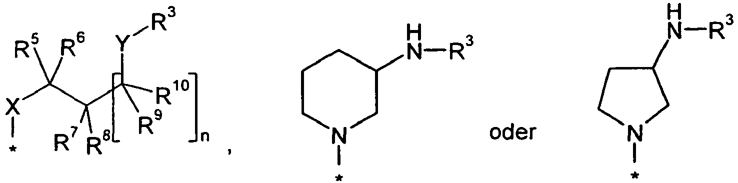 steht, wobei
* die Anknüpfstelle an den Heterocyclus bedeutet,
n für die Zahl 0 steht,
X für NR¹¹, S oder O steht, wobei R¹¹ für Wasserstoff oder Methyl steht,
Y für NR¹², S oder O steht, wobei R¹² für Wasserstoff oder Methyl steht,
R³ für 2-Pyridyl oder 1,3-Thiazol-2-yl steht, wobei 2-Pyridyl und 1,3-Thiazol-2-yl substituiert sind mit 1 bis 2 Substituenten, wobei die Substituenten unabhängig voneinander ausgewählt werden aus der Gruppe bestehend aus Cyano, Nitro, Amino, Trifluormethylcarbonyl und Methylcarbonyl,
R⁵ für Wasserstoff oder Methyl steht,
R⁶ für Wasserstoff steht,
R⁷ für Wasserstoff oder Methyl steht,
R⁸ für Wasserstoff steht,
R⁹ für Wasserstoff steht,
R¹⁰ für Wasserstoff steht,
und ihre Salze, ihre Solvate und die Solvate ihrer Salze.

Bevorzugt sind auch Verbindungen der Formel (I), in welcher
- A: für N oder CR¹⁵ steht, wobei R¹⁵ für Wasserstoff steht,
- R¹: für C₁-C₄-Alkylaminocarbonyl, 1,3-Dihydroxyprop-2-ylaminocarbonyl, Piperazinyl-carbonyl, -CH₂R¹³ oder -CH₂CH₂R¹⁴ steht, wobei Alkylaminocarbonyl substituiert ist mit einem Substituenten, wobei der Substituent ausgewählt wird aus der Gruppe bestehend aus Hydroxy, Amino, C₁-C₄-Alkylamino, Pyrrolidinyl, Piperidinyl, Morpholinyl und Piperazinyl, worin Pyrrolidinyl, Piperidinyl, Morpholinyl und Piperazinyl substituiert sein können mit 1 bis 3 Substituenten C₁-C₄-Alkyl, und wobei Piperazinylcarbonyl substituiert sein kann mit 1 bis 3 Substituenten, wobei die Substituenten unabhängig voneinander ausgewählt werden aus der Gruppe bestehend aus Oxo, C₁-C₄-Alkyl, C₁-C₄-Alkoxy und C₁-C₄-Alkylamino, und wobei
R¹³ für C₁-C₄-Alkoxy, C₁-C₄-Alkylamino, C₁-C₄-Alkylaminocarbonyl, Pyrrolidinyl, Piperidinyl, Morpholinyl oder Piperazinyl steht, worin Alkoxy, Alkylamino und Alkylaminocarbonyl substituiert sind mit einem Substituenten, wobei der Substituent ausgewählt wird aus der Gruppe bestehend aus Hydroxy, Amino, C₁-C₄-Alkoxy und C₁-C₄-Alkylamino, und worin Pyrrolidinyl, Piperidinyl, Morpholinyl und Piperazinyl substituiert sein können mit 1 bis 3 Substituenten C₁-C₄-Alkyl, und wobei
R¹⁴ für C₁-C₄-Alkoxy, C₁-C₄-Alkylamino, C₁-C₄-Alkylaminocarbonyl, Pyrrolidinyl, Piperidinyl, Morpholinyl oder Piperazinyl steht, worin Alkoxy, Alkylamino und Alkylaminocarbonyl substituiert sind mit einem Substituenten, wobei der Substituent ausgewählt wird aus der Gruppe bestehend aus Hydroxy, Amino, C₁-C₄-Alkoxy und C₁-C₄-Alkylamino, und worin Pyrrolidinyl, Piperidinyl, Morpholinyl und Piperazinyl substituiert sein können mit 1 bis 3 Substituenten C₁-C₄-Alkyl,
- R²: für Phenyl steht, wobei Phenyl substituiert sein kann mit 1 bis 3 Substituenten, wobei die Substituenten unabhängig voneinander ausgewählt werden aus der Gruppe bestehend aus Chlor, Fluor, Trifluormethyl, Trifluormethoxy und Methyl,
- R⁴: für Wasserstoff steht,
- R¹⁶: für eine Gruppe der Formel 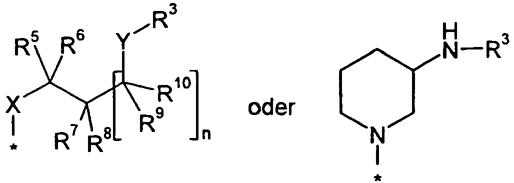 steht, wobei
* die Anknüpfstelle an den Heterocyclus bedeutet,
n für die Zahl 0 steht,
X für NR¹¹ oder O steht, wobei R¹¹ für Wasserstoff steht,
Y für NR¹² oder O steht, wobei R¹² für Wasserstoff steht,
R³ für 2-Pyridyl oder 1,3-Thiazol-2-yl steht, wobei 2-Pyridyl und 1,3-Thiazol-2-yl substituiert sind mit 1 bis 2 Substituenten, wobei die Substituenten unabhängig voneinander ausgewählt werden aus der Gruppe bestehend aus Cyano, Nitro, Amino, Trifluormethylcarbonyl und Methylcarbonyl,
R⁵ für Wasserstoff oder Methyl steht,
R⁶ für Wasserstoff steht,
R⁷ für Wasserstoff oder Methyl steht,
R⁸ für Wasserstoff steht,
R⁹ für Wasserstoff steht,
R¹⁰ für Wasserstoff steht,
und ihre Salze, ihre Solvate und die Solvate ihrer Salze.

Bevorzugt sind auch Verbindungen der Formel (I), in welcher
- A: für N oder CR¹⁵ steht, wobei R¹⁵ für Wasserstoff steht,
- R¹: für C₁-C₄-Alkylaminocarbonyl, 1,3-Dihydroxyprop-2-ylaminocarbonyl, Piperazinyl-carbonyl, -CH₂R¹³ oder -CH₂CH₂R¹⁴ steht, wobei Alkylaminocarbonyl substituiert ist mit einem Substituenten, wobei der Substituent ausgewählt wird aus der Gruppe bestehend aus Hydroxy, Amino, C₁-C₄-Alkylamino, Pyrrolidinyl, Piperidinyl, Morpholinyl und Piperazinyl, worin Pyrrolidinyl, Piperidinyl, Morpholinyl und Piperazinyl substituiert sein können mit 1 bis 3 Substituenten C₁-C₄-Alkyl, und wobei Piperazinylcarbonyl substituiert sein kann mit 1 bis 3 Substituenten, wobei die Substituenten unabhängig voneinander ausgewählt werden aus der Gruppe bestehend aus Oxo, C₁-C₄-Alkyl, C₁-C₄-Alkoxy und C₁-C₄-Alkylamino, und wobei
R¹³ für C₁-C₄-Alkoxy, C₁-C₄-Alkylamino, C₁-C₄-Alkylaminocarbonyl, Pyrrolidinyl, Piperidinyl, Morpholinyl oder Piperazinyl steht, worin Alkoxy, Alkylamino und Alkylaminocarbonyl substituiert sind mit einem Substituenten, wobei der Substituent ausgewählt wird aus der Gruppe bestehend aus Hydroxy, Amino, C₁-C₄-Alkoxy und C₁-C₄-Alkylamino, und worin Pyrrolidinyl, Piperidinyl, Morpholinyl und Piperazinyl substituiert sein können mit 1 bis 3 Substituenten C₁-C₄-Alkyl, und wobei
R¹⁴ für C₁-C₄-Alkoxy, C₁-C₄-Alkylamino, C₁-C₄-Alkylaminocarbonyl, Pyrrolidinyl, Piperidinyl, Morpholinyl oder Piperazinyl steht, worin Alkoxy, Alkylamino und Alkylaminocarbonyl substituiert sind mit einem Substituenten, wobei der Substituent ausgewählt wird aus der Gruppe bestehend aus Hydroxy, Amino, C₁-C₄-Alkoxy und C₁-C₄-Alkylamino, und worin Pyrrolidinyl, Piperidinyl, Morpholinyl und Piperazinyl substituiert sein können mit 1 bis 3 Substituenten C₁-C₄-Alkyl,
- R²: für Phenyl steht, wobei Phenyl substituiert sein kann mit 1 bis 3 Substituenten, wobei die Substituenten unabhängig voneinander ausgewählt werden aus der Gruppe bestehend aus Chlor, Fluor, Trifluormethyl, Trifluormethoxy und Methyl,
- R⁴: für Wasserstoff steht,
- R¹⁶: für eine Gruppe der Formel 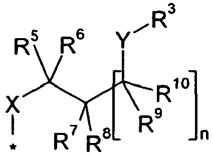 steht, wobei
* die Anknüpfstelle an den Heterocyclus bedeutet,
n für die Zahl 0 steht,
X für NR¹¹ steht, wobei R¹¹ für Wasserstoff steht,
Y für NR¹² steht, wobei R¹² für Wasserstoff steht,
R³ für 2-Pyridyl oder 1,3-Thiazol-2-yl steht, wobei 2-Pyridyl und 1,3-Thiazol-2-yl substituiert sind mit 1 bis 2 Substituenten, wobei die Substituenten unabhängig voneinander ausgewählt werden aus der Gruppe bestehend aus Cyano, Nitro, Amino, Trifluormethylcarbonyl und Methylcarbonyl,
R⁵ für Wasserstoff oder Methyl steht,
R⁶ für Wasserstoff steht,
R⁷ für Wasserstoff oder Methyl steht,
R⁸ für Wasserstoff steht,
R⁹ für Wasserstoff steht,
R¹⁰ für Wasserstoff steht,
und ihre Salze, ihre Solvate und die Solvate ihrer Salze.

Bevorzugt sind auch Verbindungen der Formel (I), in welcher
- A: für N oder CR¹⁵ steht, wobei R¹⁵ für Wasserstoff steht,
- R¹: für C₁-C₄-Alkylaminocarbonyl, 1,3-Dihydroxyprop-2-ylaminocarbonyl, Piperazinyl-carbonyl, -CH₂R¹³ oder -CH₂CH₂R¹⁴ steht, wobei Alkylaminocarbonyl substituiert ist mit einem Substituenten, wobei der Substituent ausgewählt wird aus der Gruppe bestehend aus Hydroxy, Amino, C₁-C₄-Alkylamino, Pyrrolidinyl, Piperidinyl, Morpholinyl und Piperazinyl, worin Pyrrolidinyl, Piperidinyl, Morpholinyl und Piperazinyl substituiert sein können mit 1 bis 3 Substituenten C₁-C₄-Alkyl, und wobei Piperazinylcarbonyl substituiert sein kann mit 1 bis 3 Substituenten, wobei die Substituenten unabhängig voneinander ausgewählt werden aus der Gruppe bestehend aus Oxo, C₁-C₄-Alkyl, C₁-C₄-Alkoxy und C₁-C₄-Alkylamino, und wobei
R¹³ für C₁-C₄-Alkoxy, C₁-C₄-Alkylamino, C₁-C₄-Alkylaminocarbonyl, Pyrrolidinyl, Piperidinyl, Morpholinyl oder Piperazinyl steht, worin Alkoxy, Alkylamino und Alkylaminocarbonyl substituiert sind mit einem Substituenten, wobei der Substituent ausgewählt wird aus der Gruppe bestehend aus Hydroxy, Amino, C₁-C₄-Alkoxy und C₁-C₄-Alkylamino, und worin Pyrrolidinyl, Piperidinyl, Morpholinyl und Piperazinyl substituiert sein können mit 1 bis 3 Substituenten C₁-C₄-Alkyl, und wobei
R¹⁴ für C₁-C₄-Alkoxy, C₁-C₄-Alkylamino, C₁-C₄-Alkylaminocarbonyl, Pyrrolidinyl, Piperidinyl, Morpholinyl oder Piperazinyl steht, worin Alkoxy, Alkylamino und Alkylaminocarbonyl substituiert sind mit einem Substituenten, wobei der Substituent ausgewählt wird aus der Gruppe bestehend aus Hydroxy, Amino, C₁-C₄-Alkoxy und C₁-C₄-Alkylamino, und worin Pyrrolidinyl, Piperidinyl, Morpholinyl und Piperazinyl substituiert sein können mit 1 bis 3 Substituenten C₁-C₄-Alkyl,
- R²: für Phenyl steht, wobei Phenyl substituiert sein kann mit 1 bis 3 Substituenten, wobei die Substituenten unabhängig voneinander ausgewählt werden aus der Gruppe bestehend aus Chlor, Fluor, Trifluormethyl, Trifluormethoxy und Methyl,
- R⁴: für Wasserstoff steht,
- R¹⁶: für eine Gruppe der Formel 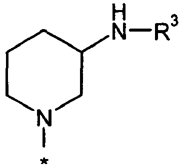 steht, wobei
* die Anknüpfstelle an den Heterocyclus bedeutet,
R³ für 2-Pyridyl oder 1,3-Thiazol-2-yl steht, wobei 2-Pyridyl und 1,3-Thiazol-2-yl substituiert sind mit 1 bis 2 Substituenten, wobei die Substituenten unabhängig voneinander ausgewählt werden aus der Gruppe bestehend aus Cyano, Nitro, Amino, Trifluormethylcarbonyl und Methylcarbonyl,
und ihre Salze, ihre Solvate und die Solvate ihrer Salze.

Bevorzugt sind auch Verbindungen der Formel (I), welche der Formel 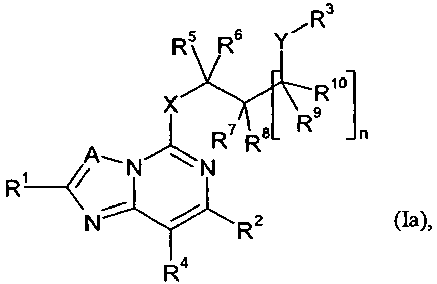 entsprechen,

in welcher
- A: für N oder CH steht,
- n: für die Zahl 0 oder 1 steht,
- X: für NR¹¹, S oder O steht, wobei R¹¹ für Wasserstoff, C₁-C₃-Alkyl oder Cyclopropyl steht,
- Y: für NR¹², S oder O steht, wobei R¹² für Wasserstoff, C₁-C₃-Alkyl oder Cyclopropyl steht,
- R¹: für Wasserstoff, Hydroxy, Amino, Hydroxycarbonyl, Aminocarbonyl, Trifluormethyl, Trifluormethoxy, Cyano, C₁-C₄-Alkyl, C₁-C₄-Alkoxy, C₁-C₄-Alkylamino, C₁-C₄-Alkylcarbonyl, C₁-C₄-Alkoxycarbonyl, C₁-C₄-Alkylaminocarbonyl, C₁-C₄-Alkylcarbonylamino, C₁-C₄-Alkylsulfonylamino, -CH₂R¹³ oder -CH₂CH₂R¹⁴ steht, wobei Alkoxy, Alkylamino, Alkylcarbonyl, Alkoxycarbonyl, Alkylaminocarbonyl, Alkylcarbonylamino und Alkylsulfonylamino substituiert sein können mit einem Substituenten, wobei der Substituent ausgewählt wird aus der Gruppe bestehend aus Hydroxy, Amino, Hydroxycarbonyl, Aminocarbonyl, C₁-C₄-Alkoxy, C₁-C₄-Alkylamino, C₁-C₄-Alkoxycarbonyl, C₁-C₄-Alkylaminocarbonyl und C₁-C₄-Alkylcarbonylamino, und wobei
R¹³ für Hydroxy, Amino, Cyano, Hydroxycarbonyl, Aminocarbonyl, C₁-C₄-Alkoxy, C₁-C₄-Alkylamino, C₁-C₄-Alkoxycarbonyl, C₁-C₄-Alkylaminocarbonyl, C₁-C₄-Alkylcarbonylamino oder 5- oder 6-gliedriges Heterocyclyl steht, worin Alkoxy, Alkylamino, Alkoxycarbonyl, Alkylaminocarbonyl und C₁-C₄-Alkylcarbonylamino substituiert sein können mit einem Substituenten, wobei der Substituent ausgewählt wird aus der Gruppe bestehend aus Hydroxy, Amino, Hydroxycarbonyl, Aminocarbonyl, C₁-C₄-Alkoxy, C₁-C₄-Alkylamino, C₁-C₄-Alkoxycarbonyl, C₁-C₄-Alkylaminocarbonyl und C₁-C₄-Alkylcarbonylamino, und worin Heterocyclyl substituiert sein kann mit 1 bis 3 Substituenten, wobei die Substituenten unabhängig voneinander ausgewählt werden aus der Gruppe bestehend aus Halogen, Oxo, C₁-C₄-Alkyl, C₁-C₄-Alkoxy, C₁-C₄-Alkylamino, C₁-C₄-Alkylcarbonyl,C₁-C₄-Alkoxycarbonyl und C_{I}-C₄-Alkylaminocarbonyl, und wobei
R¹⁴ für Hydroxy, Amino, Cyano, Hydroxycarbonyl, Aminocarbonyl, C₁-C₄-Alkoxy, C₁-C₄-Alkylamino, C₁-C₄-Alkoxycarbonyl, C₁-C₄-Alkylaminocarbonyl, C₁-C₄-Alkylcarbonylamino oder 5- oder 6-gliedriges Heterocyclyl steht, worin Alkoxy, Alkylamino, Alkoxycarbonyl, Alkylaminocarbonyl und C₁-C₄-Alkylcarbonylamino substituiert sein können mit einem Substituenten, wobei der Substituent ausgewählt wird aus der Gruppe bestehend aus Hydroxy, Amino, Hydroxycarbonyl, Aminocarbonyl, C₁-C₄-Alkoxy, C₁-C₄-Alkylamino, C₁-C₄-Alkoxycarbonyl, C₁-C₄-Alkylaminocarbonyl und C₁-C₄-Alkylcarbonylamino, und worin Heterocyclyl substituiert sein kann mit 1 bis 3 Substituenten, wobei die Substituenten unabhängig voneinander ausgewählt werden aus der Gruppe bestehend aus Halogen, Oxo, C₁-C₄-Alkyl, C₁-C₄-Alkoxy, C₁-C₄-Alkylamino, C₁-C₄-Alkylcarbonyl,C₁-C₄-Alkoxycarbonyl und C₁-C₄-Alkylaminocarbonyl,
- R²: für C₆-C₁₀-Aryl oder 5- bis 10-gliedriges Heteroaryl steht, wobei Aryl und Heteroaryl substituiert sein können mit 1 bis 3 Substituenten, wobei die Substituenten unabhängig voneinander ausgewählt werden aus der Gruppe bestehend aus Hydroxy, Hydroxymethyl, Amino, Halogen, Cyano, Trifluormethyl, Trifluormethoxy, Aminocarbonyl, C₁-C₄-Alkyl, C₁-C₄-Alkoxy, C₁-C₄-Alkoxymethyl, C₁-C₄-Alkylamino, C₁-C₄-Alkylaminomethyl, C₁-C₄-Alkylcarbonyl, C₁-C₄-Alkoxycarbonyl, C₁-C₄-Alkylaminocarbonyl, C₁-C₄-Alkylcarbonylamino, C₁-C₄-Alkylsulfonyl, C₁-C₄-Alkylsulfonylamino, C₁-C₄-Alkylaminosulfonyl, Phenyl, Benzyloxy, 5- oder 6-gliedrigem Heterocyclyl, 5- oder 6-gliedrigem Heterocyclylcarbonyl, 5- oder 6-gliedrigem Heterocyclylmethyl und 5- oder 6-gliedrigem Heteroaryl, worin Phenyl, Benzyloxy, Heterocyclyl, Heterocyclylcarbonyl, Heterocyclylmethyl und Heteroaryl substituiert sein können mit 1 bis 3 Substituenten, wobei die Substituenten unabhängig voneinander ausgewählt werden aus der Gruppe bestehend aus Halogen, Cyano, Trifluormethyl, Trifluormethoxy, Aminocarbonyl, C₁-C₄-Alkyl, C₁-C₄-Alkoxy, C₁-C₄-Alkylamino, C₁-C₄-Alkylcarbonyl, C₁-C₄-Alkoxycarbonyl, C₁-C₄-Alkylaminocarbonyl und C₁-C₄-Alkylcarbonylamino, oder zwei der Substituenten am Aryl zusammen mit den Kohlenstoffatomen, an die sie gebunden sind, ein 1,3-Dioxolan oder 1,4-Dioxan bilden,
- R³: für 2-Pyridyl, 2-Aminopyrimid-4-yl, 1,3-Thiazol-2-yl, 1,3-Thiazol-4-yl oder 1,3-Thiazol-5-yl steht, wobei 2-Pyridyl, 2-Aminopyrimid-4-yl, 1,3-Thiazol-2-yl, 1,3-Thiazol-4-yl und 1,3-Thiazol-5-yl substituiert sind mit 1 bis 3 Substituenten, wobei die Substituenten unabhängig voneinander ausgewählt werden aus der Gruppe bestehend aus Halogen, Cyano, Nitro, Amino, Trifluormethyl, Trifluormethoxy, Aminocarbonyl, C₁-C₄-Alkyl, C₁-C₄-Alkoxy, C₁-C₄-Alkylamino, C₁-C₄-Alkylcarbonyl, C₁-C₄-Alkoxycarbonyl, C₁-C₄-Alkylaminocarbonyl und C₃-C₆-Cycloalkylcarbonyl, worin Alkyl, Alkoxy, Alkylamino, Alkylcarbonyl, Alkoxycarbonyl, Alkylaminocarbonyl und Cycloalkylcarbonyl substituiert sein können mit einem Substituenten, wobei der Substituent ausgewählt wird aus der Gruppe bestehend aus Cyano, Hydroxy, Amino, Trifluormethyl und C₃-C₆-Cycloalkyl,
- oder R³: für eine Gruppe der Formel 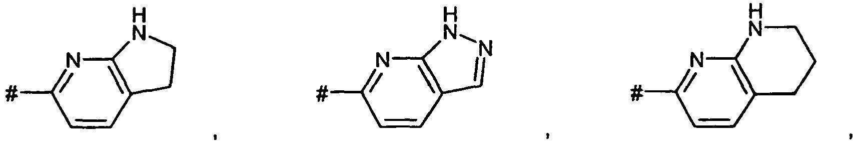 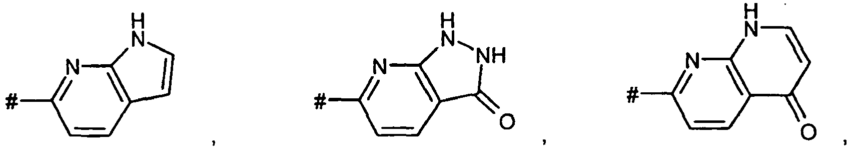 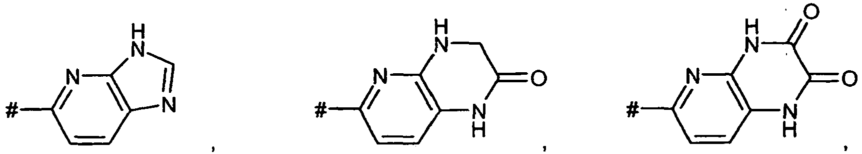 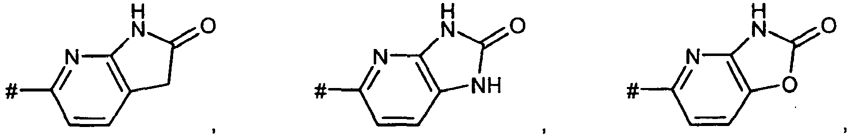 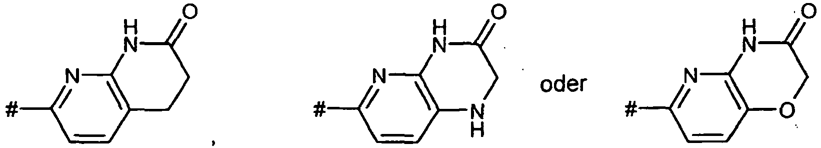 steht, wobei # die Anknüpfstelle an Y bedeutet,
- R⁴: für Wasserstoff, Halogen, Cyano, Trifluormethyl, C₁-C₃-Alkyl, Methylthio oder Cyclopropyl steht,
- R⁵: für Wasserstoff, C₁-C₃-Alkyl oder Cyclopropyl steht,
- R⁶: für Wasserstoff oder C₁-C₃-Alkyl steht,
- R⁷: für Wasserstoff, C₁-C₃-Alkyl oder Cyclopropyl steht,
- R⁸: für Wasserstoff oder C₁-C₃-Alkyl steht,
- R⁹: für Wasserstoff, C₁-C₃-Alkyl oder Cyclopropyl steht,
- R¹⁰: für Wasserstoff oder C₁-C₃-Alkyl steht,
und ihre Salze, ihre Solvate und die Solvate ihrer Salze.

Bevorzugt sind auch Verbindungen der Formel (Ia), in welcher
- A: für N oder CH steht,
- n: für die Zahl 0 oder 1 steht,
- X: für NR¹¹, S oder O steht, wobei R¹¹ für Wasserstoff oder Methyl steht,
- Y: für NR¹², S oder O steht, wobei R¹² für Wasserstoff oder Methyl steht,
- R¹: für Wasserstoff, Hydroxycarbonyl, Aminocarbonyl, Trifluormethyl, Cyano, C₁-C₄-Alkyl, C₁-C₄-Alkoxy, C₁-C₄-Alkylamino, C₁-C₄-Alkylaminocarbonyl, C₁-C₄-Alkylcarbonylamino, C₁-C₄-Alkylsulfonylamino oder -CH₂R¹³ steht, wobei Alkoxy, Alkylamino, Alkylaminocarbonyl, Alkylcarbonylamino und Alkylsulfonylamino substituiert sein können mit einem Substituenten, wobei der Substituent ausgewählt wird aus der Gruppe bestehend aus Hydroxy, Amino, Hydroxycarbonyl, Aminocarbonyl, C₁-C₄-Alkoxy, C₁-C₄-Alkylamino, C₁-C₄-Alkoxycarbonyl, C₁-C₄-Alkylaminocarbonyl und C₁-C₄-Alkylcarbonylamino, und wobei
R¹³ für Amino, Cyano, Hydroxycarbonyl, Aminocarbonyl, C₁-C₄-Alkoxy, C₁-C₄- Alkylamino, C₁-C₄-Alkoxycarbonyl, C₁-C₄-Alkylaminocarbonyl, C₁-C₄-Alkylcarbonylamino oder 5- oder 6-gliedriges Heterocyclyl steht, worin Alkoxy, Alkylamino, Alkoxycarbonyl, Alkylaminocarbonyl und C₁-C₄-Alkylcarbonylamino substituiert sein können mit einem Substituenten, wobei der Substituent ausgewählt wird aus der Gruppe bestehend aus Hydroxy, Amino, Hydroxycarbonyl, Aminocarbonyl, C₁-C₄-Alkoxy, C₁-C₄-Alkylamino, C₁-C₄-Alkoxycarbonyl, C₁-C₄-Alkylaminocarbonyl und C₁-C₄-Alkylcarbonylamino, und worin Heterocyclyl substituiert sein kann mit 1 bis 3 Substituenten, wobei die Substituenten unabhängig voneinander ausgewählt werden aus der Gruppe bestehend aus Halogen, Oxo, C₁-C₄-Alkyl, C₁-C₄-Alkoxy, C₁-C₄-Alkylamino, C₁-C₄-Alkylcarbonyl, C₁-C₄-Alkoxycarbony und C₁-C₄-Alkylaminocarbonyl,
- R²: für C₆-C₁₀-Aryl, Thienyl, Furyl, Pyrrolyl, Thiazolyl, Oxazolyl, Oxadiazolyl, Pyrazolyl, Imidazolyl, Pyridyl, Pyrimidyl, Pyridazinyl, Pyrazinyl, Indolyl, Indazolyl, Chinolinyl, Benzofuranyl oder Benzoxazolyl steht, wobei Aryl und Thienyl, Furyl, Pyrrolyl, Thiazolyl, Oxazolyl, Oxadiazolyl, Pyrazolyl, Imidazolyl, Pyridyl, Pyrimidyl, Pyridazinyl, Pyrazinyl, Indolyl, Indazolyl, Benzofuranyl und Benzoxazolyl substituiert sein können mit 1 bis 3 Substituenten, wobei die Substituenten unabhängig voneinander ausgewählt werden aus der Gruppe bestehend aus Hydroxy, Hydroxymethyl, Amino, Halogen, Cyano, Trifluormethyl, Trifluormethoxy, Aminocarbonyl, C₁-C₄-Alkyl, C₁-C₄-Alkoxy, C₁-C₄-Alkoxymethyl, C₁-C₄-Alkylamino, C₁-C₄-Alkylaminomethyl, C₁-C₄-Alkylcarbonyl, C₁-C₄-Alkoxycarbonyl, C₁-C₄-Alkylaminocarbonyl, C₁-C₄-Alkylcarbonylamino, C₁-C₄-Alkylsulfonyl, C₁-C₄-Alkylsulfonylamino, C₁-C₄-Alkylaminosulfonyl, Phenyl, Benzyloxy, 5- oder 6-gliedrigem Heterocyclyl, 5- oder 6-gliedrigem Heterocyclylcarbonyl, 5- oder 6-gliedrigem Heterocyclylmethyl und 5- oder 6-gliedrigem Heteroaryl, worin Phenyl, Benzyloxy, Heterocyclyl, Heterocyclylcarbonyl, Heterocyclylmethyl und Heteroaryl substituiert sein können mit 1 bis 3 Substituenten, wobei die Substituenten unabhängig voneinander ausgewählt werden aus der Gruppe bestehend aus Halogen, Cyano, Trifluormethyl, Trifluormethoxy, Aminocarbonyl, C₁-C₄-Alkyl, C₁-C₄-Alkoxy, C₁-C₄-Alkylamine, C₁-C₄-Alkylcarbonyl, C₁-C₄-Alkoxycarbonyl, C₁-C₄-Alkylaminocarbonyl und C₁-C₄-Alkylcarbonylamino, oder zwei der Substituenten am Aryl zusammen mit den Kohlenstoffatomen, an die sie gebunden sind, ein 1,3-Dioxolan oder 1,4-Dioxan bilden,
- R³: für 2-Pyridyl, 2-Aminopyrimid-4-yl, 1,3-Thiazol-2-yl, 1,3-Thiazol-4-yl oder 1,3-Thiazol-5-yl steht, wobei 2-Pyridyl, 2-Aminopyrimid-4-yl, 1,3-Thiazol-2-yl, 1,3-Thiazol-4-yl und 1,3-Thiazol-5-yl substituiert sind mit 1 bis 3 Substituenten, wobei die Substituenten unabhängig voneinander ausgewählt werden aus der Gruppe bestehend aus Halogen, Cyano, Nitro, Amino, Trifluormethyl, Trifluormethoxy, Aminocarbonyl, C₁-C₄-Alkyl, C₁-C₄-Alkoxy, C₁-C₄-Alkylamino, C₁-C₄-Alkylcarbonyl, C₁-C₄-Alkoxycarbonyl, C₁-C₄-Alkylaminocarbonyl und C₃-C₆-Cycloalkylcarbonyl, worin Alkyl, Alkoxy, Alkylamino, Alkylcarbonyl, Alkoxycarbonyl, Alkylaminocarbonyl und Cycloalkylcarbonyl substituiert sein können mit einem Substituenten, wobei der Substituent ausgewählt wird aus der Gruppe bestehend aus Cyano, Hydroxy, Amino, Trifluormethyl und C₃-C₆-Cycloalkyl,
- oder R³: für eine Gruppe der Formel 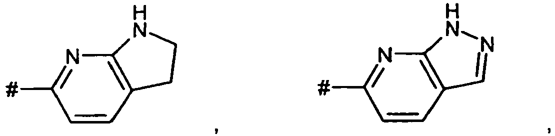 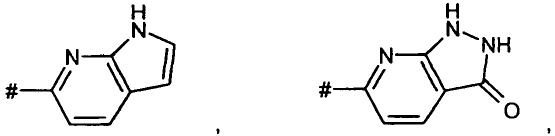 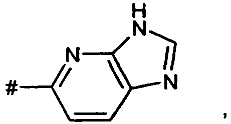 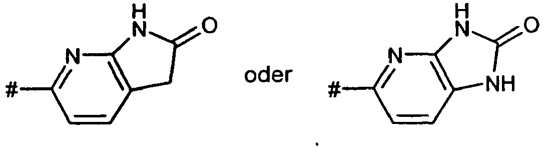 steht, wobei # die Anknüpfstelle an Y bedeutet,
- R⁴: für Wasserstoff, Halogen, Cyano oder Trifluormethyl steht,
- R⁵: für Wasserstoff oder Methyl steht,
- R⁶: für Wasserstoff oder Methyl steht,
- R⁷: für Wasserstoff oder Methyl steht,
- R⁸: für Wasserstoff oder Methyl steht,
- R⁹: für Wasserstoff oder Methyl steht,
- R¹⁰: für Wasserstoff oder Methyl steht,
und ihre Salze, ihre Solvate und die Solvate ihrer Salze.

Bevorzugt sind auch Verbindungen der Formel (Ia), in welcher
- A: für N oder CH steht,
- n: für die Zahl 0 steht,
- X: für NR¹¹, S oder O steht, wobei R¹¹ für Wasserstoff steht,
- Y: für NR¹², S oder O steht, wobei R¹² für Wasserstoff oder Methyl steht,
- R¹: für Wasserstoff, Trifluormethyl, Methyl oder -CH₂R¹³ steht, wobei R¹³ für Pyrrolidinyl, Piperidinyl oder Morpholinyl steht,
- R²: für Phenyl, Naphthyl, Thienyl, Pyrazolyl, Pyridyl, Indolyl oder Chinolinyl steht, wobei Phenyl, Naphthyl, Thienyl, Pyrazolyl, Pyridyl, Indolyl und Chinolinyl substituiert sein können mit 1 bis 3 Substituenten, wobei die Substituenten unabhängig voneinander ausgewählt werden aus der Gruppe bestehend aus Halogen, Trifluormethyl, Trifluormethoxy, Aminocarbonyl, C₁-C₄-Alkyl, C₁-C₄-Alkoxy, C₁-C₄-Alkoxycarbonyl, C₁-C₄-Alkylaminocarbonyl, C₁-C₄-Alkylsulfonyl, C₁-C₄-Alkylsulfonylamino, Benzyloxy, Pyrrolidinyl, Piperidinyl, Morpholinyl und Morpholinylcarbonyl, worin Benzyloxy substituiert sein kann mit 1 bis 3 Substituenten, wobei die Substituenten unabhängig voneinander ausgewählt werden aus der Gruppe bestehend aus Halogen, oder zwei der Substituenten am Phenyl zusammen mit den Kohlenstoffatomen, an die sie gebunden sind, ein 1,3-Dioxolan oder 1,4-Dioxan bilden,
- R³: für 2-Pyridyl oder 1,3-Thiazol-2-yl steht, wobei 2-Pyridyl und 1,3-Thiazol-2-yl substituiert sind mit 1 bis 2 Substituenten, wobei die Substituenten unabhängig voneinander ausgewählt werden aus der Gruppe bestehend aus Cyano, Nitro, Amino, Trifluormethyl und Methylcarbonyl,
- R⁴: für Wasserstoff steht,
- R⁵: für Wasserstoff oder Methyl steht,
- R⁶: für Wasserstoff steht,
- R⁷: für Wasserstoff oder Methyl steht,
- R⁸: für Wasserstoff steht,
- R⁹: für Wasserstoff steht,
- R¹⁰: für Wasserstoff steht,
und ihre Salze, ihre Solvate und die Solvate ihrer Salze.

Bevorzugt sind auch Verbindungen der Formel (I) oder (Ia), in welcher A für CH steht.

Bevorzugt sind auch Verbindungen der Formel (I) oder (Ia), in welcher A für N steht.

Bevorzugt sind auch Verbindungen der Formel (I) oder (Ia), in welcher n für die Zahl 0 steht.

Bevorzugt sind auch Verbindungen der Formel (I) oder (Ia), in welcher X für NR¹¹ steht, wobei R¹¹ für Wasserstoff steht.

Bevorzugt sind auch Verbindungen der Formel (I) oder (Ia), in welcher Y für NR¹² steht, wobei R¹² für Wasserstoff steht.

Bevorzugt sind auch Verbindungen der Formel (I) oder (Ia), in welcher R¹ für Wasserstoff, Trifluormethyl oder Methyl steht.

Bevorzugt sind auch Verbindungen der Formel (I) oder (Ia), in welcher R¹ für Wasserstoff oder Methyl steht.

Bevorzugt sind auch Verbindungen der Formel (I) oder (Ia), in welcher R¹ für -CH₂R¹³ steht, wobei R¹³ für Morpholinyl steht.

Bevorzugt sind auch Verbindungen der Formel (I) oder (Ia), in welcher
- R³: für 2-Pyridyl oder 1,3-Thiazol-2-yl steht, wobei 2-Pyridyl und 1,3-Thiazol-2-yl substituiert sind mit 1 bis 2 Substituenten, wobei die Substituenten unabhängig voneinander ausgewählt werden aus der Gruppe bestehend aus Cyano, Nitro, Amino, Trifluormethyl und Methylcarbonyl.

Bevorzugt sind auch Verbindungen der Formel (I) oder (Ia), in welcher
- R³: für 2-Pyridyl oder 1,3-Thiazol-2-yl steht, wobei 2-Pyridyl und 1,3-Thiazol-2-yl substituiert sind mit 1 bis 2 Substituenten, wobei die Substituenten unabhängig voneinander ausgewählt werden aus der Gruppe bestehend aus Cyano, Nitro, Amino, Trifluormethylcarbonyl und Methylcarbonyl.

Bevorzugt sind auch Verbindungen der Formel (I) oder (Ia), in welcher R⁴ für Wasserstoff steht.

Bevorzugt sind auch Verbindungen der Formel (I) oder (Ia), in welcher R⁵, R⁶, R⁸, R⁹ und R¹⁰ für Wasserstoff stehen und R⁷ für Wasserstoff oder Methyl steht.

Bevorzugt sind auch Verbindungen der Formel (I), in welcher
- R¹⁶: für eine Gruppe der Formel 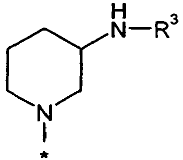 steht, wobei
* die Anknüpfstelle an den Heterocyclus bedeutet,
R³ für 2-Pyridyl oder 1,3-Thiazol-2-yl steht, wobei 2-Pyridyl und 1,3-Thiazol-2-yl substituiert sind mit 1 bis 2 Substituenten, wobei die Substituenten unabhängig voneinander ausgewählt werden aus der Gruppe bestehend aus Cyano, Nitro, Amino, Trifluormethylcarbonyl und Methylcarbonyl.

Gegenstand der Erfindung ist weiterhin ein Verfahren zur Herstellung der Verbindungen der Formel (I), oder ihrer Salze, ihrer Solvate oder der Solvate ihrer Salze, wobei
[A] die Verbindungen der Formel 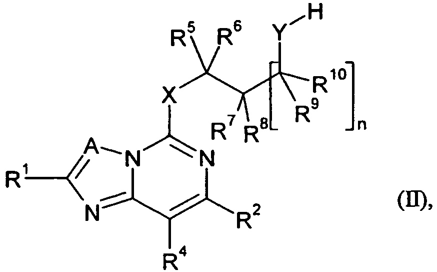 in welcher
   A, n, X, Y, R¹, R², R⁴, R⁵, R⁶, R⁷, R⁸, R⁹ und R¹⁰ die oben angegebene Bedeutung haben, mit Verbindungen der Formel

   R³—X¹ , (III)

   in welcher
   R³ die oben angegebene Bedeutung hat, und
   - X¹: für Halogen, bevorzugt Chlor oder Fluor, steht, umgesetzt werden,
   oder
[B] die Verbindungen der Formel 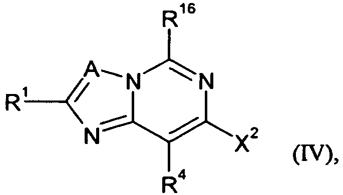 in welcher
   A, R¹, R⁴ und R¹⁶ die oben angegebene Bedeutung haben, und
   - X²: für Iod, Brom, Chlor oder Trifluormethansulfonyl, bevorzugt Iod oder Brom, steht, mit Verbindungen der Formel

   Q - R² , (V)

   in welcher
   R² die oben angegebene Bedeutung hat, und
   - Q: für -B(OH)₂, einen Boronsäure-Ester, bevorzugt Boronsäurepinakolester, oder -BF₃-K⁺ steht, unter Suzuki-Kupplungsbedingungen umgesetzt werden,
   oder
[C] die Verbindungen der Formel 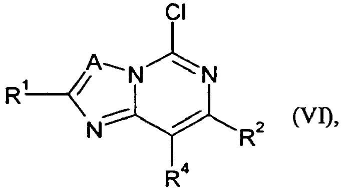 in welcher
   A, R¹, R² und R⁴ die oben angegebene Bedeutung haben, mit Verbindungen der Formel

   H—R¹⁶ , (IX)

   in welcher
   R¹⁶ die oben angegebene Bedeutung hat,
   umgesetzt werden.

Die Umsetzung nach Verfahren [A] erfolgt im Allgemeinen in inerten Lösungsmitteln, gegebenenfalls in Gegenwart einer Base, gegebenenfalls in einer Mikrowelle, bevorzugt in einem Temperaturbereich von 50°C bis 200°C bei Normaldruck bis 3 bar.

Basen sind beispielsweise Alkalicarbonate, wie z.B. Natrium-, Kalium- oder Caesiumcarbonat, oder organische Basen wie Trialkylamine, z.B. Triethylamin, N-Methylmorpholin, N-Methylpiperidin, 4-Dimethylaminopyridin oder Diisopropylethylamin, oder andere Basen wie beispielsweise Natriumhydrid oder Kalium-tert.-butylat, bevorzugt ist Diisopropylethylamin oder Natriumhydrid.

Inerte Lösungsmittel sind beispielsweise Halogenkohlenwasserstoffe wie Methylenchlorid oder Trichlormethan, Alkohole wie Methanol, Ethanol, n-Propanol oder Isopropanol, oder Ether wie Dioxan oder Tetrahydrofuran, oder andere Lösungsmittel wie beispielsweise Dimethylsulfoxid, Dimethylformamid oder N-Methylpyrrolidon, oder Gemische dieser Lösungsmittel, bevorzugt ist Isopropanol oder Dimethylsulfoxid.

Die Umsetzung nach Verfahren [B] erfolgt im Allgemeinen in inerten Lösungsmitteln, in Gegenwart eines Katalysators, gegebenenfalls in Gegenwart eines Zusatzreagenzes, gegebenenfalls in einer Mikrowelle, bevorzugt in einem Temperaturbereich von Raumtemperatur bis 150°C bei Normaldruck bis 3 bar.

Katalysatoren sind beispielsweise für Suzuki-Reaktionsbedingungen übliche Palladium-Katalysatoren, bevorzugt sind Katalysatoren wie z.B. Dichlorbis(triphenylphosphin)-palladium, Tetrakistriphenylphosphinpalladium(0), Palladium(II)acetat/Triscyclohexylphosphin, Bis-(diphenylphosphanferrocenyl)-palladium-(II)-chlorid, 1,3-Bis(2,6-diisopropylphenyl)imidazol-2-yliden(1,4-napthtochinon)palladiumdimer, Allyl(chlor)(1,3-dimesityl-1,3-dihydro-2H-imidazol-2-yliden)palladium oder Palladium(II)acetat/Dicyclohexyl-(2',4',6'-triisopropyl-biphenyl-2-yl)-phosphin. Als Palladiumquelle kann auch Tris(dibenzylidenaceton)dipalladium eingesetzt werden.

Zusatzreagenzien sind beispielsweise Kaliumacetat, Cäsium-, Kalium- oder Natriumcarbonat, Kalium-tert.-butylat, Cäsiumfluorid oder Kaliumphosphat, bevorzugt sind Zusatzreagenzien wie z.B. Kaliumacetat und/oder wässrige Natriumcarbonatlösung.

Inerte Lösungsmittel sind beispielsweise Ether wie Dioxan, Tetrahydrofuran oder 1,2-Dimethoxyethan, Kohlenwasserstoffe wie Benzol, Xylol oder Toluol, oder Carbonsäureamide wie Dimethylformamid oder Dimethylacetamid, Alkylsulfoxide wie Dimethylsulfoxid, oder N-Methylpyrrolidon oder Acetonitril, oder Gemische der Lösungsmittel mit Alkoholen wie Methanol oder Ethanol und/oder Wasser, bevorzugt ist Dioxan oder Acetonitril oder ein Gemisch aus einem dieser Lösungsmittel mit Wasser.

Die Umsetzung nach Verfahren [C] erfolgt nach den unter Verfahren [A] angegebenen Reaktionsbedingungen.

Die Verbindungen der Formeln (III), (V) und (IX) sind bekannt oder lassen sich nach bekannten Verfahren aus den entsprechenden Ausgangsverbindungen synthetisieren.

Die Verbindungen der Formel (VI) sind bekannt, lassen sich nach bekannten Verfahren aus den entsprechenden Ausgangsverbindungen synthetisieren oder können analog den im Beispielteil (Beispiel 1A bis 6A) beschriebenen Verfahren hergestellt werden.

Die Verbindungen der Formel (II) sind bekannt oder können hergestellt werden, indem Verbindungen der Formel 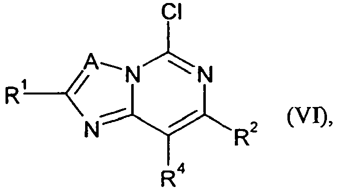 in welcher
A, R¹, R² und R⁴ die oben angegebene Bedeutung haben, mit Verbindungen der Formel 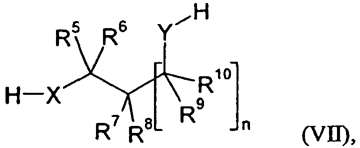 in welcher
n, X, Y, **R⁵, R⁶, R⁷, R⁸, R⁹ und R¹⁰** die oben angegebene Bedeutung haben, umgesetzt werden.

Die Umsetzung erfolgt nach den unter Verfahren [A] angegebenen Reaktionsbedingungen.

Der Rest Y ist während der Umsetzung gegebenenfalls mit einer dem Fachmann bekannten Schutzgruppe geschützt, die nach der Reaktion nach Standardverfahren abgespalten wird.

Die Verbindungen der Formel (VII) sind bekannt oder lassen sich nach bekannten Verfahren aus den entsprechenden Ausgangsverbindungen synthetisieren.

Die Verbindungen der Formel (IV) sind bekannt oder können hergestellt werden, indem Verbindungen der Formel 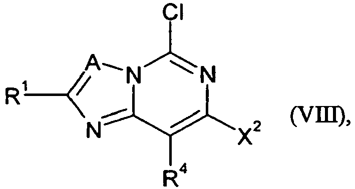 in welcher
A, R¹, R⁴ und X² die oben angegebene Bedeutung haben,
mit Verbindungen der Formel

H—R¹⁶ , (IX)

in welcher
R¹⁶ die oben angegebene Bedeutung hat,
umgesetzt werden.

Die Umsetzung erfolgt nach den unter Verfahren [A] angegebenen Reaktionsbedingungen.

Die Verbindungen der Formel (VIII) sind bekannt, lassen sich nach bekannten Verfahren aus den entsprechenden Ausgangsverbindungen synthetisieren oder können analog den im Beispielteil (Beispiel 9A bis 11A und Beispiel 50A bis 53A) beschriebenen Verfahren hergestellt werden.

Die Herstellung der Ausgangsverbindungen und der Verbindungen der Formel (I) kann durch die folgenden Syntheseschemata verdeutlicht werden.

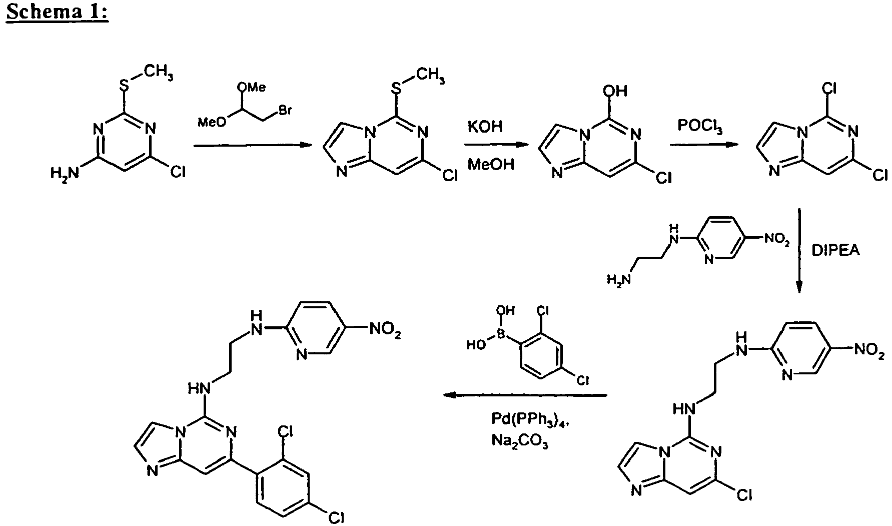

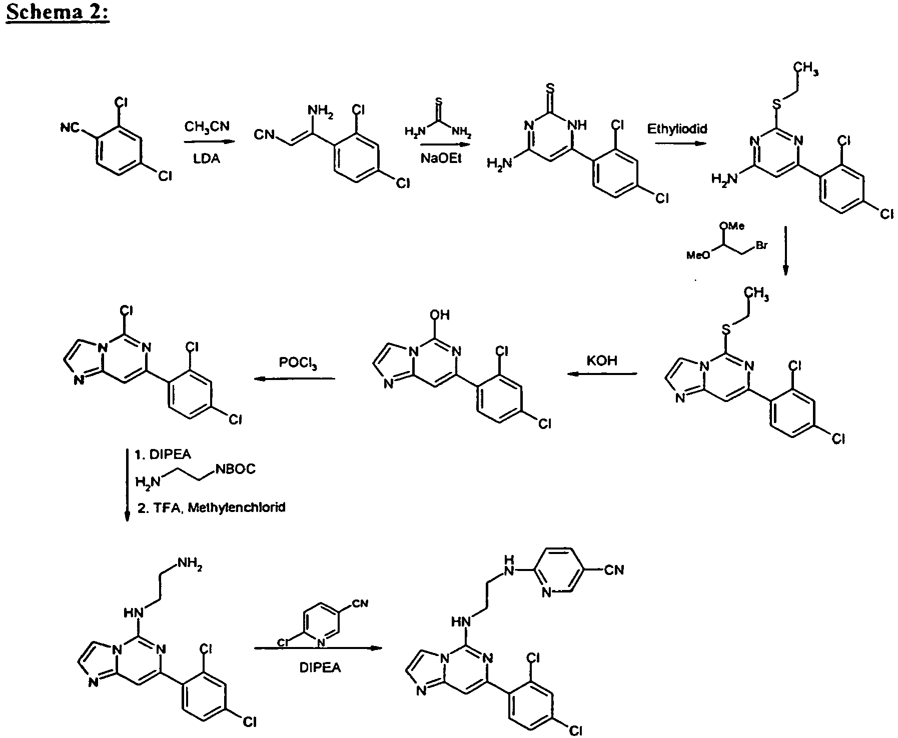

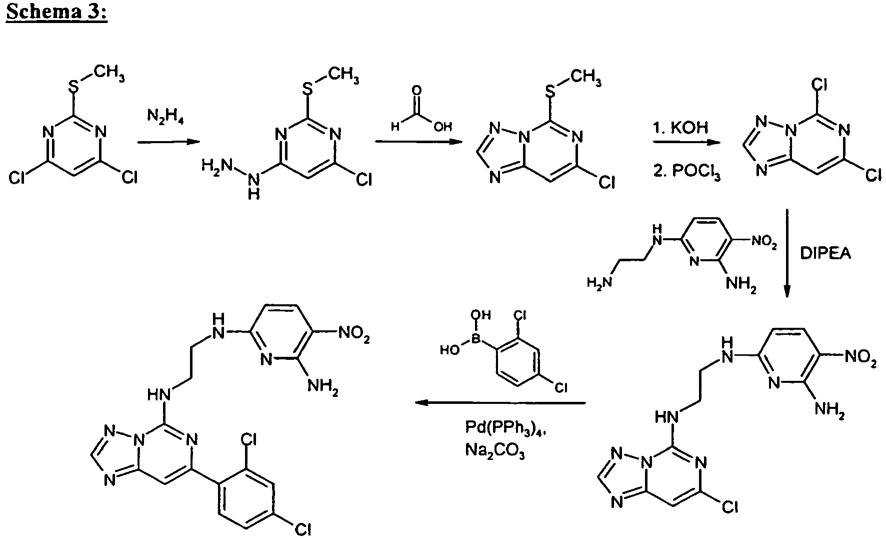

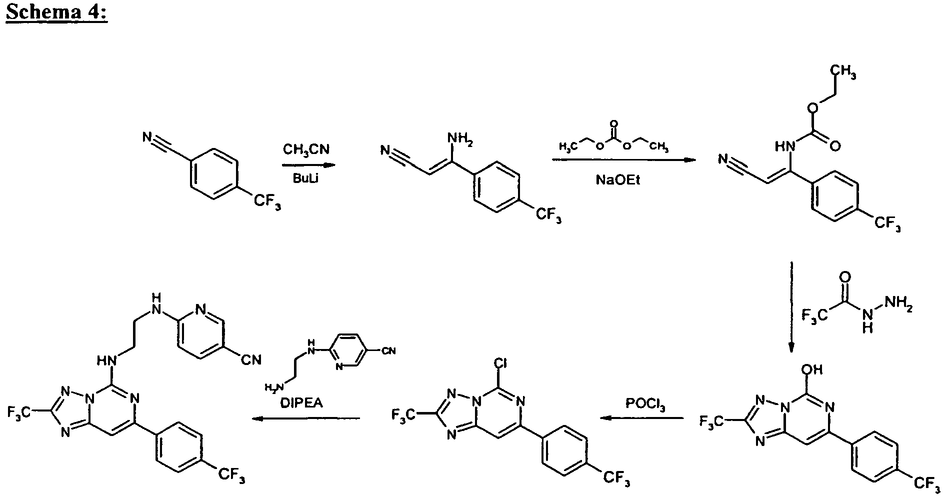

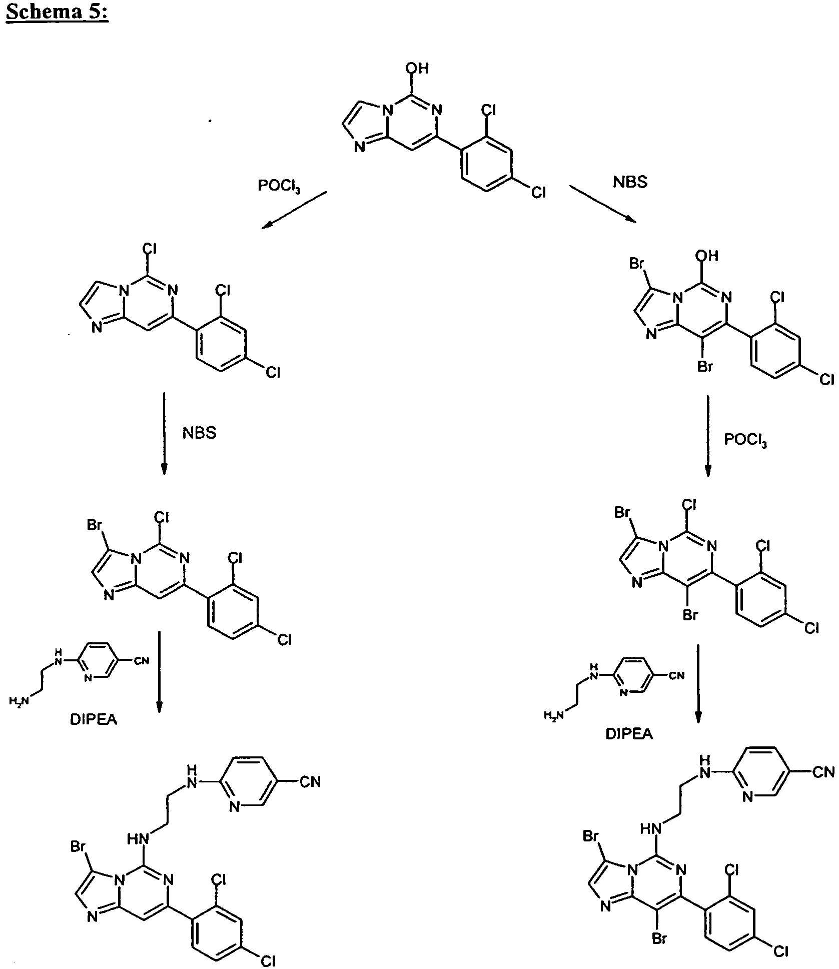

Die erfindungsgemäßen Verbindungen zeigen ein nicht vorhersehbares, wertvolles pharmakologisches und pharmakokinetisches Wirkspektrum.

Sie eigenen sich daher zur Verwendung als Arzneimittel zur Behandlung und/oder Prophylaxe von Krankheiten bei Menschen und Tieren.

Weiterer Gegenstand der vorliegenden Erfindung ist der Einsatz der erfindungsgemäßen Verbindungen zur Behandlung und/oder Prophylaxe von Erkrankungen, vorzugsweise hämatologischen Erkrankungen, insbesondere von Leukopenien und Neutropenie.

Die erfindungsgemäßen Verbindungen eignen sich daher zur Prophylaxe und/oder Behandlung von Neurodegenerativen Erkrankungen wie z.B. Alzheimer, Parkinson, Schizophrenie, Degeneration, Dementia, Depressionen; Aggression, zerebrovaskulär Ischämie, Schlafstärungen, Huntington-Chorea, neurotiaumatische Erkrankungen wie z.B. Schlaganfall; Typ 2 Diabetes Mellitus und assoziierte Erkrankungen wie z.B. das metabolische Syndrom oder Fettleibigkeit, Typ 1 Diabetes Mellitus, Diabetische Nephrophatie, Diabetische Neurophatie, Diabetische Retinophatie, Glomerulonephritis, Hyperkalzämie, Hyperglykämie, Hyperlipidimie, Glukose-Galaktose-Malabsorption, allgemeine endokrine Dysfunktionen wie z.B. Pankreatitis; hämatologische Erkrankungen, wie zum Beispiel erworbene und angeborene Neutropenie, Granulozytopenie, erworbene und angeborene Leukopenie, erworbene und angeborene Anämie, hämolytische Anämie, Sichelzellenanämie, erworbene und angeborenen Thrombozytopenie, Leukozytenfunktionssörungen, Störungen der Blutgerinnung, ex vivo Vermehrung embryonaler und adulter Stammzellen, ex vivo Differenzierung embryonaler und adulter Stammzellen, Knochenmarks, Graft-versus-host-Reaktion; Krebs, wie zum Beispiel Glaukom, Mammakarzinom, Kolontumor, gastrointestinale Tumore, Hodgkin-Lymphom, Non- Hodgkin-Lymphom, Kaposisarkom, Lebertumor, Pankreastumor, Hauttumor, Knochenmarkstumor, Leukämien wie z.B. akute lymphatische Leukämie, akute myeloische Leukämie, chronische myeloische Leukämie, chronische lymphatische Leukämie, Prostatatumore, Lungenkrebs, Nierentumore; Asthma, progrediente, nicht vollständig reversible Obstruktion der Atemwege, Lungenentzündug, Lungenfehlfunktion; Entzündungserkrankungen wie z.B. Autoimmunerkrankungen wie Multiple Sklerose, rheumatoide Arthritis, Infektionen durch gram-negative und gram-positive Bakterien, virale Infektionen, Pilzinfektionen wie z. B. durch Candida albicans, HIV- und HIV-assoziierte Infektionen, Hepatitis der Typen A, B und C, parasitäre Infektionen; Haarausfall, verminderte Beweglichkeit von Spermien, Wundheilung; Osteoporose, Knochenmarkserkrankungen, Knochen-und Gelenkserkrankungen; Herzkreislauferkrankungen wie z.B. Herzfehler, Herzinsuffizienz, Herzfibrose, Herzrhythmusstörungen, Myokardinfarkt, Medikamenten- oder Substanzinduzierte Kardiotoxizität, Atherosklerose, Bluthochdruck.

Die erfindungsgemäßen Verbindungen können darüber hinaus auch zur effizienten *ex vivo* Vermehrung von adulten hämatopoetischen Stammzellen aus dem Knochenmark, aus peripherem Blut oder Nabelschnurblut eingesetzt werden.

Diese so expandierten Zellen können dann zur Verkürzung der durch myeloablative Therapien induzierten Zytopenien oder im Rahmen von therapeutischen Transplantationsverfahren oder bei hämatologischen Systemerkrankungen, wie z.B. Leukämien, oder mit nach der Expansion gentechnisch veränderter Zellen für Gentherapien verwendet werden.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Weiterer Gegenstand der vorliegenden Erfindung sind Arzneimittel, enthaltend eine erfindungsgemäße Verbindung und einen oder mehrere weitere Wirkstoffe, insbesondere zur Behandlung und/oder Prophylaxe der zuvor genannten Erkrankungen. Als geeignete Kombinationswirkstoffe seien beispielhaft und vorzugsweise genannt:
Eine Kombination der erfindungsgemäßen Verbindungen mit in der Klinik verwendeten chemotherapeutischen Agenzien können bei unterschiedlichen Tumorerkrankungen zu einem signifikant verbesserten Behandlungserfolg führen. Bei den chemotherapeutischen Agenzien handelt es sich um Substanzen, welche entweder die Teilungsrate von Tumorzellen inhibieren und/oder die Neovaskularisierung von soliden Tumoren unterbindet. Dazu gehören u.a. Substanzen aus der Gruppe der Taxane wie z.B. Paclitaxel, oder Docetaxel, Substanzen, welche die Mitose von Tumorzellen inhibieren wie z.B. Vinblastin, Vincristin, Vindesin oder Vinorelbin. Substanzen aus der Klasse der Platinum-Derivate wie z.B. Cisplatin, Carboplatin, Oxaliplatin, Nedaplatin oder Lobaplatin. Weiterhin gehören zu den chemotherapeutischen Agentien Substanzen aus der Klasse der alkylierenden Agentien, wie z.B. Cyclophosphamid, Ifosfamid, Melphalan, Chlorambucil, Pipobroman, Triethylen-Melamin, Busulfan, Carmustin, Lomustin, Streptozin, Dacarbazin oder Temozolomid. Zu den chemotherapeutischen Agenzien werden auch Anti-Metabolite wie z.B. Folsäure-Antagonisten, Pyrimidin-Analoga, Purin-Analoga oder Adenosin-Desaminase-Inhibitoren gezählt. In diese Substanzklasse gehören u.a. Methotrexat, 5-Fluorouracil, Floxuridin, Cytarabin, Pentostatin und Gemcitabin. Als chemotherapeutische Agenzien werden auch Naturstoffe oder deren Derivate eingesetzt, zu denen u.a. Enzyme, Anti-Tumor-Antikörper und Lymphokine gezählt werden. Dazu gehören z.B. Bleomycin, Dactinomycin, Daunorubicin, Doxorubicin, Epirubicin, Idarubicin, ara-V, Paclitaxel, Mithramycin, Mitomycin-C, L-Asparaginase, Interferone (z.B. IFNalhpa) und Etoposid. Andere chemotherapeutische Agentien mit anti-proliferativer und/oder antiangiogenetischer Wirkung sind Sorafenib, Sunitinib, Bortezomib, DAST Inhibitor (BAY 73-4506) u.a.
weiterhin kännen die erfindungsgemässen Verbindungen in einem Verfahren zur *ex vivo* vermehrung von adulten hämatopoetischen Stammzellen aus dem Knochenmark, aus peripherem Blut oder Nabelschnurblut, eingesetzt werden, das dadurch gekennzeichnet ist, dass eine wirksame Menge der erfindungsgemäßen Verbindung zugegeben wird.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende schnell und/oder modifiziert die erfindungsgemäßen Verbindungen abgebende Applikationsformen, die die erfindungsgemäßen Verbindungen in kristalliner und/ oder amorphisierter und/oder gelöster Form enthalten, wie z.B. Tabletten (nicht überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Bevorzugt ist die orale Applikation.

Für die sonstigen Applikationswege eignen sich z.B. Inhalationsarzneiformen (u.a. Pulverinhalatoren, Nebulizer), Nasentropfen, -lösungen, -sprays; lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (wie beispielsweise Pflaster), Milch, Pasten, Schäume, Streupuder, Implantate oder Stents.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Laktose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und / oder Geruchskorrigentien.

Weiterer Gegenstand der vorliegenden Erfindung sind Arzneimittel, die mindestens eine erfindungsgemäße Verbindung, vorzugsweise zusammen mit einem oder mehreren inerten nichttoxischen, pharmazeutisch geeigneten Hilfsstoff enthalten, sowie deren Verwendung zu den zuvor genannten Zwecken.

Im Allgemeinen hat es sich als vorteilhaft erwiesen, bei parenteraler Applikation Mengen von etwa 5 bis 500 mg je 24 Stunden zur Erzielung wirksamer Ergebnisse zu verabreichen. Bei oraler Applikation beträgt die Menge etwa 5 bis 500 mg je 24 Stunden.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen. Die Angabe "w/v" bedeutet "weight/volume" (Gewicht/Volumen). So bedeutet beispielsweise "10% w/v": 100 ml Lösung oder Suspension enthalten 10 g Substanz.

### A) Beispiele

### Abkürzungen:

- abs.: absolut
- Boc: *tert*-Butoxycarbonyl
- CDCl₃: Deuterochloroform
- CO₂: Kohlendioxid
- d: Tag
- DIEA: *N,N*-Diisopropylethylamin
- DMAP: 4-*N,N-*Dimethylaminopyridin
- DMF: Dimethylformamid
- DMSO: Dimethylsulfoxid
- d. Th.: der Theorie
- EDC: *N'*-(3-Dimethylaminopropyl)-N ethylcarbodiimid x HCl
- eq.: Äquivalent
- ESI: Elektrospray-Ionisation (bei MS)
- ges.: gesättigt
- h: Stunde
- HOBt: 1-Hydroxy- 1H-benzotriazo x H₂O
- HPLC: Hochdruck-, Hochleistungsflüssigchromatographie
- konz.: konzentriert
- LC-MS: Flüssigchromatographie-gekoppelte Massenspektrometrie
- min.: Minuten
- MS: Massenspektrometrie
- MW: Molekulargewicht [g/mol]
- NMP: N-Methylpyrrolidon
- NMR: Kernresonanzspektroskopie
- PyBOP: 1-Benzotriazolyloxy-tripyrrolidinophosphoniumhexafluorophosphat
- R_{f}: Retentionsindex (bei DC)
- RP-HPLC: Reverse Phase HPLC
- RT: Raumtemperatur
- Rₜ: Retentionszeit (bei HPLC)
- TBTU: (Benzotriazol-1-yloxy)bisdimethylaminomethyliumfluoroborat
- TFA: Trifluoressigsäure
- THF: Tetrahydrofuran

### LC-MS Methoden:

Methode 1: Instrument: Micromass Quattro LCZ mit HPLC Agilent Serie 1100; Säule: Phenomenex Synergi 2.5 µ MAX-RP 100A Mercury 20 mm x 4 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90%A → 0.1 min 90%A → 3.0 min 5%A → 4.0 min 5%A → 4.1 min 90%A; Fluss: 2 ml/min; Ofen: 50°C; UV-Detektion: 208- 400 nm.

Methode 2: Gerätetyp MS: Micromass ZQ; Gerätetyp HPLC: Waters Alliance 2795; Säule: Merck Chromolith SpeedROD RP-18e 100 mm x 4.6 mm; Eluent A: Wasser + 500 µl 50%ige Ameisensäure / 1; Eluent B: Acetonitril + 500 µl 50%ige Ameisensäure / 1; Gradient: 0.0 min 10%B→ 7.0 min 95%B→ 9.0 min 95%B; Ofen: 35 °C; Fluss: 0.0 min 1.0 ml/min→ 7.0 min 2.0 ml/min→ 9.0 min 2.0 ml/min; UV-Detektion: 210 nm

Methode 3: Gerätetyp MS: Micromass ZQ; Gerätetyp HPLC: HP 1100 Series; UV DAD; Säule: Phenomenex Gemini 3µ 30 mm x 3.00 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min. 2 ml/min; Ofen: 50°C; UV-Detektion: 210 nm.

Methode 4: Instrument: Micromass Platform LCZ mit HPLC Agilent Serie 1100; Säule: Thermo Hypersil GOLD 3µ 20 mm x 4 mm; Eluent A: 1 1 Wasser + 0.5 ml 50%ige Ameisensäure , Eluent B: 11 Acetonitril + 0.5 ml 50%ige Ameisensäure; Gradient: 0.0 min 100%A → 0.2 min 100%A → 2.9 min 30%A → 3.1 min 10%A →5.5 min 10%A; Ofen: 50°C; Fluss: 0.8 ml/min; UV-Detektion: 210 nm.

Methode 5: Gerätetyp MS: Waters ZQ; Gerätetyp HPLC: Waters Alliance 2795; Säule: Phenomenex Onyx Monolithic C18, 100 mm x 3 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90%A → 2 min 65%A → 4.5 min 5%A → 6 min 5%A; Fluss: 2 ml/min; Ofen: 40°C; UV-Detektion: 210 nm.

Methode 6: Gerätetyp MS: Micromass ZQ; Gerätetyp HPLC: Waters Alliance 2795; Säule: Phenomenex Synergi 2.5 µ MAX-RP 100A Mercury 20 mm x 4 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90%A → 0.1 min 90%A → 3.0 min 5%A → 4.0 min 5%A → 4.01 min 90%A; Fluss: 2 ml/min;; Ofen: 50°C; UV-Detektion: 210 nm.

Methode 7: Instrument: Micromass Quattro LCZ mit HPLC Agilent Serie 1100; Säule: Phenomenex Onyx Monolithic C18, 100 mm x 3 mm. Eluent A: 1 1 Wasser + 0.5 ml 50%ige Ameisensäure, Eluent B: 1 1 Acetonitril + 0.5 ml 50%ige Ameisensäure; Gradient: 0.0 min 90%A → 2 min 65%A → 4.5 min 5%A → 6 min 5%A; Fluss: 2 ml/min; Ofen: 40°C; UV-Detektion: 208-400 nm.

Methode 8: Instrument: Micromass QuattroPremier mit Waters UPLC Acquity; Säule: Thermo Hypersil GOLD 1.9 µ 50 mm x 1 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90%A → 0.1 min 90%A → 1.5 min 10%A → 2.2 min 10%A; Ofen: 50°C; Fluss: 0.33 ml/min; UV-Detektion: 210 nm.

Methode 9: Instrument: Micromass Quattro Micro MS mit HPLC Agilent Serie 1100; Säule: Thermo Hypersil GOLD 3µ 20 mm x 4 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 100%A → 3.0 min 10% A → 4.0 min 10%A → 4.01 min 100%A (Fluss: 2.5 ml/min) → 5.00 min 100%A; Ofen: 50°C; Fluss: 2 ml/min; UV-Detektion: 210 nm.

Methode 10: Gerätetyp MS: Waters ZQ; Gerätetyp HPLC: Agilent 1100 Series; UV DAD; Säule: Thermo Hypersil GOLD 3µ 20 mm x 4 mm; Eluent A: 11 Wasser + 0.5 ml 50%ige Ameisensäure, Eluent B: 1 1 Acetonitril + 0.5 ml 50%ige Ameisensäure; Gradient: 0.0 min 100%A → 3.0 min 10%A → 4.0 min 10%A → 4.1 min 100% (Fluss: 2.5 ml/min); Ofen: 55°C; Fluss: 2 ml/min; UV-Detektion: 210 nm.

### Ausgangsverbind ungen

### Beispiel 1A

3-Amino-3-(2,4-dichlorphenyl)acrylnitril

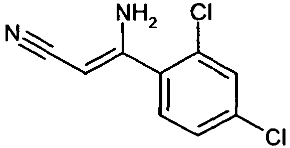

In einem Dreihalskolben mit mechanischem Rührer werden unter Argon 90 g (889.46 mmol) Diisopropylamin in 1660 ml THF bei -70°C vorgelegt. 124.66 ml N-Butyllithium-Lösung (2.5 M in Hexan, 758.66 mmol) werden so schnell zugetropft, dass die Temperatur nicht über -60°C steigt. Man lässt 10 min nachrühren und tropft dann eine Lösung von 32.22 g (784.82 mmol) Acetonitril in 340 ml THF langsam zu und lässt die Suspension für 30 min rühren. Dann wird eine Lösung von 90 g (523.21 mmol) 2,4-Dichlorbenzonitril in 340 ml THF zugetropft und 20 min bei -70°C nachgerührt. Man lässt langsam auf RT kommen und rührt für weitere 16 h bei RT. Es werden 600 ml Wasser zugegeben, der größte Teil des THF abdestilliert und mit Wasser und Dichlormethan versetzt. Die organische Phase wird mit ges. wässriger Natriumchloridlösung gewaschen. Nach Entfernen des Lösungsmittels erhält man dunkle Kristalle, die durch Verrühren mit Diethylether gereinigt werden. Es werden 76.5 g (69% d. Th.) des Produktes als Feststoff erhalten.

LCMS (Methode 5): Rₜ = 3.07 min. (m/z = 213 (M+H)⁺)

¹H-NMR (400MHz, DMSC)-d₆): δ = 7.70 (d, 1H), 7.47 (dd, 1H), 7.40 (d, 1H), 7.02 (s, breit, 2H), 3.79 (s, 1H).

### Beispiel 2A

4-Amino-6-(2,4-dichlorphenyl)pyrimidin-2(1H)-thion

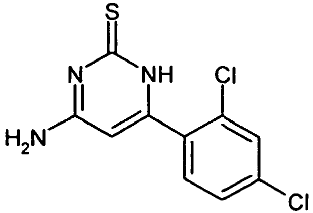

In 353 ml Ethanol werden 16.51 g (718.08 mmol) Natrium aufgelöst und in die so hergestellte Natriumethanolat-Lösung 40.996 g (538.56 mmol) Thioharnstoff gegeben und danach 76.5 g (359.04 mmol) 3-Amino-3-(2,4-dichlorphenyl)acrylnitril. Man erhitzt für 18 h unter Rückfluß und gibt nach dem Abkühlen 500 ml Wasser zu. Man neutralisiert mit 1M Salzsäure und saugt den ausfallenden Niederschlag ab. Dieser wird mit Wasser gewaschen, mit THF aufgeschlämmt und erneut abgesaugt. Es wird mit 300 ml Petrolether gewaschen und anschließend im Hochvakuum getrocknet. Es werden 67.5 g (55% d. Th.) des Produktes als Feststoff erhalten.

LCMS (Methode 3): Rₜ = 1.54 min. (m/z = 272 (M+H)⁺)

¹H-NMR (400MHz, DMSO-d₆): δ = 12.30 (s, 1H), 7.78 (d, 1H), 7.75 (s, breit, 1H), 7.62 (s, breit, 1H), 7.53 (m, 2H), 5.89 (s, 1H).

### Beispiel 3A

6-(2,4-Dichlorphenyl)-2-(ethylthio)pyrimidin-4-amin

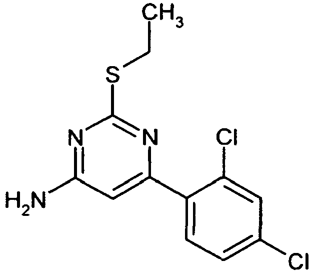

In 560 ml trockenem DMSO werden 67.5 g (248 mmol) 4-Amino-6-(2,4-dichlorphenyl)pyrimidin-2(1H)-thion und 77.37 g (496.04 mmol) Iodethan gelöst und langsam bei RT 270 ml eine gesättigte wässrige Natriumhydrogencarbonat-Lösung zugegeben. Man rührt bei RT für 5 h und gibt dann 440 ml Wasser zu. Der Niederschlag wird abgesaugt, mit Wasser gewaschen und trocken gesaugt. Man verrührt zweimal mit je 100 ml Isopropanol und saugt erneut ab. Danach wäscht man noch einmal mit Petrolether und trocknet den Rückstand im Hochvakuum. Es werden 50.0 g (67% d. Th.) des Produktes als Feststoff erhalten.

LCMS (Methode 5): Rₜ = 3.49 min. (m/z = 300 (M+H)⁺)

¹H-NMR (400MHz, DMSO-d₆): δ = 7.73 (d, 1 H), 7.60 (d, 1H), 7.53 (dd, 1H), 7.11 (s, 2H), 6.39 (s, 1H), 3.01 (q, 2H), 1.29 (t, 3H).

### Beispiel 4A

7-(2,4-Dichlorphenyl)-5-(ethylthio)imidazo[1,2-c]pyrimidin

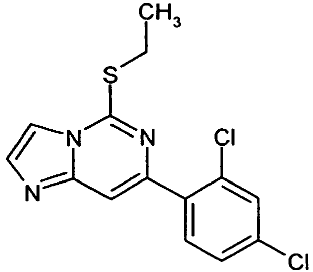

In einem Gemisch aus 1164 ml Dioxan und 291 ml Wasser werden 48.5 g (161.55 mmol) 6-(2,4-Dichlorphenyl)-2-(ethylthio)pyrimidin-4-amin (Beispiel 3A) und 163.84 g (969.32 mmol) Bromacetaldehyddimethylacetal vorgelegt und für 16 h unter Rückfluß erhitzt. Man entfernt das Dioxan weitgehend unter Vakuum, gibt vorsichtig 1000 ml Essigsäureethylester und 1000 ml gesättigte wässrige Natriumhydrogencarbonat-Lösung zu. Man trennt die Phasen und extrahiert noch ein weiteres Mal mit Essigsäureethylester. Die vereinigten organischen Phasen werden mit gesättigter wässriger Natriumchlorid-Lösung gewaschen, über Natriumsulfat getrocknet und danach vom Lösungsmittel befreit. Der Rückstand wird durch Kieselgelchromatographie gereinigt. Man erhält 16.6 g (27% d. Th.) des Produktes, welches so weiter umgesetzt wird.

LCMS (Methode 5): Rₜ = 3.84 min. (m/z = 324 (M+H)⁺)

¹H-NMR (400MHz, DMSO-d₆): δ = 7.92 (s, 1H), 7.79 (s, 2H), 7.78 (d, 1H), 7.71 (s, 1H), 7.59 (dd, 1H), 3.41 (q, 2H), 1.43 (t, 3H).

### Beispiel 5A

7-(2,4-Dichlorphenyl)imidazo[1,2-c]pyrimidin-5-ol 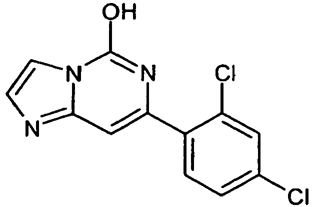 16.6 g (51.19 mmol) 7-(2,4-Dichlorphenyl)-5-(ethylthio)imidazo[1,2-c]pyrimidin werden in 330 ml Methanol gelöst und 77 ml einer 2 molaren wässrigen Kaliumhydroxidlösung zugegeben. Man rührt bei Rückflusstemperatur für 2 h. Nach Abkühlen werden 300 ml Wasser zugegeben und dann unter Eiskühlung 140 ml 0.1 molare Salzsäure. Dann wird mit gesättigter wässriger Ammoniumchloridlösung neutralisiert, für 10 min gerührt und der Feststoff abgesaugt. Man wäscht gut mit Wasser und trocknet im Hochvakuum. Es werden 13.6 g (92% d. Th.) des Produktes als Feststoff erhalten.

MS (ES+): m/z = 280 (M)⁺.

¹H-NMR (400MHz, DMSO-d₆): δ = 11.90 (s, 1H), 7.83 (dd, 2H), 7.56-7.64 (m, 2H), 7.45 (d, 1H), 6.69 (s, 1H).

### Beispiel 6A

5-Chlor-7-(2,4-dichlorphenyl)imidazo[1,2-c]pyrimidin 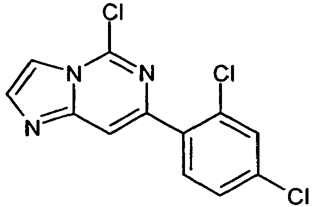 12.0 g (42.84 mmol) 7-(2,4-Dichlorphenyl)imidazo[1,2-c]pyrimidin-5-ol werden in 131.6 g (858.27 mmol) Phosphorylchlorid vorgelegt und für 5 h auf 120°C erhitzt. Nach 10 min entsteht eine klare Lösung. Man lässt abkühlen und gibt dann vorsichtig 2000 ml gesättigte wässrige Natriumhydrogencarbonat-Lösung zu. Zum Ende werden noch ca. 100 g festes Natriumhydrogencarbonat zugegeben und 10 min bei pH 7 gerührt. Man saugt den Niederschlag ab, wäscht gut mit Wasser nach, schlämmt mit wenig Isopropanol auf und saugt trocken. Es wird dann noch einmal mit Petrolether gewaschen und im Hochvakuum bei 50°C getrocknet. Es werden 11 g (81% d. Th.) des Produktes als Feststoff erhalten.

MS (CI+): m/z = 298 (M)⁺.

¹H-NMR (400MHz, DMSO-d₆): δ = 8.17 (s, 1H), 8.00 (s, 1H), 8.88 (d, 1H), 7.82 (d, 1H), 7.73 (d, 1H), 7.56 (dd, 1H).

### Beispiel 7A

tert-Butyl-(2-{[7-(2,4-dichlorphenyl)imidazo[1,2-c]pyrimidin-5-yl]amino}ethyl)carbamat 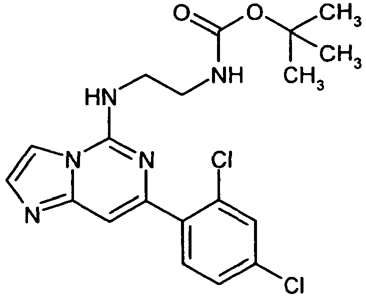 3.0 g (10.05 mmol) 5-Chlor-7-(2,4-dichlorphenyl)imidazo[1,2-c]pyrimidin, 2.41 g (15.07 mmol) N-Boc-ethylendiamin und 3.89 g (30.15 mmol) Diisopropylethylamin werden in 50 ml DMSO gelöst und für 16 h auf 120°C erhitzt. Es werden Essigsäureethylester und Wasser zugeben und die abgetrennte organische Phase noch zweimal mit Wasser gewaschen. Die organische Phase wird mit Magnesiumsulfat getrocknet. Nach Entfernen des Lösungsmittels erhält man 4.49 g (99% d. Th.) Produkt.

LCMS (Methode 5): Rₜ = 2.80 min. (m/z = 422 (M+H)⁺)

¹H-NMR (400MHz, DMSO-d₆): δ = 8.04 (s, 1H), 7.99 (t, 1H), 7.75 (d, 1H), 7.71 (d, 1H), 7.57 (d, 1H), 7.52 (dd, 1H), 7.11 (s, 1H), 6.95 (t, 1H), 3.53 (m, 2H), 3.2-3.32 (m, 2H), 1.33 (s, 9H).

### Beispiel 8A

N-[7-(2,4-Dichlorphenyl)imidazo[1,2-c]pyrimidin-5-yl)ethan-1,2-diamin-trifluoracetat 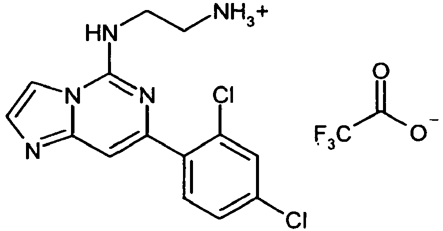 4.42 g (10.47 mmol) tert-Butyl-(2-{[7-(2,4-dichlorphenyl)imidazo[1,2-c]pyrimidin-5-yl]amino}-ethyl)carbamat werden in 160 ml Dichlormethan gelöst und 16 ml (209 mmol) Trifluoressigsäure zugegeben. Man rührt für 2 h bei RT und entfernt danach von allen flüchtigen Bestandteilen im Vakuum. Man erhält 4.56 g (99% d. Th.) Produkt, das ohne weitere Aufreinigung eingesetzt wird.

LCMS (Methode 4): Rₜ = 2.23 min. (m/z = 322 (M+H)⁺)

¹H-NMR (400MHz, DMSO-d₆): δ = 8.90 (s, 1H), 8.20 (s, 1H), 8.04 (s, 1H), 7.90 (s, breit, 2H), 7.82 (d, 1H), 7.76 (d, 1H), 7.60 (dd, 1H), 7.42 (s, 1H), 3.80 (dd, 2H), 3.18 (dd, 2H).

### Beispiel 9A

7-Chlor-5-(methylthio)imidazo[1,2-c]pyrimidin 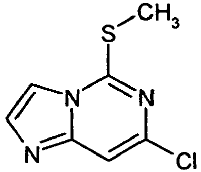 75 g (427 mmol) 4-Amino-6-chlor-2-(methylthio)pyrimidin werden in 3000 ml Dioxan und 750 ml Wasser gelöst. Es werden 101.05 g (597.81 mmol) Bromacetaldehyddimethylacetal zugegeben und 24 h unter Rückfluß erhitzt. Nach beendeter Reaktion wird das Dioxan im Vakuum entfernt und die wäßrige Suspension in THF aufgeschlämmt, abgesaugt und mit etwas THF nachgewaschen. Nach Trocknen des Feststoffes bei 40°C im Hochvakuum erhält man 56.5 g (66% d. Th.) des Produktes.

MS (ES+): m/z = 200 (M+H)⁺.

¹H-NMR (400MHz, DMSO)-d₆): δ = 8.12 (s, 1H), 7.97 (s, 1H), 7.77 (s, 1H), 2.80 (s, 3H).

### Beispiel 10A

7-Chlorimidazo[1,2-c]pyrimidin-5-ol

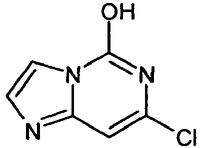

Eine Lösung von 73.25 g (366.87 mmol) 7-Chlor-5-(methylthio)imidazo[1,2-c]pyrimidin werden in 732 ml Methanol gelöst und 1275 ml einer 2 molaren wässrigen Kaliumhydroxidlösung zugegeben. Man erhitzt für 3 h unter Rückfluß. Nach beendeter Reaktion wird das Methanol im Vakuum entfernt, mit halbkonzentrierter Salzsäure neutralisiert und die ausgefallenen Kristalle abgesaugt. Diese werden mit Wasser und Methanol gewaschen und danach im Hochvakuum getrocknet. Man erhält 40.0 g (64% d. Th.) des Produktes.

LCMS (Methode 4): Rₜ = 1.32 min. (m/z = 170 (M+H)⁺).

¹H-NMR (400MHz, DMSO-d₆): δ = 12.98 (s, 1H), 7.71 (s, 1H), 7.54 (s, 1H), 6.63 (s, 1H).

### Beispiel 11A

5,7-Dichlorimidazo[1,2-c]pyrimidin

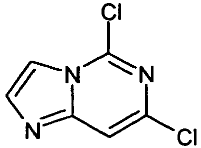

Unter Argon werden 38.3 g (225.86 mmol) 7-Chlorimidazo[1,2-c]pyrimidin-5-ol in 450.2 g (2936 mmol) Phosphorylchlorid suspendiert und 4 h auf 120°C erhitzt. Nach dieser Zeit wird das überschüssige Phosphorylchlorid im Vakuum entfernt und der Rückstand mit Toluol zusammen destilliert. Der Rückstand wird mit Wasser suspendiert, mit gesättigter wässriger Natriumhydrogencarbonat-Lösung auf pH7 gebracht und der ausfallende Feststoff abgesaugt. Dieser wird mit Wasser gewaschen und für 18 h über Phosphorpentoxid getrocknet. Man erhält 33.5 g (67% d. Th.) des Produktes als Feststoff:

LCMS (Methode 6): Rₜ = 1.01 min. (m/z = 189 (M+H)⁺).

¹H-NMR (400MHz, DMSO-d₆): δ = 8.11 (s, 1H), 7.94 (s, 1H), 7.81 (s, 1H).

### Beispiel 12A

tert-Butyl-{2-[(5-cyanopyridin-2-yl)amino]ethyl}carbamat 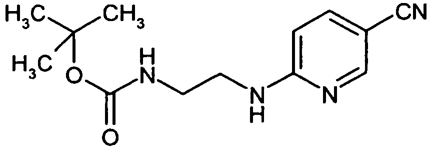 3.0 g (18.73 mmol) N-Boc-ethylendiamin, 5.19 g (37.45 mmol) 6-Chlornicotinnitril und 3.75 g (37.45 mmol) Kaliumhydrogencarbonat werden in 360 ml DMF suspendiert und auf 90°C für 16 h erhitzt. Der Ansatz wird mit Wasser versetzt und dreimal mit Essigsäureethylester extrahiert. Die vereinigten organischen Phasen werden mit gesättigter wässriger Natriumchlorid-Lösung gewaschen. Nach Trocknen über Magnesiumsulfat wird das Lösungsmittel entfernt. Nach Trocknen im Hochvakuum erhält man 3.58 g (68% d. Th.) des Produktes.

LCMS (Methode 7): Rₜ = 2.63 min. (m/z = 263 (M+H)⁺).

¹H-NMR (400MHz, DMSO-d₆): δ = 8.37 (d, 1H), 7.66 (d, 1H), 7.60 (s, br, 1H), 6.88 (t, 1H), 6.53 (d, 1H), 3.31 (dd, 2H), 3.08 (dd, 2H), 1.37 (s, 9H).

### Beispiel 13A

6-[(2-Aminoethyl)amino]nicotinonitril-trifluoracetat 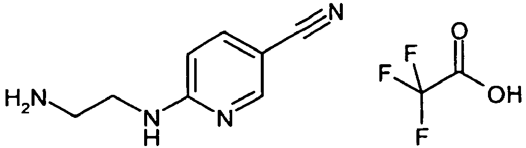 4.0 g (15.25 mmol) tert-Butyl-{2-[(5-cyanopyridin-2-yl)amino]ethyl}carbamat (Beispiel 12A) werden in 150 ml Dichlormethan gelöst und 17.39 g (152.5 mmol) Trifluoressigsäure langsam zugespritzt. Man rührt für 16 h bei RT und entfernt dann alle flüchtigen Bestandteile im Vakuum. Es werden 4.3 g (91% d. Th.) des Produktes als Harz gewonnen.

LCMS (Methode 4): Rₜ = 0.91 min. (m/z = 161 (M-H)⁺).

¹H-NMR (400MHz, DMSO-d₆): δ = 11.53 (s, br, 3H), 8.44 (d, 1H), 7.84 (s, br, 1H), 7.75 (dd, 1H), 6.61 (d, 1H), 3.55 (m, 2H), 3.0 (dd, 2H).

### Beispiel 14A

tert-Butyl-{2-[(6-amino-5-nitropyridin-2-yl)amino]ethyl}carbamat

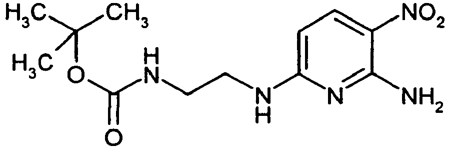

Analog der Herstellung des Cyanopyridins (Beispiel 12A) werden aus 3.0 g (18.73 mmol) N-Boc-ethylendiamin und 6.5 g (37.4 mmol) 6-Chlor-3-nitropyridin-2-amin nach Aufreinigung über Kieselgelchromatographie 5.5 g (86% d. Th.) des Produktes erhalten.

LCMS (Methode 4): Rₜ = 3.05 min. (m/z = 296 (M-H)⁺).

¹H-NMR (400MHz, DMSO-d₆): δ = 8.13 (s, br, 1H), 7.94 (s, 1H), 7.92 (d, 1H), 7.71 (s, br, 1H), 6.87 (s, br, 1H), 5.94 (d, 1H), 3.37 (dd, 2H), 3.11 (dd, 2H), 1.37 (s, 9H).

### Beispiel 15A

N⁶-(2-Aminoethyl)-3-nitropyridin-2,6-diamin-trifluoracetat

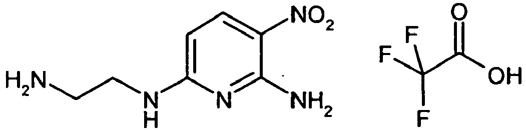

Analog der Herstellung des Cyanopyridins (Beispiel 13A) werden aus 5.5 g (16.1 mmol) des Aminopyridins (Beispiel 14A) und 18.7 g (160.9 mmol) Trifluoressigsäure nach Entfernen aller flüchtigen Bestandteile 4.98 g (99% d. Th.) des Produktes erhalten.

MS (CI): m/z = 198 (M+H)⁺.

¹H-NMR (400MHz, DMSO-d₆): δ = 8.15 (s, br, 2H), 8.03 (s, br, 1H), 8.00 (d, 1H), 7.79 (s, br, 2H), 5.97 (d, 1H), 3.51 (dd, 2H), 3.06 (dd, 2H).

### Beispiel 16A

6-({2-[(7-Chlorimidazo[1,2-c]pyrimidin-5-yl)amino]ethyl}amino)nicotinnitril

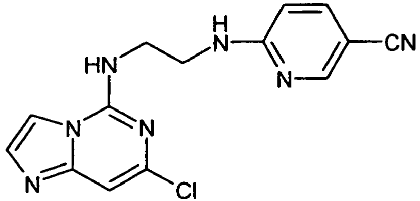

In 25 ml 2-Propanol werden 707 mg (3.20 mmol) 5,7-Dichlorimidazo[1,2-c]pyrimidin (Beispiel 11A) suspendiert und 1.35 g (3.52 mmol) 6-[(2-Aminoethyl)amino]nicotinonitril-trifluoracetat (Beispiel 13A) und 1.03 g (7.99 mmol) DIPEA zugegeben. Man erhitzt für 16 h bei 80°C. Nach dieser Zeit wird Wasser zugeben und der ausfallende Niederschlag abgesaugt. Man wäscht mit wenig 2-Propanol/Wasser nach und trocknet den erhaltenen Feststoff im Hochvakuum. Man erhält 523 mg (50% d. Th.) des Produktes.

LCMS (Methode 3): Rₜ = 1.31 min. (m/z = 314 (M+H)⁺).

¹H-NMR (400MHz, DMSO-d₆): δ = 8.37 (d, 1H), 8.33 (t, 1H), 7.93 (s, 1H), 7.80 (s, 1H), 7.68 (d, 1H), 7.50 (d, 1H), 6.90 (s, 1H), 6.56 (s, br, 1H), 3.62 (m, 2H), 3.31 (m, 2H).

### Beispiel 17A

N⁶-{2-[(7-Chlorimidazo[1,2-c]pyrimidin-5-yl)amino]ethyl}-3-nitropyridin-2,6-diamin

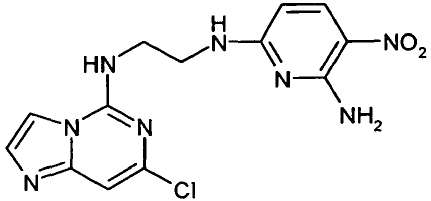

In 20 ml DMSO werden 800 mg (4.13 mmol) 5,7-Dichlorimidazo[1,2-c]pyrimidin (Beispiel 11A) suspendiert und 1.62 g (4.54 mmol) N⁶-(2-Aminoethyl)-3-nitropyridin-2,6-diamin-trifluoracetat (Beispiel 15A) und 1.6 g (12.38 mmol) DIPEA zugegeben. Man erhitzt für 16 h bei 120°C. Nach dieser Zeit wird Wasser zugeben und der ausfallende Niederschlag abgesaugt. Man wäscht mit wenig 2-Propanol/Wasser nach und trocknet den erhaltenen Feststoff im Hochvakuum. Man erhält 1.34 g (92% d. Th.) des Produktes als Feststoff.

LCMS (Methode 7): Rₜ = 1.44 min. (m/z = 349 (M+H)⁺).

¹H-NMR (400MHz, DMSO-d₆): δ = 8.29 (t, 1H), 8.15 (s, br, 1H), 8.09 (t, 1H), 7.95 (s, 1H), 7.93 (s, 1H), 7.69 (s, br, 1H), 7.50 (d, 1H), 6.92 (s, 1H), 5.92 (d, 1H), 3.67 (m, 4H).

### Beispiel 18A

4-Amino-2-(methylsulfonyl)-1,3-thiazol-5-carbonitril

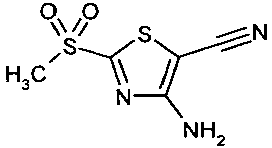

In 200 ml Dichlormethan werden 2.7 g (15.77 mmol) 4-Amino-2-(methylthio)-1,3-thiazol-5-carbonitril gelöst und 11.97 g (34.7 mmol) 3-Chlorperbenzoesäure zugegeben. Man lässt für 30 min bei RT rühren, gibt dann 6 ml DMSO zu und dann gesättigte wässrige Natriumhydrogencarbonat-Lösung und extrahiert dreimal mit Dichlormethan. Nach Trocknen der organischen Phase über Magnesiumsulfat erhält man nach Entfernen des Lösungsmittels 2.22 g (46% d. Th.) des Produktes als Öl, das ohne weitere Aufreinigung eingesetzt wird.

LCMS (Methode 3): Rₜ = 1.19 min. (m/z = 204 (M+H)⁺).

### Beispiel 19A

tert-Butyl-{2-[(4-amino-5-cyano-1,3-thiazol-2-yl)amino]ethyl}carbamat

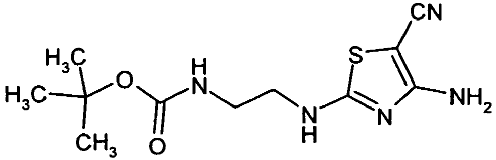

In 24 ml DMSO werden 2.2 g (7.22 mmol) 4-Amino-2-(methylsulfonyl)-1,3-thiazol-5-carbonitril (Beispiel 18A) gelöst und 1.74 g (10.84 mmol) N-Boc-ethylendiamin und 933 mg (7.22 mmol) DIPEA zugegeben. Man lässt für 16 h bei 120°C rühren und gibt nach beendeter Reaktion Wasser und Essigsäureethylester zu. Man extrahiert die wässrige Phase dreimal mit Essigsäureethylester. Die vereinigten organischen Phasen werden über Magnesiumsulfat getrocknet und mittels Kieselgelchromatographie gereinigt. Man erhält 633 mg (31% d. Th.) des Produktes.

LCMS (Methode 6): Rₜ = 1.45 min. (m/z = 284 (M+H)⁺).

¹H-NMR (400MHz, DMSO-d₆): δ = 8.35 (s, br, 1H), 6.90 (t, 1H), 6.68 (s, 2H), 3.22 (s, br, 2H), 3.07 (dd, 2H), 1.37 (s, 9H).

### Beispiel 20A

4-Amino-2-[(2-aminoethyl)amino]-1,3-thiazol-5-carbonitril-trifluoracetat

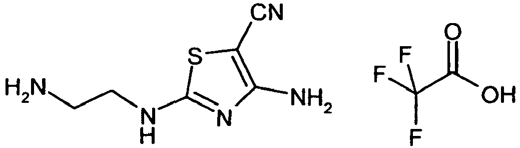

Analog der Herstellung des Cyanopyridins (Beispiel 13A) werden aus 130 mg (0.46 mmol) des Boc-geschützten Amin (Beispiel 19A) und 1.05 g (9.18 mmol) Trifluoessigsäure nach Entfernen aller flüchtigen Bestandteile 130 mg (96% d. Th.) des Produktes erhalten werden.

LCMS (Methode 4): Rₜ = 0.61 min. (m/z = 184 (M+H)⁺).

¹H-NMR (400MHz, DMSO-d₆): δ = 8.45 (t, 1H), 7.84 (s, br, 2H), 6.80 (s, br, 1H), 3.93 (s, 1H), 3.43 (dd, 2H), 3.01 (dd, 2H).

### Beispiel 21A

7-Chlor-5-(methylthio)-2-(trifluormethyl)imidazo[1,2-c]pyrimidin

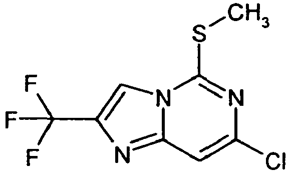

In 200 ml DMF werden 5 g (28.5 mmol) 6-Chlor-2-(methylthio)pyrimidin-4-amin und 6.26 g (42.7 mmol) 3-Chlor-1,1,1-trifluorpropanon gelöst und bei 120°C für 16 h erhitzt. Nach beendeter Reaktion wird Wasser zugegeben und mit Essigsäureethylester dreimal extrahiert. Die vereinigten organischen Phasen werden über Natriumsulfat getrocknet und das Produkt durch Kieselgelchromatographie gereinigt. Nach Trocknen im Hochvakuum erhält man 3.5 g (45% d. Th.) des Produktes als Feststoff.

LCMS (Methode 5): Rₜ = 3.40 min. (m/z = 268 (M+H)⁺)

¹H-NMR (400MHz, DMSO-d₆): δ = 8.65 (s, 1H), 7.75 (s, 1H), 2.78 (s, 3H).

### Beispiel 22A

7-Chlor-2-(trifluormethyl)imidazo[1,2-c]pyrimidin-5-ol

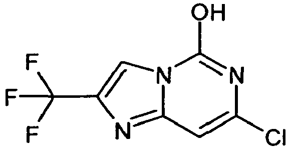

Analog der Herstellung des Hydroxypyrimidins (Beispiel 10A) werden aus 3.8 g (14.2 mmol) des 7-Chlor-5-(methylthio)-2-(trifluormethyl)imidazo[1,2-c]pyrimidin (Beispiel 21A) und 33 ml 2 molarer wässriger Kaliumhydroxid-Lösung in 100 ml Methanol 2.27 g (67% d. Th.) des Produktes erhalten.

LCMS (Methode 3): Rₜ = 1.73 min. (m/z = 238 (M+H)⁺)

¹H-NMR (400MHz, DMSO-d₆): δ = 8.40 (s, 1H), 6.94 (s, 1H).

### Beispiel 23A

5,7-Dichlor-2-(trifluormethyl)imidazo[1,2-c]pyrimidin

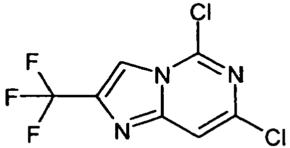

Analog der Herstellung von (Beispiel 11A) werden aus 2.76 g (8.95 mmol) des 7-Chlor-2-(trifluormethyl)imidazo[1,2-c]pyrimidin-5-ol (Beispiel 22A) und 38.7 g (252 mmol) Phosphorylchlorid 1.11 g (40% d. Th.) des Produktes erhalten.

LCMS (Methode 3): Rₜ = 2.29 min. (m/z = 256 (M+H)⁺)

¹H-NMR (400MHz, DMSO-d₆): δ = 8.84 (s, 1H), 8.09 (s, 1H).

### Beispiel 24A

6-[(2-{[7-Chlor-2-(trifluormethyl)imidazo[1,2-c]pyrimidin-5-yl]amino}ethyl)amino]nicotinnitril

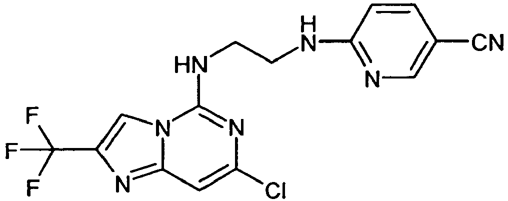

Analog der Herstellung von (Beispiel 16A) werden aus 140 mg (0.41 mmol) 5,7-Dichlor-2-(trifluormethyl)imidazo[1,2-c]pyrimidin (Beispiel 23A) und 173.1 mg (0.45 mmol) 6-[(2-Aminoethyl)amino]nicotinonitril-trifluoracetat (Beispiel 13A) 29 mg (19% d. Th.) des Produktes erhalten werden.

LCMS (Methode 5): Rₜ = 3.12 min. (m/z = 382 (M+H)⁺)

¹H-NMR (400MHz, DMSO-d₆): δ = 8.55 (t, 1H), 8.53 (s, 1H), 8.34 (d, 1H), 7.74 (s, 1H), 7.69 (d, 1H), 7.02 (s, 1H), 6.54 (s, 1 H), 3.64 (m, 4H).

### Beispiel 25A

N⁶-(2-{[7-Chlor-2-(trifluormethyl)imidazo[1,2-c]pyrimidin-5-yl]amino}ethyl)-3-nitropyridin-2,6-diamin

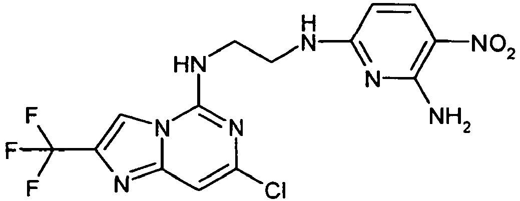

Analog der Herstellung von (Beispiel 16A) werden aus 140 mg (0.41 mmol) 5,7-Dichlor-2-(trifluormethyl)imidazo[1,2-c]pyrimidin (Beispiel 23A) und 161 mg (0.45 mmol) N⁶-(2-Aminoethyl)-3-nitropyridin-2,6-diamin-trifluoracetat (Beispiel 15A) 69 mg (38% d. Th.) des Produktes erhalten.

LCMS (Methode 7): Rₜ = 3.21 min. (m/z = 417 (M+H)⁺)

¹H-NMR (400MHz, DMSO-d₆): δ = 8.55 (s, 1H), 8.52 (t, 1H), 8.13 (s, br, 1H), 8.02 (t, 1H), 7.94 (d, 1H), 7.67 (s, br, 1 H), 7.03 (s, 1H), 5.90 (d, 1H), 3.67 (m, 4H).

### Beispiel 26A

7-Chlor-2-(chlormethyl)-5-(methylthio)imidazo[1,2-c]pyrimidin

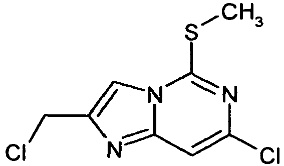

In 53 ml Eisessig werden 8 g (45.5 mmol) 6-Chlor-2-(methylthio)pyrimidin-4-amin und 5.78 g (45.55 mmol) 1,3-Dichloraceton gelöst und bei 105°C für 16 h erhitzt. Nach beendeter Reaktion wird Wasser zugegeben und der Niederschlag abgesaugt. Das Rohprodukt wird durch Kieselgelchromatographie weiter gereinigt. Nach Trocknen im Hochvakuum erhält man 5.53 g (49% d. Th.) Produkt.

LCMS (Methode 4): Rₜ = 3.33 min. (m/z = 248 (M+H)⁺)

¹H-NMR (400MHz, DMSO-d₆): δ = 7.99 (s, 1H), 7.58 (s, 1H), 4.85 (s, 2H), 2.76 (s, 3H).

### Beispiel 27A

7-Chlor-2-(chlormethyl)-5-(methylthio)imidazo[1,2-c]pyrimidin

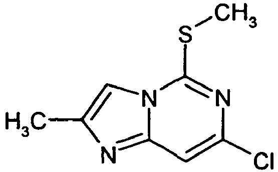

In 32 ml THF werden 640 mg (2.58 mmol) 7-Chlor-2-(chlormethyl)-5-(methylthio)imidazo[1,2-c]pyrimidin (Beispiel 26A) gelöst und 6 g Raney-Nickel (Suspension in Wasser) zugeben und für 16 h unter Rückfluß erhitzt. Nach beendeter Reaktion wird das Lösungsmittel entfernt und das Rohprodukt durch Kieselgelchromatographie gereinigt. Nach Trocknen im Hochvakuum erhält man 344.8 mg (63% d. Th.) Produkt.

LCMS (Methode 7): Rₜ = 1.99 min. (m/z = 214 (M+H)⁺)

¹H-NMR (400MHz, DMSO-d₆): δ = 7.66 (s, 1H), 7.48 (s, 1H), 2.75 (s, 3H), 2.35 (s, 3H).

### Beispiel 28A

7-Chlor-2-methylimidazo[1,2-c]pyrimidin-5-ol

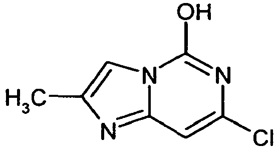

Analog der Herstellung des Hydroxypyrimidins (Beispiel 10A) werden aus 850 mg (3.98 mmol) des 7-Chlor-2-(chlormethyl)-S-(methylthio)imidazo[1,2-c]pyrimidin (Beispiel 27A) und 10 ml 2 molarer wässriger Kaliumhydroxid-Lösung in 33 ml Methanol 652 mg (89% d. Th.) des Produktes erhalten.

LCMS (Methode 4): Rₜ = 1.89 min. (m/z = 185 (M+H)⁺)

¹H-NMR (400MHz, DMSO-d₆): δ = 13.07 (s, br, 1H), 7.43 (s, 1H), 6.52 (s, 1H), 2.27 (s, 3H).

### Beispiel 29A

5,7-Dichlor-2-methylimidazo[1,2-c]pyrimidin

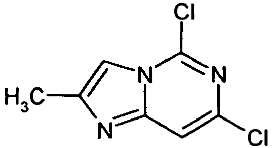

Analog der Herstellung von Beispiel 11A werden aus 716 mg (3.9 mmol) des 7-Chlor-2-methylimidazo[1,2-c]pyrimidin-5-ol (Beispiel 28A) und 10.0 g (65.5 mmol) Phosphorylchlorid 501.6 mg (64% d. Th.) des Produktes erhalten.

LCMS (Methode 6): Rₜ = 1.22 min. (m/z = 203 (M+H)⁺)

¹H-NMR (400MHz, DMSO-d₆): δ = 7.87 (s, 1H), 7.80 (s, 1H), 2.38 (s, 3H).

### Beispiel 30A

6-({2-[(7-Chlor-2-methylimidazo[1,2-c]pyrimidin-5-yl)amino]ethyl}amino)nicotinnitril

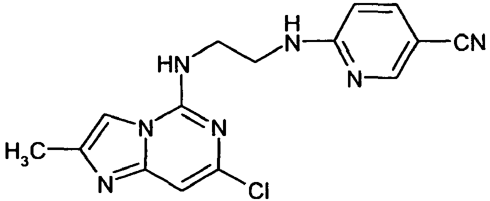

Analog der Herstellung von Beispiel 17A werden aus 150 mg (0.74 mmol) 5,7-Dichlor-2-methylimidazo[1,2-c]pyrimidin (Beispiel 29A) und 313.3 mg (0.82 mmol) 6-[(2-Aminoethyl)amino]nicotinonitril-trifluoracetat (Beispiel 13A) 129.9 mg (52% d. Th.) des Produktes erhalten werden.

LCMS (Methode 6): Rₜ = 1.01 min. (m/z = 328 (M+H)⁺)

¹H-NMR (400MHz, DMSO-d₆): δ = 8.36 (s, 1H), 8.15 (s, 1H), 7.78 (s, 1H), 7.68 (d, 1H), 7.63 (s, 1H), 6.78 (s, 1H), 6.56 (s, br, 1H), 3.60 (s, br, 4H), 2.27 (s, 3H).

### Beispiel 31A

N6-{2-[(7-Chlor-2-methylimidazo[1,2-c]pyrimidin-5-yl)amino]ethyl}-3-nitropyridin-2,6-diamin

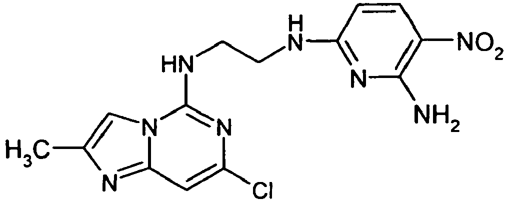

Analog der Herstellung von Beispiel 16A werden aus 200 mg (0.99 mmol) 5,7-Dichlor-2-methylimidazo[1,2-c]pyrimidin (Beispiel 29A) und 389.5 mg (1.09 mmol) N⁶-(2-Aminoethyl)-3-nitropyridin-2,6-diamin-trifluoracetat (Beispiel 15A) 320.9 mg (87% d. Th.) des Produktes erhalten.

LCMS (Methode 6): Rₜ = 0.96 min. (m/z = 363 (M+H)⁺)

¹H-NMR (400MHz, DMSO-d₆): δ = 8.16 (s, br, 1H), 8.10 (s, 1H), 8.07 (s, 1H), 7.93 (d, 1H), 7.68 (s, br, 1H), 7.65 (s, 1H), 6.79 (s, 1H), 5.92 (d, 1H), 3.65 (s, br, 4H), 2.27 (s, 3H).

### Beispiel 32A

7-Chlor-5-(methylthio)-2-(morpholin-4-ylmethyl)imidazo[1,2-c]pyrimidin

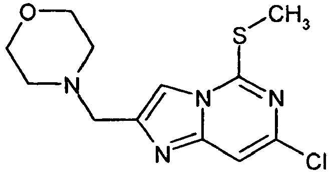

In 10 ml DMF werden 158 mg (1.81 mmol) Morpholin bei 0°C vorgelegt, 367 mg (3.63 mmol) Triethylamin und eine katalytische Menge Kaliumiodid zugegeben. Dann wird eine Lösung von 300 mg 7-Chlor-2-(chlormethyl)-5-(methylthio)imidazo[1,2-c]pyrimidin (Beispiel 26A) in DMF langsam zugetropft. Man läßt auf RT kommen und rührt für weitere 16 h bei dieser Temperatur. Nach Reinigung des Rohproduktes durch präparative HPLC werden nach Trocknen im Hochvakuum 221.8 mg (41% d. Th.) Produkt gewonnen.

LCMS (Methode 6): R₁ = 0.52 min. (m/z = 299 (M+H)⁺)

¹H-NMR (400MHz, DMSO-d₆): δ = 7.73 (s, 1H), 7.53 (s, 1H), 3.61 (s, 2H), 3.57 (dd, 4H), 2.76 (s, 3H), 2.45 (dd, 4H).

### Beispiel 33A

7-Chlor-2-(morpholin-4-ylmethyl)imidazo[1,2-c]pyrimidin-5-ol-hydrochlorid

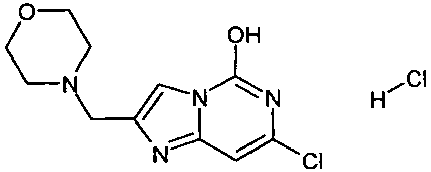

Analog der Herstellung des Hydroxypyrimidins Beispiel 10A werden aus 200 mg (0.67 mmol) des 7-Chlor-5-(methylthio)-2-(morpholin-4-ylmethyl)imidazo[1,2-c]pyrimidin (Beispiel 32A) und 1.7 ml 2 molarer wässriger Kaliumhydroxid-Lösung in 3 ml Methanol und abschließendes Sauerstellen mit Salzsäure 194 mg (95% d. Th.) des Produktes erhalten.

LCMS (Methode 4): Rₜ = 3.33 min. (m/z = 248 (M+H)⁺)

¹H-NMR (400MHz, DMSO-d₆): δ = 7.97 (s, 1H), 6.90 (s, 1H), 4.37 (s, 2H), 3.00-4.0 (m, 8H).

### Beispiel 34A

5,7-Dichlor-2-(morpholin-4-ylmethyl)imidazo[1,2-c]pyrimidin

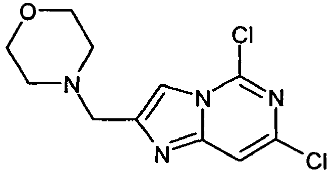

Analog der Herstellung von (Beispiel 11A) werden aus 180 mg (3.9 mmol) des 7-Chlor-2-(morpholin-4-ylmethyl)imidazo[1,2-c]pyrimidin-5-ol-hydrochlorid (Beispiel 33A) und 1.06 g (6.9 mmol) Phosphorylchlorid 120.5 mg (71% d. Th.) des Produktes erhalten.

LCMS (Methode 4): Rₜ = 2.02 min. (m/z = 287 (M+H)⁺)

¹H-NMR (400MHz, DMSO-d₆): δ = 7.95 (s, 1H), 7.86 (s, 1H), 3.67 (s, 2H), 3.59 (dd, 4H), 3.32 (s, br, 4H).

### Beispiel 35A

N⁶-(2-{[7-Chlor-2-(morpholin-4-ylmethyl)imidazo[1,2-c]pyrimidin-5-yl]amino}ethyl)-3-nitro-pyridin-2,6-diamin

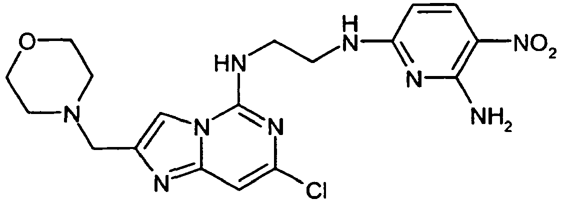

Analog der Herstellung von Beispiel 17A werden aus 60 mg (0.2 mmol) 5,7-Dichlor-2-(morpholin-4-ylmethyl)imidazo[1,2-c]pyrimidin (Beispiel 34A) und 79.8 mg (0.22 mmol) N⁶-(2-Aminoethyl)-3-nitropyridin-2,6-diamin-trifluoracetat (Beispiel 15A) 74.6 mg (82% d. Th.) des Produktes erhalten.

LCMS (Methode 6): Rₜ = 0.86 min. (m/z = 448 (M+H)⁺)

¹H-NMR (400MHz, DMSO-d₆): δ = 8.18 (s, 1H), 8.08 (s, 1H), 7.93 (d, 1H), 7.82 (s, 1H), 7.64 (s, br, 1 H), 6.83 (s, 1H), 5.92 (d, 1H), 3.65 (s, br, 4H), 3.58 (dd, 4H), 3.54 (s, 2H).

### Beispiel 36A

tert-Butyl-2-{[7-(2,4-dichlorphenyl)imidazo[1,2-c]pyrimidin-5-yl]amino}propyl)carbamat 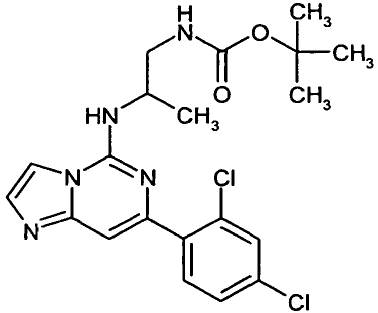 5-Chlor-7-(2,4-dichlorphenyl)imidazo[1,2-c]pyrimidin (Beispiel 6A) (800 mg, 2.7 mmol), tert-Butyl-(2-aminopropyl)carbamat (700 mg, 4.0 mmol) und N,N-Diisopropylethylamin (1.00 g, 8.0 mmol) werden in DMSO (13 ml) gelöst und bei 120°C unter Argon 12 Stunden gerührt. Das Reaktionsgemisch wird mit Wasser (100 ml) verdünnt, und mit Ethylacetat (3 x 75 ml) extrahiert. Die vereinigten organischen Phasen werden mit gesättigter wässriger Natriumchloridlösung gewaschen, über Magnesiumsulfat getrocknet und eingeengt. Nach Entfernen des Lösemittels erhält man einen Schaum, der in Diethylether aufgenommen wird. Das Produkt fällt als Feststoff aus der Diethyletherlösung aus, und wird abgesaugt und getrocknet. Es werden 800 mg (68% d. Th.) des Produktes als Feststoff erhalten.

LCMS (Methode 3): Rₜ = 2.04 min. (m/z = 437 (M+H)⁺)

¹H-NMR (400MHz, DMSO-d₆): δ = 8.04 (s, 1H), 7.75 (dd, 1H), 7.70 (d, 1H), 7.56 (d, 1 H), 7.51 (m, 2H), 7.10 (s, 1H), 6.96 (t, 1H), 4.34 (m, 1H), 3.21 (m, 2H), 1.32 (s, 9H), 1.23 (d, 3H).

### Beispiel 37A

N²-[7-(2,4-Dichlorphenyl)imidazo[1,2-c]pyrimidin-5-yl]propan-1,2-diamin-trifluoracetat 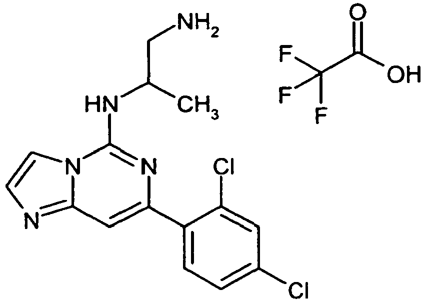 tert-Butyl-(2-{[7-(2,4-dichlorphenyl)imidazo[1,2-c]pyrimidin-5-yl]amino}propyl)carbamat (Beispiel 36A) (700 mg, 1.6 mmol) wird in Dichlormethan (20 ml) gelöst und Trifluoressigsäure (2.5 ml, 32 mmol) wird zugegeben. Die resultierende Lösung wird 1 h bei RT gerührt, einrotiert und auf der Vakuumlinie getrocknet. Man erhält 1 g des Produktes.

LCMS (Methode 4): Rₜ = 2.31 min. (m/z = 337 (M+H)⁺)

¹H-NMR (400MHz, DMSO-d₆): δ = 8.58 (br d, 1H), 8.28 (d; 1H), 8.06 (d, 1H), 7.94 (br s, 2H), 7.81 (d, 1H), 7.79 (d, 1H), 7.61 (dd, 1H), 7.44 (s, 1H), 4.58 (m, 1H), 3.14 (m, 2H), 1.35 (d, 3H).

### Beispiel 38A

tert-Butyl-{2-[(5-cyanopyridin-2-yl)amino]propyl}carbamat 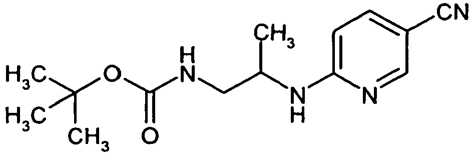 2-Chlor-5-cyanopyridin (477 mg, 3.4 mmol), tert-Butyl-(2-aminopropyl)carbamat (300 mg, 1.7 mmol) und Kaliumhydrogencarbonat (345 mg, 3.4 mmol) werden in DMF (10 ml) gelöst und bei 90°C unter Argon 12 h gerührt. Das Reaktionsgemisch wird mit Wasser (100 ml) verdünnt, und mit Ethylacetat (3 x 75 ml) extrahiert. Die vereinigten organischen Phasen werden mit gesättigter wässriger Natriumchloridlösung gewaschen, über Magnesiumsulfat getrocknet und eingeengt. Nach Entfernen des Lösemittels wird der Rückstand über Kieselgel durch Flashchromatographie gereinigt (Eluent: Cyclohexan-Ethylacetat 10:1). Es werden 265 mg (55% d. Th.) des Produktes als Feststoff erhalten.

LCMS (Methode 4): Rₜ = 3.21 min. (m/z = 277 (M+H)⁺)

¹H-NMR (400MHz, DMSO-d₆): δ = 8.37 (d, 1H), 7.66 (d, 1H), 7.37 (d, 1H), 6.87 (br t, 1H), 6.50 (d, 1H), 4.05 (m, 1H), 3.02 (m, 2H), 1.37 (s, 9H), 1.07 (d, 3H).

### Beispiel 39A

tert-Butyl-{2-[(6-amino-5-nitropyridin-2-yl)amino]propyl}carbamat 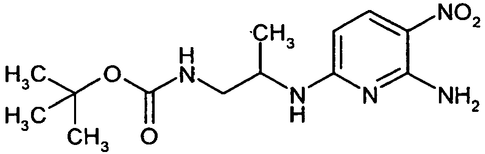 tert-Butyl-{2-[(6-amino-5-nitropyridin-2-yl)amino]propyl}carbamat (Beispiel 39A) wird in Analogie zu Beispiel 38A aus 2-Amino-6-chlor-3-nitropyridin (637 mg, 3.7 mmol), tert-Butyl-(2-aminopropyl)carbamat (320 mg, 1.8 mmol) und Kaliumhydrogencarbonat (368 mg, 3.7 mmol) hergestellt. Es werden 561 mg (98% d. Th.) des Produktes als Feststoff erhalten.

LCMS (Methode 3): Rₜ = 2.10 min. (m/z = 312 (M+H)⁺)

¹H-NMR (400MHz, DMSO-d₆): δ = 8.13 (br m, 1H), 7.95 (d, 1H), 7.70 (br m6.86 (br s, 1H), 5.92 (d, 1H), 4.19 (br m, 1H), 3.06 (br m, 2H), 1.34 (s, 9H), 1.09 (d, 3H).

### Beispiel 40A

6-[(2-Amino-1-methylethyl)amino]nicotinnitril-trifluoracetat 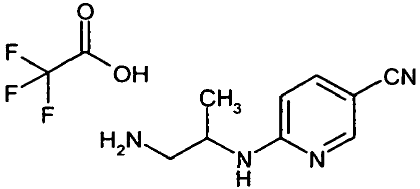 tert-Butyl-{2-[(5-cyanopyridin-2-yl)amino]propyl}carbamat (Beispiel 38A) (262 mg, 0.9 mmol) wird in Dichlormethan (12 ml) vorgelegt, Trifluoressigsäure (1.0 ml, 14 mmol) wird zugegeben, und das Reaktionsgemisch wird 1 h bei RT gerührt. Das Lösemittel wird eingedampft, und das Produkt wird auf der Vakuumlinie getrocknet. Das auf dieser Weise isolierte Rohprodukt erhält immer noch TFA, kann aber so weiter umgesetzt werden. Man erhält 400 mg des Produktes.

LCMS (Methode 4): Rₜ = 1.25 min. (m/z = 177 (M+H)⁺)

¹H-NMR (400MHz, DMSO-d₆): δ = 8.44 (d, 1H), 7.83 (br s, 3H), 7.74 (dd, 1H), 7.55 (d, 1H), 6.58 (d, 1H), 4.29 (br t, 1H), 3.45 (m, 1H), 2.94 (m, 2H), 1.19 (d, 3H).

### Beispiel 41A

N⁶-(2-Amino-1-methylethyl)-3-nitropyridin-2,6-diamin-trifluoracetat 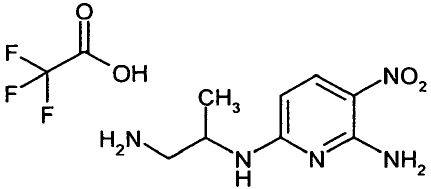 N⁶-(2-Amino-1-methylethyl)-3-nitropyridin-2,6-diamin-trifluoracetat (Beispiel 40A) wird in Analogie zu Beispiel 40A aus tert-Butyl-{2-[(6-amino-5-nitropyridin-2-yl)amino]propyl}carbamat (Beispiel 39A) (560 mg, 1.8 mmol), Trifluoressigsäure (2.0 ml, 27 mmol) in Dichlormethan (20 ml) hergestellt. 900 mg des Rohproduktes werden so als Feststoff erhalten.

LCMS (Methode 4): Rₜ = 1.82 min. (m/z = 212 (M+H)⁺)

### Beispiel 42A

tert-Butyl-{2-[(5-cyanopyridin-2-yl)thio]ethyl}carbamat 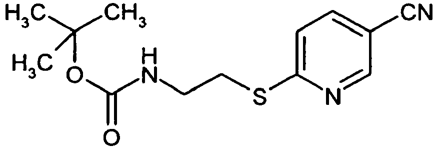 2-Chlor-5-cyanopyridin (500 mg, 3.6 mmol), tert-Butyl-(2-mercaptoethyl)carbamat (426 mg, 2.4 mmol) und 1,8-Diazabicyclo(5.4.0)undec-7-en (549 mg, 3.6 mmol) werden in DMF (5 ml) gelöst und bei RT unter Argon 12 h gerührt. Das Reaktionsgemisch wird mit Wasser (100 ml) verdünnt, und mit Ethylacetat (3 x 75 ml) extrahiert. Die vereinigten organischen Phasen werden mit gesättigter wässriger Natriumchloridlösung gewaschen, über Magnesiumsulfat getrocknet und eingeengt. Nach Entfernen des Lösemittels wird der Rückstand durch präparativer RP-HPLC gereinigt (Gradient Eluent: Wasser-Acetonitril 90:10 bis 10:90). Es werden 612 mg (91% d. Th.) des Produktes als Feststoff erhalten.

LCMS (Methode 6): Rₜ = 1.80 min. (m/z = 280 (M+H)⁺)

¹H-NMR (400MHz, DMSO-d₆): δ = 8.86 (d, 1H), 8.07 (dd, 1H), 7.54 (dd, 1H), 7.08 (br s, 1H), 3.24 (m, 4H), 1.36 (s, 9H).

### Beispiel 43A

tert-Butyl-(2-[(6-amino-5-nitropyridin-2-yl)thio]ethyl}carbamat 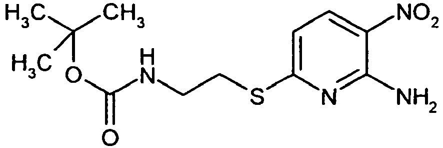 tert-Butyl-(2-[(6-amino-5-nitropyridin-2-yl)thio]ethyl)carbamat (Beispiel 43A) wird in Analogie zu Beispiel 42A aus 2-Amino-6-chlor-3-nitropyridin (500 mg, 2.9 mmol), tert-Butyl-(2-mercaptoethyl)carbamat (255 mg, 1.4 mmol) und 1,8-Diazabicyclo(5.4.0)undec-7-en (439 mg, 2.9 mmol) hergestellt. Es werden 422 mg (93% d. Th.) an Produkt erhalten.

LCMS (Methode 1): Rₜ = 2.10 min. (m/z = 315 (M+H)⁺)

¹H-NMR (400MHz, DMSO-d₆): δ = 8.17 (d, 1H), 8.07 (br s, 1H), 7.03 (br t. 1H), 6.64 (d, 1H), 3.22 (m, 4H), 1.36 (s, 9H).

### Beispiel 44A

tert-Butyl-(2-{[7-(2,4-dichlorphenyl)imidazo[1,2-c]pyrimidin-5-yl]thio}ethyl)carbamat 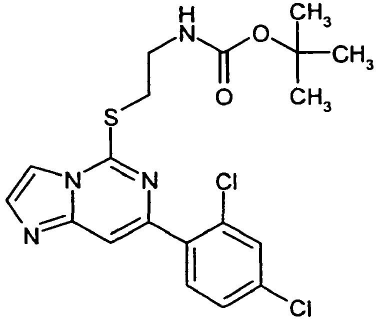 tert-Butyl-(2-{[7-(2,4-dichlorphenyl)imidazo[1,2-c]pyrimidin-5-yl]thio}ethyl)carbamat (Beispiel 44A) wird in Analogie zur Herstellung von Beispiel 42A aus 5-Chlor-7-(2,4-dichlorphenyl)imidazo[1,2-c]pyrimidin (Beispiel 6A) (500 mg, 1.7 mmol), tert-Butyl-(2-mercaptoethyl)carbamat (445 mg, 2.5 mmol) und 1,8-Diazabicyclo(5.4.0)undec-7-en (382 mg, 2.5 mmol) synthetisiert. Das Rohprodukt wird durch Flashchromatographie (Eluent: Cyclohexan:Ethylacetat) aufgereinigt und man erhält 422 mg (93% d. Th.) des Produktes nach Eindampfen des Lösemittels.

LCMS (Methode 1): Rₜ = 2.40 min. (m/z = 439 (M+H)⁺)

### Beispiel 45A

6-[(2-Aminoethyl)thio]nicotinnitril-trifluoracetat 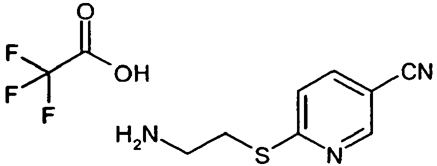 6-[(2-Aminoethyl)thio]nicotinonitril-trifluoracetat (Beispiel 45A) wird in Analogie zu Beispiel 40A aus tert-Butyl-{2-[(5-cyanopyridin-2-yl)thio]ethyl}carbamat (Beispiel 42A) (580 mg, 2.1 mmol) und Trifluoressigsäure (3.2 ml, 42 mmol) in Dichlormethan (20 ml) hergestellt. Es werden 500 mg des Produktes erhalten.

LCMS (Methode 4): Rₜ = 1.01 min. (m/z = 180 (M+H)⁺)

¹H-NMR (400MHz, DMSO-d₆): δ = 8.89 (d, 1H), 8.13 (dd, 1H), 8.03 (br s, 3H), 7.62 (d, 1H), 3.43 (t, 2H), 3.14 (m, 2H).

### Beispiel 46A

6-[(2-Aminoethyl)thio]-3-nitropyridin-2-amin-trifluoracetat 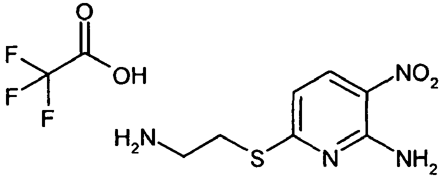 6-[(2-Aminoethyl)thio]-3-nitropyridin-2-amine-trifluoracetat (Beispiel 46A) wird in Analogie zu Beispiel 40A aus tert-Butyl-{2-[(6-amino-5-nitropyridin-2-yl)thio]ethyl}carbamat (Beispiel 43A) (380 mg, 1.2 mmol) und Trifluoressigsäure (1.9 ml, 24 mmol) in Dichlormethan (20 ml) hergestellt. Es werden 400 mg des Produktes erhalten.

LCMS (Methode 4): Rₜ = 2.03 min. (m/z = 215 (M+H)⁺)

¹H-NMR (400MHz, DMSO-d₆): δ = 8.20 (d, 1H), 8.12 (br s, 2H), 7.92 (br s, 3H), 6.69 (d, 1H), 3.35 (t, 2H), 3.16 (m, 2H).

### Beispiel 47A

2-{[7-(2,4-Dichlorphenyl)imidazo[1,2-c]pyrimidin-5-yl]thio}ethanamin-trifluoracetat 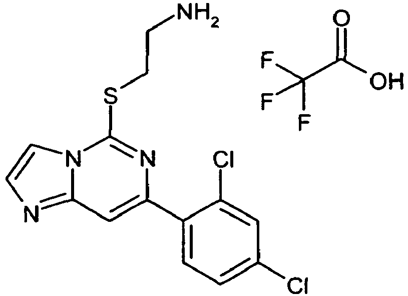 2-{[7-(2,4-Dichlorphenyl)imidazo[1,2-c]pyrimidin-5-yl]thio}ethanamine-trifluoracetat (Beispiel 47A) wird in Analogie zu Beispiel 40A aus tert-Butyl-(2-{[7-(2,4-dichlorphenyl)imidazo[1,2-c]pyrimidin-5-yl]thio}ethyl)carbamat (Beispiel 44A) (950 mg, 2.2 mmol) und Trifluoressigsäure (3.3 ml, 43 mmol) in Dichlormethan (20 ml) hergestellt. Das Rohprodukt besteht aus einem 50:50 Gemisch von Beispiel 47A und dem regioisomeren Produkt 2-{[7-(2,4-Dichlorphenyl)imidazo[1,2-c]pyrimidin-5-yl]amino}ethanethiol. Das Gemisch wird ohne Reinigung weiter verarbeitet.

### Beispiel 48A

2-[(6-Amino-5-nitropyridin-2-yl)amino]ethanol 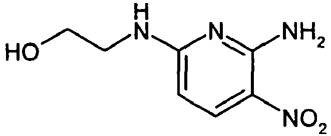 2-[(6-Amino-5-nitropyridin-2-yl)amino]ethanol (Beispiel 48A) wird in Analogie zu Beispiel 36A aus 2-Amino-6-chlor-3-nitropyridin (400 mg, 2.3 mmol) und 2-Aminoethanol (282 mg, 4.6 mmol) in DMSO (110°C, 4 h) hergestellt. Nach Entfernen des Lösemittels erhält man ein Rohprodukt, das aus Dichlormethan ausgefällt wird. Das Produkt wird abgesaugt und getrocknet. Es werden 283 mg (62% d. Th.) des Produktes erhalten.

LCMS (Methode 1): Rₜ = 0.51 min. (m/z = 199 (M+H)⁺)

¹H-NMR (400MHz, DMSO-d₆): δ = 8.15 (br s, 1H), 8.02 (br t, 1H), 8.03 (d, 1H), 7.74 (br m, 1H), 6.00 (d, 1H), 4.79 (t, 1H), 3.54 (m, 2H), 3.43 (m, 2H).

### Beispiel 49A

2-{[7-(2,4-Dichlorphenyl)imidazo[1,2-c]pyrimidin-5-yl)amino}ethanol 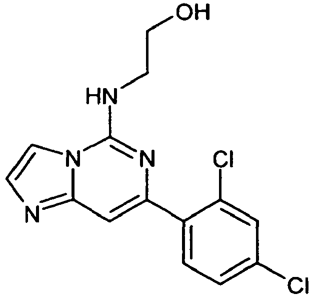 2-{[7-(2,4-Dichlorphenyl)imidazo[1,2-c]pyrimidin-5-yl]amino}ethanol (Beispiel 49A) wird in Analogie zu Beispiel 36A aus 5-Chlor-7-(2,4-dichlorphenyl)imidazo[1,2-c]pyrimidin (Beispiel 6A) (200 mg, 0.7 mmol) und 2-Aminoethanol (82 mg, 1.3 mmol) in DMSO (80°C, 4 h) hergestellt. Nach Entfernen des Lösemittels erhält man ein Rohprodukt, das aus Dichlormethan ausgefällt wird. Das Produkt wird abgesaugt und getrocknet. Es werden 216 mg (99% d. Th.) Produkt als Feststoff erhalten.

LCMS (Methode 3): Rₜ = 1.44 min. (m/z = 323 (M+H)⁺)

¹H-NMR (400MHz, DMSO-d₆): δ = 8.15 (s, 1H), 8.12 (t, 1H), 7.74 (m, 2H), 7.58 (s, 1H), 7.54 (dd, 1H), 7.09 (s, 1H), 4.90 (br s, 1H), 3.67 (m, 2H), 2.85 (t, 2H).

### Beispiel 50A

4-Chlor-6-hydrazino-2-(methylthio)pyrimidin

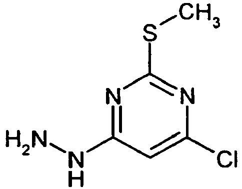

In 300 ml THF werden bei -20°C 33 g (169.2 mmol) 4,6-Dichlor-2-(methylthio)pyrimidin vorgelegt und 169 ml einer 1M Lösung von Hydrazin in THF so schnell zugetropft, dass die Temperatur nicht über -15°C steigt. Man lässt weitere 2 h bei 0°C rühren. Nach beendeter Reaktion wird das Lösungsmittel abdestilliert und der Rückstand mit Diethylether verrührt. Man erhält 18.4 g (57% d. Th.) des Produktes als Feststoff.

LCMS (Methode 4): Rₜ = 2.43 min. (m/z = 191 (M+H)⁺)

¹H-NMR (400MHz, DMSO-d₆): δ = 7.26 (s, 1H), 6.48 (s, 1H), 6.39 (s, br, 1H), 3.82 (s, br, 2H), 2.51 (s, 3H).

### Beispiel 51A

7-Chlor-5-(methylthio)[1,2,4]triazolo[1,5-c]pyrimidin

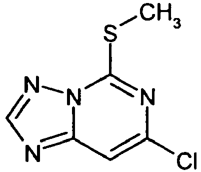

In 30 ml Ameisensäure werden 10 g (52.5 mmol) 4-Chlor-6-hydrazino-2-(methylthio)pyrimidin (Beispiel 50A) gelöst und für 16 h unter Rückfluß erhitzt. Das Rohgemisch wird von allen flüchtigen Bestandteilen im Vakuum entfernt und der Rückstand über Chromatographie an Kieselgel (Dichlormethan/Methanol 10+1) gereinigt. Man erhält 2.58 g (24% d. Th.) des Produktes.

LCMS (Methode 6): Rₜ = 0.97 min. (m/z = 201 (M+H)⁺)

¹H-NMR (400MHz, DMSO-d₆): δ = 8.78 (s, 1H), 7.45 (s, 1H), 2.84 (s, 3H).

### Beispiel 52A

7-Chlor[1,2,4]triazolo[1,5-c]pyrimidin-5-ol

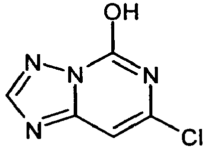

Eine Lösung von 2.58 g (12.9 mmol) 7-Chlor-5-(methylthio)[1,2,4]triazolo[1,5-c]pyrimidin (Beispiel 51A) wird in 300 ml Methanol gelöst und 28 ml einer 2 molaren wässrigen Kaliumhydroxidlösung zugegeben. Man erhitzt für 3 h unter Rückfluß: Nach beendeter Reaktion wird das Methanol im Vakuum entfernt, mit halbkonzentrierter Salzsäure neutralisiert und die ausgefallenen Kristalle abgesaugt. Diese werden mit Wasser und Methanol gewaschen und danach im Hochvakuum getrocknet. Man erhält 1.82 g (83% d. Th.) des Produktes.

LCMS (Methode 4): Rₜ = 1.72 min. (m/z = 171 (M+H)⁺).

¹H-NMR (400MHz, DMSO-d₆): δ = 8.40 (s, 1H), 7.06 (s, 1H).

### Beispiel 53A

5,7-Dichlor[1,2,4]triazolo[1,5-c]pyrimidin

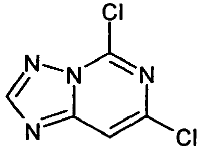

Unter Argon werden 927 mg (4.19 mmol) 7-Chlor[1,2,4]triazolo[1,5-c]pyrimidin-5-ol (Beispiel 52A) in 16.9 g (110.13 mmol) Phosphorylchlorid suspendiert und 4 h auf 120°C erhitzt. Nach dieser Zeit wird das überschüssige Phosphorylchlorid im Vakuum entfernt und der Rückstand mit Toluol zusammen destilliert. Der Rückstand wird mit Wasser suspendiert, mit gesättigter wässriger Natriumhydrogencarbonat-Lösung auf pH7 gebracht und der ausfallende Feststoff abgesaugt. Dieser wird mit Wasser gewaschen und für 18 h über Phosphorpentoxid getrocknet. Man erhält 389 mg (49% d. Th.) des Produktes.

LCMS (Methode 4): Rₜ = 2.55 min. (m/z = 189 (M+H)⁺).

¹H-NMR (400MHz, DMSO-d₆): δ = 8.79 (s, 1H), 8.26 (s, 1H).

### Beispiel 54A

6-({2-[(7-Chlor[1,2,4]triazolo[1,5-c]pyrimidin-5-yl)amino]ethyl}amino)nicotinnitril

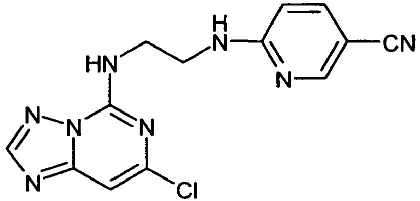

In 25 ml 2-Propanol werden 707 mg (3.20 mmol) 5,7-Dichlorimidazo[1,2-c]pyrimidin (Beispiel 53A) suspendiert und 1.35 g (3.52 mmol) 6-[(2-Aminoethyl)amino]nicotinonitrile-trifluoracetat (Beispiel 13A) und 1.03 g (7.99 mmol) DIPEA zugegeben. Man erhitzt für 16 h bei 80°C. Nach dieser Zeit wird Wasser zugeben und der ausfallende Niederschlag abgesaugt. Man wäscht mit wenig 2-Propanol/Wasser nach und trocknet den erhaltenen Feststoff im Hochvakuum. Man erhält 523 mg (50% d. Th.) des Produktes als Feststoff.

LCMS (Methode 3): Rₜ = 1.31 min. (m/z = 314 (M+H)⁺).

¹H-NMR (400MHz, DMSO-d₆): δ = 8.37 (d, 1H), 8.33 (t, 1H), 7.93 (s, 1H), 7.80 (s, 1H), 7.68 (d, 1H), 7.50 (d, 1H), 6.90 (s, 1H), 6.56 (s, br, 1H), 3.62 (m, 2H), 3.31 (m, 2H).

### Beispiel 55A

N⁶-{2-[(7-Chlor[1,2,4]triazolo[1,5-c]pyrimidin-5-yl)amino]ethyl}-3-nitropyridin-2,6-diamin

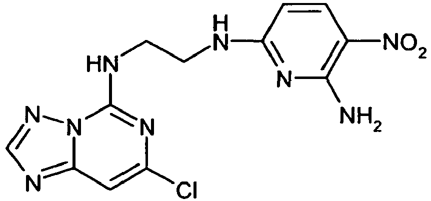

In 8 ml 2-Propanol werden 194 mg (3.20 mmol) 5,7-Dichlorimidazo[1,2-c]pyrimidin suspendiert und 403.9 mg (1.13 mmol) N⁶-(2-Aminoethyl)-3-nitropyridin-2,6-diamin-trifluoracetat und 331.7 mg (2.57 mmol) DIPEA zugegeben. Man erhitzt für 16 h bei 80°C. Nach dieser Zeit wird Wasser zugeben und der ausfallende Niederschlag abgesaugt. Man wäscht mit wenig 2-Propanol/Wasser nach und trocknet den erhaltenen Feststoff im Hochvakuum. Man erhält 265 mg (74% d. Th.) des Produktes als Feststoff.

LCMS (Methode 3): Rₜ = 1.97 min. (m/z = 350 (M+H)⁺).

¹H-NMR (400MHz, DMSO-d₆): δ = 8.81 (t, 1H), 8.53 (s, 1H), 8.16 (s, br, 1H), 8.06 (t, 1H), 7.94 (d, 1H), 7.67 (s, br, 1 H), 7.13 (s, 1H), 5.90 (d, 1 H), 3.57-3.73 (m, 4H).

### Beispiel 56A

tert-Butyl-{3-[(6-amino-5-nitropyridin-2-yl)amino]propyl}carbamat 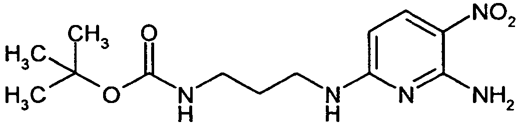 150.6 mg (0.864 mmol) tert-Butyl-(3-aminopropyl)carbamat, 300 mg (1.73 mmol) 6-Chlor-3-nitropyridin-2-amin und 173 mg (1.73 mmol) Kaliumhydrogencarbonat werden in 10 ml DMF suspendiert und auf 90°C für 16 h erhitzt. Der Ansatz wird mit Wasser versetzt und dreimal mit Essigsäureethylester extrahiert. Nach Reinigung des Rohproduktes durch präparative HPLC werden nach Trocknen im Hochvakuum 195 mg (65% d. Th.) Produkt gewonnen.

LCMS (Methode 7): Rₜ = 2.85 min. (m/z = 312 (M+H)⁺).

¹H-NMR (400MHz, DMSO-d₆): δ = 8.12 (s, br, 1H), 7.91 (d, 1H), 7.73 (s, br, 1H), 6.84 (t, 1H), 5.93 (d, 1H), 4.09 (dd, 1H), 3.32 (m, 2H), 2.97 (q, 2H), 1.64 (m, 2H), 1.37 (s, 9H).

### Beispiel 57A

N⁶-(3-Aminopropyl)-3-nitropyridin-2,6-diamin-trifluoroacetat 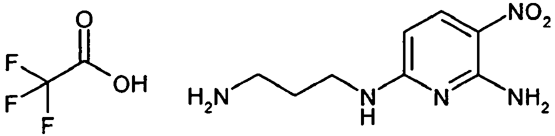 195 mg (0.56 mmol) tert-Butyl-{3-[(6-amino-5-nitropyridin-2-yl)amino]propyl}carbamat (Beispiel 56A) werden in 6 ml Dichlormethan gelöst und 656 mg (5.6 mmol) Trifluoressigsäure langsam zugespritzt. Man rührt für 16 h bei RT und entfernt dann alle flüchtigen Bestandteile im Vakuum. Es werden 185 mg (96% d. Th.) des Produktes erhalten.

LCMS (Methode 4): Rₜ = 1.41 min. (m/z = 212 (M+1)⁺).

¹H-NMR (400MHz, DMSO-d₆): δ = 8.18 (s, br, 1H), 8.04 (t, 1H), 7.95 (d, 1H), 7.70 (s, br, 3H), 5.94 (d, 1H), 3.40 (dd, 2H), 2.82 (dd, 2H), 1.80 (pent, 2H).

### Beispiel 58A

6-({2-[(4-Amino-6-chlorpyrimidin-2-yl)amino]ethyl}amino)pyridin-3-carbonitril

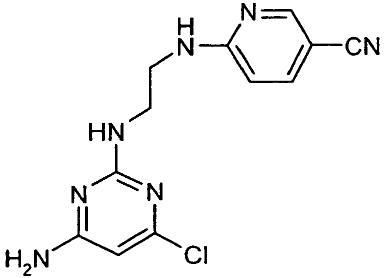

In 164 ml Acetonitril werden 10.3 g (63 mmol) 2,6-Dichlorpyrimidin-4-amin und 10.22 g (63 mmol) 6-[(2-Aminoethyl)amino]pyridin-3-carbonitril gelöst und für 24 h unter Rückflussbedingungen erhitzt. Nach Abkühlen wird die Hauptmenge des ausfallenden Produktes abgesaugt. Man erhält 11.9 g (50% d. Th.) des Produktes als Feststoff.

LCMS (Methode 8): Rₜ = 0.75 min. (m/z = 290 (M+H)⁺)

### Beispiel 59A

Ethyl-7-chlor-5-({2-[(5-cyanopyridin-2-yl)amino]ethyl}amino)imidazo[1,2-c]pyrimidin-2-carboxylat

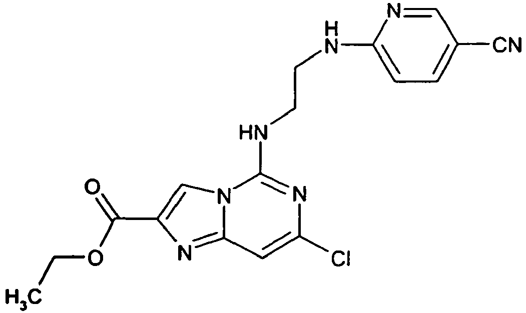

In 8 ml DMF werden 480 mg (1.66 mmol) 6-({2-[(4-Amino-6-chlorpyrimidin-2-yl)amino]ethyl}-amino)pyridin-3-carbonitril und 538 mg (2.49 mmol) Brombrenztraubensäureethylester gelöst und auf 100°C für 16 h erhitzt. Das Rohgemisch wird nach beendeter Reaktion mittels präparativer HPLC gereinigt. Man erhält 190 mg (27% d. Th.) des Produktes als Feststoff:

LCMS (Methode 1): Rₜ = 1.69 min. (m/z = 486 (M+H)⁺)

¹H-NMR (400MHz, DMSO-d₆): δ = 8.64 (s, 1H), 8.58 (t, 1H), 8.35 (d, 1H), 7.77 (m, 1H), 7.68 (d, 1H), 6.94 (s, 1H), 6.57 (d, 1H), 4.31 (q, 2H), 3.63 (m, 4H), 1.30 (t, 3H).

### Beispiel 60A

tert-Butyl 3-[(5-cyanopyridin-2-yl)amino]piperidin-1-carboxylat 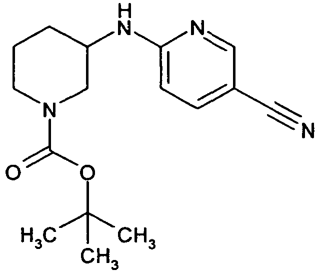 1.0 g (4.99 mmol) tert-Butyl-3-aminopiperidin-1-carboxylat und 1.383 g (9.99 mmol) 6-Chlorpyridin-3-carbonitril und 1.29 g (9.99 mmol) Diisopropylethylamin werden in 40 ml DMSO suspendiert und für 45 min in einer Mikrowelle auf 140°C erhitzt. Der Ansatz wird durch Kugelrohrdestillation weitgehend vom DMSO befreit, mit Wasser versetzt und der ausfallende Niederschlag abfiltriert. Nach Trocknen im Hochvakuum erhält man 2.24 g (46% d. Th.) des Produktes.

LCMS (Methode 3): Rₜ = 2.23 min. (m/z = 303 (M+H)⁺).

### Beispiel 61A

6-(Piperidin-3-ylamino)pyridin-3-carbonitril Hydrochlorid

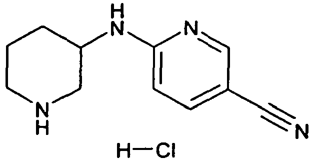

In 4.3 ml einer Salzsäurelösung in Dioxan (4 M) werden 2.24 g (3.4 mmol) tert-Butyl-3-[(5-cyanopyridin-2-yl)amino]piperidin-1-carboxylat (Beispiel 60A) gelöst und 3 h bei RT gerührt. Nach vollständiger Reaktion wird das Lösungsmittel komplett entfernt. Es werden 1.74 g (90% d. Th.) des Produktes als Feststoff erhalten.

LCMS (Methode 8): Rₜ = 0.27 min. (m/z = 203 (M+H)⁺)

¹H-NMR (400MHz, DMSO-d₆): δ = 9.13 (m, 1H), 9.0 (m, 1H), 8.44 (d, 1H), 7.89 (m, 1H), 7.74 (dd, 1H), 6.63 (d, 1H), 5.58 (s, br), 4.19 (s, br, 1H), 3.57 (s, 1H), 3.34 (d, 1H), 3.14 (d, 1H), 2.88 (m, 1H), 2.7-2.81 (m, 1H), 1.82-2.0 (m, 2H), 1.63-1.79 (m, 1H), 1.48-1.59 (m, 1H).

### Beispiel 62A

2-Amino-6-[(2-{[7-chlor-2-(trifluormethyl)imidazo[1,2-c]pyrimidin-5-yl]amino}ethyl)amino]-pyridin-3-carbonitril

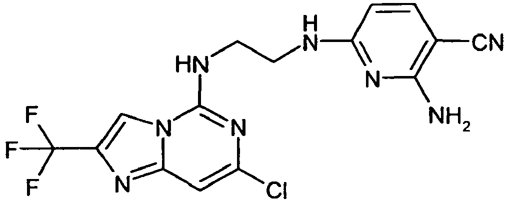

Analog der Herstellung von Beispiel 16A werden aus 338 mg (1.29 mmol) 5,7-Dichlor-2-(trifluormethyl)imidazo[1,2-c]pyrimidin (Beispiel 23A) und 338.4 mg (1.55 mmol) 2-Amino-6-[(2-aminoethyl)amino]pyridin-3-carbonitril (Beispiel 111A) 433 mg (84% d. Th.) des Produktes als Feststoff erhalten.

LCMS (Methode 3): Rₜ = 2.12 min. (m/z = 397 (M+H)⁺)

¹H-NMR (400MHz, DMSO-d₆): δ = 8.56 (s, 1H), 8.49 (t, 1H), 7.32 (d, 1H), 7.23 (s, br, 1H), 7.01 (s, 1H), 6.27 (s, 2H), 5.79 (d, 1H), 3.59-3.67 (m, 2H), 3.50-3.58 (m, 2H).

### Beispiel 63A

tert-Butyl-2-{[(5-cyanopyridin-2-yl)amino]methyl}pyrrolidin-1-carboxylat

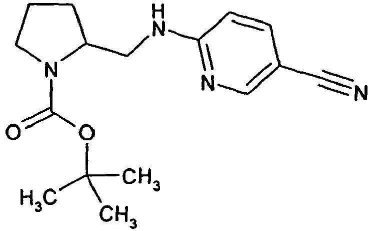

Analog der Herstellung von Beispiel 60A werden aus 250 mg (1.25 mmol) tert-Butyl-2-(aminomethyl)pyrrolidin-1-carboxylat und 345.9 mg (2.5 mmol) 6-Chlorpyridin-3-carbonitril 214.5 mg (57% d. Th.) des Produktes als Feststoff erhalten.

LCMS (Methode 9): Rₜ = 2.13 min. (m/z = 303 (M+H)⁺).

¹H-NMR (400MHz, DMSO-d₆): δ = 8.34 (s, 1H), 7.68 (s, br, 2H), 6.56 (d, 1H), 3.88 (s, 1H), 3.52 (s, br, 1H), 3.23 (m, 2H), 1.65-1.9 (m, 5H), 1.38 (s, 9H).

### Beispiel 64A

6-[(Pyrrolidin-2-ylmethyl)amino]pyridin-3-carbonitril Dihydrochlorid

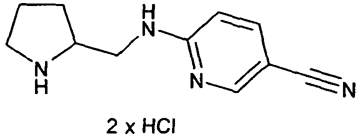

Analog der Herstellung von Beispiel 61A werden aus 190 mg (0.43 mmol) tert-Butyl-2-{[(5-cyanopyridin-2-yl)amino]methyl}pyrrolidin-1-carboxylat und 10 ml Salzsäure in Dioxan (4M) 117 mg (99% d. Th.) des Produktes als Feststoff erhalten.

LCMS (Methode 9): Rₜ = 0.84 min. (m/z = 203 (M+H)⁺).

¹H-NMR (400MHz, DMSO-d₆): δ = 9.35 (s, br, 1H), 8.98 (s, br, 1H), 8.44 (s, 1H), 8.03 (s, 1H), 7.76 (dd, 1H), 6.67 (d, 1 H), 5.10 (s, br), 3.71 (m, 1H), 3.62 (m, 2H), 3.67 (s, 1H), 3.08-3.25 (m, 2H), 1.98-2.09 (m, 1H), 1.80-1.98 (m, 2H), 1.59-1.70 (m, 1H).

### Beispiel 65A

tert-Butyl-3-[(6-amino-5-nitropyridin-2-yl)amino]piperidin-1-carboxylat

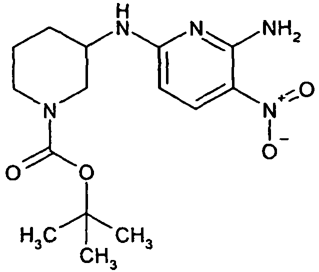

Analog der Herstellung von Beispiel 60A werden aus 500 mg (2.11 mmol) tert-Butyl-3-aminopiperidin-1-carboxylat Hydrochlorid und 771.7 mg (4.2 mmol) 6-Chlor-3-nitropyridin-2-amin 615 mg (81% d. Th.) des Produktes als Feststoff erhalten.

LCMS (Methode 6): Rₜ = 1.77 min. (m/z = 338 (M+H)⁺).

¹H-NMR (400MHz, DMSO-d₆): δ = 8.11 (s, br, 1H), 7.95 (d, 1H), 7.81 (d, 1H), 7.69 (s, br, 1 H), 6.0 (d, 1H), 3.49 (s, br, 1H), 3.48 (s, 1H), 1.89 (m, 1H), 1.76 (m, 1H), 1.50 (m, 1H), 1.2-1.55 (s, 11 H).

### Beispiel 66A

3-Nitro-N⁶-piperidin-3-ylpyridin-2,6-diamin Dihydrochlorid

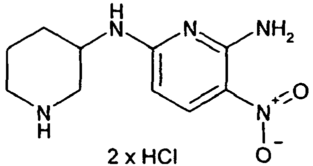

Analog der Herstellung von Beispiel 61A werden aus 610 mg (1.62 mmol) tert-Butyl-3-[(6-amino-5-nitropyridin-2-yl)amino]piperidin-1-carboxylat und 40 ml Salzsäure in Dioxan (4M) 500 mg (99% d. Th.) des Produktes als Feststoff erhalten.

LCMS (Methode 9): Rₜ = 0.86 min. (m/z = 238 (M+H)⁺).

¹H-NMR (400MHz, DMSO-d₆): δ = 9.13 (s, br, 1H), 8.99 (s, br, 1H), 8.17 (s, 1H), 7.99 (d, 1H), 7.7 (s, br, 1H), 6.0 (d, 1H), 4.18 (s, br, 1H), 3.57 (s, 1H), 3.35 (d, 1H), 3.10 (m, 1H), 2.94 (m, 2H), 1.83-2.01 (m, 2H), 1.63-1.78 (m, 1H), 1.50-1.62 (m, 1H).

### Beispiel 67A

tert-Butyl 3-[(5-cyanopyridin-2-yl)amino]piperidin-1-carboxylat

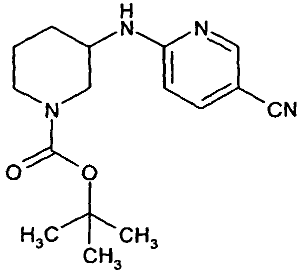

Analog der Herstellung von Beispiel 60A werden aus 100 mg (0.49 mmol) tert-Butyl-3-aminopiperidin-1-carboxylat Hydrochlorid und 138 mg (0.99 mmol) 6-Chlorpyridin-3-carbonitril 85 mg (56% d. Th.) des Produktes als Feststoff erhalten.

LCMS (Methode 6): Rₜ = 1.81 min. (m/z = 303 (M+H)⁺).

¹H-NMR (400MHz, DMSO-d₆): δ = 8.40 (d, 1H), 7.68 (d, 1H), 7.53 (d, 1H), 6.59 (d, 1H), 4.12 (s, br, 3H), 3.80 (s, br, 1H), 3.1-3.7 (m, 2H), 1.90 (m, 1H), 1.74 (m, 1H), 1.3-1.6 (m, 1H), 1.27 (s, 9H).

### Beispiel 68A

6-(Piperidin-3-ylamino)pyridin-3-carbonitril Dihydrochlorid

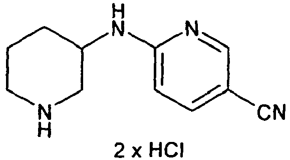

Analog der Herstellung von Beispiel 61A werden aus 2.24 g (3.4 mmol) tert-Butyl-3-[(5-cyanopyridin-2-yl)amino]piperidin-1-carboxylat durch Reaktion mit Salzsäure in Dioxan 1.74 g (90% d. Th.) des Produktes als Feststoff erhalten.

LCMS (Methode 8): Rₜ = 0.27 min. (m/z = 203 (M+H)⁺).

¹H-NMR (400MHz, DMSO-d₆): δ = 9.13 (s, br, 1H), 8.99 (s, br, 1H), 8.44 (d, 1H), 7.89 (m, 1H), 7.74 (dd, 1H), 6.63 (d, 1H), 5.58 (s, br), 4.19 (m, 1H), 3.57 (s, 1H), 3.34 (d, 1H), 3.15 (d, 1H), 2.82-2.95 (m, 1H), 2.7-2.82 (m, 1H), 1.83-2.0 (m, 2H), 1.64-1.8 (m, 1H), 1.47-1.61 (m, 1H).

### Beispiel 69A

Methyl-4-amino-2-(methylsulfonyl)-1,3-thiazol-5-carboxylat

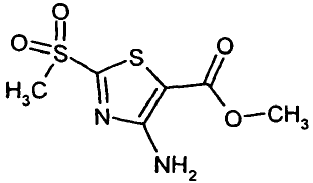

In 170 ml Wasser werden 5.12 g (8.32 mmol) Oxone^{®} gelöst und auf 5°C abgekült. Dann tropft man eine Lösung von 1 g (4.90 mmol) Methyl-4-amino-2-(methylsulfanyl)-1,3-thiazol-5-carboxylat in 18 ml Methanol zu und rührt die Lösung für 3 h bei RT. Die Hauptmenge Methanol wird entfernt und dreimal mit Dichlormethan extrahiert. Die vereinigten organischen Phasen werden mit Natriumsulfat getrocknet. Nach Entfernen des Lösungsmittels und Trocknen des Rückstandes im Hochvakuum wird der erhaltene Feststoff (824 mg (43% d. Th.)) ohne weitere Reinigung eingesetzt.

LCMS (Methode 3): Rₜ = 1.52 min. (m/z = 237 (M+H)⁺).

¹H-NMR (400MHz, DMSO-d₆): δ = 7.36 (s, br, 2H), 3.79 (s, 3H), 3.45 (s, 3H).

### Beispiel 70A

1-[4-Amino-2-(methylsulfonyl)-1,3-thiazol-5-yl]ethanon

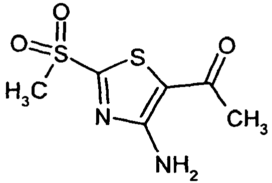

Analog der Herstellung von Beispiel 69A werden aus 500 mg (2.66 mmol) 1-[4-Amino-2-(methylsulfanyl)-1,3-thiazol-5-yl]ethanon durch Umsetzung mit 2775 mg (4.52 mmol) Oxone^{®} 395 mg (52% d. Th.) des Produktes als Feststoff erhalten. Das Reaktionsprodukt enthält noch ca. 30% des entsprechenden Sulfoxides, welches nicht weiter abgetrennt wird.

LCMS (Methode 3): Rₜ = 1.17 min. (m/z = 221 (M+H)⁺).

¹H-NMR (400MHz, DMSO-d₆): δ = 7.76 (s, br, 2H), 3.46 (s, 3H), 2.41 (s, 3H).

### Beispiel 71A

2-(Methylsulfonyl)-5-nitro-1,3-thiazol-4-amin

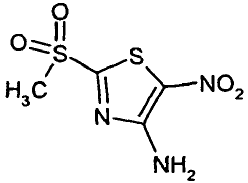

Analog der Herstellung von Beispiel 18A werden aus 250 mg (1.3 mmol) 2-(Methylsulfanyl)-5-nitro-1,3-thiazol-4-amin durch Umsetzung mit 3-Chlorperbenzoesäure 116 mg (40% d. Th.) des Produktes erhalten.

LCMS (Methode 9): Rₜ = 0.96 min. (m/z = 224 (M+H)⁺).

### Beispiel 72A

tert-Butyl[2-({[(3,5-dimethyl-1H-pyrazol-1-yl)-(imino)methyl]carbamothioyl}-amino)ethyl]-carbamat

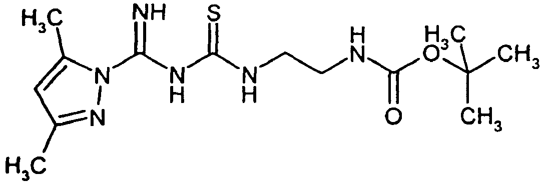

In 12 ml DMSO werden unter leichter Eiskühlung 554.7 mg (4.94 mmol) Kalium-tert-butylat gelöst und anschließend 596.8 mg (2.96 mmol) 3,5-Dimethyl-1H-pyrazol-1-carboximidamid Nitrat zugegeben. Nach ca. 5 minütigem Rühren entsteht eine klare Lösung. Dann werden 500 mg (2.47 mmol) tert-Butyl-(2-isothiocyanatoethyl)carbamat zugegeben und man entfernt das Eisbad. Man erhitzt für weitere 3 h auf 60°C und setzt die so erhaltene Lösung für die weiteren Folgereaktionen ein.

### Beispiel 73A

tert-Butyl(2-{[(2Z)-3-cyano-4-hydroxy-4-(trifluormethyl)-1,3-thiazolidin-2-yliden]amino}-ethyl)carbamat

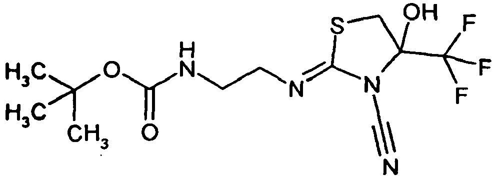

Zu dem Rohansatz aus Beispiel 72A wird unter Eiskühling eine Lösung von 484 mg (2.4 mmol) 3-Brom-1,1,1-trifluorpropan-2-on in 12 ml DMSO getropft und dann für 1 h auf 60°C erhitzt. Der Rohansatz wird auf Eiswasser gegossen und dreimal mit Essigsäureethylester extrahiert. Nach Trocknen der vereinigten organischen Phasen wird das Lösungsmittel vollständig entfernt und der Rückstand durch anschließende Reinigung mittels präparativer HPLC gereinigt. Man erhält 640 mg (67% d. Th.) des Produktes als Feststoff.

LCMS (Methode 3): Rₜ = 2.23 min. (m/z = 355 (M+H)⁺).

¹H-NMR (400MHz, DMSO-d₆): δ = 8.87 (s, 1H), 6.95 (t, 1H), 3.95 (d, 1H), 3.65 (d, 1H), 3.51-3.62 (m, 1H), 3.35(m, 1 H), 3.06-3.21 (m, 2H), 1.38 (s, 9H).

### Beispiel 74A

(2Z)-2-[(2-Aminoethyl)imino]-4-hydroxy-4-(trifluormethyl)-1,3-thiazolidin-3-carbonitril Hydrochlorid

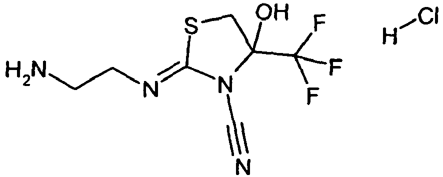

Analog der Herstellung von Beispiel 61A werden aus 436 mg (1.23 mmol) tert-Butyl(2-{[4-amino-5(trifluoracetyl)-1,3-thiazol-2-yl]amino}ethyl)carbamat und 20 ml Salzsäure in Dioxan (4M) 400 mg (99% d. Th.) des Produktes als Feststoff erhalten.

LCMS (Methode 8): Rₜ = 0.26 min. (m/z = 255 (M+H)⁺).

¹H-NMR (400MHz, DMSO-d₆): δ = 9.31 (s, 3H), 7.94 (s, br, 2H), 3.92 (dd, 2H), 3.3-3.8 (m, 2H).

### Beispiel 75A

tert-Butyl(2-{[4-amino-5-(cyclopropylcarbonyl)-1,3-thiazol-2-yl]amino}ethyl)carbamat

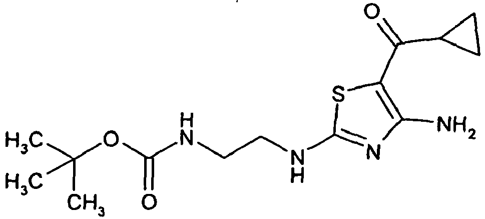

Zu 845 mg (2.48 mmol) des Rohansatz aus Beispiel 72A wird unter Eiskühlung eine Lösung von 404 mg (2.48 mmol) 2-Brom-1-cyclopropylethanon in 15 ml DMSO getropft und dann für 1 h auf 100°C erhitzt. Der Rohansatz wird auf Eiswasser gegossen und dreimal mit Essigsäureethylester extrahiert. Nach Trocknen der vereinigten organischen Phasen wird das Lösungsmittel vollständig entfernt und der Rückstand durch anschließende Reinigung mittels präparativer HPLC gereinigt. Man erhält 428 mg (48% d. Th.) des Produktes als Feststoff.

LCMS (Methode 9): Rₜ = 1.73 min. (m/z = 327 (M+H)⁺).

¹H-NMR (400MHz, CDCl₃): δ = 5.58 (s, br, 2H), 6.35 (s, 1H), 4.91 (s, 1H), 3.3-3.5 (m, 4H), 1.65-1.74 (m, 1H), 1.45 (s, 9H), 1.06-1.12 (m, 2H), 0.78-0.88 (m, 2H).

### Beispiel 76A

{4-Amino-2-[(2-aminoethyl)amino]-1,3-thiazol-5-yl}(cyclopropyl)methanon Dihydrochlorid

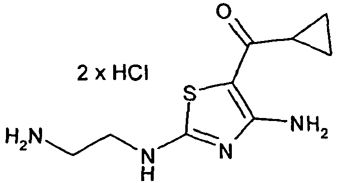

Analog der Herstellung von Beispiel 61A werden aus 420 mg (1.28 mmol) tert-Butyl(2-{[4-amino-5-(cyclopropylcarbonyl)-1,3-thiazol-2-yl]amino}ethyl)carbamat und 16 ml Salzsäure in Dioxan (4M) 400 mg (99% d. Th.) des Produktes als Feststoff erhalten.

LCMS (Methode 9): Rₜ = 0.83 min. (m/z = 227 (M+H)⁺).

¹H-NMR (400MHz, DMSO-d₆): δ = 8.73 (s, 1H), 8.09 (s, br, 2H), 3.96 (s, br, 2H), 3.51 (dd, 2H), 3.02 (dd, 2H), 1.64 (m, 1H), 0.79-0.85 (m, 2H), 0.7-0.79 (m, 2H).

Analog der für Beispiel 75A und Beispiel 76A beschriebenen Vorschriften werden folgende Produkte bzw. deren Vorstufen erhalten:

| **Beispiel** | **Struktur** | **Charakterisierung** |
|---|---|---|
| **77A** | 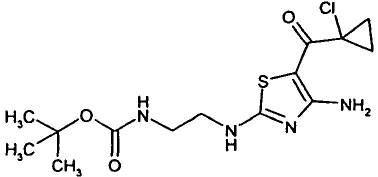 | LC/MS (Methode 8): Rₜ = 1.03 min MS (ESIpos): m/z = 361 (M+H)⁺. |
| **78A** | 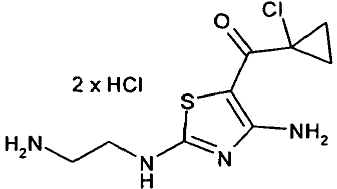 | LC/MS (Methode 8): Rₜ = 0.35 min MS (ESIpos): m/z = 261 (M+H)⁺. |
| **79A** | 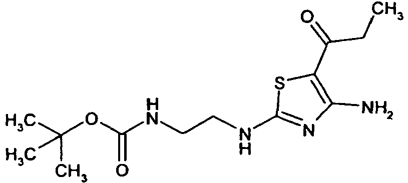 | LC/MS (Methode 6): Rₜ = 1.36 min MS (ESIpos): m/z = 315 (M+H)⁺. |
| **80A** | 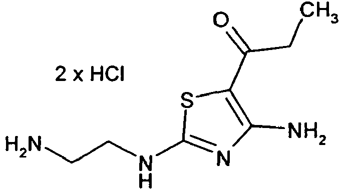 | LC/MS (Methode 9): Rₜ = 0.77 min MS (ESIpos): m/z = 215 (M+H)⁺. |

### Beispiel 81A

tert-Butyl-3-{[4-amino-5-(methoxycarbonyl)-1,3-thiazol-2-yl]amino}piperidin-1-carboxylat

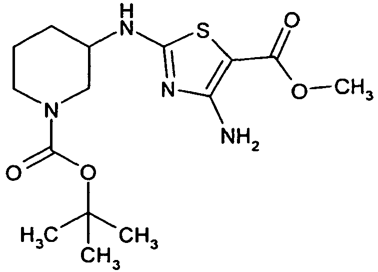

Analog der Herstellung von Beispiel 60A werden aus 335 mg (1.42 mmol) tert-Butyl-3-aminopiperidin-1-carboxylat Hydrochlorid und 3190 mg (2.84 mmol) Methyl-4-amino-2-(methylsulfonyl)-1,3-thiazol-5-carboxylat 158 mg (29% d. Th.) des Produktes als Feststoff erhalten.

LCMS (Methode 8): Rₜ = 1.06 min. (m/z = 357 (M+H)⁺).

¹H-NMR (400MHz, DMSO-d₆): δ = 8.32 (d, 1H), 6.79 (s, br, 2H), 3.60 (s, 3H), 3.55 (m, 2H), 1.89 (m, 1H), 1.71 (m, 1H), 1.5 (m, 1H), 1.35 (s, 11H).

### Beispiel 82A

Methyl-4-amino-2-(piperidin-3-ylamino)-1,3-thiazol-5-carboxylat Dihydrochlorid

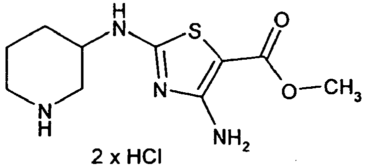

Analog der Herstellung von Beispiel 61A werden aus 150 mg (0.39 mmol) tert-Butyl-3-{[4-amino-5-(methoxycarbonyl)-1,3-thiazol-2-yl]amino}piperidin-1-carboxylat und 20 ml Salzsäure in Dioxan (4M) 130 mg (99% d. Th.) des Produktes als Feststoff erhalten.

LCMS (Methode 8): Rₜ = 0.26 min. (m/z = 257 (M+H)⁺).

¹H-NMR (400MHz, DMSO-d₆): δ = 9.04 (s, br, 2H), 8.51 (d, 1H), 3.9 (m, 2H), 3.6 (s, 3H), 3.34 (d, 1H), 3.11 (d, 1H), 2.75-2.94 (m, 2H), 1.93-2.04 (m, 1H), 1.8-1.91 (m, 1H), 1.60-1.76 (m, 1H), 1.43-1.57 (m, 1H).

Analog der für Beispiel 82A beschriebenen Vorschrift werden folgende Produkte bzw. deren Vorstufen erhalten:

| **Beispiel** | **Struktur** | **Charakterisierung** |
|---|---|---|
| **83A** | 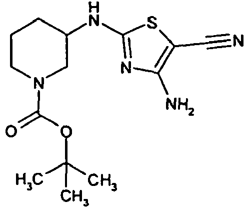 | LC/MS (Methode 7): Rₜ = 2.25 min MS (ESIpos): m/z = 390 (M+H)⁺. |
| **84A** | 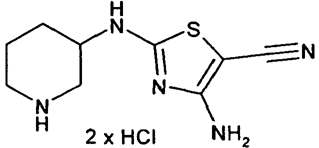 | LC/MS (Methode 1): Rₜ = 1.58 min MS (ESIpos): m/z = 458 (M+H)⁺. |
| **85A** | 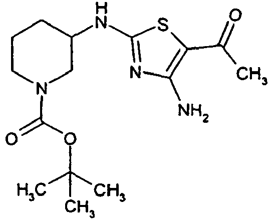 | LC/MS (Methode 4): Rₜ = 4.54 min MS (ESIpos): m/z = 455 (M+H)⁺. |
| **86A** | 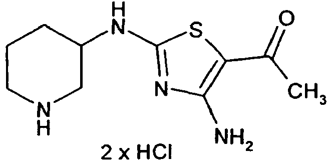 | LC/MS (Methode 4): Rₜ = 4.54 min MS (ESIpos): m/z = 455 (M+H)+. |
| **87A** | 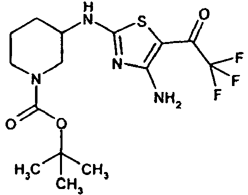 | LC/MS (Methode 4): Rₜ = 4.54 min MS (ESIpos): m/z = 455 (M+H)+. |
| **88A** | 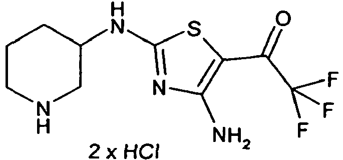 | LC/MS (Methode 4): Rₜ = 4.54 min MS (ESIpos): m/z = 455 (M+H)+. |

### Beispiel 89A

4-Amino-2-[(2-hydroxyethyl)amino]-1,3-thiazol-5-carbonitril

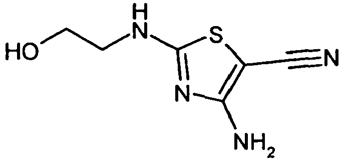

Beispiel 89A wird in Analogie zu Beispiel 48A aus 4-Amino-2-(methylsulfonyl)-1,3-thiazol-5-carbonitril (Beispiel 18A) (499 mg, 2.45 mmol) und 2-Aminoethanol (300 mg, 4.91 mmol) in DMSO (80°C, 20 h) hergestellt. Nach Entfernen des Lösemittels erhält man ein Rohprodukt 527 mg (50% d. Th.), welches ohne weitere Aufreinigung eingesetzt wird.

LCMS (Methode 8): Rₜ = 0.26 min. (m/z = 185 (M+H)⁺)

### Beispiel 90A

2-(Chlormethyl)-7-(2,4-dichlorphenyl)-5-(ethylsulfanyl)imidazo[1,2-c]pyrimidin

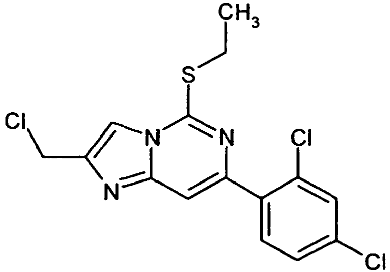

Zu einer Lösung von 1.395 g (10.99 mmol) 1,3-Dichlorpropan-2-on in 33 ml trockenem Ethanol werden 3 g (9.99 mmol) 6-(2,4-Dichlorphenyl)-2-(ethylsulfanyl)pyrimidin-4-amin (Beispiel 3A) gegeben und dann unter Rückflußbedingungen für 20 h erhitzt. Das Lösungsmittel wird weitgehend entfernt und der Rückstand über präparative HPLC gereinigt. Man erhält 1.5 g (37% d. Th.) des Produktes.

LCMS (Methode 3): Rₜ = 3.04 min. (m/z = 372 (M+H)⁺)

¹H-NMR (400MHz, DMSO-d₆): δ = 8.0 (s, 1H), 7.79 (dd, 1H), 7.78 (s, 1H), 7.69 (s, 1H), 7.59 (dd, 1H), 4.9 (s, 2H), 3.4 (q, 2H), 1.45 (t, 3H).

### Beispiel 91A

7-(2,4-Dichlorphenyl)-5-(ethylsulfanyl)-2-(morpholin-4-ylmethyl)imidazo[1,2-c]pyrimidin

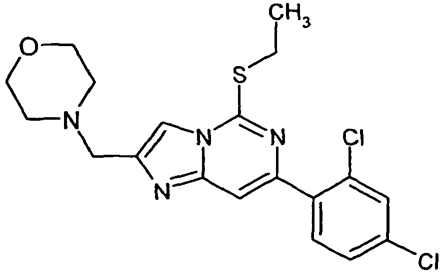

In 7 ml DMF werden 102 mg (1.17 mmol) Morpholin bei 0°C vorgelegt, 236 mg (0.78 mmol) Triethylamin und eine katalytische Menge Kaliumiodid zugegeben. Dann wird eine Lösung von 290 mg 2-(Chlormethyl)-7-(2,4-dichlorphenyl)-5-(ethylsulfanyl)imidazo[1,2-c]pyrimidin (Beispiel 91A) in DMF langsam zugetropft. Man läßt auf RT kommen und rührt für weitere 16 h bei dieser Temperatur. Nach Reinigung des Rohproduktes durch präparative HPLC werden nach Trocknen im Hochvakuum 214 mg (43% d. Th.) Produkt gewonnen.

LCMS (Methode 3): Rₜ = 1.95 min. (m/z = 423 (M+H)⁺)

¹H-NMR (400MHz, DMSO-d₆): δ = 7.78 (m, 1H), 7.74 (d, 1H), 7.63 (s, 1H), 7.58 (dd, 1H), 3.65 (s, 2H), 3.58 (dd, 4H), 3.40 (q, 2H), 2.47 (m, 4H), 1.42 (t, 3H).

### Beispiel 92A

7-(2,4-Dichlorphenyl)-5-(ethylsulfanyl)-2-[(4-methylpiperazin-1-yl)methyl]imidazo[1,2-c]-pyrimidin

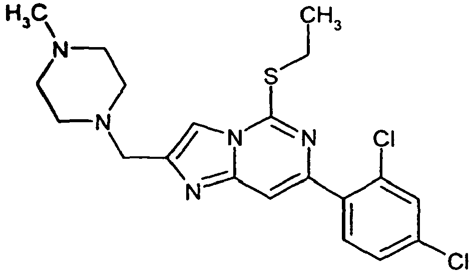

In Analogie zu **Beispiel 91A** werden aus 400 mg (0.44 mmol) 2-(Chlormethyl)-7-(2,4-dichlorphenyl)-5-(ethylsulfanyl)imidazo[1,2-c]pyrimidin (Beispiel 91A) und 161 mg (0.179 mmol) Methylpiperazin 465 mg (99% d. Th.) des Produktes erhalten.

LCMS (Methode 6): Rₜ = 1.33 min. (m/z = 436 (M+H)⁺)

### Beispiel 93A

7-(2,4-Dichlorphenyl)-2-(morpholin-4-ylmethyl)imidazo[1,2-c]pyrimidin-5-ol

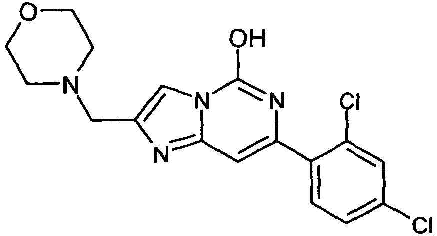

Analog der Herstellung des Hydroxypyrimidins Beispiel 10A werden aus 114 mg (0.27 mmol) 7-(2,4-Dichlorphenyl)-5-(ethylsulfanyl)-2-(morpholin-4-ylmethyl)imidazo[1,2-c]pyrimidin (Beispiel 92A) und 1.7 ml 2 molarer wässriger Kaliumhydroxid-Lösung in 3 ml Methanol und abschließendem Sauerstellen mit Salzsäure 104 mg (99% d. Th.) des Produktes erhalten.

LCMS (Methode 10): Rₜ = 1.31 min. (m/z = 379 (M+H)⁺)

¹H-NMR (400MHz, DMSO-d₆): δ = 11.85 (s, br, 1H), 7.81 (d, 1H), 7.65 (s, 1H), 7.61 (d, 1H), 7.57 (dd, 1H), 6.62 (s, 1H), 3.57 (dd, 4H), 3.52 (s, 2H), 2.45 (m, 4H).

### Beispiel 94A

7-(2,4-Dichlorphenyl)-2-[(4-methylpiperazin-1-yl)methyl]imidazo[1,2-c]pyrimidin-5-ol

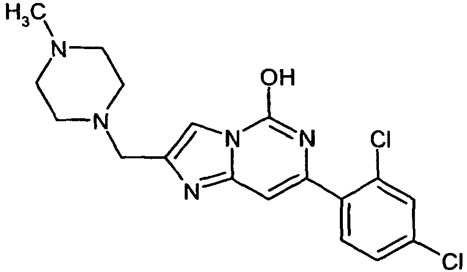

In Analogie zu Beispiel 10A werden aus 675 mg (1.31 mmol) 7-(2,4-Dichlorphenyl)-5-(ethylsulfanyl)-2-[(4-methylpiperazin-1-yl)methyl]imidazo[1,2-c]pyrimidin (Beispiel 93A) durch Erhitzen in methanolischer Kaliumhydroxidlösung 500 mg (97% d. Th.) des Produktes erhalten.

LCMS (Methode 6): Rₜ = 0.71 min. (m/z = 392 (M+H)⁺)

### Beispiel 95A

5-Chlor-7-(2,4-dichlorphenyl)-2-(morpholin-4-ylmethyl)imidazo[1,2-c]pyrimidin 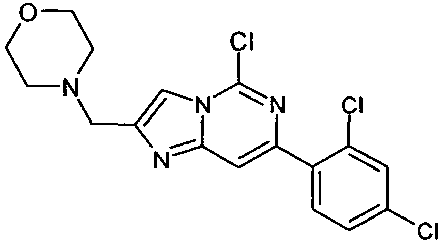 103 mg (0.267 mmol) 7-(2,4-Dichlorphenyl)-2-(morpholin-4-ylmethyl)imidazo[1,2-c]pyrimidin-5-ol (Beispiel 93A) werden in 5 ml (53.64 mmol) Phosphorylchlorid vorgelegt und für 3 h auf Rückflusstemperatur erhitzt. Man lässt abkühlen und gibt den Ansatz vorsichtig auf ein Eis/Wasser-Gemisch. Der ausfallende Niederschlag wird abfiltriert. Nach Trocknen im Hochvakuum erhält man 105 mg (99% d. Th.) des Produktes.

LCMS (Methode 8): Rₜ = 0.93 min. (m/z = 397 (M+H)⁺)

¹H-NMR (400MHz, DMSO-d₆): δ = 8.01 (s, br, 1H), 7.93 (s, 1H), 7.80 (d, 1H), 7.72 (d, 1H), 7.59 (dd, 1H), 3.73 (s, br, 2H), 3.61 (m, 4H), 3.32 (m, 4H).

### Beispiel 96A

5-Chlor-7-(2,4-dichlorphenyl)-2-[(4-methylpiperazin-1-yl)methyl]imidazo[1,2-c]pyrimidin

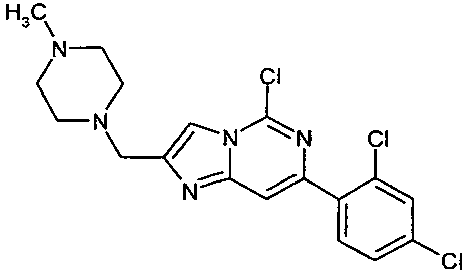

In Analogie zu Beispiel 95A werden aus 225 mg (0.49 mmol) 7-(2,4-Dichlorphenyl)-2-[(4-methylpiperazin-1-yl)methyl]imidazo[1,2-c]pyrimidin-5-ol (Beispiel 94A) durch Umsetzung mit Phosphorylchlorid 42 mg (17% d. Th.) des Produktes erhalten.

LCMS (Methode 3): Rₜ = 1.71 min. (m/z = 410 (M+H)⁺)

### Beispiel 97

1-[7-Chlor-5-(methylsulfanyl)imidazo[1,2-c]pyrimidin-2-yl]-N,N-dimethyl-methanamin

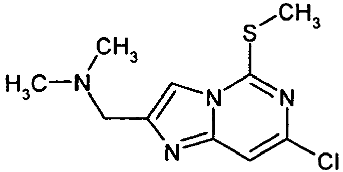

In Analogie zu Beispiel 32A werden aus 300 mg (1.21 mmol) 7-Chlor-2-(chlormethyl)-5-(methylsulfanyl)imidazo[1,2-c]pyrimidin (Beispiel 26A) und 81.8 mg (1.81 mmol) Dimethylamin 154 mg (31% d. Th.) des Produktes erhalten.

LCMS (Methode 7): Rₜ = 0.89 min. (m/z = 257 (M+H)⁺)

### Beispiel 98A

7-Chlor-2-[(dimethylamino)methyl]imidazo[1,2-c]pyrimidin-5-ol

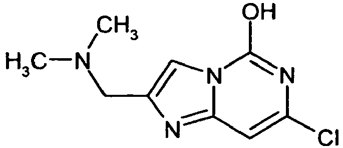

In Analogie zu Beispiel 33A werden aus 150 mg (0.58 mmol) 1-[7-Chlor-5-(methylsulfanyl)imidazo[1,2-c]pyrimidin-2-yl]-N,N-dimethyl-methanamin (Beispiel 97A) durch Erhitzen in methanolischer Kaliumhydroxidlösung 150 mg (98% d. Th.) des Produktes erhalten.

LCMS (Methode 4): Rₜ = 0.97 min. (m/z = 227 (M+H)⁺)

### Beispiel 99A

1-(5,7-Dichlorimidazo[1,2-c]pyrimidin-2-yl)-N,N-dimethyl-methanamin

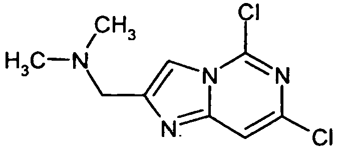

In Analogie zu Beispiel 11A werden aus 131 mg (0.58 mmol) 7-Chlor-2-[(dimethylamino)methyl]imidazo[1,2-c]pyrimidin-5-ol (Beispiel 98A) durch Umsetzung mit Phosphorylchlorid 30 mg (18% d. Th.) des Produktes erhalten.

LCMS (Methode 4): Rₜ = 1.95 min. (m/z = 245 (M+H)⁺)

### Beispiel 100A

N⁶-[2-({7-Chlor-2-[(dimethylamino)methyl]imidazo[1,2-c]pyrimidin-5-yl}amino)ethyl]-3-nitro-pyridin-2,6-diamin

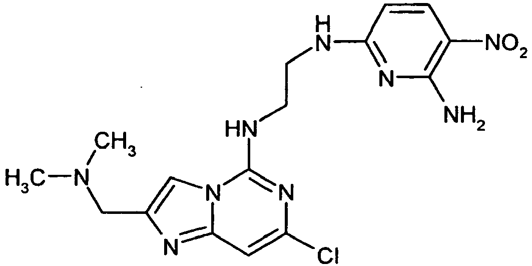

Analog der Herstellung von Beispiel 17A werden aus 147 mg (0.58 mmol) 1-(5,7-Dichlorimidazo[1,2-c]pyrimidin-2-yl)-N,N-dimethylmethanamin (Beispiel 99A) und 228 mg (0.638 mmol) N⁶-(2-Aminoethyl)-3-nitropyridin-2,6-diamin Trifluoracetat (Beispiel 15A) 11 mg (5% d. Th.) des Produktes erhalten.

LCMS (Methode 1): Rₜ = 1.00 min. (m/z = 406 (M+H)⁺)

### Beispiel 101A

7-Chlor-2-ethyl-5-(methylsulfanyl)[1,2,4]triazolo[1,5-c]pyrimidin

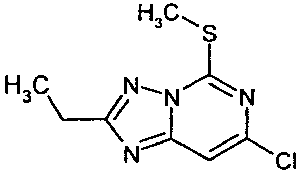

In 15 ml Orthopropionsäuretriethylester und 10 ml Propionsäure werden 3 g (13.2 mmol) 4-Chlor-6-hydrazino-2-(methylthio)pyrimidin (Beispiel 50A) gelöst und für 1 h unter Rückfluß erhitzt. Man gibt den Ansatz auf Wasser, stellt mit Natronlauge pH=7 ein und extrahiert dreimal mit Dichlormethan. Die gesammelten organischen Phasen werden mit Magnesiumsulfat getrocknet und das Lösungsmittel vollständig entfernt. Man erhält 2.3 g (54% d. Th.) des Produktes als Feststoff, der ohne weitere Aufreinigung eingesetzt wird.

LCMS (Methode 9): Rₜ = 1.64 min. (m/z = 229 (M+H)⁺).

¹H-NMR (400MHz, DMSO-d₆): δ = 7.64 (s, 1H), 3.38 (q, 2H), 2.70 (s, 3H), 1.41 (t, 3H).

### Beispiel 102A

7-Chlor-2-ethyl[1,2,4]triazolo[1,5-c]pyrimidin-5-ol

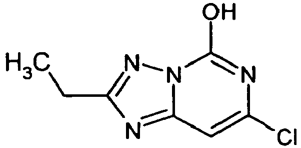

In Analogie zu Beispiel 33A werden aus 280 mg (1.22 mmol) 7-Chlor-2-ethyl-5-(methylsulfanyl)[1,2,4]triazolo[1,5-c]pyrimidin (Beispiel 101A) durch Erhitzen in methanolischer Kaliumhydroxidlösung 242 mg (99% d. Th.) des Produktes erhalten.

LCMS (Methode 3): Rₜ = 0.86 min. (m/z = 199 (M+H)⁺)

### Beispiel 103A

5,7-Dichlor-2-ethyl[1,2,4]triazolo[1,5-c]pyrimidin

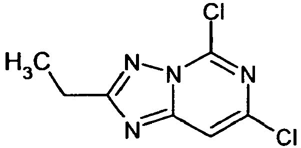

In Analogie zu Beispiel 11A werden aus 312 mg (1.57 mmol) 7-Chlor-2-ethyl[1,2,4]triazolo[1,5-c]pyrimidin-5-ol (Beispiel 102A) durch Umsetzung mit Phosphorylchlorid 285 mg (80% d. Th.) des Produktes erhalten.

LCMS (Methode 9): Rₜ = 1.61 min. (m/z = 217 (M+H)⁺)

¹H-NMR (400MHz, DMSO-d₆): δ = 8.11 (s, 1H), 2.90 (q, 2H), 1.33 (t, 3H).

### Beispiel 104A

6-({2-[(7-Chlor-2-ethyl[1,2,4]triazolo[1,5-c]pyrimidin-5-yl)amino]ethyl}amino)pyridin-3-carbonitril Trifluoracetat

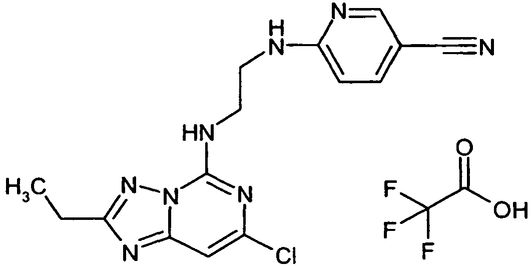

In Analogie zu Beispiel 54A werden aus 150 mg (0.62 mmol) 5,7-Dichlor-2-ethyl[1,2,4]triazolo[1,5-c]pyrimidin (Beispiel 103A) durch Umsetzung mit 297 mg (1.07 mmol) 6-[(2-Aminoethyl)amino]pyridin-3-carbonitril 64 mg (23% d. Th.) des Produktes erhalten.

LCMS (Methode 3): Rₜ = 2.17 min. (m/z = 343 (M+H)⁺).

¹H-NMR (400MHz, DMSO-d₆): δ = 8.64 (t, 1H), 8.35 (d, 1H), 7.75 (m, 1H), 7.68 (m, 1H), 6.99 (s, 1H), 6.55 (s, br, 1H), 3.55-3.68 (m, 4H), 2.81 (q, 2H), 1.31(t, 3H).

### Beispiel 105A

tert-Butyl-(6-chlorpyridin-2-yl)carbamat 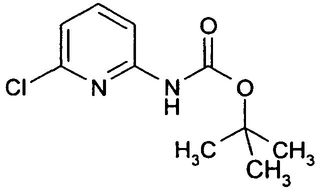 23.4 g (181.8 mmol) 2-Chlor-5-aminopyridin werden unter Argon mit 150 ml THF versetzt und auf 0°C gekühlt. Es werden 73.3 g (400 mmol) Bis-(trimethylsilyl)-natriumamid und 43.65 g (200 mmol) Di-tert-butyldicarbonat, gelöst in 150 ml THF, zugetropft. Nach 15 min wird das Kühlbad entfernt und weitere 15 min bei RT nachgerührt. Das THF wird abrotiert und der Rückstand mit Essigsäureethylester und 0.5 N Salzsäure versetzt und extrahiert. Die organische Phase wird abgetrennt, über Magnesiumsulfat getrocknet und am Rotationsverdampfer eingeengt. Man chromatographiert das Reaktionsgemisch an Kieselgel (Laufmittel Dichlormethan/Methanol 100% → 100:3). Es werden 36.54 g (88% d. Th.) des Produktes als Feststoff erhalten.

LCMS (Methode 3): Rₜ = 2.41 min. (m/z = 175 (M+H)⁺).

¹H-NMR (400MHz, DMSC)-d₆): δ = 10.11 (s, 1H), 7.78 (d, 2H), 7,1 (t, 1H), 1.47 (s, 9H).

### Beispiel 106A

tert-Butyl-(6-chlor-3-formylpyridin-2-yl)carbamat 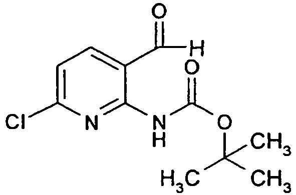 Die Reaktion läuft unter Argon, die Aparatur wird ausgeheizt und mit einem KPG-Rührwerk betrieben. 15 g (65.6 mmol) tert-Butyl-(6-chlorpyridin-2-yl)carbamat (Beispiel 25A) und 19 g (164 mmol) 1,2-Bis(dimethylamino)ethan werden in 270 ml THF vorgelegt und auf -78 °C gekühlt. Es werden 102.5 ml (164 mmol) Butyllithium (1.6N) zugetropft. Nach Beendigung des Zutropfens wird die Reaktion langsam auf -10°C erwärmt und bei -10 °C für 2 h gehalten, dann wieder auf -78 °C gekühlt und 10 ml (131 mmol) DMF werden zugegeben. Die Reaktion wird langsam auf RT erwärmt und das Reaktionsgemisch wird auf 11 Essigsäureethylester und 350 ml 1N Salzsäure gegeben, 15 min nachgerührt und die organische Phase wird abgetrennt. Es wird mit Wasser und gesättigter Natriumhydrogencarbonat-Lösung gewaschen, über Magnesiumsulfat getrocknet und am Rotationsverdampfer eingeengt. Der Rückstand wird mit Diethylether versetzt und der Feststoff wird abgesaugt und nachgetrocknet. Es werden 12.3 g (73% d. Th.) des Produktes als Feststoff erhalten.

LCMS (Methode 3): Rₜ = 2.19 min. (m/z = 255 (M+H)⁻).

¹H-NMR (400MHz. DMSO-d₆): δ = 10.37 (s, 1H), 9.83 (s, 1H), 8.2 (d, 1H), 7.42 (d, 1H), 1.46 (s, 9H).

### Beispiel 107A

tert-Butyl-{6-chlor-3-[(hydroxyimino)methyl]pyridin-2-yl}carbamat 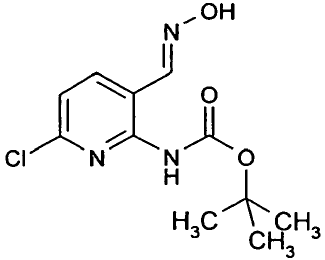 15.45 g (60.2 mmol) tert-Butyl-(6-chlor-3-formylpyridin-2-yl)carbamat (Beispiel 106A) werden in 750 ml Ethanol vorgelegt und mit einer Lösung aus 225 ml Wasser und 9.38 g (120.4 mmol) Natriumacetat versetzt und 5 min gerührt. Eine Lösung aus 225 ml Wasser und 8.36 g (114.4 mmol) Hydroxylamin-Hydrochlorid wird zugegeben und 4 h bei RT gerührt. Bei 20°C wird das Reaktionsgemisch am Rotationsverdampfer eingeengt. Der Rückstand wird in Essigsäureethylester aufgenommen und zweimal mit gesättigter Natriumhydroxydcarbonat-Lösung und einmal mit gesättigter Natriumchlorid-Lösung gewaschen. Die organische Phase wird abgetrennt, getrocknet über Magnesiumsulfat und bei 20°C am Rotationsverdampfer eingeengt. Es werden 15.5 g (80% d. Th.) des Produktes als Feststoff erhalten.

LCMS (Methode 3): Rₜ = 2.08 min. (m/z = 270 (M+H)⁻).

¹H-NMR (400MHz, DMSO-d₆): δ = 11.71 (s, 1H), 9.91 (s, 1H), 8.14 (s, 1H), 8.02 (d, 1H), 7.3 (d, 1H), 1.49 (s, 9H).

### Beispiel 108A

2-Amino-6-chlorpyridin-3-carbaldehydoxim Hydrochlorid 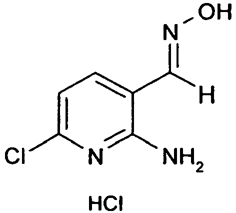 15.5 g (57 mmol) tert-Butyl-{6-chlor-3-[(hydroxyimino)methyl]pyridin-2-yl}carbamat (Beispiel 107A) werden in 285 ml 4N Chlorwasserstoff in Dioxan gelöst und 30 min nachgerührt. Man engt das Reaktionsgemisch um die Hälfte ein und gibt den gleichen Teil an Diethylether zu. Das Reaktionsgemisch wird 20 min nachgerührt und das Produkt abfiltriert und mit Diethylether nachgewaschen. Es werden 11 g (94% d. Th.) des Produktes als Feststoff erhalten.

LCMS (Methode 6): Rₜ = 1.09 min. (m/z = 172 (M+H)⁺)

¹H-NMR (400MHz, DMSO-d₆): δ = 8.27 (s, 1H), 7.61 (d, 1H), 6.65 (d, 1H).

### Beispiel 109A

2-Amino-6-chlorpyridin-3-carbonitril 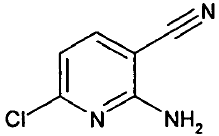 11.15 g (53.6 mmol) 2-Amino-6-chlorpyridin-3-carbaldehydoxim Hydrochlorid (Beispiel 108A) werden in Dioxan vorgelegt, mit 13 ml (161 mmol) Pyridin versetzt und auf 0°C abgekühlt. Es werden 8.3 ml (58.95 mmol) Trifluoressigsäureanhydrid zugegeben, man lässt die Reaktion auf RT erwärmen und anschließend wird 2 h bei 60°C gerührt. Das Reaktionsgemisch wird in einem Gemisch von Essigsäureethylester und Natriumhydrogencarbonat-Lösung aufgenommen. Die organische Phase wird mit gesättigter wässriger Natriumchloridlösung gewaschen, über Magnesiumsulfat getrocknet und am Rotationsverdampfer eingeengt. Der Rückstand wird in Dichlormethan:Diethylether 3:1 suspendiert, und der Feststoff wird abgesaugt und getrocknet. Es werden 5.56 g (66% d. Th.) des Produktes als Feststoff erhalten.

LCMS (Methode 6): Rₜ = 1.0 min. (m/z = 154 (M+H)⁺).

¹H-NMR (400MHz, DMSO-d₆): δ = 7.91 (d, 1H), 7.38 (s, 2H), 6.69 (d, 1H).

### Beispiel 110A

tert-Butyl-{2-[(6-amino-5-cyanopyridin-2-yl)amino]ethyl}carbamat 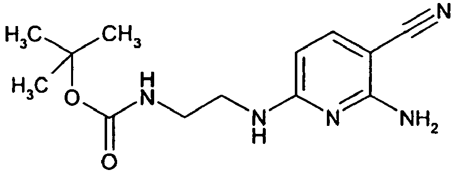 2 g (13 mmol) 2-Amino-6-chlorpyridin-3-carbonitril (Beispiel 109A) werden in 15 ml DMSO vorgelegt und mit 2.71 g (16.93 mmol) N-Boc-Ethylenamin und 3.4 ml (19.54 mmol) N,N-Diisopropylethylamin versetzt. Das Reaktionsgemisch wird 1.5 h bei 115°C in dem Mikrowellenreaktor bestrahlt. Das Reaktionsgemisch wird in einem Gemisch von Essigsäureethylester und Wasser aufgenommen. Die organische Phase wird mit gesättigter wässriger Natriumchloridlösung gewaschen, über Magnesiumsulfat getrocknet und am Rotationsverdampfer eingeengt. Es werden 23.38 g (88% d. Th.) des Produktes als Feststoff erhalten.

LCMS (Methode 3): Rₜ = 1.7 min. (m/z = 278 (M+H)⁺).

¹H-NMR (400MHz, DMSO-d₆): δ = 7.3 (s, 1H), 7.0 (br, s, 1H), 6.83 (s, 1H), 6.25 (s, 2H), 5.78 (d, 1H), 3.25 (q, 2H), 3.06 (q, 2H), 1.36 (s, 9H).

### Beispiel 111A

2-Amino-6-[(2-aminoethyl)amino]pyridin-3-carbonitril Dihydrochlorid 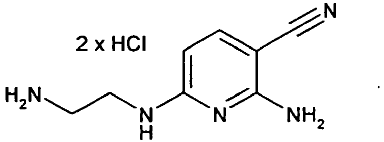 6.76 g (24.38 mmol) tert-Butyl-{2-[(6-amino-5-cyanopyridin-2-yl)amino]ethyl}carbamat (Beispiel 110A) werden in 122 ml 4N Chlorwasserstoff in Dioxan gelöst und 30 min nachgerührt. Man engt das Reaktionsgemisch um die Hälfte ein und gibt den gleichen Teil an Diethylether zu. Das Reaktionsgemisch wird 20 min nachgerührt und das Produkt abfiltriert und mit Diethylether nachgewaschen. Es werden 5.43 g (89% d. Th.) des Produktes als Feststoff erhalten.

LCMS (Methode 6): Rₜ = 0.92 min. (m/z = 177 (M+H)⁺)

¹H-NMR (400MHz, DMSO-d₆): δ = 8.1 (s, 2H), 7.5 (d, 1H), 5.96 (d, 1H), 3.53 (q, 2H), 3.0 (q, 2H).

### Beispiel 112A

4-(Trifluoracetyl)morpholin 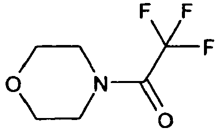 15 g (172 mmol) Morpholin werden in 750 ml Dichlormethan vorgelegt und es werden bei 0°C 29 ml (206 mmol) Trifluoressigsäureanhydrid und 119 ml (688 mmol) N,N-Diisopropylethylamin zugegeben. Das Reaktionsgemisch wird auf RT aufgetaut und 3 h bei RT nachgerührt. Das Reaktionsgemisch wird eingeengt und der Rückstand in Essigsäureethylester aufgenommen und nacheinander mit wässriger Natriumhydrogencarbonatlösung, 1N Salzsäure und wieder mit wässriger Natriumhydrogencarbonatlösung gewaschen. Die organische Phase wird über Magnesiumsulfat getrocknet und am Rotationsverdampfer eingeengt. Es werden 28 g (88% d. Th.) des Produktes als Öl erhalten.

LCMS (Methode 9): Rₜ = 1.22 min. (m/z = 184 (M+H)⁺)

¹H-NMR (400MHz, DMSO-d₆): δ = 3.65 (m, 2H), 3.56 (m, 2H).

### Beispiel 113A

tert-Butyl-[6-chlor-3-(trifluoracetyl)pyridin-2-yl]carbamat 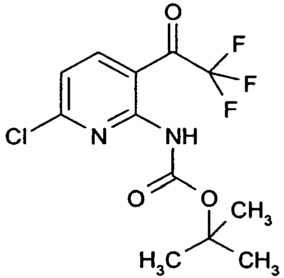 8 g (35 mmol) tert-Butyl-(6-chlorpyridin-2-yl)carbamat (Beispiel 105A) werden in 100 ml THF vorgelegt und auf -50°C gekühlt. Es werden 55 ml (87 mmol) Butyllithium (1.6N) zugetropft. Nach Beendigung des Zutropfens wird die Reaktion langsam auf -10°C erwärmt und bei 0°C für 2 h gehalten. Anschließend wird wieder auf -40°C abgekühlt und 12.8 g (70 mmol) 4-(Trifluoracetyl)morpholin (Beispiel 35A), gelöst in 4 ml THF, werden zugegeben. Die Reaktionslösung wird 1 h bei -40°C nachgerührt, danach bei - 40°C auf 11 Essigsäureethylester und 350 ml Ammoniumchloridlösung gegossen und extrahiert. Die organische Phase wird abgetrennt, über Magnesiumsulfat getrocknet und am Roationsverdampfer eingeengt. Man chromatographiert das Reaktionsgemisch an Kieselgel (Laufmittel Cyclohexan/ Essigsäureethylester 10:1). Es werden 9 g (79% d. Th.) des Produktes als Öl erhalten.

¹H-NMR (400MHz, DMSO-d₆): δ = 10.957 (s, 1H), 7.99 (d, 1H), 7.4 (d, 1H), 1.43 (s, 9H).

### Beispiel 114A

tert-Butyl-[6-({2-[(tert-butoxycarbonyl)amino]ethyl}amino)-3-(trifluoracetyl)pyridin-2-yl]-carbamat 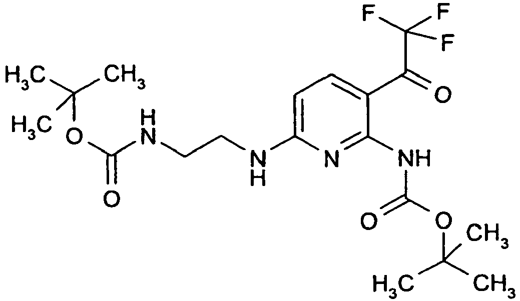 5 g (15.4 mmol) tert-Butyl-[6-chlor-3-(trifluoracetyl)pyridin-2-yl]carbamat (Beispiel 113A) werden in 37.5 ml DMSO vorgelegt und mit 3.2 g (20 mmol) N-Boc-Ethylendiamin und 4 ml (23 mmol) N,N-Diisopropylethylamin versetzt. Das Reaktionsgemisch wird 0.5 h bei 90°C in dem Mirkrowellenreaktor bestrahlt. Das Reaktionsgemisch wird in einem Gemisch von Essigsäureethylester und Wasser aufgenommen. Die organische Phase wird mit gesättigter wässriger Natriumchloridlösung gewaschen, über Magnesiumsulfat getrocknet und am Rotationsverdampfer eingeengt. Man chromatographiert das Reaktionsgemisch an Kieselgel (Laufmittel Cyclohexan/Essigsäureethylester 5:1 → 1:1). Es werden 2.5 g (34% d. Th.) des Produktes als Feststoff erhalten.

LCMS (Methode 6): Rₜ = 2.44 min. (m/z = 449 (M+H)⁺).

¹H-NMR (400MHz, DMSO-d₆): δ = 10.75 (s, 1H), 8.44 (s, 1H), 7.70 (d, 1H), 6.77 (s, 1H), 6.28 (d, 1H), 3.48 (br, s, 2H), 3.17 (br, s, 2H), 1.46 (s, 9H), 1.30 (s, 9H).

### Beispiel 115A

1-{2-Amino-6-[(2-aminoethyl)amino]pyridin-3-yl}-2,2,2-trifluorethanon Hydrochlorid 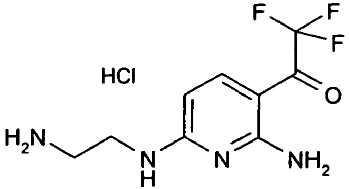 2.5 g (5.57 mmol) tert-Butyl-[6-({2-[(tert-butoxycarbonyl)amino]ethyl}amino)-3-(trifluoracetyl)-pyridin-2-yl]carbamat (Beispiel 114A) in 15 ml 4N Chlorwasserstoff in Dioxan gelöst und 20 h nachgerührt. Man engt das Reaktionsgemisch um die Hälfte ein und gibt den gleichen Teil an Diethylether zu. Das Reaktionsgemisch wird 20 min nachgerührt und das Produkt abfiltriert und mit Diethylether nachgewaschen. Es werden 1.4 g (89% d. Th.) des Produktes als Feststoff erhalten.

LCMS (Methode 6): Rₜ = 0.73 min. (m/z = 249 (M+H)⁺).

### Beispiel 116A

tert-Butyl-[6-chlor-3-acetylpyridin-2-yl]carbamat 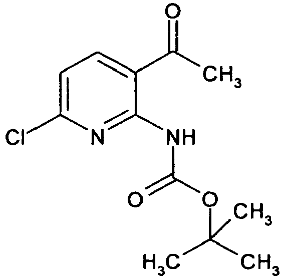 0.65 g (2.9 mmol) tert-Butyl-(6-chlorpyridin-2-yl)carbamat (Beispiel 105A) werden in 10 ml THF vorgelegt und auf -50°C gekühlt. Es werden 4.5 ml (7.2 mmol) Butyllithium (1.6 M) zugetropft. Nach Beendigung des Zutropfens wird die Reaktion langsam auf -10°C erwärmt und bei 0°C für 2 h gehalten. Anschließend wird wieder auf -40°C abgekühlt und 740 mg (5.7 mmol) N-Acetylmorpholin gelöst in 4 ml THF, werden zugegeben. Die Reaktionslösung wird 1 h bei -40°C nachgerührt, danach bei - 40°C auf 11 Essigsäureethylester und 350 ml Ammoniumchloridlösung gegossen und extrahiert. Die organische Phase wird abgetrennt, über Magnesiumsulfat getrocknet und am Roationsverdampfer eingeengt. Man chromatographiert das Reaktionsgemisch an Kieselgel (Laufmittel Cyclohexan/Essigsäureethylester 2:1). Es werden 218 mg (28% d. Th.) des Produktes als Öl erhalten.

LCMS (Methode 8): Rₜ = 1.16 min. (m/z = 269 (M-H)⁻)

### Beispiel 117A

tert-Butyl-{3-acetyl-6-[(2-{[7-(2,4-dichlorphenyl)imidazo[1,2-c]pyrimidin-5-yl]amino}ethyl)-amino]pyridin-2-yl}carbamat

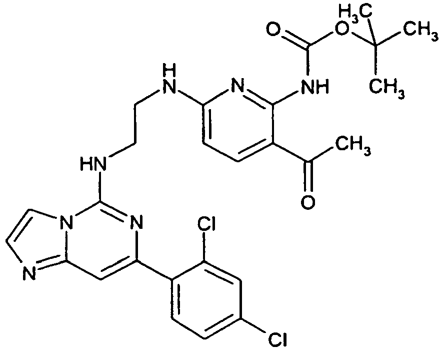

Die Verbindung wird wie in Beispiel 1 beschrieben aus N-[7-(2,4-Dichlorphenyl)imidazo[1,2-c]pyrimidin-5-yl]ethan-1,2-diamin-trifluoracetat (Beispiel 8A) und tert-Butyl-[6-chlor-3-acetylpyridin-2-yl]carbamat (Beispiel 116A) hergestellt.

LCMS (Methode 6): Rₜ = 1.61 min. (m/z = 556 (M+H-Boc)⁺)

### Beispiel 118A

Methyl-2-[(tert-butoxycarbonyl)amino]-6-chlorpyridin-3-carboxylat 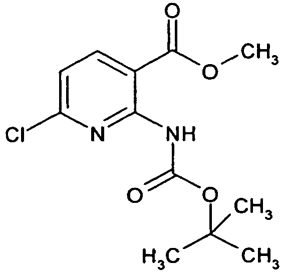 2.0 g (8.7 mmol) tert-Butyl-(6-chlorpyridin-2-yl)carbamat (Beispiel 105A) werden in 50 ml THF vorgelegt und auf -78°C gekühlt. Es werden 13.7 ml (22 mmol) Butyllithium (1.6 M) zugetropft. Nach Beendigung des Zutropfens wird die Reaktion langsam auf -10°C erwärmt und bei -10°C für 2 h gehalten. Anschließend wird wieder auf -78°C gekühlt und 870 mg (9.2 mmol) Chlorameisensäuremethylester werden zugegeben. Die Reaktionslösung wird über 12 h auf RT erwärmt, danach wird das Reaktionsgemisch auf 150 ml Essigsäureethylester und 80 ml Salzsäure-Lösung (1N) gegossen und 15 min nachgerührt. Die organische Phase wird abgetrennt, mit Wasser und gesättigter Natriumhydrogencarbonat-Lösung gewaschen, über Magnesiumsulfat getrocknet und am Roationsverdampfer eingeengt. Man chromatographiert das Reaktionsgemisch an Kieselgel (Laufmittel Cyclohexan/Essigsäureethylester 10:1). Es werden 1018 mg (33% d. Th.) des Produktes als Öl erhalten.

LCMS (Methode 8): Rₜ = 1.25 min. (m/z = 187 (M+H-Boc)⁺)

### Beispiel 119A

Methyl-2-[(tert-butoxycarbonyl)amino]-6-({2-[(tert-butoxycarbonyl)amino]ethyl}amino)pyridin-3-carboxylat

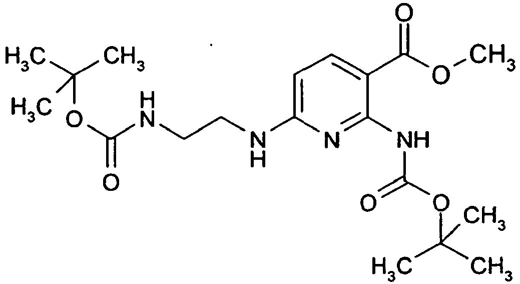

Die Verbindung wird hergestellt aus 650 mg (2.3 mmol) Methyl-2-[(tert-butoxycarbonyl)amino]-6-chlorpyridin-3-carboxylat (Beispiel 118A) und 363 mg (2.3 mmol) N-Boc-ethylendiamin wie in Beispiel 114A beschrieben. Es werden 500 mg (50% d. Th.) des Produktes als Feststoff erhalten.

LCMS (Methode 8): Rₜ = 1.32 min. (m/z = 411 (M+H)⁺).

### Beispiel 120A

Methyl-2-amino-6-[(2-aminoethyl)amino]pyridin-3-carboxylat Dihydrochlorid

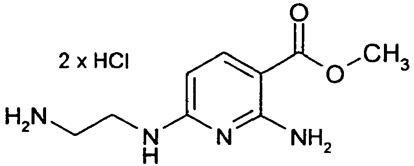

Die Verbindung wird hergestellt aus 496 mg (1.2 mmol) Methyl-2-[(tert-butoxycarbonyl)amino]-6-({2-[(tert-butoxycarbonyl)amino]ethyl}amino)pyridin-3-carboxylat wie in Beispiel 115A beschrieben. Es werden 363 mg (82% d. Th.) des Produktes als Feststoff erhalten.

LCMS (Methode 9): Rₜ = 0.75 min. (m/z = 212 (M+H-2HCl)⁺).

### Beispiel 121A

3-Brom-5-chlor-7-(2,4-dichlorphenyl)imidazo[1,2-c]pyrimidin 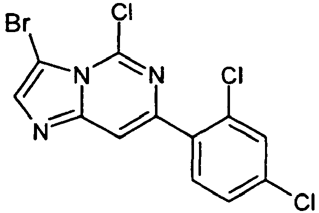 200 mg (0.67 mmol) 5-Chlor-7-(2,4-dichlorphenyl)imidazo[1,2-c]pyrimidin (Beispiel 6A) werden in Chloroform (30 ml) suspendiert, 238 mg (1.34 mmol) N-Bromsuccinimid werden zugegeben, und das Reaktionsgemisch wird 12 h bei RT nachgerührt. Das Reaktionsgemisch wird mit Ethylacetat (100 ml) und Wasser (75 ml) verdünnt, und die organische Phase wird abgetrennt, über Magnesiumsulfat getrocknet und am Roationsverdampfer eingeengt. Der Rückstand wird mit Acetonitril (20 ml) und anschließend Diethylether (20 ml) verrührt und der Feststoff wird abgesaugt und getrocknet. Es werden 103 mg (41% d. Th.) des Produktes isoliert.

LCMS (Methode 3): Rₜ = 2.78 min. (m/z = 378 (M+H)⁺)

### Beispiel 122A

3,5-Dichlor-7-(2,4-dichlorphenyl)imidazo[1,2-c]pyrimidin 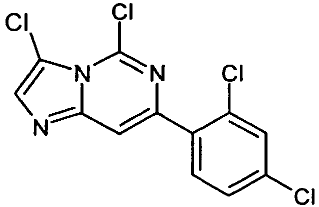 300 mg (1.0 mmol) 5-Chlor-7-(2,4-dichlorphenyl)imidazo[1,2-c]yrimidin (Beispiel 6A) werden in Chloroform (15 ml) suspendiert, 240 mg (1.8 mmol) N-Chlorsuccinimid werden zugegeben, und das Reaktionsgemisch wird 12 h bei RT nachgerührt. Das Reaktionsgemisch wird mit Ethylacetat (100 ml) und Wasser (75 ml) verdünnt, und die organische Phase wird abgetrennt, über Magnesiumsulfat getrocknet und am Roationsverdampfer eingeengt. Der Rückstand wird mit Diethylether (20 ml) verrührt und der Feststoff wird abgesaugt und getrocknet. Es werden 63 mg (18% d. Th.) des Produktes isoliert.

LCMS (Methode 8): Rₜ = 1.42 min. (m/z = 334 (M+H)⁺)

### Beispiel 123A

3,8-Dibrom-7-(2,4-dichlorphenyl)imidazo[1,2-c]pyrimidin-5-ol 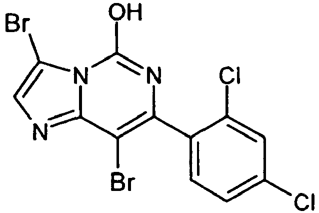 500 mg (1.8 mmol) 7-(2,4-Dichlorphenyl)imidazo[1,2-c]pyrimidin-5-ol (Beispiel 5A) werden in Chloroform (25 ml) suspendiert, 314 mg (1.8 mmol) N-Bromsuccinimid werden zugegeben, und das Reaktionsgemisch wird 12 h bei RT nachgerührt. Der Feststoff wird abfiltriert, das Filtrat wird mit Ethylacetat (100 ml) und Wasser (75 ml) verdünnt, und die organische Phase wird abgetrennt, über Magnesiumsulfat getrocknet und am Roationsverdampfer eingeengt. Der Rückstand wird mittels präparativer HPLC gereinigt. Man erhält 101 mg (12% d. Th.) des Produktes.

LCMS (Methode 8): Rₜ = 1.12 min. (m/z = 438 (M+H)⁺)

### Beispiel 124A

3,8-Dichlor-7-(2,4-dichlorphenyl)imidazo[1,2-c]pyrimidin-5-ol 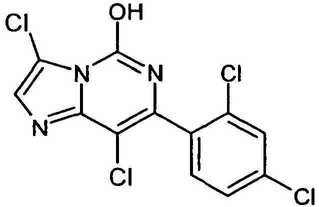 500 mg (1.8 mmol) 7-(2,4-Dichlorphenyl)imidazo[1,2-c]pyrimidin-5-ol (Beispiel 5A) werden in Chloroform (15 ml) suspendiert, 477 mg (3.6 mmol) N-Chlorsuccinimid werden zugegeben, und das Reaktionsgemisch wird 12 h bei 50°C nachgerührt. Das Reaktionsgemisch wird bei RT auf Ethylacetat (100 ml) gegeben und mit Wasser (75 ml) verdünnt, und die organische Phase wird abgetrennt, über Magnesiumsulfat getrocknet und am Roationsverdampfer eingeengt. Der Rückstand wird in Acetonitril verrührt und filtriert, und das Filtrat wird mittels präparativer HPLC gereinigt. Man erhält 214 mg (26% d. Th.) des Rohproduktes, das ohne weitere Reinigung weiter umgesetzt wird.

LCMS (Methode 8): Rₜ = 1.11 min. (m/z = 350 (M+H)⁺)

### Beispiel 125A

3,8-Dibrom-5-chlor-7-(2,4-dichlorphenyl)imidazo[1,2-c]pyrimidin 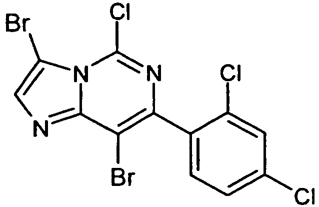 575 mg (1.3 mmol) 3,8-Dibrom-7-(2,4-dichlorphenyl)imidazo[1,2-c]pyrimidin-5-ol (Beispiel 123A) werden in Phosphorylchlorid (5 ml) vorgelegt, 900 mg (4 mmol) Benzyltriethylammoniumchlorid werden zugegeben, und das Reaktionsgemisch wird 12 h bei 120°C nachgerührt. Das Reaktionsgemisch wird langsam unter kräftigem Rühren auf gesättigte Natriumhydrogencarbonat-Lösung gegossen, und festes Natriumhydrogencarbonat (ca. 3 g) wird zugegeben, bis ein pH-Wert von 10 erreicht ist. Es wird 10 min nachgerührt, Ethylacetat (100 ml) wird zugesetzt und geschüttelt, und die organische Phase wird abgetrennt, mit Wasser gewaschen und über Magnesiumsulfat getrocknet und einrotiert. Der Rückstand wird mittels Kieselgelchromatographie (Cyclohexan-Ethylacetat 10:1) gereinigt. Man erhält 480 mg (80% d. Th.) des Produktes.

LCMS (Methode 3): Rₜ = 2.91 min. (m/z = 454 (M+H)⁺)

### Beispiel 126A

3,5,8-Trichlor-7-(2,4-dichlorphenyl)imidazo[1,2-c]pyrimidin 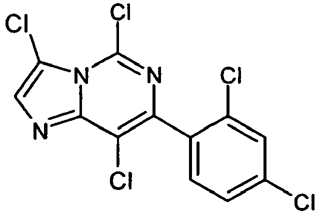 200 mg (0.4 mmol) 3,8-Dichlor-7-(2,4-dichlorphenyl)imidazo[1,2-c]pyrimidin-5-ol (Beispiel 124A) werden in Phosphorylchlorid (2 ml) vorgelegt, 300 mg (1.3 mmol) Benzyltriethylammoniumchlorid werden zugegeben, und das Reaktionsgemisch wird 12 h bei 120°C nachgerührt. Das Reaktionsgemisch wird langsam unter kräftigem Rühren auf gesättigte Natriumhydrogencarbonat-Lösung gegossen, und festes Natriumhydrogencarbonat (ca. 1.5 g) wird zugegeben, bis ein pH-Wert von 10 erreicht ist. Es wird 10 min nachgerührt, Ethylacetat (100 ml) wird zugesetzt und geschüttelt, und die organische Phase wird abgetrennt, mit Wasser gewaschen und über Magnesiumsulfat getrocknet und einrotiert. Der Rückstand wird mittels Kieselgelchromatographie (Cyclohexan-Ethylacetat 10:1) gereinigt. Man erhält 150 mg (94% d. Th.) des Produktes.

LCMS (Methode 8): Rₜ = 1.45 min. (m/z = 366 (M+H)⁺)

### Beispiel 127A

(2Z)-3-Amino-3-[4-(trifluormethyl)phenyl]prop-2-enonitril

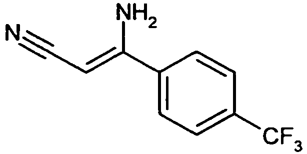

In einem Dreihalskolben mit mechanischem Rührer werden unter Argon 28.1 g (278 mmol) Diisopropylamin in 450 ml THF bei -70°C vorgelegt. 148 ml N-Butyllithium-Lösung (1.6 M in Hexan, 237 mmol) werden so schnell zugetropft, dass die Temperatur nicht über -60°C steigt. Man lässt 10 min nachrühren und tropft dann eine Lösung von 12.9 ml (245 mmol) Acetonitril in 100 ml THF langsam zu und lässt die Suspension für 30 min rühren. Dann wird eine Lösung von 28 g (164 mmol) 4-(Trifluormethyl)benzoesäurenitril in 100 ml THF zugetropft und 20 min bei -70°C nachgerührt. Man lässt langsam auf RT kommen und rührt für weitere 16 h bei RT. Es werden 150 ml Wasser zugegeben, der größte Teil des THF abdestilliert und mit Wasser und Dichlormethan versetzt. Die organische Phase wird mit gesättigter wässriger Natriumchloridlösung gewaschen. Nach Entfernen des Lösungsmittels erhält man dunkle Kristalle, die durch Verrühren mit Diisopropylether (einmal 40 ml, zweimal 20 ml) gereinigt werden. Die Kristalle werden abfiltriert, mit Petrolether gewaschen und getrocknet. Es werden 27 g (78% d. Th.) des Produktes als Feststoff erhalten.

LCMS (Methode 9): Rₜ = 2.05 min. (m/z = 213 (M+H)⁺)

¹H-NMR (400MHz, DMSO-d₆): δ = 7.79 (s, 4H), 7.98 (br s, 1H), 4.30 (s, 1H).

### Beispiel 128A

Ethyl-[2-cyan-1-(2,4-dichlorphenyl)ethenyl]carbamat 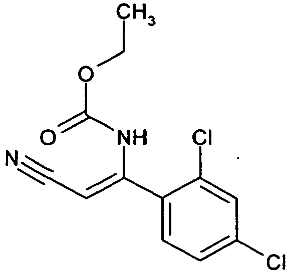 4.85 g (211 mmol) Natrium wird in Ethanol (260 ml) gelöst, 30.0 g (141 mmol) 3-Amino-3-(2,4-dichlorphenyl)acrylnitril sowie 36.59 g (310 mmol) Diethylcarbonat werden zugegeben, und die Reaktionslösung wird 4 h unter Rückfluss gerührt. Das Reaktionsgemisch wird mit Ethylacetat und Wasser versetzt, und der pH-Wert wird auf pH = 5 mit konzentrierter Salzsäure eingestellt. Die organische Phase wird abgetrennt, über Magnesiumsulfat getrocknet und einrotiert. Man chromatographiert den Rückstand an Kieselgel (Laufmittel Cyclohexan/Ethylacetat 7:1 bis 1:1), das Produkt hat einen R_{f} = 0.5 in Cyclohexan-Ethylaceat 1:1. Es werden 17.1 g (43% d. Th.) des Produktes erhalten.

LCMS (Methode 6): Rₜ = 1.87 min. (m/z = 285 (M+H)⁺)

¹H-NMR (400MHz, DMSO-d₆): δ = 10.35 (s, 1H), 7.79 (d, 1H), 7.54 (m, 2H), 6.16 (s, 1H), 4.13 (q, 2H), 1.22 (t, 3H).

### Beispiel 129A

Ethyl-{2-cyan-1-[4-(trifluormethyl)phenyl]ethenyl}carbamat 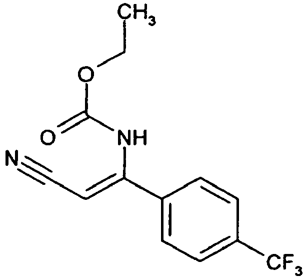 4.88 g (212 mmol) Natrium wird in Ethanol (260 ml) gelöst, 30.0 g (141 mmol) 3-Amino-3-(2,4-dichlorphenyl)acrylnitril sowie 36.75 g (311 mmol) Diethylcarbonat werden zugegeben, und die Reaktionslösung wird 5 h unter Rückfluss gerührt. Das Reaktionsgemisch wird mit Ethylacetat und Wasser versetzt, und der pH-Wert wird auf pH = 5 mit 2M Salzsäure eingestellt. Die organische Phase wird abgetrennt, über Magnesiumsulfat getrocknet und einrotiert. Man chromatographiert den Rückstand an Kieselgel (Laufmittel Cyclohexan/Ethylacetat 9:1 bis 2:1). Es werden 13.3 g (33% d. Th.) des Produktes als ein Gemisch von E- und Z-Isomeren erhalten.

LCMS (Methode 6): Rₜ = 1.92 min. (m/z = 285 (M+H)⁺)

### Beispiel 130A

7-(2,4-Dichlorphenyl)-2-(trifluormethyl)[1,2,4]triazolo[1,5-c]pyrimidin-5-ol 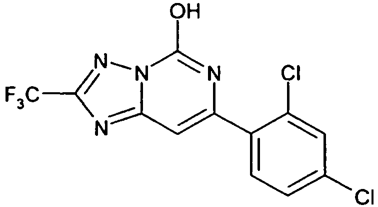 2.10 g (7.4 mmol) Ethyl-[2-cyan-1-(2,4-dichlorphenyl)ethenyl]carbamat (Beispiel 128A) und 0.95 g (7.4 mmol) 2,2,2-Trifluoracetohydrazid werden in NMP (10 ml) unter Argon gelöst und 4 h bei einer Ölbadtemperatur von 160°C in einem Kolben mit Calciumchlorid-Trockenrohr gerührt. Das Reaktionsgemisch wird auf RT abgekühlt und mit Wasser (20 ml) versetzt. Ethylacetat (150 ml) und Wasser (100 ml) werden zugegeben, die organische Phase wird abgetrennt, mit Magnesiumsulfat getrocknet und eingeengt. Man chromatographiert den Rückstand an Kieselgel (Laufmittel Cyclohexan/Ethylacetat 4:1 bis 1:1). Es werden 0.97 g (38% d. Th.) des Produktes erhalten.

LCMS (Methode 8): Rₜ = 1.17 min. (m/z = 349 (M+H)⁺)

¹H-NMR (400MHz, DMSO-d₆): δ = 12.82 (br s, 1H), 7.88 (d, 1H), 7.65 (m, 2H), 7.07 (s, 1H).

### Beispiel 131A

2-(Trifluormethyl)-7-[4-(trifluormethyl)phenyl][1,2,4]triazolo[1,5-c]pyrimidin-5-ol 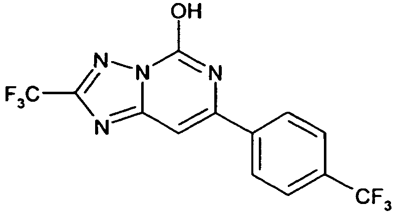 2.42 g (8.5 mmol) Ethyl-{2-cyan-1-[4-(trifluormethyl)phenyl]ethenyl}carbamat (Beispiel 129A) und 1.09 g (8.5 mmol) 2,2,2-Trifluoracetohydrazid werden in NMP (10 ml) gelöst und 4 h bei einer Ölbadtemperatur von 160°C in einem Kolben mit Calciumchlorid-Trockenrohr gerührt. Das Reaktionsgemisch wird auf RT abgekühlt und mit Ethylacetat (150 ml) und Wasser (100 ml) versetzt. Die organische Phase wird abgetrennt, mit Magnesiumsulfat getrocknet und eingeengt. Der Rückstand wird mit Diethylether (25 ml) verrührt, und der Feststoff wird abfiltriert und getrocknet. Es werden 0.67 g (23% d. Th.) des Produktes erhalten.

LCMS (Methode 3): Rₜ = 2.29 min. (m/z = 349 (M+H)⁺)

¹H-NMR (400MHz, DMSO-d₆): δ = 12.81 (br s, 1H), 8.05 (d, 2H), 7.95 (d, 2H), 7.37. (s, 1H).

### Beispiel 132A

Ethyl-7-(2,4-dichlorphenyl)-5-hydroxy[1,2,4]triazolo[1,5-c]pyrimidin-2-carboxylat 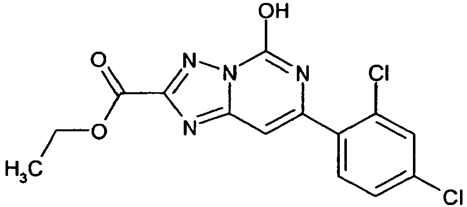 3.0 g (10.5 mmol) Ethyl-[2-cyan-1-(2,4-dichlorphenyl)ethenyl]carbamat (Beispiel 128A) und 1.39 g (10.5 mmol) Ethylhydrazino(oxo)acetat werden in NMP (16 ml) gelöst und 4 h bei einer Ölbadtemperatur von 160°C in einem Kolben mit Calciumchlorid-Trockenrohr gerührt. Das Reaktionsgemisch wird auf RT abgekühlt und mit Ethylacetat (150 ml) und Wasser (100 ml) versetzt. Die organische Phase wird abgetrennt, mit Magnesiumsulfat getrocknet und eingeengt. Man chromatographiert den Rückstand an Kieselgel (Laufmittel Cyclohexan/Ethylacetat 10:1 bis 1:1, dann mit Dichlormethan-Methanol 100:1). Das Rohprodukt wird eingeengt, mit Wasser versetzt und 1 h nachgerührt. Der Feststofff wird abgesaugt und getrocknet; es werden 1.31 g (35% d. Th.) des Produktes erhalten.

LCMS (Methode 8): Rₜ = 1.00 min. (m/z = 353 (M+H)⁺)

¹H-NMR (400MHz, DMSO-d₆): δ = 12.63 (br s, 1H), 7.88 (d, 1H), 7.63 (d, 2H), 6.99 (s, 1H), 4.42 (q, 2H), 1.46 (t, 3H).

### Beispiel 133A

Ethyl-5-hydroxy-7-[4-(trifluormethyl)phenyl][1,2,4]triazolo[1,5-c]pyrimidin-2-carboxylat 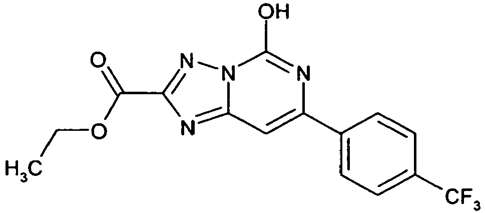 2.4 g (8.4 mmol) Ethyl-{2-cyan-1-[4-(trifluormethyl)phenyl]ethenyl}carbamat (Beispiel 129A) und 1.24 g (8.4 mmol) Ethylhydrazino(oxo)acetat werden in NMP (16 ml) gelöst und 4 h bei einer Ölbadtemperatur von 160°C in einem Kolben mit Calciumchlorid-Trockenrohr gerührt. Das Reaktionsgemisch wird auf RT abgekühlt und auf Wasser (100 ml) gegossen, und der Niederschlag wird abfiltriert, mit Wasser und Diethylether nachgewaschen und getrocknet. Es werden 1.90 g (64% d. Th.) des Rohproduktes erhalten.

LCMS (Methode 8): Rₜ = 1.0 min. (m/z = 353 (M+H)⁺)

### Beispiel 134A

7-(2,4-Dichlorphenyl)-2-(morpholin-4-ylmethyl)[1,2,4]triazolo[1,5-c]pyrimidin-5-ol 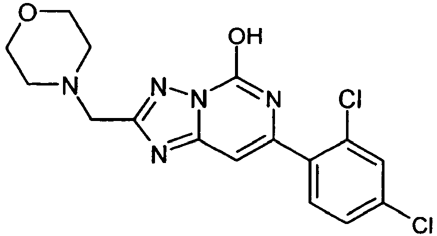 1.33 g (4.7 mmol) Ethyl-[2-cyan-1-(2,4-dichlorphenyl)ethenyl]carbamat (Beispiel 128A) und 740 mg (4.7 mmol) 2-Morpholin-4-ylacetohydrazid werden in NMP (6 ml) gelöst und 3 h bei einer Ölbadtemperatur von 160°C in einem Kolben mit Calciumchlorid-Trockenrohr gerührt. Das Reaktionsgemisch wird auf RT abgekühlt und mit Ethylacetat (100 ml) und Wasser (50 ml) versetzt. Die organische Phase wird abgetrennt, mit Magnesiumsulfat getrocknet und eingeengt. Man chromatographiert den Rückstand an Kieselgel (Laufmittel Dichlormethan-Methanol 100:1 bis 50:1). Es werden 1.25 g (70% d. Th.) des Produktes erhalten.

LCMS (Methode 8): Rₜ = 0.68 min. (m/z = 380 (M+H)⁺)

¹H-NMR (400MHz, DMSO-d₆): δ = 12.37 (br s, 1H), 7.85 (d, 1H), 7.64 (m, 2H), 6.85 (s, 1H), 4.44 (br s, 2H), 3.71 (s, 2H), 3.59 (m, 4H), 3.40 (s, 2H).

### Beispiel 135A

2-(Morpholin-4-ylmethyl)-7-[4-(trifluormethyl)phenyl][1,2,4]triazolo[1,5-c]pyrimidin-5-ol 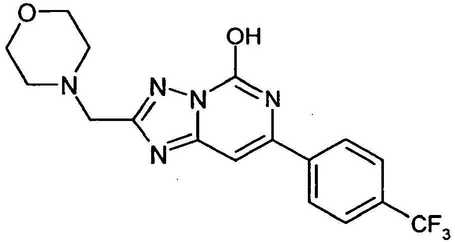 2.4 g (8.4 mmol) Ethyl-{2-cyan-1-[4-(trifluormethyl)phenyl]ethenyl}carbamat (Beispiel 129A) und 1.34 g (8.4 mmol) 2-Morpholin-4-ylacetohydrazid werden in NMP (12 ml) gelöst und 4 h bei einer Ölbadtemperatur von 190°C in einem Kolben mit Calciumchlorid-Trockenrohr gerührt. Das Reaktionsgemisch wird auf RT abgekühlt und mit Ethylacetat (150 ml) und Wasser (100 ml) versetzt. Die organische Phase wird mit gesättiger Natriumhydrogencarbonat-Lösung gewaschen, abgetrennt, mit Magnesiumsulfat getrocknet und eingeengt. Man chromatographiert den Rückstand an Kieselgel (Laufmittel Cyclohexan/Ethylacetat 10:1 bis 1:1, dann mit Dichlormethan-Methanol 100:1). Es werden 850 mg (26% d. Th.) des Produktes erhalten.

LCMS (Methode 8): Rₜ = 0.74 min. (m/z = 380 (M+H)⁺)

¹H-NMR (400MHz, DMSO-d₆): δ = 12.36 (br s, 1H), 8.05 (d, 2H), 7.92 (d, 2H), 7.18 (s, 1H), 4.45 (m, 2H), 3.70 (s, 2H), 3.60 (m, 4H), 3.29 (s, 2H).

### Beispiel 136A

7-(2,4-Dichlorphenyl)-2-methyl[1,2,4]triazolo[1,5-c]pyrimidin-5-ol

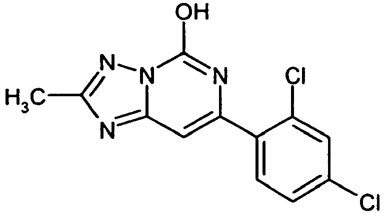

In Analogie zu Beispiel 11A werden aus 1000 mg (3.5 mmol) Ethyl-[(Z)-2-cyano-1-(2,4-dichlorphenyl)ethenyl]carbamat (Beispiel 129A) durch Umsetzung mit 289 mg (3.5 mmol) Acetohydrazid 686 mg (60% d. Th.) des Produktes erhalten.

LCMS (Methode 3): Rₜ = 1.75 min. (m/z = 295 (M+H)⁺)

¹H-NMR (400MHz, DMSO-d₆): δ = 12.35 (s, 1H), 7.84 (d, 1H), 5.57-7.66 (m, 2H), 6.79 (s, 1H), 2.43 (s, 3H).

### Beispiel 137A

7-(2,4-Dichlorphenyl)[1,2,4]triazolo[1,5-c]pyrimidin-5-ol 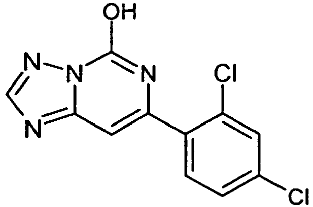 500 mg (1.75 mmol) Ethyl-[2-cyan-1-(2,4-dichlorphenyl)ethenyl]carbamat (Beispiel 128A) und 182 mg (1.75 mmol) Hydrazino(oxo)essigsäure werden in NMP (2 ml) unter Argon gelöst und 4 h bei einer Ölbadtemperatur von 160°C in einem Kolben mit Calciumchlorid-Trockenrohr gerührt. Das Reaktionsgemisch wird auf RT abgekühlt, mit Wasser (20 ml) versetzt und 20 min nachgerührt. Ethylacetat (150 ml) und Wasser (100 ml) werden zugegeben, die organische Phase wird abgetrennt, mit Magnesiumsulfat getrocknet und eingeengt. Der Rückstand wird mit Diethylether/Dichlormethan verrührt, und der Feststoff wird abfiltriert und getrocknet. Es werden 150 mg (31% d. Th.) des Produktes erhalten.

LCMS (Methode 8): Rₜ = 0.85 min. (m/z = 281 (M+H)⁺)

¹H-NMR (400MHz, DMSO-d₆): δ = 12.45 (br s, 1H), 8.49 (s, 1H), 7.86 (d, 1H), 7.63 (m, 2H), 6.92 (s, 1H).

### Beispiel 138A

7-(4-Trifluormethylphenyl)[1,2,4]triazolo[1,5-c]pyrimidin-5-ol 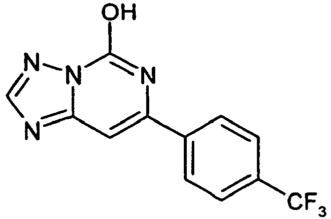 2.00 g (7.0 mmol) Ethyl-[2-cyan-1-(4-trifluormethylphenyl)ethenyl]carbamat (Beispiel 129A) und 0.73 g (7.0 mmol) Hydrazino(oxo)essigsäure werden in NMP (7 ml) unter Argon gelöst und 4 h bei einer Ölbadtemperatur von 160°C in einem Kolben mit Calciumchlorid-Trockenrohr gerührt. Das Reaktionsgemisch wird auf RT abgekühlt, mit Wasser (20 ml) versetzt und 20 min nachgerührt. Ethylacetat (150 ml) und Wasser (100 ml) werden zugegeben, die organische Phase wird abgetrennt, mit Magnesiumsulfat getrocknet und eingeengt. Der Rückstand wird 10 min in Wasser auf dem Ultraschallbad suspendiert, und anschließend 30 min nachgerührt. Der Feststoff wird abfiltriert und getrocknet. Es werden 0.97 g (38% d. Th.) des Produktes erhalten.

LCMS (Methode 3): Rₜ = 1.73 min. (m/z = 281 (M+H)⁺)

¹H-NMR (400MHz, DMSO-d₆): δ = 12.44 (br s, 1H), 8.47 (s, 1H), 8.05 (d, 2H), 7.92 (d, 2H), 7.26 (s, 1H).

Analog der für die vorangegangenen Beispiele beschriebenen Vorschriften werden folgende Produkte erhalten:

| **Bsp.** | **Struktur** | **LC/MS: Retentionszeit [min] (Methode)** | **Charakterisierung** | **Ausbeute [d. Th.]** |
|---|---|---|---|---|
| **139A** | 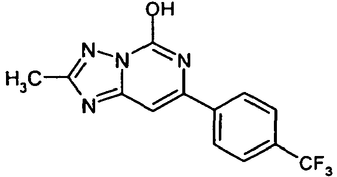 | 0.90 (Methode 8) | ¹H-NMR (400MHz, DMSO-d₆): δ = 12.31 (s, 1H), 8.04 (d, 2H), 7.90 (d, 2H), 7.13 (s, 1 H), 2.45 (s, 3H). | 18% |
| **140A** | 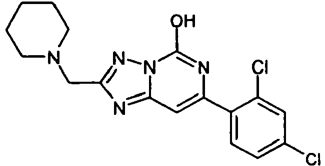 | 0.91 (Methode 6) | ¹H-NMR (400MHz, DMSO-d₆): δ = 12.28 (br s, 1H), 7.83 (d, 1H), 7.63 (m, 2H), 6.78 (s, 1H), 3.65 (s, 2H), 2.48 (m, 4H), 1.50 (m, 4H), 1.37 (m, 2H). | 96% |
| **141A** | 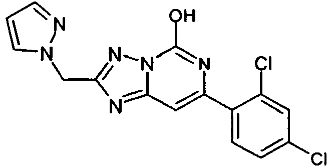 | 0.93 (Methode 8) | ¹H-NMR (400MHz, DMSO-d₆): δ = 12.44 (s, 1H), 7.87 (d, 1H), 7.85 (s, 1H), 7.62 (d, 2H), 7.46 (d, 1H), 6.85 (s, 1H), 6.30 (d, 1H), 5.57 (s, 2H). | 84% |
| **142A** | 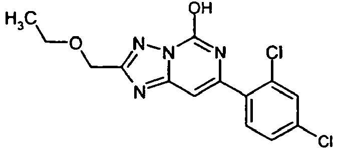 | 2.58 (Methode 3) | ¹H-NMR (400MHz, DMSO-d₆): δ = 12.40 (s, 1H), 7.85 (d, 1H), 7.60-7-66 (m, 2H), 6.86 (s, 1H), 4.60 (s, 2H), 3.57 (q, 2H), 1.15 (s, 3H). | - |
| **143A** | 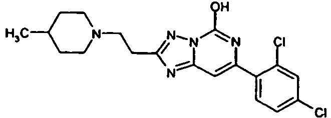 | 0.81 (Methode 8) | m/z = 406 (M+H)⁺ | 31% |
| **144A** | 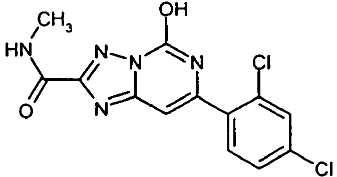 | 0.84 (Methode 8) | m/z=338(M+H)⁺ | 39% |
| **145A** | 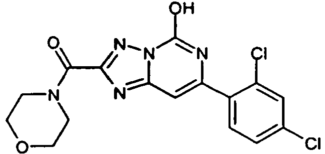 | 0.88 (Methode 8) | m/z = 394 (M+11)+ | 62% |

### Beispiel 146A

5-Chlor-7-(2,4-dichlorphenyl)-2-(trifluormethyl)[1,2,4]triazolo[1,5-c]pyrimidin 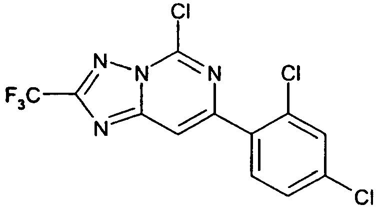 950 mg (2.7 mmol) 7-(2,4-Dichlorphenyl)-2-(trifluormethyl)[1,2,4]triazolo[1,5-c]pyrimidin-5-ol (Beispiel 130A) werden in Phosphorylchlorid (10 ml) vorgelegt, 1.86 g (8.2 mmol) Benzyltriethylammoniumchlorid wird zugegeben, und das Reaktionsgemisch wird 12 h bei 120°C nachgerührt. Das Reaktionsgemisch wird langsam unter kräftigem Rühren auf gesättigte Natriumhydrogencarbonat-Lösung und Eis gegossen, und festes Natriumhydrogencarbonat (ca. 5 g) wird zugegeben, bis ein pH-Wert von 8 erreicht ist. Der Feststoff wird abgesaugt. Das Reaktionsgefäß wird mit Dichlormethan ausgewaschen, und die organische Phase wird über Magnesiumsulfat getrocknet, eingeengt und mit dem abfiltrierten Feststoff vereinigt. Das Rohprodukt wird mittels Kieselgelchromatographie (Cyclohexan-Ethylacetat 10:1) gereinigt. Man erhält 784 mg (78% d. Th.) des Produktes.

LCMS (Methode 8): Rₜ = 1.50 min. (m/z = 367 (M+H)⁺)

### Beispiel 147A

5-Chlor-2-(trifluormethyl)-7-[4-(trifluormethyl)phenyl][1,2,4]triazolo[1,5-c]pyrimidin 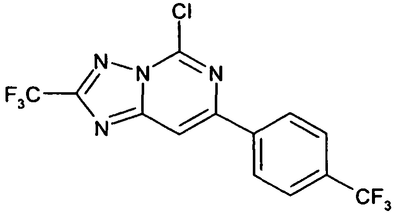 650 mg (1.9 mmol) 2-(Trifluormethyl)-7-[4-(trifluormethyl)phenyl][1,2,4]triazolo[1,5-c]pyrimidin-5-ol (Beispiel 131A) werden in Phosphorylchlorid (10 ml) vorgelegt, 1.28 g (5.6 mmol) Benzyltriethylammoniumchlorid werden zugegeben, und das Reaktionsgemisch wird 12 h bei 120°C nachgerührt. Das Reaktionsgemisch wird langsam unter kräftigem Rühren auf gesättigte Natriumhydrogencarbonat-Lösung (50 ml) und Eis gegossen, und festes Natriumhydrogencarbonat (ca. 1 g) wird zugegeben, bis ein pH-Wert von 8 erreicht ist. Der Feststoff wird abgesaugt. Man erhält 635 mg (93% d. Th.) des Rohproduktes.

LCMS (Methode 8): Rₜ = 1.48 min. (m/z = 367 (M+H)⁺)

¹H-NMR (400MHz, DMSO-d₆): δ = 8.90 (s, 18.46 (d, 2H), 7.97 (d, 2H).

### Beispiel 148A

Ethyl-5-chlor-7-(2,4-dichlorphenyl)[1,2,4]triazolo[1,5-c]pyrimidin-2-carboxylat 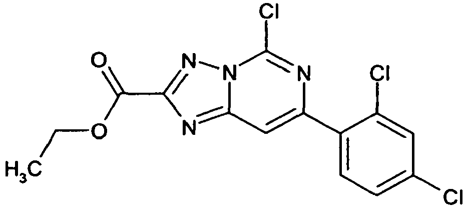 1311 mg (3.7 mmol) Ethyl-7-(2,4-dichlorphenyl)-5-hydroxy[1,2,4]triazolo[1,5-c]pyrimidin-2-carboxylat (Beispiel 132A) werden in Phosphorylchlorid (15 ml) vorgelegt, 2.54 g (11.1 mmol) Benzyltriethylammoniumchlorid werden zugegeben, und das Reaktionsgemisch wird 12 h bei 120°C nachgerührt. Das Reaktionsgemisch wird langsam unter kräftigem Rühren auf gesättigte Natriumhydrogencarbonat-Lösung (150 ml) und Eis gegossen, und festes Natriumhydrogencarbonat (ca. 10 g) wird zugegeben, bis ein pH-Wert von 8 erreicht ist. Der Feststoff wird abgesaugt. Man erhält 635 mg (93% d. Th.) des Produktes.

LCMS (Methode 3): Rₜ = 2.60 min. (m/z = 371 (M+H)⁺)

¹H-NMR (400MHz, DMSO-d₆): δ = 8.40 (s, 1H), 7.88 (d, 1H), 7.75 (d, 1H), 7.65 (dd, 1H), 4.47 (q, 2H), 1.40 (t, 3H).

### Beispiel 149A

Ethyl-5-chlor-7-[4-(trifluormethyl)phenyl][1,2,4]triazolo[1,5-c]pyrimidin-2-carboxylat 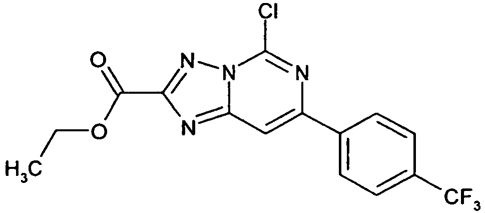 2300 mg (6.5 mmol) Ethyl-5-hydroxy-7-[4-(trifluormethyl)phenyl][1,2,4]triazolo[1,5-c]pyrimidin-2-carboxylat (Beispiel 133A) wird in Phosphorylchlorid (30 ml) vorgelegt, 5.95 g (26.1 mmol) Benzyltriethylammoniumchlorid wird zugegeben, und das Reaktionsgemisch wird 20 h bei 120°C nachgerührt. Das Reaktionsgemisch wird langsam unter kräftigem Rühren auf gesättigte Natriumhydrogencarbonat-Lösung (200 ml) und Eis gegossen, und festes Natriumhydrogencarbonat (ca. 20 g) wird zugegeben, bis ein pH-Wert von 8 erreicht ist. Der Feststoff wird abgesaugt, und mit Wasser und Diethylether gewaschen und getrocknet. Man erhält 2300 mg (92% d. Th.) des Produktes.

LCMS (Methode 3): Rₜ = 1.34 min. (m/z = 371 (M+H)⁺)

¹H-NMR (400MHz, DMSO-d₆): δ = 8.80 (s, 1H), 8.45 (d, 2H), 7.95 (d, 2H), 4.47 (q, 2H), 1.39 (t, 3H).

### Beispiel 150A

5-Chlor-7-(2,4-dichlorphenyl)-2-(morpholin-4-ylmethyl)[1,2,4]triazolo[1,5-c]pyrimidin 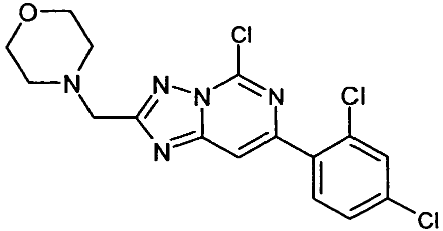 1250 mg (3.3 mmol) 7-(2,4-Dichlorphenyl)-2-(morpholin-4-ylmethyl)[1,2,4]triazolo[1,5-c]pyrimidin-5-ol (Beispiel 134A) wird in Phosphorylchlorid (20 ml) vorgelegt, 2.25 g (9.9 mmol) Benzyltriethylammoniumchlorid wird zugegeben, und das Reaktionsgemisch wird 12 h bei 120°C nachgerührt. Das Reaktionsgemisch wird langsam unter kräftigem Rühren auf gesättigte Natriumhydrogencarbonat-Lösung (150 ml) und Eis gegossen, und festes Natriumhydrogencarbonat (ca. 10 g) wird zugegeben, bis ein pH-Wert von 8 erreicht ist. Der Feststoff wird abgesaugt und getrocknet. Man erhält 508 mg (39% d. Th.) des Produktes.

LCMS (Methode 8): Rₜ = 1.01 min. (m/z = 398 (M+H)⁺)

### Beispiel 151A

5-Chlor-2-(morpholin-4-ylmethyl)-7-[4-(trifluormethyl)phenyl] [1,2,4]triazolo[1,5-c]pyrimidin 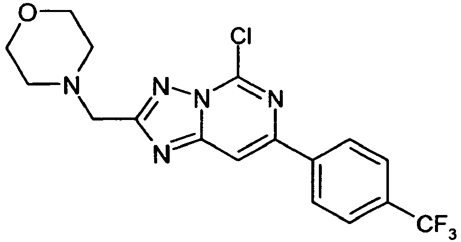 850 mg (2.2 mmol) 2-(Morpholin-4-ylmethyl)-7-[4-(trifluoromethyl)phenyl][1,2,4]triazolo-[1,5-c]pyrimidin-5-ol (Beispiel 135A) wird in Phosphorylchlorid (20 ml) vorgelegt, 1.98 g (8.7 mmol) Benzyltriethylammoniumchlorid wird zugegeben, und das Reaktionsgemisch wird 2 h bei 120°C nachgerührt. Das Reaktionsgemisch wird langsam unter kräftigem Rühren auf Eiswasser gegossen, und festes Natriumhydrogencarbonat wird zugegeben, bis ein pH-Wert von 8 erreicht ist. Das Reaktionsgemisch wird mit Ethylacetat (150 ml) extrahiert, und die organische Phase wird abgetrennt, getrocknet (Magnesiumsulfat) und eingeengt. Man chromatographiert den Rückstand an Kieselgel (Laufmittel Dichlormethan-Ethanol 100:1 bis 20:1). Es werden 650 mg (75% d. Th.) des Produktes als ein Feststoff erhalten.

LCMS (Methode 8): Rₜ = 1.04 min. (m/z = 398 (M+H⁺)

### Beispiel 152A

5-Chlor-7-(2,4-dichlorphenyl)-2-methyl[1,2,4]triazolo[1,5-c]pyrimidin

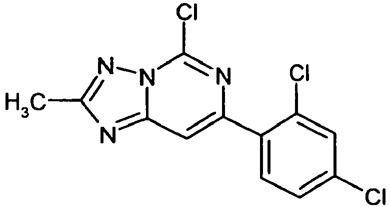

In Analogie zu Beispiel 53A werden aus 685 mg (2.31 mmol) 7-(2,4-Dichlorphenyl)-2-methyl[1,2,4]triazolo[1,5-c]pyrimidin-5-ol (Beispiel 136A) durch Umsetzung in 6 ml Phosphorylchlorid und 1.59 g (6.96 mmol) Benzyltriethylammoniumchlorid 676 mg (49% d. Th.) des Produktes erhalten.

LCMS (Methode 3): Rₜ = 2.44 min. (m/z = 313 (M+H)⁺).

¹H-NMR (400MHz, DMSO-d₆): δ = 8.14 (s, 1H), 7.84 (d, 1H), 7.73 (d, 1H), 7.62 (dd, 1H), 2.58 (s, 3H).

### Beispiel 153A

5-Chlor-7-[4-(trifluormethyl)phenyl] [1,2,4]triazolo[1,5-c]pyrimidin 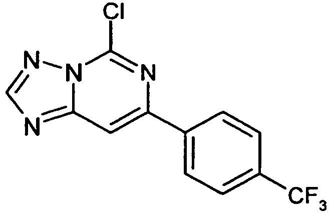 1320 mg (4.7 mmol) 7-[4-(Trifluormethyl)phenyl][1,2,4]triazolo[1,5-c]pyrimidin-5-ol (Beispiel 138A) wird in Phosphorylchlorid (20 ml) vorgelegt, 3.20 g (14 mmol) Benzyltriethylammoniumchlorid wird zugegeben, und das Reaktionsgemisch wird 4 h bei 120°C nachgerührt. Das Reaktionsgemisch wird langsam unter kräftigem Rühren auf gesättigte Natriumhydrogencarbonat-Lösung (150 ml) und Eis gegossen, und festes Natriumhydrogencarbonat (ca. 10 g) wird zugegeben, bis ein pH-Wert von 8 erreicht ist. Der Feststoff wird abgesaugt. Man erhält 1300 mg (92% d. Th.) des Rohproduktes.

LCMS (Methode 6): Rₜ = 2.00 min. (m/z = 299 (M+H)⁺)

¹H-NMR (400MHz, DMSO-d₆): δ = 8.81 (s, 1H), 8.74 (s, 1H), 8.45 (d, 2H), 7.94 (d, 2H).

### Beispiel 154A

5-Chlor-7-[2,4-dichlorphenyl][1,2,4]triazolo[1,5-c]pyrimidin 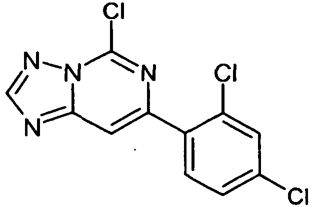 2.07 g (7.4 mmol) 7-[2,4-Dichlorphenyl][1,2,4]triazolo[1,5-c]pyrirnidin-5-ol (Beispiel 137A) wird in Phosphorylchlorid (20 ml) vorgelegt, 5.0 g (22 mmol) Benzyltriethylammoniumchlorid wird zugegeben, und das Reaktionsgemisch wird 16 h bei 120°C nachgerührt. Das Reaktionsgemisch wird eingeengt und vorsichtig unter kräftigem Rühren auf Eis gegossen, und es wird 10 min nachgerührt. Der Feststoff wird abgesaugt. Man erhält 1100 mg (50% d. Th.) des Produktes.

LCMS (Methode 3): Rₜ = 2.39 min. (m/z = 299 (M+H)⁺)

¹H-NMR (400MHz, DMSO-d₆): δ = 8.84 (s, 1H), 8.29 (s, 1H), 7.85 (d, 1H), 7.75 (d, 1H), 7.64 (dd, 1H).

Analog der für die vorangegangenen Beispiele beschriebenen Vorschriften werden folgende Produkte erhalten:

| **Bsp.** | **Struktur** | **LC/MS: Retentionszeit [min] (Methode)** | **Charakterisierung** | **Ausbeute [d. Th.]** |
|---|---|---|---|---|
| **155A** | 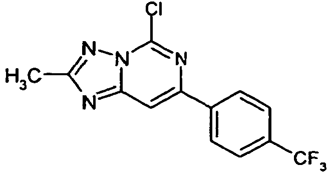 | 1.28 (Methode 8) | ¹H-NMR (400MHz, DMSO-d₆): δ = 8.59 (s, 1H), 8.43 (d, 2H), 7.93 (d, 2H), 2.59 (s, 3H). | 82% |
| **156A** | 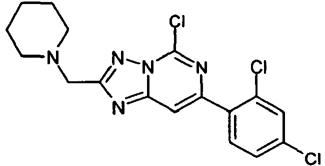 | 1.51 (Methode 3) | ¹H-NMR (400MHz, DMSO-d₆): δ = 10.54 (br s, 1H), 8.35 (s, 1H), 7.89 (d, 1H), 7.74 (d, 1H), 7.66 (dd, 1H), 4.74 (br s, 2H), 3.04 (br m, 2H), 3.07 (br m, 2H), 1.78 (br m, 6H). | 6% |
| **157A** | 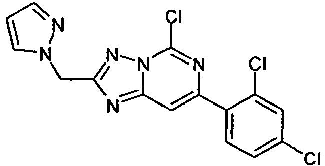 | 2.45 (Methode 3) | ¹H-NMR (400MHz, DMSO-d₆): δ = 8.22 (s, 1H), 7.93 (d, 1H), 7.84 (d, 1H), 7.73 (d, 1H), 7.63 (dd, 1H), 7.47 (d, 1H), 6.32 (t, 1H), 5.73 (s, 2H). | 73% |
| **158A** | 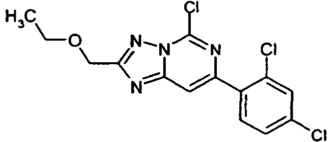 | 2.58 (Methode3) | ¹H-NMR (400MHz, DMSO-d₆): δ = 8.22 (s, 1H), 7.85 (d, 1H), 7.72 (d, 1H), 7.63 (dd, 1H), 4.75 (s, 2H), 3.62 (q, 2H), 1.17 (s, 3H). | - |
| **159A** | 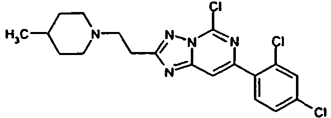 | 1.04 (Methode 8) | m/z = 424 (M+H)⁺ | 58% |
| **160A** | 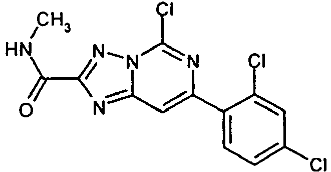 | 2.09 (Methode 9) | m/z = 356 (M+H)⁺ | 95% |
| **161A** | 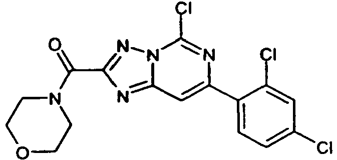 | 2.19 (Methode 9) | m/z = 412 (M+H)⁺ | 94% |

### Ausführungsbeispiele

### Beispiel 1

6-[(2-{[7-(2,4-Dichlorphenyl)imidazo[1,2-c]pyrimidin-5-yl]amino}ethyl)amino]nicotinnitril

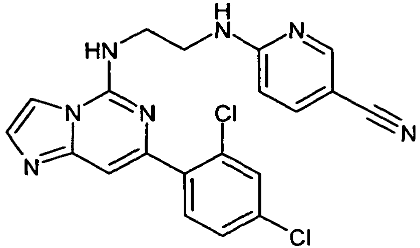

In 2 ml Isopropanol werden 50 mg (0.155 mmol) des Amins (Beispiel 8A) vorgelegt, mit 32.3 mg (0.233 mmol) 6-Chlornicotinonitril und 30.09 mg (0.233 mmol) DIPEA versetzt und bei 150°C für 1 h in der Mikrowelle erhitzt. Man erhält nach Reinigung mittels präparativer HPLC 47 mg (71% d. Th.) des Produktes.

LCMS (Methode 5): Rₜ = 2.34 min. (m/z = 424 (M+H)+)

¹H-NMR (400MHz, DMSO-d₆): δ = 8.36 (d, 1H), 8.05 (t, 1H), 7.98 (s, 1H), 7.76 (s, br, 1H), 7.71 (d, 1H), 7.65 (d, 1H), 7.60 (m, 1H), 7.58 (s, 1H), 7.50 (dd, 1H), 7.10 (s, 1H), 6.51 (d, 1H), 3.69 (t, 2H), 3.66 (t, br, 2H).

### Beispiel 2

N-[7-(2,4-Dichlorphenyl)imidazo[1,2-c]pyrimidin-5-yl]-N'-[5-(trifluormethyl)pyridin-2-yl]ethan-1,2-diamin

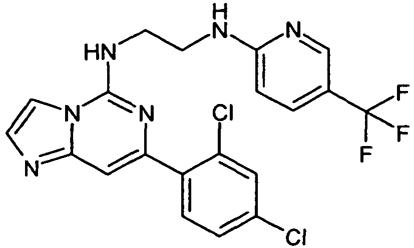

In 3 ml trockenem DMSO werden 50 mg (0.155 mmol) des Amins (Beispiel 8A) vorgelegt, mit 51.2 mg (0.31 mmol) 2-Fluor-5-(trifluormethyl)pyridin und 31.1 mg (0.31 mmol) Kaliumhydrogencarbonat versetzt und bei 130°C für 16 h unter Argon erhitzt. Man erhält nach Reinigung mittels präparativer HPLC 22 mg (30% d. Th.) des Produktes als Feststoff.

LCMS (Methode 3): Rₜ = 2.06 min. (m/z = 468 (M+H)⁺)

¹H-NMR (400MHz, DMSO-d₆): δ = 8.27 (s, 1H), 8.07 (t, 1H), 8.00 (s, 1H), 7.71 (d, 1H), 7.66 (d, 1H), 7.58 (d, 1H), 7.54 (m, breit, 2H), 7.49 (dd, 1H), 7.10 (s, 1H), 6.57 (d, 1H), 3.68 (t, 2H), 3.64 (t, 2H).

### Beispiel 3

1-{6-[(2-{[7-(2,4-Dichlorphenyl)imidazo[1,2-c]pyrimidin-5-yl]amino}ethyl)amino]pyridin-3-yl}ethanon

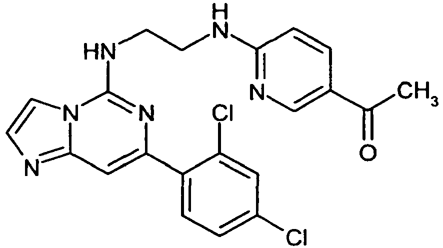

In 3 ml trockenem N-Methylpyrrolidin werden 50 mg (0.155 mmol) des Amins (Beispiel 8A) vorgelegt, mit 48.3 mg (0.31 mmol) 1-(6-Chlorpyridin-3-yl)ethanon und 40.1 mg (0.31 mmol) DIPEA versetzt und bei 130°C für 1.5 h in der Mikrowelle erhitzt. Man erhält nach Reinigung mittels präparativer HPLC 11 mg (15% d. Th.) des Produktes als Feststoff.

LCMS (Methode 5): Rₜ = 1.82 min. (m/z = 439 (M-H)⁺)

¹H-NMR (400MHz, DMSO-d₆): δ = 8.76 (s, br, 1H), 8.53 (d, 1H), 8.19 (s, 1H), 8.00 (s, 1H), 7.76 (d, 2H), 7.75 (dd, 1H), 7.66 (d, 1H), 7.53 (dd, 1H), 7.33 (s, 1H), 6.45 (d, 1H), 3.78 (dt, 2H), 3.69 (dt, 2H), 2.40 (s, 3H).

### Beispiel 4

N⁶-(2-{[7-(2,4-Dichlorphenyl)imidazo[1,2-c]pyrimidin-5-yl]amino}ethyl)-3-nitropyridin-2,6-diamin

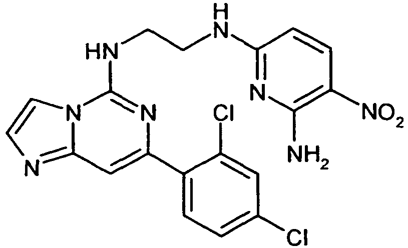

Analog der Vorschrift zur Herstellung von Beispiel 3 werden aus 40 mg (0.124 mmol) des Amins (Beispiel 8A) und 43.1 mg (0.25 mmol) 2-Amino-6-chlor-3-nitropyridin nach Reinigung mittels präparativer HPLC 30.1 mg (53% d. Th.) des Produktes als Feststoff erhalten.

LCMS (Methode 3): Rₜ = 1.68 min. (m/z = 459 (M+H)⁺)

¹H-NMR (400MHz, DMSO-d₆): δ = 8.10 (t, 1H), 8.04 (t, 1H), 8.00 (s, 1H), 7.88 (d, 1H), 7.70 (d, 1H), 7.65 (d, 1H), 7.58 (d, 1H), 7.46 (d, 1H), 7.11 (s, 1H), 5.88 (d, 1H), 3.73 (dt, 2H), 3.69 (dt, 2H).

### Beispiel 5

4-Amino-2-[(2-{[7-(2,4-dichlorphenyl)imidazo[1,2-c]pyrimidin-5-yl]amino}ethyl)amino]-1,3-thiazol-5-carbonitril

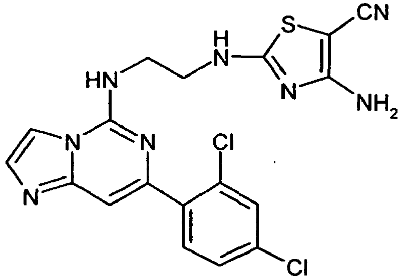

In 28 ml trockenem DMSO werden 214.27 mg (0.72 mmol) Chlorpyrimidin (Beispiel 6A) vorgelegt, mit 320 mg (1.08 mmol) 4-Amino-2-[(2-aminoethyl)amino]-1,3-thiazol-5-carbonitril-trifluoracetat (Beispiel 20A) und 742 mg (5.74 mmol) DIPEA versetzt und bei 120°C für 16 h unter Argon erhitzt. Es wird mit Wasser versetzt und mit 1N Salzsäure neutralisiert und dreimal mit Essigsäureethylester extrahiert. Die vereinigten organischen Phasen werden vom Lösungsmittel befreit und der Rückstand mittels präparativer HPLC gereinigt. Man erhält 48.4 mg (14% d. Th.) des Produktes als Feststoff.

LCMS (Methode 6): Rₜ = 1.18 min. (m/z = 445 (M+H)⁺)

¹H-NMR (400MHz, DMSO-d₆): δ = 8.53 (t, 1H), 8.08 (t, 1H), 8.00 (s, 1H), 7.99 (s, 1H), 7.71 (d, 1H), 7.66 (d, 1H), 7.59 (s, 1H), 7.51 (dd, 1H), 7.13 (s, 1H), 6.70 (s, 1H), 3.70 (dt, 2H), 3.56 (dt, 2H).

### Beispiel 6

N-[7-(2,4-Dichlorphenyl)imidazo[1,2-c]pyrimidin-5-yl]-N'-(5-nitropyridin-2-yl)ethan-1,2-diamin

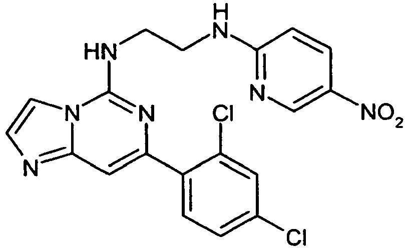

Analog der Vorschrift zur Herstellung von Beispiel 7A wird durch Reaktion von 5-Chlor-7-(2,4-dichlorphenyl)imidazo[1,2-c]pyrimidin (Beispiel 6A) mit N-(5-nitropyridin-2-yl)ethan-1,2-diamin das Produkt als Feststoff erhalten.

LCMS (Methode 5): Rₜ = 2.48 min. (m/z = 444 (M+H))

¹H-NMR (400MHz, DMSO-d₆): δ = 8.85 (s, 1H), 8.30 (s, 1H), 8.10 (s, 1H), 8.05 (s, 1H), 7.98 (s, 1H), 7.68 (s, 1H), 7.64 (d, 1H), 7.57 (d, 1H), 7.46 (dd, 1H), 7.10 (s, 1H), 6.48 (d, 1H), 3.73 (m, 4H).

### Beispiel 7

6-{[2-({7-[4-(Trifluormethyl)phenyl]imidazo[1,2-c]pyrimidin-5-yl}amino)ethyl]amino}-nicotinonitril

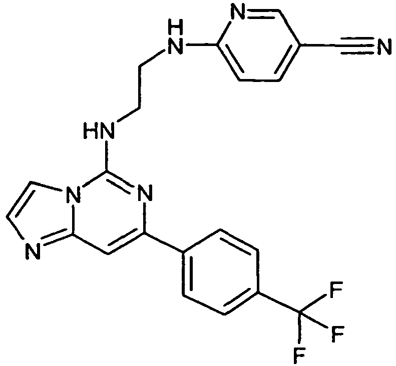

Unter Argon werden in einer Mischung aus 2.5 ml Dioxan und 0.83 ml gesättigter wässriger Natriumcarbonat-Lösung 50 mg (0.123 mmol) 6-({2-[(7-Chlorimidazo[1,2-c]pyrimidin-5-yl)amino]ethyl}amino)nicotinonitril (Beispiel 16A), 23.3 mg (0.123 mmol) [4-(Trifluormethyl)-phenyl]boronsäure und 14.18 mg (0.012 mmol) Tetrakis(triphenylphosphin)palladium(0) vorgelegt. Die Mischung wird mit Argon entgast und danach für 30 min bei 150°C in der Mikrowelle erhitzt. Nach Abkühlen filtriert man über eine Extrelut^{®}-Kartusche. Man erhält nach Reinigung mittels präparativer HPLC 43 mg (83% d. Th.) des Produktes als Feststoff.

LCMS (Methode 3): Rₜ = 1.79 min. (m/z = 424 (M+H)⁺)

¹H-NMR (400MHz, DMSO-d₆): δ = 8.85 (t, 1H), 8.40 (s, 1H), 8.28 (d, 2H), 8.21 (d, 1H), 8.06 (d, 1H), 7.85 (d, 2H), 7.80 (t, 1H), 7.71 (s, 1H), 7.58 (d, 1H), 6.46 (d, 1H), 3.89 (dd, 2H), 3.71 (dd, 2H).

Analog der für Beispiel 7 beschriebenen Vorschrift werden folgende Produkte durch Palladium-katalysierte Kupplung mit den entsprechenden Boronsäuren erhalten:

| **Beispiel** | **Struktur** | **Charakterisierung** |
|---|---|---|
| **8** | 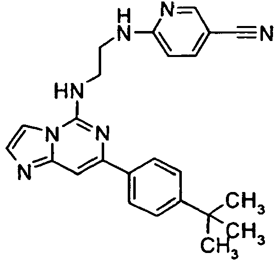 | LC/MS (Methode): Rₜ = 2.89 min MS (ESIpos): m/z = 412 (M+H)⁺. |
| **9** | 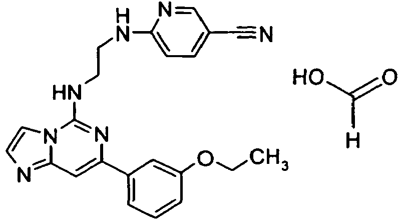 | LC/MS (Methode 3): Rₜ = 1.60 min MS (ESIpos): m/z = 400 (M+H)⁺. |
| **10** | 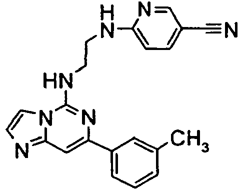 | LC/MS (Methode 3): Rₜ = 1.59 min MS (ESIpos): m/z = 370 (M+H)⁺. |
| **11** | 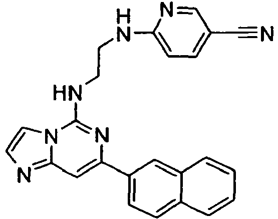 | LC/MS (Methode 5): Rₜ = 2.28 min MS (ESIpos): m/z = 406 (M+H)+. |
| **12** | 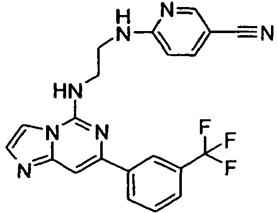 | LC/MS (Methode 3): Rₜ = 1.74 min MS (ESIpos): m/z = 424 (M+H)+. |
| **13** | 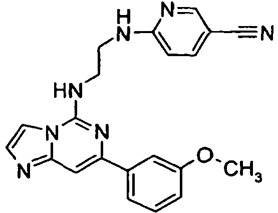 | LC/MS (Methode 3): Rₜ = 1.51 min MS (ESIpos): m/z = 386 (M+H)+. |
| **14** | 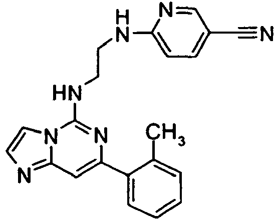 | LC/MS (Methode 3): Rₜ = 1.53 min MS (ESIpos): m/z = 370 (M+H)+. |
| **15** | 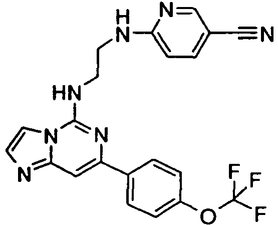 | LC/MS (Methode 3): Rₜ = 1.80 min MS (ESIpos): m/z = 440 (M+H)+. |
| **16** | 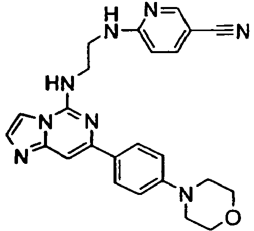 | LC/MS (Methode 2): Rₜ = 2.64 min MS (ESIpos): m/z = 441 (M+-)+. |
| **17** | 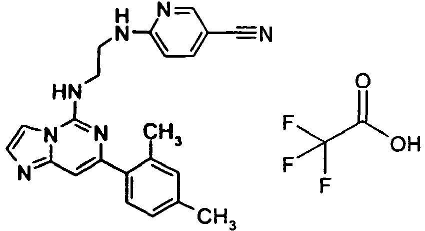 | LC/MS (Methode 3): Rₜ = 1.61 min MS (ESIpos): m/z = 384 (M+H)+. |
| **18** | 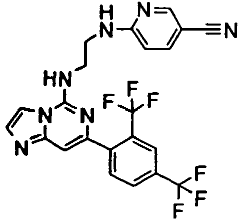 | LC/MS (Methode 7): Rₜ = 2.90 min MS (ESIpos): m/z = 492 (M+H)+. |
| **19** | 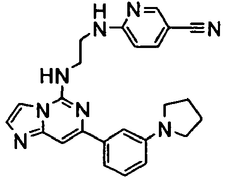 | LC/MS (Methode 3): Rₜ = 1.74 min MS (ESIpos): m/z = 425 (M+H)+. |
| **20** | 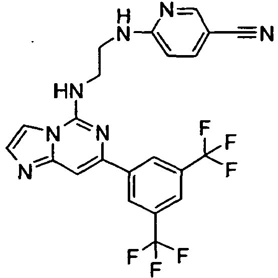 | LC/MS (Methode 3): Rₜ = 2.07 min MS (ESIpos): m/z = 492 (M+H)+. |
| **21** | 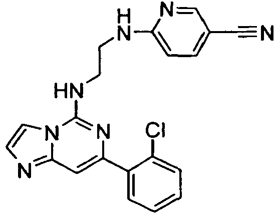 | LC/MS (Methode 5): Rₜ = 1.94 min MS (ESIpos): m/z = 390 (M+H)+. |
| **22** | 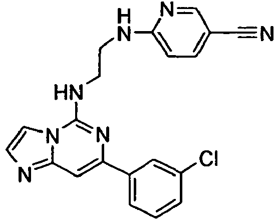 | LC/MS (Methode 5): Rₜ = 2.13 min MS (ESIpos): m/z = 390 (M+H)+. |
| **23** | 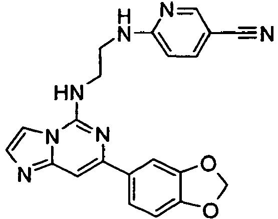 | LC/MS (Methode 7): R, = 2.09 min MS (ESIpos): m/z = 400 (M+H)+. |
| **24** | 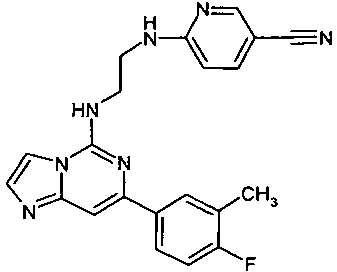 | LC/MS (Methode 5): Rₜ = 2.16 min MS (ESIpos): m/z = 388 (M+H)+. |
| **25** | 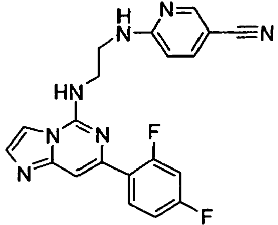 | LC/MS (Methode 6): Rₜ = 1.1 min MS (ESIpos): m/z = 392 (M+H)+. |
| **26** | 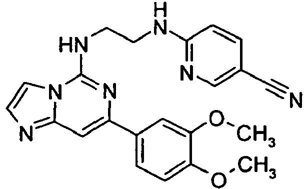 | LC/MS (Methode 7): Rₜ = 1.94 min MS (ESIpos): m/z = 416 (M+H)+. |
| **27** | 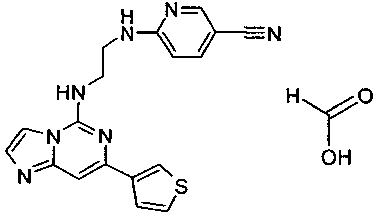 | LC/MS (Methode 5): Rₜ = 1.83 min MS (ESIpos): m/z = 362 (M+H)+. |
| **28** | 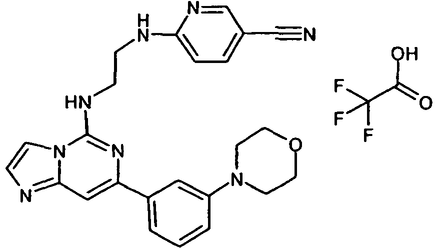 | LC/MS (Methode 6): Rₜ = 1.01 min MS (ESIpos): m/z = 441 (M+H)+. |
| **29** | 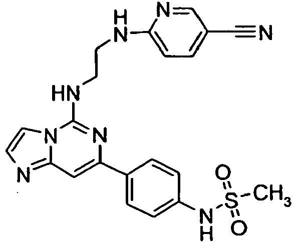 | LC/MS (Methode 6): Rₜ = 0.87 min MS (ESIpos): m/z = 449 (M+H)+. |
| **30** | 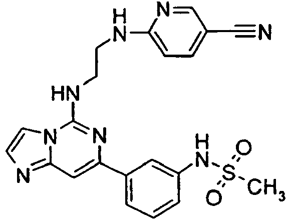 | LC/MS (Methode 6): Rₜ = 1.09 min MS (ESIpos): m/z = 449 (M+H)+. |

Analog der für Beispiel 7 beschriebenen Vorschrift werden ausgehend von N⁶-{2-[(7-Chlorimidazo[1,2-c]pyrimidin-5-yl)amino]ethyl}-3-nitropyridin-2,6-diamin (Beispiel 17A) folgende Produkte durch Palladium-katalysierte Kupplung mit den entsprechenden Boronsäuren erhalten:

| **Beispiel** | **Struktur** | **Charakterisierung** |
|---|---|---|
| **31** | 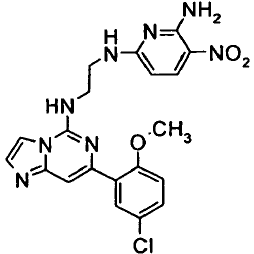 | LC/MS (Methode 6): Rₜ = 1.30 min MS (ESIpos): m/z = 455 (M+H)⁺. |
| **32** | 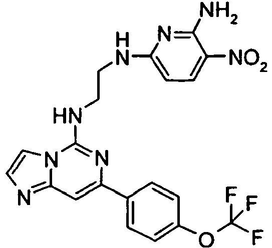 | LC/MS (Methode 4): Rₜ = 3.07 min MS (ESIpos): m/z = 475 (M+H)+ |
| **33** | 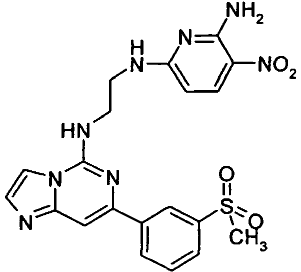 | LC/MS (Methode 7): Rₜ = 1.65 min MS (ESIpos): m/z = 469 (M+H)+. |
| **34** | 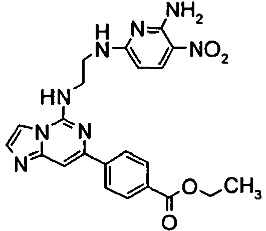 | LC/MS (Methode 7): Rₜ = 2.34 min MS (ESIpos): m/z = 463 (M+H)+. |
| **35** | 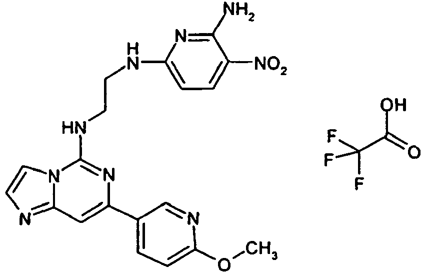 | LC/MS (Methode 6): Rₜ = 0.91 min MS (ESIpos): m/z = 422 (M+H)+. |
| **36** | 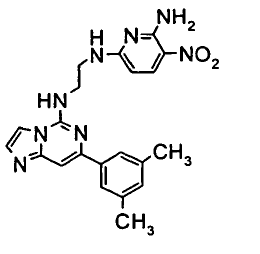 | LC/MS (Methode 7): Rₜ = 2.49 min MS (ESIpos): m/z = 419 (M+H)+. |
| **37** | 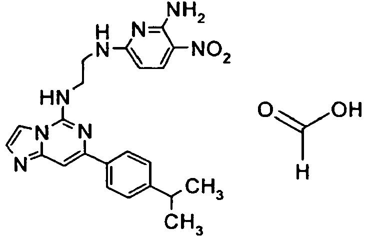 | LC/MS (Methode 3): Rₜ = 1.74 min MS (ESIpos): m/z = 433 (M+H)+. |
| **38** | 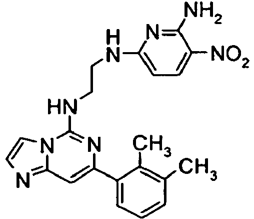 | LC/MS (Methode 6): Rₜ = 1.29 min MS (ESIpos): m/z = 419 (M+H)+. |
| **39** | 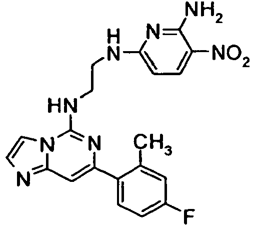 | LC/MS (Methode 3): Rₜ = 1.54 min MS (ESIpos): m/z = 423 (M+H)+. |
| **40** | 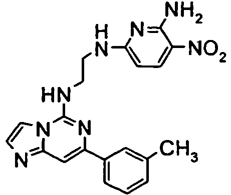 | LC/MS (Methode 3): Rₜ = 1.55 min MS (ESIpos): m/z = 405 (M+H)+. |
| **41** | 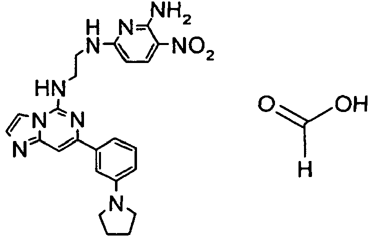 | LC/MS (Methode 5): Rₜ = 2.31 min MS (ESIpos): m/z = 460 (M+H)+. |
| **42** | 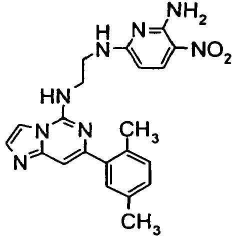 | LC/MS (Methode 3): Rₜ = 1.58 min MS (ESIpos): m/z = 419 (M+H)+. |
| **43** | 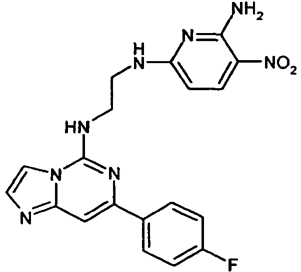 | LC/MS (Methode 3): Rₜ = 1.50 min MS (ESIpos): m/z = 409 (M+H)+. |
| **44** | 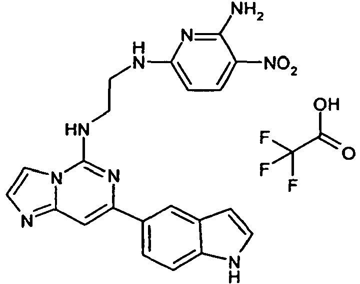 | LC/MS (Methode 6): Rₜ = 1.70 min MS (ESIpos): m/z = 530 (M+H)+. |
| **45** | 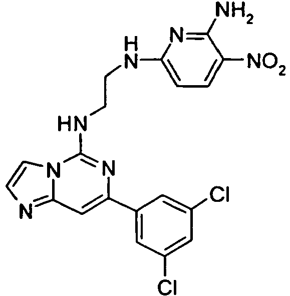 | LC/MS (Methode 7): Rₜ = 2.68 min MS (ESIpos): m/z = 459 (M+H)+. |
| **46** | 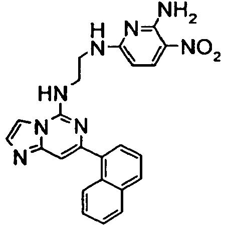 | LC/MS (Methode 3): Rₜ = 1.60 min MS (ESIpos): m/z = 441 (M+H)+. |
| **47** | 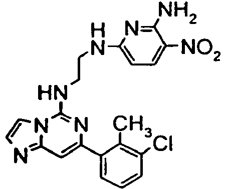 | LC/MS (Methode 7): Rₜ = 3.08 min MS (ESIpos): m/z = 439 (M+H)+. |
| **48** | 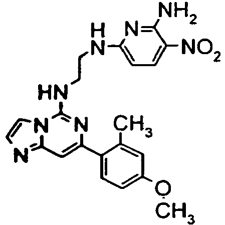 | LC/MS (Methode 3): Rₜ = 1.45 min MS (ESIpos): m/z = 435 (M+H)+. |
| **49** | 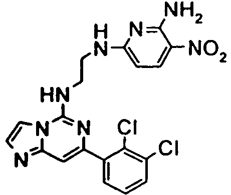 | LC/MS (Methode 3): Rₜ = 1.66 min MS (ESIpos): m/z = 459 (M+H)+. |
| **50** | 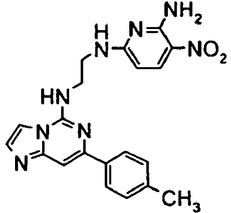 | LC/MS (Methode 3): Rₜ = 1.59 min MS (ESIpos): m/z = 405 (M+H)+. |
| **51** | 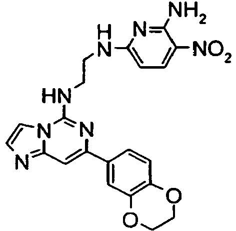 | LC/MS (Methode 7): Rₜ = 2.01 min MS (ESIpos): m/z = 449 (M+H)+. |
| **52** | 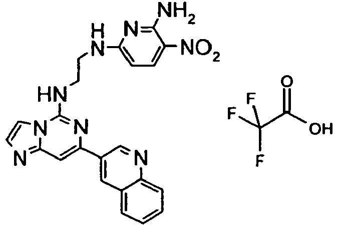 | LC/MS (Methode 5): Rₜ = 2.90 min MS (ESIpos): m/z = 441 (M+H)+. |
| **53** | 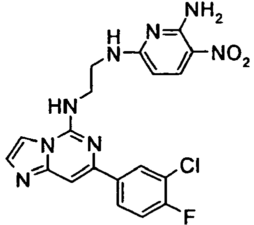 | LC/MS (Methode 3): Rₜ = 1.65 min MS (ESIpos): m/z = 443 (M+H)+. |
| **54** | 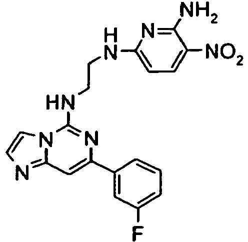 | LC/MS (Methode 3): Rₜ = 1.51 min MS (ESIpos): m/z = 409 (M+H)+. |
| **55** | 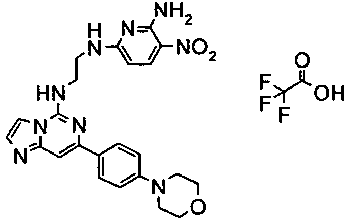 | LC/MS (Methode 6): Rₜ = 1.01 min MS (ESIpos): m/z = 476 (M+H)+. |
| **56** | 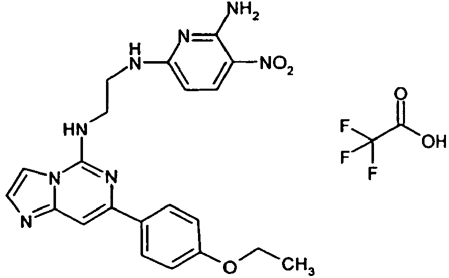 | LC/MS (Methode 6): Rₜ = 1.12 min MS (ESIpos): m/z = 435 (M+H)+. |
| **57** | 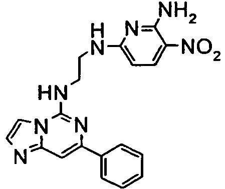 | LC/MS (Methode 3): Rₜ = 1.45 min MS (ESIpos): m/z = 391 (M+H)+. |
| **58** | 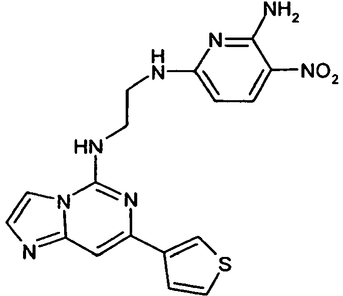 | LC/MS (Methode 5): Rₜ = 2.94 min MS (ESIpos): m/z = 397 (M+H)+. |
| **59** | 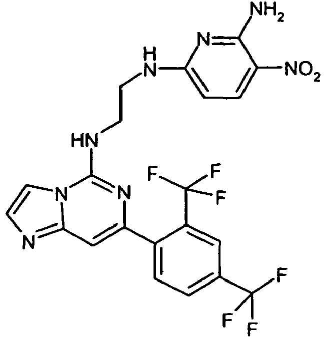 | LC/MS (Methode 5): Rₜ = 2.71 min MS (ESIpos): m/z = 527 (M+H)+. |
| **60** | 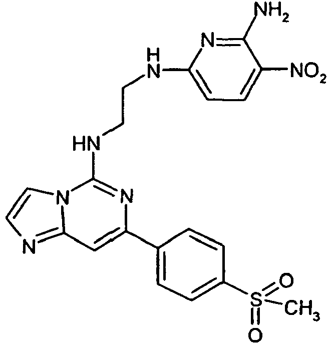 | LC/MS (Methode 5): Rₜ = 1.42 min MS (ESIpos): m/z = 469 (M+H)+. |
| **61** | 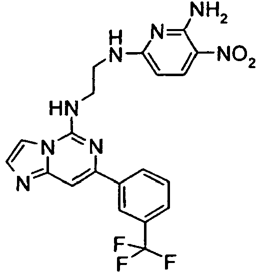 | LC/MS (Methode 3): Rₜ = 1.70 min MS (ESIpos): m/z = 459 (M+H)+. |
| **62** | 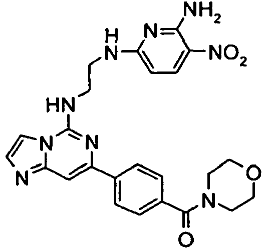 | LC/MS (Methode 6): Rₜ = 1.02 min MS (ESIpos): m/z = 504 (M+H)+. |
| **63** | 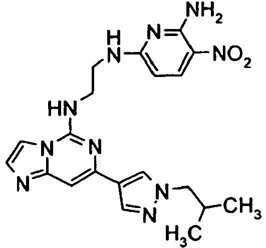 | LC/MS (Methode 3): Rₜ = 1.45 min MS (ESIpos): m/z = 437 (M+H)+. |
| **64** | 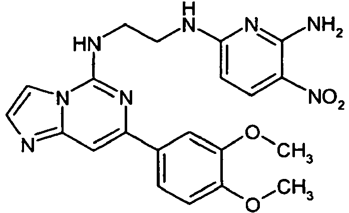 | LC/MS (Methode 3): Rₜ = 1.37 min MS (ESIpos): m/z = 451 (M+H)+. |
| **65** | 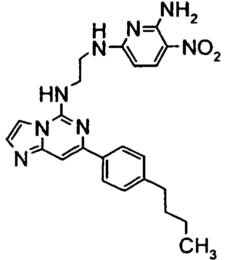 | LC/MS (Methode 6): Rₜ = 1.43 min MS (ESIpos): m/z = 447 (M+H)+. |
| **66** | 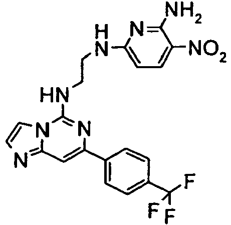 | LC/MS (Methode 5): Rₜ = 2.35 min MS (ESIpos): m/z = 459 (M+H)+. |
| **67** | 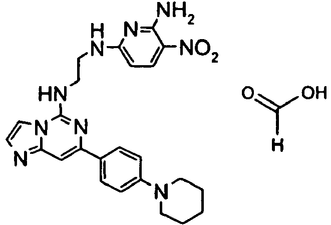 | LC/MS (Methode): Rₜ = 2.08 min MS (ESIpos): m/z= 474 (M+H)+. |
| **68** | 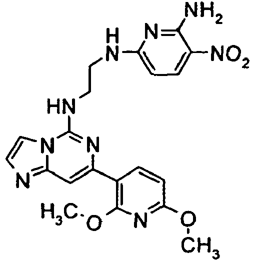 | LC/MS (Methode 6): Rₜ = 1.31 min MS (ESIpos): m/z = 452 (M+H)+. |
| **69** | 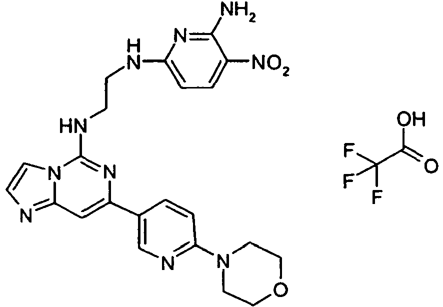 | LC/MS (Methode 6): Rₜ = 0.79 min MS (ESIpos): m/z = 477 (M+H)+. |
| **70** | 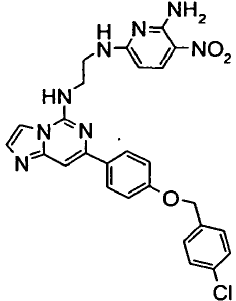 | LC/MS (Methode 5): Rₜ = 2.91 min MS (ESIpos): m/z = 531 (M+H)+. |
| **71** | 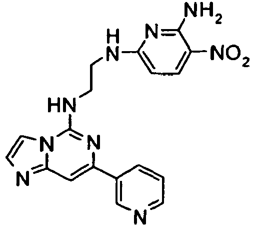 | LC/MS (Methode 6): Rₜ = 0.63 min MS (ESIpos): m/z = 392 (M+H)+. |
| **72** | 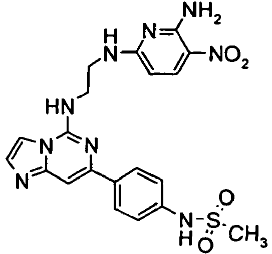 | LC/MS (Methode 6): Rₜ = 0.84 min MS (ESIpos): m/z = 484 (M+H)+. |
| **73** | 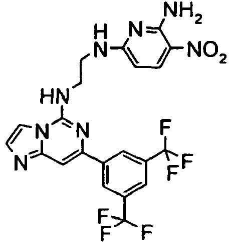 | LC/MS (Methode 3): Rₜ = 2.03 min MS (ESIpos): m/z = 527 (M+H)+. |
| **74** | 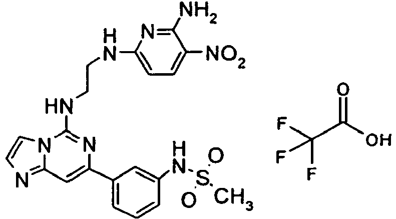 | LC/MS (Methode 6): Rₜ = 0.86 min MS (ESIpos): m/z = 484 (M+H)+. |
| **75** | 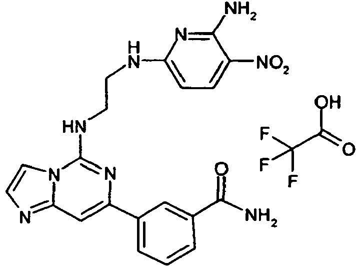 | LC/MS (Methode 6): Rₜ = 0.71 min MS (ESIpos): m/z = 434 (M+H)+. |

### Beispiel 76

N⁶-(2-{[7-(2,4-Dichlorphenyl)imidazo[1,2-c]pyrimidin-5-yl]amino}ethyl)-N⁶-methyl-3-nitropyridin-2,6-diamin-formiat

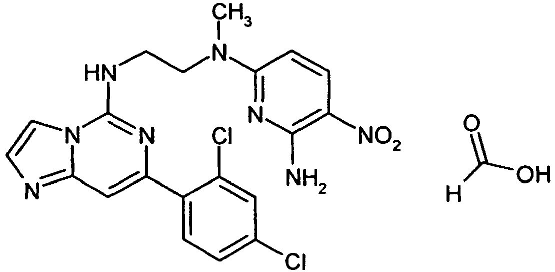

Analog der Vorschrift zur Herstellung von Beispiel 5 wird durch Reaktion von 5-Chlor-7-(2,4-dichlorphenyl)imidazo[1,2-c]pyrimidin (Beispiel 6A) mit N⁶-(2-Aminoethyl)-N⁶-methyl-3-nitropyridin-2,6-diamin-formiat (Herstellung analog Beispiel 14A) das Produkt erhalten.

LCMS (Methode 7): Rₜ = 2.67 min. (m/z = 473 (M+H)⁺)

¹H-NMR (400MHz, DMSO-d₆): δ = 12.74 (s, 1H), 8.20 (s, 1H), 8.13 (s, 1H), 7.97 (s, 1H), 7.93 (s, 1H), 7.73 (s, 1H), 7.67 (d, 1H), 7.58 (s, 1H), 7.49 (d, 1H), 7.10 (s, 1H), 6.37 (s, br, 1H), 6.10 (s, br, 1H), 3.73 (s, br, 2H), 3.14 (s, br, 2H), 2.54 (s, 3H).

### Beispiel 77

N⁶-(2-{[7-(2,4-Dichlorphenyl)-2-methylimidazo[1,2-c]pyrimidin-5-yl]amino}ethyl)-3-nitropyridin-2,6-diamin

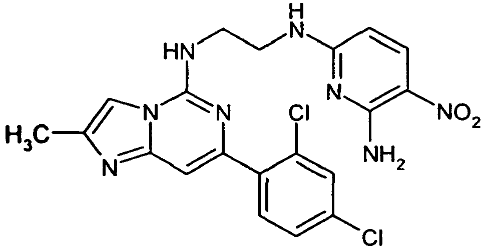

Analog der für Beispiel 7 beschriebenen Vorschrift wird ausgehend von 60 mg (0.1 mmol) N⁶-{2-[(7-Chlor-2-methylimidazo[1,2-c]pyrimidin-5-yl)amino]ethyl}-3-nitropyridin-2,6-diamin (Beispiel 23A) durch Kupplung mit 29.3 mg (0.1 mmol) (2,4-Dichlorphenyl)boronsäure und anschließende Reinigung durch präparativer HPLC 9.3 mg (13% d. Th.) des Produktes erhalten.

LCMS (Methode 7): Rₜ = 2.67 min. (m/z = 473 (M+H)⁺)

¹H-NMR (400MHz, DMSO-d₆): δ = 8.05 (s, br, 1H), 7.88 (d, 1H), 7.83 (s, 1H), 7.68 (d, 1H), 7.65 (d, 1 H), 7.45 (d, 1H), 7.00 (s, 1H), 5.90 (d, 1H), 3.71 (s, br, 4H), 3.67 (s, br, 4H), 2.32 (s, 3H).

### Beispiel 78

6-[(2-{[7-(2,4-Dichlorphenyl)-2-methylimidazo[1,2-c]pyrimidin-5-yl]amino}ethyl)amino]-nicotinnitril

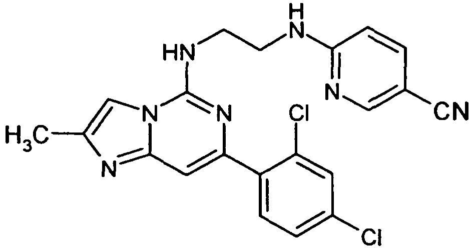

Analog der für Beispiel 7 beschriebenen Vorschrift wird ausgehend von 120 mg (0.36 mmol) 6-({2-[(7-Chlor-2-methylimidazo[1,2-c]pyrimidin-5-yl)amino]ethyl}amino)nicotinnitril (Beispiel 30A) durch Kupplung mit 68.4 mg (0.36 mmol) (2,4-Dichlorphenyl)boronsäure und anschließende Reinigung durch präparativer HPLC 11.9 mg (7% d. Th.) des Produktes erhalten.

LCMS (Methode 3): Rₜ = 1.76 min. (m/z = 438 (M+H)⁺)

¹H-NMR (400MHz, DMSO-d₆): δ = 8.67 (s, br, 1H), 8.32 (s, 1H), 7.93 (s, 1H), 7.77 (d, 1H), 7.70 (s, br, 2H), 7.65 (d, 1H), 7.57 (dt, 1H), 7.29 (s, 1H), 6.48 (d, 1H), 3.74 (dd, 2H), 3.65 (dd, 2H), 2.45 (s, 3H).

### Beispiel 79

N⁶-(2-{[7-(2,4-Dichlorphenyl)-2-(trifluormethyl)imidazo[1,2-c]pyrimidin-5-1]amino}ethyl)-3-nitropyridin-2,6-diamin

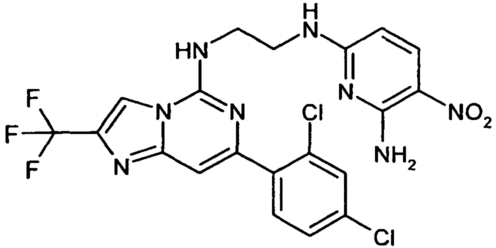

Analog der für Beispiel 7 beschriebenen Vorschrift wird ausgehend von 68 mg (0.15 mmol) N⁶-{2-[(7-chlor-2-methylimidazo[1,2-c]pyrimidin-5-yl)amino]ethyl}-3-nitropyridin-2,6-diamin (Beispiel 25A) durch Kupplung mit 28 mg (0.15 mmol) (2,4-Dichlorphenyl)boronsäure und anschließende Reinigung durch präparativer HPLC 23.5 mg (30% d. Th.) des Produktes als Feststoff erhalten.

LCMS (Methode 3): Rₜ = 2.84 min. (m/z = 527 (M+H)⁺)

¹H-NMR (400MHz, DMSO-d₆): δ = 8.61 (s, 1H), 8.23 (t, 1H), 8.12 (s, br, 1H), 8.04 (t, 1H), 7.87 (d, 1H), 7.72 (s, 1H), 7.63 (d, 2H), 7.47 (d, 1H), 7.19 (s, 1H), 5.86 (d, 1H), 3.75 (dd, 2H), 3.68 (dd, 2H).

### Beispiel 80

6-[(2-{[7-(2,4-Dichlorphenyl)-2-(trifluormethyl)imidazo[1,2-c]pyrimidin-5-yl]amino} ethyl)-amino]nicotinnitril

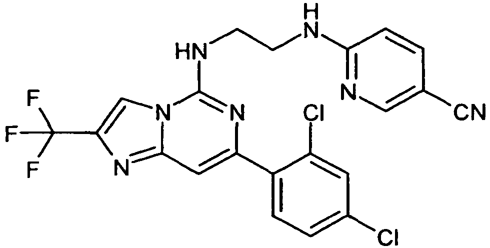

Analog der für Beispiel 7 beschriebenen Vorschrift wird ausgehend von 29 mg (0.07 mmol) 6-[(2-{[7-Chlor-2-(trifluormethyl)imidazo[1,2-c]pyrimidin-5-yl]amino}ethyl)amino]nicotinnitril (Beispiel 24A) durch Kupplung mit 13 mg (0.07 mmol) (2,4-Dichlorphenyl)boronsäure und anschließende Reinigung durch präparativer HPLC 11.4 mg (33% d. Th.) des Produktes als Feststoff erhalten.

LCMS (Methode 5): Rₜ = 3.89 min. (m/z = 492 (M+H)⁺)

¹H-NMR (400MHz, DMSO-d₆): δ = 8.59 (s, 1H), 8.32 (d, 2H), 7.74 (s, br, 2H), 7.62 (d, 1H), 7.58 (d, 1 H), 7.52 (d, 1H), 7.18 (s, 1H), 6.48 (d, 1 H), 3.70 (dd, 2H), 3.65 (dd, 2H).

### Beispiel 81

N⁶-(2-{[7-(2,4-Dichlorphenyl)-2-(morpholin-4-ylmethyl)imidazo[1,2-c]pyrimidin-5-yl]amino}ethyl)-3-nitropyridin-2,6-diamin-trifluoacetat

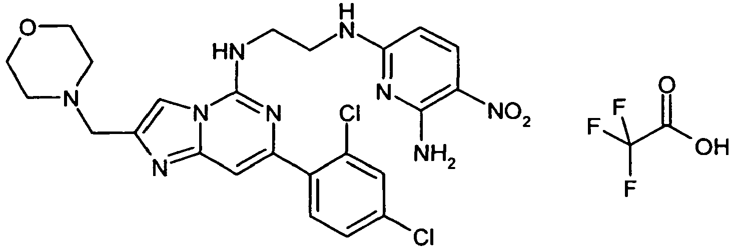

Analog der für Beispiel 7 beschriebenen Vorschrift wird ausgehend von 60 mg (0.13 mmol) N⁶-(2-{[7-Chlor-2-(morpholin-4-ylmethyl)imidazo[1,2-c]pyrimidin-5-yl]amino}ethyl)-3-nitropyridin-2,6-diamin (Beispiel 35A) durch Kupplung mit 25.6 mg (0.07 mmol) (2,4-Dichlorphenyl)boronsäure und anschließende Reinigung durch präparativer HPLC 7.9 mg (8% d. Th.) des Produktes erhalten.

LCMS (Methode 6): Rₜ = 1.28 min. (m/z = 558 (M+H)⁺)

¹H-NMR (400MHz, DMSO-d₆): δ = 8.21 (s, br, 1H), 8.10 (s, 1H), 8.03 (s, br, 1H), 7.89 (d, 1H), 7.72 (s, 1 H), 7.66 (d, 1H), 7.49 (d, 1H), 7.13 (s, 1H), 5.89 (d, 1H), 5.74 (s, 1 H), 4.47 (s, br, 2H), 3.82 (m, br, 8H), 3.74 (s, br, 2H), 3.69 (s, br, 2H).

### Beispiel 82

6-[(2-{[7-(2,4-Dichlorphenyl)imidazo[1,2-c]pyrimidin-5-yl]amino}propyl)amino]nicotinnitril

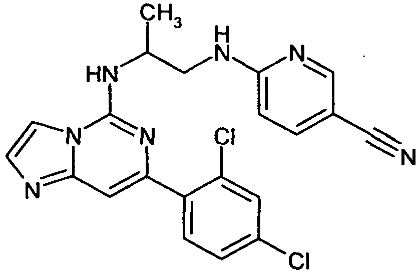

In DMSO (6 ml) werden das Amin (Beispiel 37A) (300 mg, 0.46 mmol), 2-Chlor-5-cyanopyridin (129 mg, 0.93 mmol) und N,N-Diisopropylethylamin (600 mg, 4.6 mmol) vorgelegt, und 30 min in dem Mikrowellenofen bei 150°C erhitzt. Das Reaktionsgemisch wird auf Wasser gegossen und mit Ethylacetat extrahiert (3 x 25 ml). Die vereinigten organischen Phasen werden mit gesättigter wässriger Natriumchloridlösung gewaschen, über Magnesiumsulfat getrocknet und eingeengt. Der Rückstand wird in Acetonitril aufgenommen, das Produkt fällt aus, und wird mit Acetonitril nachgewaschen und getrocknet. Es werden 87 mg (41% d. Th.) des Produktes als Feststoff erhalten.

LCMS (Methode 3): Rₜ = 1.86 min. (m/z = 439 (M+H)⁺)

¹H-NMR (400MHz, DMSO-d₆): δ = 8.31 (d, 1H), 8.07 (s, 1H), 7.76 (t, 1H), 7.70 (d, 1H), 7.65 (d, 1H), 7.59 (d, 1H), 7.52 (m, 3H), 7.10 (s, 1H), 6.50 (br s, 1H), 4.56 (br m, 1H), 3.45 (m, 2H), 1.31 (d, 3H).

**Enantiomerentrennung** von 6-[(2-{[7-(2,4-Dichlorphenyl)imidazo[1,2-c]pyrimidin-5-yl]amino}propyl)amino]nicotinnitril (Beispiel 82) wird unter den folgenden Bedingungen durchgeführt:
Eine Probe von Beispiel 82 (87 mg) wird in Ethanol:Acetonitril (4:1, 100 ml) aufgenommen und über eine Daicel Chiralpak AD-H 250 mm x 20 mm Säule chromatographiert (Fluß: 15 ml/min; Detektion bei 230 nm; Injektionsvolumen: 500 µl; Eluent iso-Hexan:Ethanol:N,N-Diisopropylethylamin (350:150:1), Temperatur: 40°C). Es werden zwei Fraktionen isoliert:

### Beispiel Ent-A-82: Es werden 27 mg Produkt in > 99% ee isoliert.

Retentionszeit 4.38 min

### Beispiel Ent-B-82: Es werden 24 mg Produkt in > 99% ee isoliert.

Retentionszeit 5.85 min

### Beispiel 83

N⁶-(2-{[7-(2,4-Dichlorphenyl)imidazo [1,2-c]pyrimidin-5-yl]amino} propyl)-3-nitropyridin-2,6-diamin

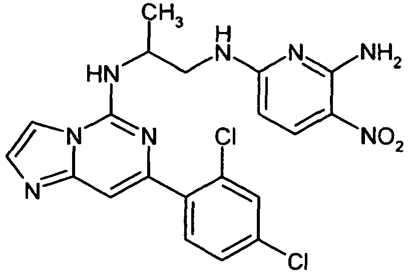

N⁶-(2-{[7-(2,4-Dichlorphenyl)imidazo[1,2-c]pyrimidin-5-yl]amino}propyl)-3-nitropyridin-2,6-diamin (Beispiel 83) wird in Analogie zu Beispiel 82 aus Beispiel 37A (300 mg, 0.47 mmol), 2-Amino-6-chlor-3-nitropyridin (161 mg, 0.93 mmol) und N,N-Diisopropylethylamin (600 mg, 4.6 mmol) hergestellt. Das Rohprodukt wird aus N,N-Dimethylformamid und Acetonitril umgefällt. Das Produkt wird abfiltriert und mit Acetonitril nachgewaschen und getrocknet. Es werden 99 mg (45% d. Th.) des Produktes als Feststoff erhalten.

LCMS (Methode 3): Rₜ = 1.78 min. (m/z = 473 (M+H)⁺)

¹H-NMR (400MHz, DMSO-d₆): δ = 8.07 (br m, 3H), 7.85 (d, 1H), 7.64 (m, 3 H), 7.58 (m, 2H), 7.38 (dd, 1H), 7.12 (s, 1H), 5.38 (d, 1H), 4.60 (br m, 1H), 3.75 (m, 1H), 3.47 (s, 1H), 1.33 (d, 3H).

**Enandomerentrennung** von N⁶-(2-{[7-(2,4-Dichlorphenyl)imidazo[1,2-c]pyrimidin-5-yl]amino}propyl)-3-nitropyridin-2,6-diamin (Beispiel 83) wird unter den folgenden Bedingungen durchgeführt:
Eine Probe von 99 mg Beispiel 83 wird in Ethanol:Acetonitril (4:1, 80 ml) warm aufgenommen und über eine Daicel Chiralpak AD-H 5 µM 250 mm x 20 mm Säule chromatographiert (Fluß: 15 ml/min; Detektion bei 220 nm; Injektionsvolumen 700 µl; Eluent iso-Hexan:Ethanol:N,N-Diisopropylethylamin (350:150:1), Temperatur: 40°C). Es werden zwei Fraktionen isoliert:

### Beispiel Ent-A-83: Es werden 23 mg Produkt in > 99.5% ee isoliert.

Retentionszeit 4.88 min

### Beispiel Ent-B-83: Es werden 28 mg Produkt in > 98% ee isoliert.

Retentionszeit 5.93 min

### Beispiel 84

6-[(2-{[7-(2,4-Dichlorphenyl)imidazo[1,2-c]pyrimidin-5-yl]amino}-1-methylethyl)arnino]nicotin-nitril 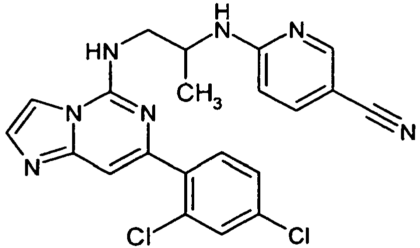 6-[(2-([7-(2,4-Dichlorphenyl)imidazo[1,2-c]pyrimidin-5-yl]amino)-1-methylethyl)amino]nicotin-nitril (Beispiel 84) wird in Analogie zu Beispiel 82 aus Beispiel 40A (187 mg, 0.63 mmol), 5-Chlor-7-(2,4-dichlorphenyl)imidazo[1,2-c]pyrimidin (Beispiel 6A) (273 mg, 0.94 mmol) und N,N-Diisopropylethylamin (810 mg, 6.3 mmol) hergestellt. Das Rohprodukt wird aus Acetonitril ausgefällt, das Produkt wird abfiltriert und mit Acetonitril und Diethylether nachgewaschen und getrocknet. Es werden 220 mg (75% d. Th.) des Produktes als Feststoff erhalten.

LCMS (Methode 3): Rₜ = 1.85 min. (m/z = 438 (M+H)⁺)

¹H-NMR (400MHz, DMSO-d₆): δ = 8.29 (d, 1H), 8.08 (s, 2H), 7.72 (s, 1H), 7.65 (d, 1H), 7.58 (d, 2H), 7.50 (d, 2H), 7.11 (s, 1H), 6.49 (br s, 1H), 4.43 (br m, 1H), 3.80 (br m, 1H), 3.55 (br m, 1H), 1.22 (d, 3H).

**Enantiomerentrennung** von 6-[(2-([7-(2,4-Dichlorphenyl)imidazo[1,2-c]pyrimidin-5-yl]amino)-1-methylethyl)amino]nicotinonitril (Beispiel 84) wird unter den folgenden Bedingungen durchgefiihrt:
Eine Probe von 220 mg Beispiel 84 wird in 2-Propanol:Acetonitril (4:1, 160 ml) warm aufgenommen und über eine Daicel Chiralpak AD-H 5µm 250 mm x 20 mm Säule chromatographiert (Fluß: 15 ml/min; Detektion bei 220 nm; Injektionsvolumen: 500 µl; Eluent iso-Hexan:2-Propanol:N,N-Diisopropylethylamin (400:100:1), Temperatur: 40°C). Es werden zwei Fraktionen isoliert:

### Beispiel Ent-A-84: Es werden 63 mg Produkt in > 99% ee isoliert.

Retentionszeit 5.22 min

### Beispiel Ent-B-84: Es können 59 mg Produkt in > 97% ee isoliert.

Retentionszeit 5.98 min

### Beispiel 85

N°-(2-([7-(2,4-Dichlorphenyl)imidazo[1,2-c]pyrimidin-5-yl]amino)-1-methylethyl)-3-nitropyridin-2,6-diamin 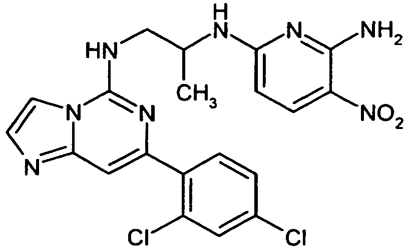 N6-(2-{[7-(2,4-Dichlorphenyl)imidazo[1,2-c]pyrimidin-5-yl]amino}-1-methylethyl)-3-nitropyridin-2,6-diamin (Beispiel 85) wird in Analogie zu Beispiel 82 aus Beispiel 41A (467 mg, 1.0 mmol), 5-Chlor-7-(2,4-dichlorphenyl)imidazo[1,2-c]pyrimidin (Beispiel 6A) (200 mg, 0.67 mmol) und N,N-Diisopropylethylamin (866 mg, 6.7 mmol) hergestellt. Das Rohprodukt wird aus Acetonitril ausgefällt, das Produkt wird abfiltriert und mit Acetonitril und Diethylether nachgewaschen und getrocknet. Es werden 234 mg (72% d. Th) Produkt als Feststoff erhalten.

LCMS (Methode 3): Rₜ = 1.80 min. (m/z = 473 (M+H)⁺)

¹H-NMR (400MHz, DMSO-d₆): δ = 8.04 (br s, 2H), 7.94 (br t, 1H), 7.87 (d, 1H), 7.83 (d, 1H), 7.69 (s, 1H), 7.64 (d, 1H), 7.57 (s, 1H), 7.41 (br m, 2H), 7.12 (s, 1H), 5.83 (d, 1H), 4.48 (br t, 1H), 3.43 (m, 1H), 3.54 (m, 1H), 1.25 (d, 3H).

**Enantiomerentrennung** von N⁶-(2-{[7-(2,4-Dichlorphenyl)imidazo[1,2-c]pyrimidin-5-yl]amino}-1-methylethyl)-3-nitropyridin-2,6-diamin (Beispiel 85) wird unter den folgenden Bedingungen durchgeführt:
Eine Probe von 234 mg Beispiel 85 wird in Methanol:Dichlormethan: tert-Butylmethylether (15:15:35, 65 ml) warm aufgenommen und über eine Daicel Chiralpak IA 5µm 250 mm x 20 mm Säule chromatographiert (Fluß: 15 ml/min; Detektion bei 230 nm; Injektionsvolumen: 700 µl; Eluent tert-Butylmethylether:Methanol:N,N-Diisopropylethylamin (225:25:1), Temperatur: 40°C). Es werden zwei Fraktionen isoliert:

### Beispiel Ent-A-85: Es werden 27 mg Produkt in > 98.5% ee isoliert.

Retentionszeit 6.10 min

### Beispiel Ent-B-85: Es werden 55 mg Produkt in > 96% ee isoliert.

Retentionszeit 7.49 min

### Beispiel 86

6-[(2-{[7-(2,4-Dichlorphenyl)imidazo[1,2-c]pyrimidin-5-yl]amino}ethyl)thio]nicotinnitril 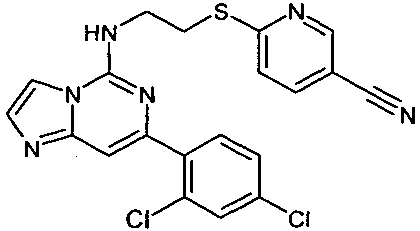 6-[(2-{[7-(2,4-Dichlorphenyl)imidazo[1,2-c]pyrimidin-5-yl]amino}ethyl)thio]nicotinonitril (Beispiel 86) wird in Analogie zu Beispiel 82 aus Beispiel 45A (179 mg, 1.0 mmol), 5-Chlor-7-(2,4-dichlorphenyl)imidazo[1,2-c]pyrimidin (Beispiel 6A) (358 mg, 1.2 mmol) und N,N-Diisopropylethylamin (1.7 ml, 10 mmol) hergestellt. Es werden 169 mg (38% d. Th.) Produkt als Feststoff nach Aufreinigung durch präparative RP-HPLC (Gradient Eluent: Wasser:Acetonitril 90:10 bis 10:90) erhalten.

LCMS (Methode 3): Rₜ = 2.06 min. (m/z = 441 (M+H)⁺)

¹H-NMR (400MHz, DMSO-d₆): δ = 9.71 (t, 1H), 8.73 (d, 1H), 8.64 (m, 1H), 8.16 (d, 1H), 7.93 (dd, 1H), 7.81 (d, 1H), 7.68 (d, 1H), 7.58 (dd, 1H), 7.53 (d, 1H), 7.42 (s, 1H), 3.94 (m, 2H), 3.57 (t, 2H).

### Beispiel 87

N-(2-[(6-Amino-5-nitropyridin-2-yl)thio]ethyl)-7-(2,4-dichlorphenyl)imidazo[1,2-c]pyrimidin-5-amin

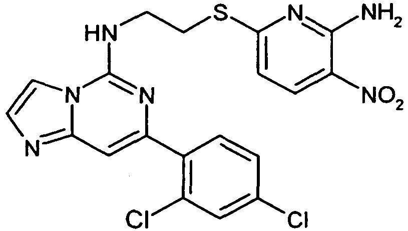

N-{2-[(6-Amino-5-nitropyridin-2-yl)thio]ethyl}-7-(2,4-dichlorphenyl)imidazo[1,2-c]pyrimidin-5-amine (Beispiel 87) wird in Analogie zur Beispiel 82 aus Beispiel 46A (129 mg, 0.6 mmol), 5-Chlor-7-(2,4-dichlorphenyl)imidazo[1,2-c]pyrimidin (Beispiel 6A) (197 mg, 0.7 mmol) und N,N-Diisopropylethylamin (1.0 ml, 6 mmol) hergestellt. Es werden 83 mg (29% d. Th.) Produkt als Feststoff nach Aufreinigung durch präparative RP-HPLC (Gradient Eluent: Wasser:Acetonitril 90:10 bis 10:90) erhalten werden. Als Nebenkomponente wird Beispiel 89 isoliert.

LCMS (Methode 1): Rₜ = 1.70 min. (m/z = 476 (M+H)⁺)

¹H-NMR (400MHz, DMSO-d₆): δ = 9.28 (br m, 1H), 8.42 (br m, 1H), 8.14 (br s, 2H), 7.98 (d, 1H), 7.77 (d, 1H), 7.71 (d, 1H), 7.56 (dd, 1H), 7.37 (s, 1H), 6.66 (d, 1H), 3.94 (m, 2H), 3.50 (m, 2H).

### Beispiel 88

6-[(2-{[7-(2,4-Dichlorphenyl)imidazo[1,2-c]pyrimidin-5-yl]thio}ethyl)amino]nicotinonitril 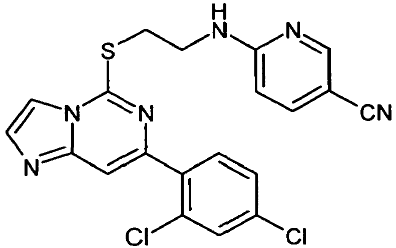 6-[(2-{[7-(2,4-Dichlorphenyl)imidazo[1,2-c]pyrimidin-5-yl]thio} ethyl)amino]nicotinonitril (Beispiel 88) wird in Analogie zu Beispiel 82 aus Beispiel 47A (200 mg, 0.4 mmol), 2-Chlor-5-cyanopyridin (75 mg, 0.5 mmol) und N,N-Diisopropylethylamin (0.8 ml, 4 mmol) hergestellt. Es werden 92 mg (47% d. Ph.) Produkt als Feststoff nach Aufreinigung durch präparative RP-HPLC (Gradient Eluent: Wasser:Acetonitril 90:10 bis 10:90) erhalten.

LCMS (Methode 1): Rₜ = 1.66 min. (m/z = 441 (M+H)⁺)

¹H-NMR (400MHz, DMSO-d₆): δ = 9.53 (br t, 1H), 8.71 (d, 1H), 8.52 (m, 1H), 8.13 (d, 1H), 7.94 (dd, 1H), 7.81 (d, 1H), 7.69 (d, 1H), 7.59 (dd, 1H), 7.53 (dd, 1H), 7.42 (s, 1H), 3.93 (m, 2H), 3.58 (t, 2H).

### Beispiel 89

N⁶-(2-{[7-(2,4-Dichlorphenyl)imidazo[1,2-c]pyrimidin-5-yl]thio}ethyl)-3-nitropyridin-2,6-diamin 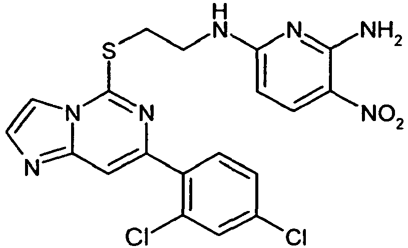 N⁶-(2-{[7-(2,4-Dichlorphenyl)imidazo[1,2-c]pyrimidin-5-yl]thio}ethyl)-3-nitropyridin-2,6-diamin (Beispiel 89) wird in Analogie zu Beispiel 82 aus Beispiel 46A (129 mg, 0.6 mmol), 5-Chlor-7-(2,4-dichlorphenyl)imidazo[1,2-c]pyrimidin (Beispiel 6A) (197 mg, 0.7 mmol) und N,N-Diisopropylethylamin (1.0 ml, 6 mmol) hergestellt. Es werden 949 mg (32% d. Th.) Produkt als Feststoff nach Aufreinigung durch präparative RP-HPLC (Gradient Eluent: Wasser:Acetonitril 90:10 bis 10:90) erhalten. Als Nebenkomponente wird Beispiel 87 isoliert.

LCMS (Methode 1): Rₜ = 2.00 min. (m/z = 476 (M+H)⁺)

¹H-NMR (400MHz, DMSO-d₆): δ = 8.38 (br s, 1H), 8.26 (d, 1H), 8.17 (d, 1H), 7.96 (s, 1H), 7.86 (d, 1H), 7.77 (m, 2H), 7.55 (dd, 1H), 6.20 (br, 2H), 5.85 (d, 1H), 3.80 (m, 4H).

### Beispiel 90

N⁶-(2-{[7-(2,4-Dichlorphenyl)imidazo[1,2-c]pyrimidin-5-yl]oxy}ethyl)-3-nitropyridin-2,6-diamin 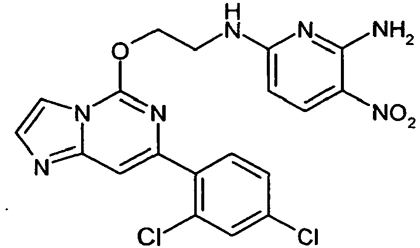 2-[(6-Amino-5-nitropyridin-2-yl)amino]ethanol (Beispiel 48A) wird in DMF (3 ml) bei 0°C vorgelegt und Natriumhydrid (60% Öl-Dispersion, 15 mg, 0.4 mmol) wird zugegeben. Es wird 10 min bei 0°C nachgerührt. 5-Chlor-7-(2,4-dichlorphenyl)imidazo[1,2-c]pyrimidin (Beispiel 6A) (125 mg, 0.4 mmol) in DMF (1 ml) wird zugetropft, und das Reaktionsgemisch wird 12 h bei RT gerührt. Eisessig (200 µl) wird zugegeben, das Reaktionsgemisch wird auf Wasser gegossen und mit Ethylacetat extrahiert (3 x 50 ml). Die vereinigten organischen Phasen werden mit gesättigter wässriger Natriumchloridlösung gewaschen, über Magnesiumsulfat getrocknet und eingeengt. Der Rückstand wird aus Acetonitril asugefällt, abfiltriert, mit Acetonitril nachgewaschen und getrocknet. Es werden 36 mg (18% d. Th.) Produkt als Feststoff erhalten.

LCMS (Methode 3): Rₜ = 2.24 min. (m/z = 460 (M+H)⁺)

¹H-NMR (400MHz, DMSO-d₆): δ = 8.17 (br t, 1H), 8.13 (br s, 2H), 7.93 (d, 1H), 7.88 (s, 1H), 7.75 (d, 1H), 7.71 (d, 1H), 7.68 (d, 1H), 7.55 (s, 1H), 7.51 (dd, 1H), 5.93 (d, 1H), 4.81 (m, 2H), 3.88 (m, 2H).

### Beispiel 91

N-{2-[(6-Amino-5-nitropyridin-2-yl)oxy]ethyl}-7-(2,4-dichlorphenyl)imidazo[1,2-c]pyrimidin-5-amin

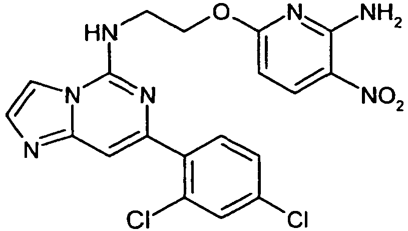

N-{2-[(6-Amino-5-nitropyridin-2-yl)oxy]ethyl}-7-(2,4-dichlorphenyl)imidazo[1,2-c]pyrimidin-5-amin (Beispiel 91) wird in Analogie zu Beispiel 90 aus Beispiel 49A (156 mg, 0.5 mmol), 2-Amino-6-chlor-3-nitropyridin (70 mg, 0.4 mmol) und Natriumhydrid (60% Öl-Dispersion, 15 mg, 0.4 mmol) hergestellt. Es werden 70 mg (38% d. Th.) des Produktes als Feststoff nach Aufreinigung des Rohproduktes durch präparative RP-HPLC (Gradient Eluent: Wasser:Acetonitril 90:10 bis 10:90) erhalten.

LCMS (Methode 1): Rₜ = 1.54 min. (m/z = 460 (M+H)⁺

¹H-NMR (400MHz, DMSO-d₆): δ = 9.32 (br m, 1H), 8.50 (br s, 1H), 8.15 (d, 1H), 8.09 (m, 3H), 7.77 (d, 1H), 7.70 (d, 1H), 7.55 (dd, 1H), 7.40 (s, 1H), 6.04 (d, 1H), 4.59 (m, 2H), 4.03 (m, 2H).

### Beispiel 92

6-[(2-{[7-(2,4-Dichlorphenyl)[1,2,4]triazolo[1,5-c]pyrimidin-5-yl]amino}ethyl)amino]nicotinnitril

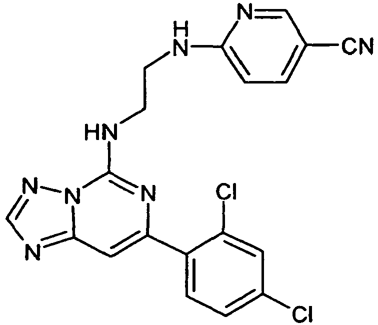

Unter Argon werden in einer Mischung aus 4 ml Dioxan und 1.3 ml gesättigter wässriger Natriumcarbonat-Lösung 60 mg (0.19 mmol) 6-({2-[(7-Chlor[1,2,4]triazolo[1,5-c]pyrimidin-5-yl)amino]ethyl}amino)nicotinnitril (Beispiel 54A), 35.6 mg (0.19 mmol) (2,4-Dichlorphenyl)boronsäure und 21.6 mg (0.019 mmol) Tetrakis(triphenylphosphin)palladium(0) vorgelegt. Die Mischung wird mit Argon entgast und danach für 30 min bei 150°C in der Mikrowelle erhitzt. Nach Abkühlen filtriert man über eine Extrelut^{®}-Kartusche. Man erhält nach Reinigung mittels präparativer HPLC 15.3 mg (19% d. Th.) des Produktes als Feststoff.

LCMS (Methode 5): Rₜ = 3.41 min. (m/z = 425 (M+H)⁺)

¹H-NMR (400MHz, DMSO-d₆): δ = 8.55 (s, 1H), 8.48 (t, 1H), 8.32 (d, 1H), 7.73 (s, 1H), 7.71 (t, 1H), 7.59 (d, 1 H), 7.55 (dd, 1H), 7.51 (d, 1H), 7.24 (s, 1H), 6.48 (s, br, 1H), 3.72 (dd, 2H), 3.63 (s, br, 2H).

### Beispiel 93

6-{[2-({7-[4-(Trifluormethyl)phenyl][1,2,4]triazolo[1,5-c]pyrimidin-5-yl}amino)ethyl]amino}-nicotinnitril

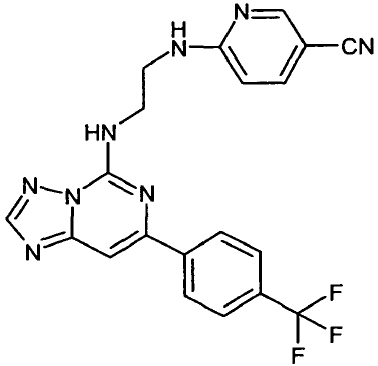

Unter Argon werden in einer Mischung aus 3.3 ml Dioxan und 1.1 ml gesättigter wässriger Natriumcarbonat-Lösung 50 mg (0.16 mmol) 6-({2-[(7-Chlor[1,2,4]triazolo[1,5-c]pyrimidin-5-yl)amino]ethyl}amino)nicotinnitril (Beispiel 54A), 29.5 mg (0.16 mmol) [4-(Trifluormethyl)phenyl]boronsäure und 18 mg (0.016 mmol) Tetrakis(triphenylphosphin)palladium(0) vorgelegt. Die Mischung wird mit Argon entgast und danach für 30 min bei 150°C in der Mikrowelle erhitzt. Nach Abkühlen filtriert man über eine Extrelut^{®}-Kartusche. Man erhält nach Reinigung mittels präparativer HPLC 6.5 mg (10% d. Th.) des Produktes als Feststoff.

LCMS (Methode 5): Rₜ = 3.42 min. (m/z = 425 (M+H)⁺)

¹H-NMR (400MHz, DMSO-d₆): δ = 8.54 (s, 1H), 8.45 (t, 1H), 8.42 (s, br, 1H), 8.22 (d, 2H), 7.78 (d, 2H), 7.76 (t, 1H), 7.72 (s, 1H), 7.54 (s, br, 1H), 6.44 (s, br, 1H), 3.85 (dd, 2H), 3.68 (m, 2H).

### Beispiel 94

N⁶-(2-{[7-(2,4-Dichlorphenyl)[1,2,4]triazolo[1,5-c]pyrimidin-5-yl]amino}ethyl)-3-nitropyridin-2,6-diamin

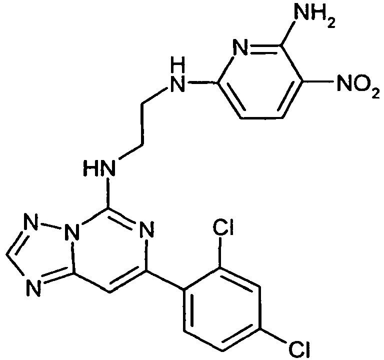

Unter Argon werden in einer Mischung aus 3.4 ml Dioxan und 1.1 ml gesättigter wässriger Natriumcarbonat-Lösung 60 mg (0.16 mmol) N⁶-{2-[(7-Chlor[1,2,4]triazolo[1,5-c]pyrimidin-5-yl)amino]ethyl}-3-nitropyridine-2,6-diamin (Beispiel 6A), 30.3 mg (0.16 mmol) (2,4-Dichlorphenyl)boronsäure und 18.4 mg (0.016 mmol) Tetrakis(triphenylphosphin)palladium(0) vorgelegt. Die Mischung wird mit Argon entgast und danach für 30 min bei 150°C in der Mikrowelle erhitzt. Nach Abkühlen filtriert man über eine Extrelut^{®}-Kartusche. Man erhält nach Reinigung mittels präparativer HPLC 10.9 mg (13% d. Th.) des Produktes als Feststoff.

LCMS (Methode 5): Rₜ = 3.40 min. (m/z = 460 (M+H)⁺)

¹H-NMR (400MHz, DMSO-d₆): δ = 8.58 (s, 1H), 8.53 (t, 1H), 8.14 (s, br, 1H), 8.07 (t, 1H), 7.85 (d, 1H), 7.72 (s, 1H), 7.63 (s, br, 1H), 7.60 (d, 1H), 7.43 (d, 1H), 7.26 (s, 1H), 5.84 (d, 1H), 3.76 (m, 2H), 3.65 (m, 2H).

### Beispiel 95

N⁶-(2-{[7-(3,5-Dimethylphenyl)[1,2,4]triazolo[1,5-c]pyrimidin-5-yl]amino}ethyl)-3-nitropyridin-2,6-diamin

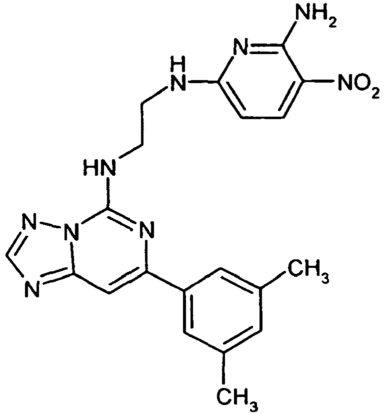

Analog der für Beispiel 94 beschriebenen Vorschrift wird ausgehend von 50 mg (0.133 mmol) N⁶-{2-[(7-Chlor[1,2,4]triazolo[1,5-c]pyrimidin-5-yl)amino]ethyl}-3-nitropyridin-2,6-diamin (Beispiel 55A) durch Kupplung mit 19.9 mg (0.13 mmol) (3,5-Dimethylphenyl)boronsäure und anschließende Reinigung durch präparative HPLC 39.9 mg (72% d. Th.) des Produktes als Feststoff erhalten.

LCMS (Methode 5): Rₜ = 3.42 min. (m/z = 420 (M+H)⁺)

¹H-NMR (400MHz, DMSO-d₆): δ = 8.50 (s, 1H), 8.37 (t, 1H), 8.18 (s, br, 1H), 8.12 (t, 1H), 7.83 (d, 1H), 7.74 (s, br, 1H), 7.67 (s, 2H), 7.53 (s, 1 H), 7.04 (s, 1 H), 5.83 (d, 1), 3.86 (m, 2H), 3.71 (dd, 2H), 2.28 (s, 6H).

### Beispiel 96

3-Nitro-N⁶-[2-({7-[4-(trifluonmethyl)phenyl][1,2,4]triazolo[1,5-c]pyrimidin-5-yl}amino)ethyl]pyridin-2,6-diamin

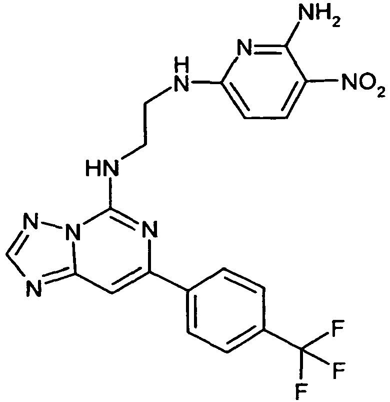

Analog der für Beispiel 94 beschriebenen Vorschrift wird ausgehend von 50 mg (0.133 mmol) N⁶-{2-[(7-Chlor[1,2,4]triazolo[1,5-c]pyrimidin-5-yl)amino]ethyl}-3-nitropyridin-2,6-diamin (Beispiel 55A) durch Kupplung mit 25.2 mg (0.13 mmol) [4-(Trifluormethyl)phenyl]boronsäure und anschließende Reinigung durch präparative HPLC 2.3 mg (4% d. Th.) des Produktes als Feststoff erhalten.

LCMS (Methode 5): Rₜ = 3.41 min. (m/z = 460 (M+H)⁺)

¹H-NMR (400MHz, DMSO-d₆): δ = 8.55 (s, 1H), 8.47 (t, 1H), 8.24 (d, 3H), 8.08 (t, 1H), 7.77 (d, 1H), 7.73 (s, 1H), 7.70 (d, 3H), 5.79 (d, 1H), 3.92 (dd, 2H), 3.69 (dd, 2H).

### Beispiel 97

3-Nitro-N⁶-(2-{[7-(3-pyrrolidin-1-ylphenyl)[1,2,4]triazolo[1,5-c]pyrimidin-5-yl]amino}-ethyl)pyridin-2,6-diamin

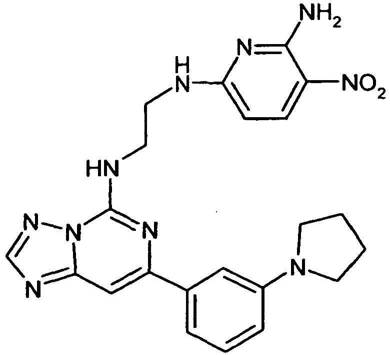

Analog der für Beispiel 94 beschriebenen Vorschrift wird ausgehend von 50 mg (0.133 mmol) N⁶-{2-[(7-Chlor[1,2,4]triazolo[1,5-c]pyrimidin-5-yl)amino]ethyl}-3-nitropyridin-2,6-diamin (Beispiel 55A) durch Kupplung mit 25.3 mg (0.13 mmol) (3-Pyrrolidin-1-ylphenyl)boronsäure und anschließende Reinigung durch präparative HPLC 17.5 mg (28% d. Th.) des Produktes als Feststoff erhalten.

LCMS (Methode 5): Rₜ = 3.46 min. (m/z = 461 (M+H)⁺)

¹H-NMR (400MHz, DMSO-d₆): δ = 8.48 (s, 1H), 8.34 (t, 1H), 8.18 (s, br, 1H), 8.08 (t, 1H), 7.84 (d, 1H), 7.67 (s, br, 1H), 7.53 (s, 1H), 7.28 (d, 1H), 7.25 (s, 1H), 7.13 (t, 1H), 6.60 (d, 1H), 5.85 (d, 1H), 3.85 (dd, 2H), 3.74 (dd, 2H), 3.25 (m, 4H), 1.91-1.96 (m, 4H).

### Beispiel 98

N⁶-(3-{[7-(2,4-Dichlorphenyl)imidazo[1,2-c]pyrimidin-5-yl]amino}propyl)-3-nitropyridin-2,6-diamin

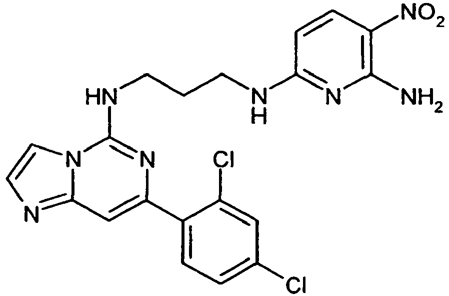

In 3 ml Isopropanol werden 104 mg (0.32 mmol) des Amins (Beispiel 57A) vorgelegt, mit 73.6 mg (0.25 mmol) 5-Chlor-7-(2,4-dichlorphenyl)imidazo[1,2-c]pyrimidin und 95.6 mg (0.74 mmol) DIPEA versetzt und bei 150°C für 2 h in der Mikrowelle erhitzt. Man erhält nach Reinigung mittels präparativer HPLC 96 mg (82% d. Th.) des Produktes.

LCMS (Methode 7): Rₜ = 2.53 min. (m/z = 475 (M+H)⁺)

¹H-NMR (400MHz, DMSO-d₆): δ = 8.10 (s, br, 1H), 8.01 (s, 1H), 7.97 (t, 1H), 7.90 (m, 2H), 7.70 (s, 1H), 7.69 (d, 1H), 7.58 (d, 1H), 7.49 (dd, 1H), 7.10 (s, 1H), 5.89 (d, 1H), 3.59 (dd, 2H), 3.45 (dd, 2H), 3.16 (d, 1H), 1.97 (pent, 2H).

Analog der für Beispiel 7 beschriebenen Vorschrift werden folgende Produkte durch Palladium-katalysierte Kupplung mit den entsprechenden Boronsäuren erhalten:

| **Beispiel** | **Struktur** | **Charakterisierung** |
|---|---|---|
| **99** | 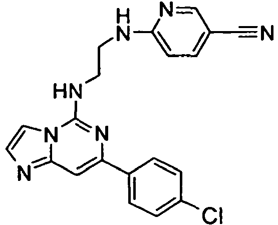 | LC/MS (Methode 7): Rₜ = 2.25 min MS (ESIpos): m/z = 390 (M+H)⁺. |
| **100** | 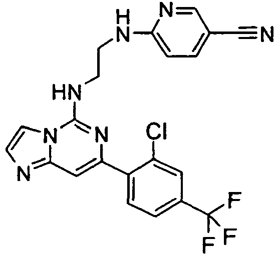 | LC/MS (Methode 1): Rₜ = 1.58 min MS (ESIpos): m/z = 458 (M+H)⁺. |
| **101** | 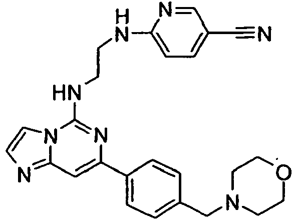 | LC/MS (Methode 4): Rₜ = 4.54 min MS (ESIpos): m/z = 455 (M+H)⁺. |

### Beispiel 102

N⁶-[2-({2-Methyl-7-[4-(trifluormethyl)phenyl]imidazo[1,2-c]pyrimidin-5-yl}amino)ethyl]-3-nitropyridin-2,6-diamin

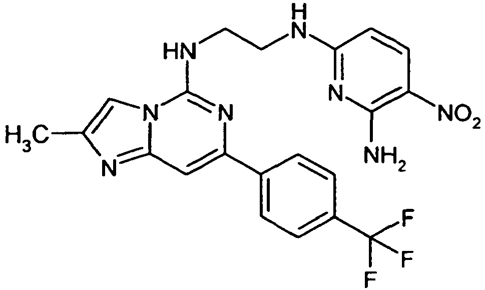

Analog der für Beispiel 7 beschriebenen Vorschrift wird ausgehend von 50 mg (0.13 mmol) N⁶-{2-[(7-Chlor-2-methylimidazo[1,2-c]pyrimidin-5-yl)amino]ethyl}-3-nitropyridin-2,6-diamin (Beispiel 31A) durch Kupplung mit 30.2 mg (0.15 mmol) [4-(Trifluormethyl)phenyl]boronsäure und anschließende Reinigung durch präparative HPLC 30.2 mg (48% d. Th.) des Produktes erhalten.

LCMS (Methode 1): Rₜ = 1.46 min. (m/z = 473 (M+H)⁺)

¹H-NMR (400MHz, DMSO-d₆): δ = 8.69 (s, br, 1H), 8.30 (d, 2H), 8.03 (t, 1H), 7.95 (s, 1H), 7.75-7.8 (m, 3H), 7.67 (s, 1H), 5.76 (d, 1H), 3.93 (s, br, 2H), 3.7 (s, br, 2H), 2.45 (s, 3H).

### Beispiel 103

N⁶-(2-{[7-(3,4-Dimethoxyphenyl)-2-methylimidazo[1,2-c]pyrimidin-5-yl]amino}ethyl)-3-nitropyridin-2,6-diamin

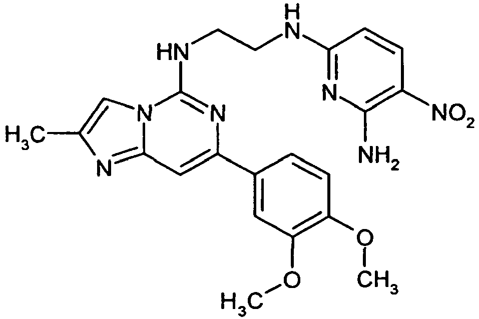

Analog der für Beispiel 7 beschriebenen Vorschrift wird ausgehend von 120 mg (0.32 mmol) N⁶-{2-[(7-Chlor-2-methylimidazo[1,2-c]pyrimidin-5-yl)amino]ethyl}-3-nitropyridin-2,6-diamin (Beispiel 31A) durch Kupplung mit 69 mg (0.38 mmol) (3,4-Dimethoxyphenyl)boronsäure und anschließende Reinigung durch präparative HPLC 57 mg (39% d. Th.) des Produktes erhalten.

LCMS (Methode 1): Rₜ = 1.17 min. (m/z = 465 (M+H)⁺)

¹H-NMR (400MHz, DMSO-d₆): δ = 8.61 (s, br, 1H), 8.03 (t, br, 1H), 7.89 (s, 1H), 7.81 (d, 1H), 7.70 (d, 1H), 7.68 (s, 1H), 7.50 (s, 1H), 6.97 (d, 1H), 5.79 (d, 1H), 3.90 (s, br, 2H), 3.85 (s, 3H), 3.82 (s, 3H), 3.72 (s, br, 2H), 2.44 (s, 3H).

### Beispiel 104

N⁶-(2-{[7-(4-Tert-butylphenyl)-2-methylimidazo[1,2-c]pyrimidin-5-yl]amino}ethyl)-3-nitropyridin-2,6-diamin

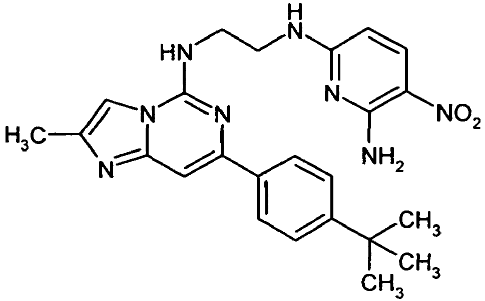

Analog der für Beispiel 7 beschriebenen Vorschrift wird ausgehend von 120 mg (0.32 mmol) N⁶-{2-[(7-Chlor-2-methylimidazo[1,2-c]pyrimidin-5-yl)amino]ethyl}-3-nitropyridin-2,6-diamin (Beispiel 31A) durch Kupplung mit 68 mg (0.38 mmol) (4-tert-Butylphenyl)boronsäure und anschließende Reinigung durch präparative HPLC 34 mg (23% d. Th.) des Produktes erhalten.

LCMS (Methode 1): Rₜ = 1.57 min. (m/z = 461 (M+H)⁺)

¹H-NMR (400MHz, DMSO-d₆): δ = 8.59 (s, br, 1H), 8.19 (s, br, 1H), 8.04 (t, 1H), 7.95 (d, 2H), 7.90 (s, 1H), 7.77 (d, 1H), 7.7 (s, br, 1H), 7.45 (s, 1H), 7.41 (d, 2H), 5.75 (d, 1H), 3.93 (m, 2H), 3.68 (m, 2H), 2.44 (s, 3H), 1.30 (s, 9H).

### Beispiel 105

N⁶-(2-{[7-(3,5-Dimethylphenyl)-2-methylimidazo[1,2-c]pyrimidin-5-yl]amino}ethyl)-3-nitropyridin-2,6-diamin

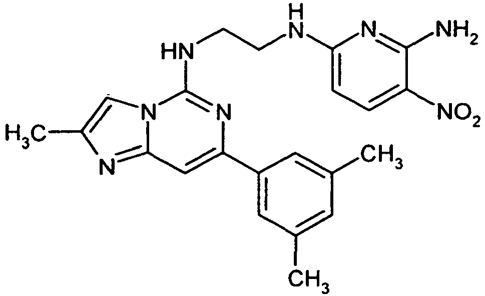

Analog der für Beispiel 7 beschriebenen Vorschrift wird ausgehend von 120 mg (0.32 mmol) N⁶-{2-[(7-Chlor-2-methylimidazo[1,2-c]pyrimidin-5-yl)amino]ethyl}-3-nitropyridin-2,6-diamin (Beispiel 31A) durch Kupplung mit 57 mg (0.38 mmol) (3,5-Dimethylphenyl)boronsäure und anschließende Reinigung durch präparative HPLC 4 mg (3% d. Th.) des Produktes erhalten.

LCMS (Methode 1): Rₜ = 1.44 min. (m/z = 433 (M+H)⁺) ¹H-NMR (400MHz, DMSO-d₆): δ = 8.59 (s, br, 1H), 8.12 (s, br, 1H), 8.03 (t, 1H), 7.92 (s, 1H), 7.81 (d, 1H), 7.70 (s, 1H), 7.6 (s, br, 1H), 7.49 (s, 1H), 7.13 (s, 1H), 5.79 (d, 1H), 3.89 (m, 2H), 3.73 (m, 2H), 2.44 (s, 3H), 2.32 (s, 6H).

### Beispiel 106

N⁶-(2-{[7-(2,3-Dimethylphenyl)-2-methylimidazo[1,2-c]pyrimidin-5-yl]amino}ethyl)-3-nitropyridin-2,6-diamin Trifluoracetat

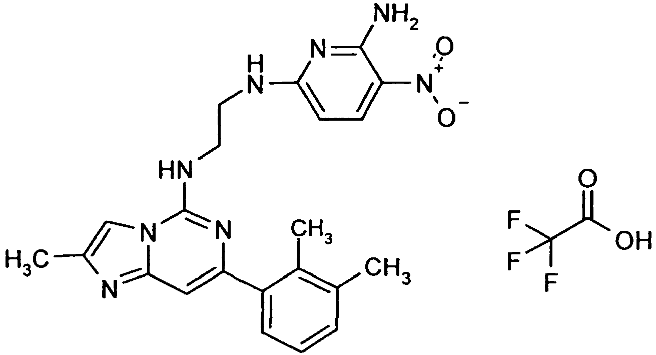

Analog der für Beispiel 7 beschriebenen Vorschrift wird ausgehend von 50 mg (0.13 mmol) N⁶-{2-[(7-Chlor-2-methylimidazo[1,2-c]pyrimidin-5-yl)amino]ethyl}-3-nitropyridin-2,6-diamin (Beispiel 31A) durch Kupplung mit 24 mg (0.16 mmol) (2,3-Dimethylphenyl)boronsäure und anschließende Reinigung durch präparative HPLC 14 mg (19% d. Th.) des Produktes erhalten.

LCMS (Methode 6): Rₜ = 1.15 min. (m/z = 433 (M+H)⁺)

¹H-NMR (400MHz, DMSO-d₆): δ = 8.69 (s, br, 1H), 8.0 (t, br, 1H), 7.95 (s, 1H), 7.84 (d, 1H), 7.45 (s, br, 1H), 7.12-7.29 (m, 3H), 7.07 (s, 1H), 5.81 (d, 1H), 3.77 (m, 2H), 3.64 (m, 2H), 2.46 (s, 3H), 2.29 (s, 3H), 2.23 (s, 3H).

### Beispiel 107

N-(2-{[7-(2,4-Dichlorphenyl)imidazo[1,2-c]pyrimidin-5-yl]oxy}ethyl)-5-(trifluormethyl)pyridin-2-amin

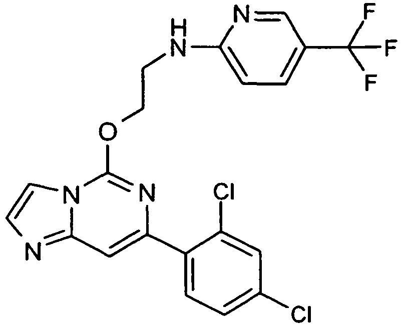

Analog der für Beispiel 90 beschriebenen Vorschrift werden ausgehend von 50 mg (0.17 mmol) 5-Chlor-7-(2,4-dichlorphenyl)imidazo[1,2-c]pyrimidin (Beispiel 6A) durch Reaktion mit 48 mg (0.23 mmol) 2-{[5-(Trifluormethyl)pyridin-2-yl]amino}ethanol, welches analog der Synthese von Beispiel 48A aus 2-Chlor-5-(trifluormethyl)pyridin und Aminoethanol hergestellt werden kann, nach anschließender Reinigung durch präparative HPLC 46 mg (59% d. Th.) des Produktes erhalten.

LCMS (Methode 7): Rₜ = 3.78 min. (m/z = 468 (M+H)⁺)

### Beispiel 108

6-[(2-{[7-(2,4-Dichlorphenyl)imidazo[1,2-c]pyrimidin-5-yl]oxy}ethyl)amino]pyridin-3-carbonitril

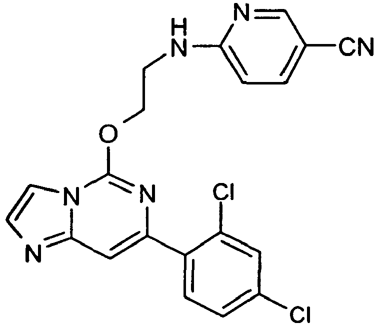

Analog der für Beispiel 90 beschriebenen Vorschrift werden ausgehend von 50 mg (0.17 mmol) 5-Chlor-7-(2,4-dichlorphenyl)imidazo[1,2-c]pyrimidin (Beispiel 6A) durch Reaktion mit 38 mg (0.23 mmol) 6-[(2-Hydroxyethyl)amino]pyridin-3-carbonitril, welches analog der Synthese von Beispiel 48A aus 6-Chlorpyridin-3-carbonitril und Aminoethanol hergestellt werden kann, nach anschließender Reinigung durch präparative HPLC 35 mg (48% d. Th.) des Produktes erhalten.

LCMS (Methode 3): Rₜ = 2.31 min. (m/z = 425 (M+H)⁺) ¹H-NMR (400MHz, DMSO-d₆): δ = 8.33 (d, 1H), 7.87 (t, 1H), 7.84 (s, 1H), 7.75 (d, 1H), 7.6-7.7 (m, 3H), 7.51-7.56 (m, 2H), 6.54 (d, 1H), 4.77 (t, 2H), 3.85 (dd, 2H).

### Beispiel 109

5-({2-[(5-Cyanopyridin-2-yl)amino]ethyl}amino)-7-(2,4-dichlorphenyl)imidazo[1,2-c]pyrimidin-2-carbonsäure

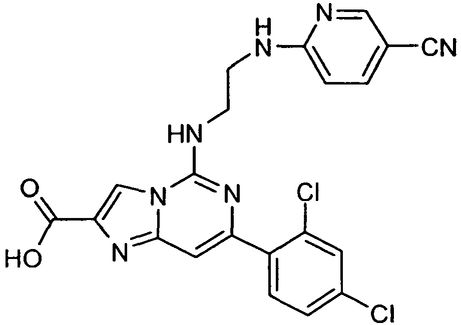

Unter Argon werden in einer Mischung aus 25 ml 1,2-Dimethoxyethan und 10 ml Wasser 1 g (2.59 mmol) Ethyl-7-chlor-5-({2-[(5-cyanopyridin-2-yl)amino)ethyl}amino)imidazo[1,2-c]pyrimidin-2-carboxylat (Beispiel 59A), 580 mg (2.98 mmol) (2,4-Dichlorphenyl)boronsäure und 716 mg (5.18 mmol) Kaliumcarbonat vorgelegt und das Gemisch sorgfältig entgast, bevor 300 mg (0.26 mmol) Tetrakis(triphenylphosphin)palladium(0) zugegeben werden. Die Mischung wird für 16 h auf 120°C erhitzt. Nach Filtration und anschließender Reinigung mittels präparativer HPLC erhält man 185 mg (14% d. Th.) des Produktes als Feststoff.

LCMS (Methode 8): Rₜ = 1.01 min. (m/z = 468 (M+H)⁺)

¹H-NMR (400MHz, DMSO-d₆): δ = 8.66 (s, 1H), 8.36 (d, 1H), 8.35 (t, 1H), 7.77 (m, 1H), 7.73 (d, 1H), 7.63 (d, 1H), 7.59 (dd, 1H), 7.52 (dd, 1H), 7.13 (s, 1H), 6.50 (d, 1H), 7.36-7.8 (m, 4H).

### Beispiel 110

5-({2-[(5-Cyanopyridin-2-yl)amino]ethyl}amino)-7-[4-(trifluormethyl)phenyl]imidazo[1,2-c]pyrimidin-2-carbonsäure

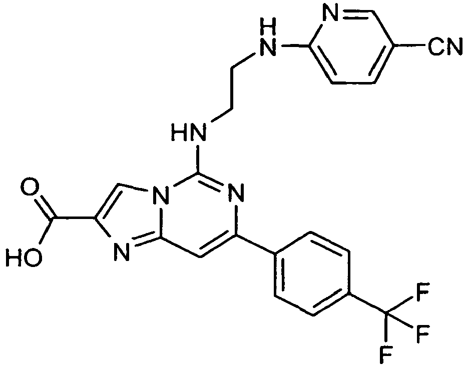

Analog der Herstellung von Beispiel 109 wird durch Pd-katalysierte Kupplung ausgehend von Ethyl-7-chlor-5-({2-[(5-cyanopyridin-2-yl)amino]ethyl}amino)imidazo[1,2-c]pyrimidin-2-carboxylat (Beispiel 59A) und [4-(Trifluormethyl)phenyl]boronsäure das gewünschte Produkt hergestellt.

LCMS (Methode 8): Rₜ = 1.03 min. (m/z = 468 (M+H)⁺)

¹H-NMR (400MHz, DMSO-d₆): δ = 8.69 (s, 1H), 8.44 (m, 2H), 8.24 (d, 2H), 7.79 (d, 3H), 7.59 (m, 1H), 7.56 (s, 1H), 6.48 (d, 1H), 3.85 (dd, 2H), 3.66-3.74 (m, 2H).

Unter Standardkupplungsbedingungen (HATU, DIEA in DMF) werden ausgehend von Beispiel 109 bzw. Beispiel 110 mit den entsprechenden Aminen folgende Amide hergestellt:

| **Beispiel** | **Struktur** | **Charakterisierung** |
|---|---|---|
| **111** | 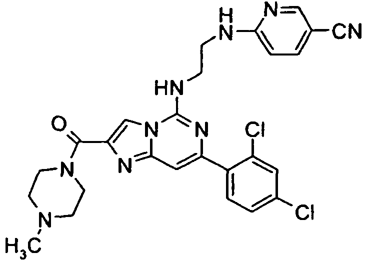 | LC/MS (Methode 1): Rₜ = 1.42 min MS (ESIpos): m/z = 550 (M+H)⁺. |
| **112** | 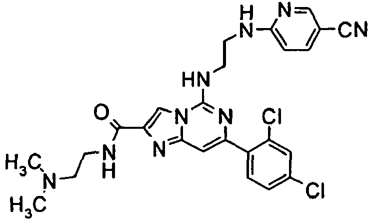 | LC/MS (Methode 1): Rₜ = 1.45 min MS (ESIpos): m/z = 538 (M+H)⁺. |
| **113** | 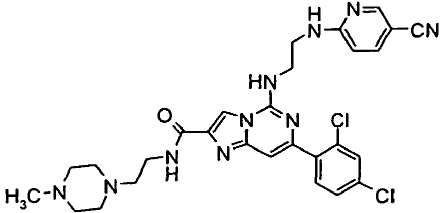 | LC/MS (Methode 6): Rₜ = 1.29 min MS (ESIpos): m/z = 593 (M+H)⁺. |
| **114** | 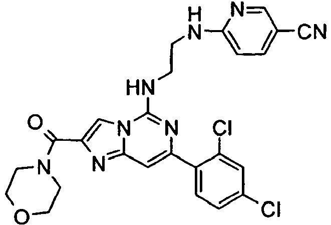 | LC/MS (Methode 3): Rₜ = 2.48 min MS (ESIpos): m/z = 537 (M+H)⁺. |
| **115** | 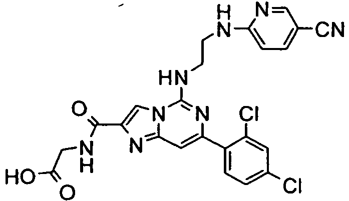 | LC/MS (Methode 6): Rₜ = 1.82 min MS (ESIpos): m/z = 525 (M+H)⁺. |
| **116** | 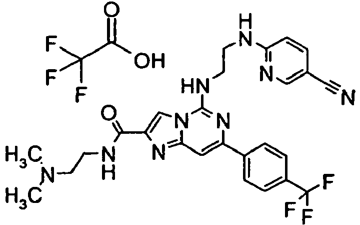 | LC/MS (Methode 3): Rₜ = 1.66 min MS (ESIpos): m/z= 538 (M+H)⁺. |
| **117** | 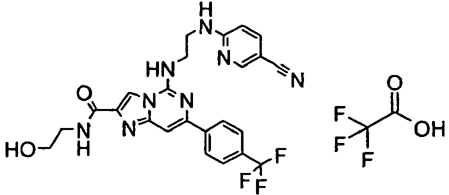 | LC/MS (Methode 6): Rₜ = 1.77 min MS (ESIpos): m/z = 511 (M+H)⁺. |
| **118** | 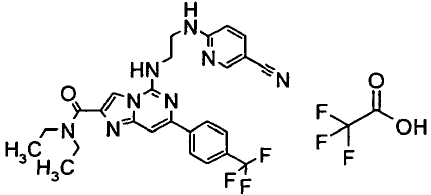 | LC/MS (Methode 6): Rₜ = 2.11 min MS (ESIpos): m/z = 523 (M+H)⁺. |
| **119** | 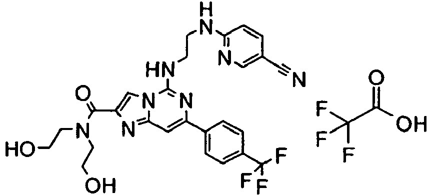 | LC/MS (Methode 3): Rₜ = 2.12 min MS (ESIpos): m/z= 555 (M+H)⁺. |
| **120** | 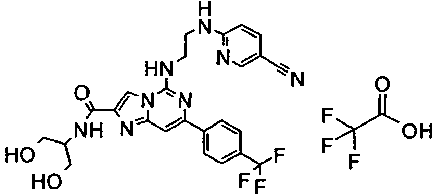 | LC/MS (Methode 6): Rₜ = 1.70 min MS (ESIpos): m/z = 541 (M+H)⁺. |
| **121** | 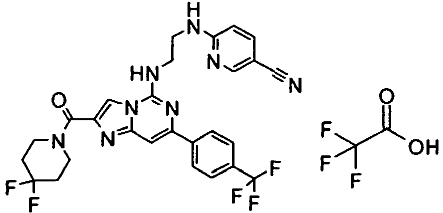 | LC/MS (Methode 6): Rₜ = 2.17 min MS (ESIpos): m/z = 571 (M+H)⁺. |
| **122** | 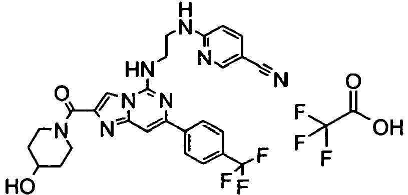 | LC/MS (Methode 3): Rₜ = 2.18 min MS (ESIpos): m/z = 551 (M+H)⁺. |

### Beispiel 123

6-({1-[7-(2,4-Dichlorphenyl)imidazo[1,2-c]pyrimidin-5-yl]piperidin-3-yl}amino)pyridin-3-carbonitril Trifluoracetat

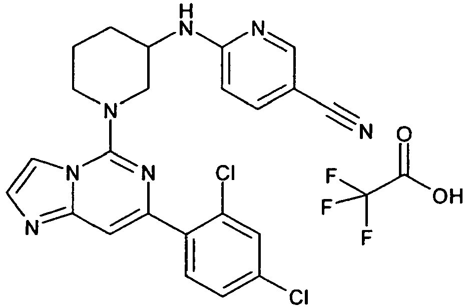

In 2 ml DMSO werden 28.4 mg (0.095 mmol) 5-Chlor-7-(2,4-dichlorphenyl)imidazo[1,2-c]pyrimidin (Beispiel 6A) gelöst und 25 mg (0.124 mmol) 6-(Piperidin-3-ylamino)pyridin-3-carbonitril-Hydrochlorid (Beispiel 61A) und 36.9 mg (0.29 mmol) DIEA zugegeben. Man erhitzt für 2 h bei 150°C in der Mikrowelle. Nach dieser Zeit wird Wasser zugeben und der ausfallende Niederschlag abgesaugt. Dieser wird mittels präparativer HPLC weiter gereinigt. Man erhält 55 mg (98% d. Th.) des Produktes als Feststoff.

LCMS (Methode 1): Rₜ = 1.89 min. (m/z = 464 (M+H)⁺).

¹H-NMR (400MHz, DMSO-d₆): δ = 8.51 (d, 1H), 8.28 (s, 1H), 8.13 (s, 1H), 7.73-7.82 (m, 3H), 7.69 (dd, 1H), 7.58-7.63 (m, 2H), 6.61 (d, 1H), 4.08-4.22 (m, 2H), 3.94 (d, 2H), 3.24 (t, 1H), 2.98 (dd, 1H), 2.03-2.14 (m, 1H), 1.92-2.02 (m, 1H), 1.75-1.89 (m, 1H), 1.62-1.74 (m, 1H).

**Enantiomerentrennung** von 6-({1-[7-(2,4-Dichlorphenyl)imidazo[1,2-c]pyrimidin-5-yl]piperidin-3-yl}amino)pyridin-3-carbonitril Trifluoracetat (Beispiel 123) wird unter den folgenden Bedingungen durchgeführt:
Eine Probe von Beispiel 123 (50 mg) wird in 2 ml Methanol und 6 ml tert-Buthylmethylether aufgenommen und über eine Daicel Chiralpak IA-H 250 mm x 20 mm Säule chromatographiert (Fluß: 15 ml/min; Detektion bei 220 nm; Injektionsvolumen: 450 µl; Eluent: Methanol:tert-Butylmethylether (10:90), Temperatur: 25°C). Es werden zwei Fraktionen isoliert:

### Beispiel Ent-A-123: Es werden 20 mg Produkt in > 98% ee isoliert.

Retentionszeit 6.55 min

### Beispiel Ent-B-123: Es werden 16 mg Produkt in > 95% ee isoliert.

Retentionszeit 6.94 min

Analog der für Beispiel 123 beschriebenen Vorschrift werden folgende Produkte aus den entsprechenden Aminen erhalten:

| **Beispiel** | **Struktur** | **Charakterisierung** |
|---|---|---|
| **124** | 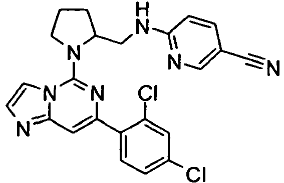 | LC/MS (Methode 8): Rₜ = 1.00 min MS (ESIpos): m/z = 464 (M+H)⁺. |
| **125** | 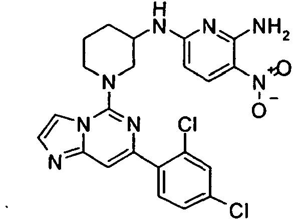 | LC/MS (Methode 8): Rₜ = 1.10 min MS (ESIpos): m/z = 499 (M+H)⁺. |
| **126** | 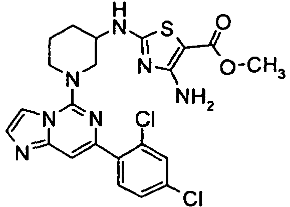 | LC/MS (Methode 6): Rₜ = 1.61 min MS (ESIpos): m/z = 518 (M+H)⁺. |
| **127** | 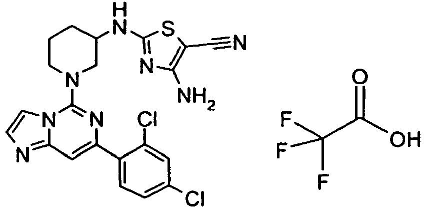 | LC/MS (Methode 3): Rₜ = 1.93 min MS (ESIpos): m/z = 485 (M+H)⁺. |
| **128** | 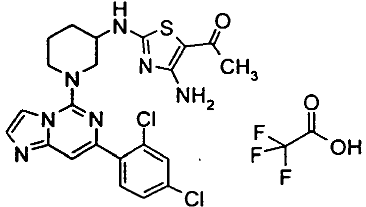 | LC/MS (Methode 6): Rₜ = 1.47 min MS (ESIpos): m/z = 502 (M+H)⁺. |

**Enantiomerentrennung** von 6-[({1-[7-(2,4-Dichlorphenyl)imidazo[1,2-c]pyrimidin-5-yl]pyrrolidin-2-yl}methyl)amino]pyridin-3-carbonitril (Beispiel 124) wird unter den folgenden Bedingungen durchgeführt:
Eine Probe von Beispiel 124 (110 mg) wird in 3 ml Ethanol aufgenommen und über eine Daicel Chiralcel OJ-H, 5µm, 250 mm x 20 mm Säule chromatographiert (Fluß: 15 ml/min; Detektion bei 220 nm; Injektionsvolumen: 500 µl; Eluent: iso-Hexan:Ethanol (50:50), Temperatur: 45°C). Es werden zwei Fraktionen isoliert:

### Beispiel Ent-A-124: Es werden 39 mg Produkt in > 99% ee isoliert.

Retentionszeit 4.54 min

### Beispiel Ent-B-124: Es werden 40 mg Produkt in > 95% ee isoliert.

Retentionszeit 5.55 min

**Enantiomerentrennung** von N⁶-{1-[7-(2,4-Dichlorphenyl)imidazo[1,2-c]pyrimidin-5-yl]piperidin-3-yl}-3-nitropyridin-2,6-diamin (Beispiel 125) wird unter den folgenden Bedingungen durchgeführt:
Eine Probe von Beispiel 125 (171 mg) wird in 8 ml Acetonitril und 5 ml tert-Butylmethylether (TBME) aufgenommen und über eine Daicel Chiralpak IA, 5µm, 250 mm x 20 mm Säule chromatographiert (Fluß: 15 ml/min; Detektion bei 220 nm; Injektionsvolumen: 1500 µl; Eluent: Methanol:TBME (10:90), Temperatur: 30°C). Es werden zwei Fraktionen isoliert:

### Beispiel Ent-A-125: Es werden 81 mg Produkt in > 98% ee isoliert.

Retentionszeit 4.58 min

### Beispiel Ent-B-125: Es werden 80 mg Produkt in > 93% ee isoliert.

Retentionszeit 6.01 min

**Enantiomerentrennung** von 1-[4-Amino-2-({1-[7-(2,4-dichlorphenyl)imidazo[1,2-c]pyrimidin-5-yl]piperidin-3-yl}amino)-1,3-thiazol-5-yl]ethanon Trifluoracetat (Beispiel 128) wird unter den folgenden Bedingungen durchgeführt:
Eine Probe von Beispiel 128 (50 mg) wird in 2 ml Acetonitril und 2 ml Methanol aufgenommen und über eine Daicel Chiralpak IA-H 250 mm x 20 mm Säule chromatographiert (Fluß: 15 ml/min; Detektion bei 320 nm; Injektionsvolumen: 500 µl; Eluent: Methanol:tert-Butylmethylether (20:80), Temperatur: 25°C). Es werden zwei Fraktionen isoliert:

### Beispiel Ent-A-128: Es werden 20 mg Produkt in > 99% ee isoliert.

Retentionszeit 5.31 min

### Beispiel Ent-B-128: Es werden 16 mg Produkt in > 95% ee isoliert.

Retentionszeit 6.72 min

Analog der für Beispiel 123 beschriebenen Vorschrift werden folgende Produkte aus den entsprechenden Aminen erhalten:

| **Beispiel** | **Struktur** | **Charakterisierung** |
|---|---|---|
| **129** | 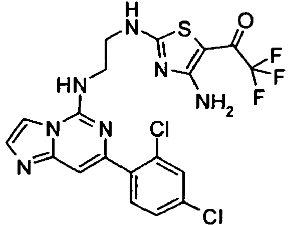 | LC/MS (Methode 6): Rₜ= 1.38 min MS (ESIpos): m/z = 516 (M+H)⁺. |
| **130** | 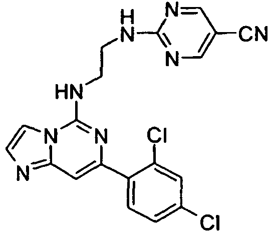 | LC/MS (Methode 3): Rₜ = 1.69 min MS (ESIpos): m/z = 425 (M+H)⁺. |
| **131** | 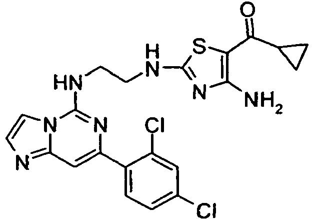 | LC/MS (Methode 3): Rₜ = 1.76 min MS (ESIpos): m/z = 488 (M+H)⁺. |
| **132** | 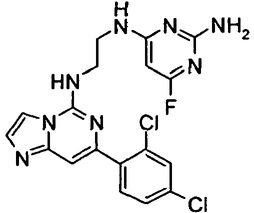 | LC/MS (Methode 3): Rₜ = 1.73 min MS (ESIpos): m/z = 433 (M+H)⁺. |
| **133** | 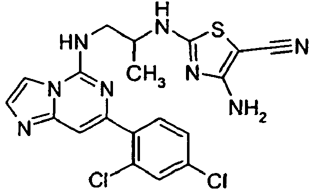 | LC/MS (Methode 3): Rₜ = 1.64 min MS (ESIpos): m/z = 459 (M+H)⁺. |
| **134** | 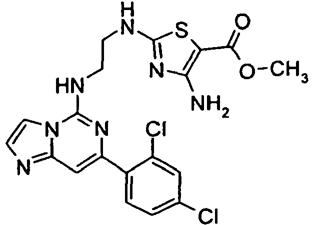 | LC/MS (Methode 6): Rₜ = 1.24 min MS (ESIpos): m/z = 478 (M+H)⁺. |
| **135** | 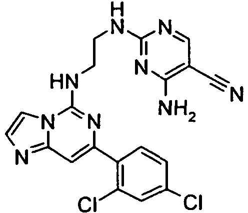 | LC/MS (Methode 8): Rₜ = 0.84 min MS (ESIpos): m/z = 441 (M+H)⁺. |
| **136** | 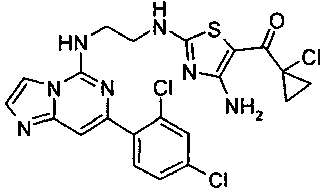 | LC/MS (Methode 8): Rₜ = 1.00 min MS (ESIpos): m/z = 522 (M+H)⁺. |
| **137** | 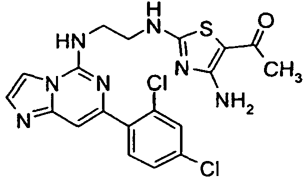 | LC/MS (Methode 8): Rₜ = 0.88 min MS (ESIpos): m/z = 462 (M+H)⁺. |
| **138** | 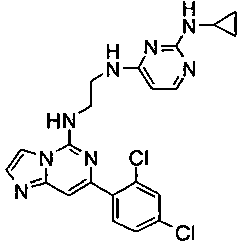 | LC/MS (Methode 3): Rₜ = 1.80 min MS (ESIpos): m/z = 455 (M+H)⁺. |
| **139** | 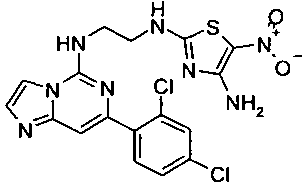 | LC/MS (Methode 3): Rₜ = 1.36 min MS (ESIpos): m/z = 465 (M+H)⁺. |
| **140** | 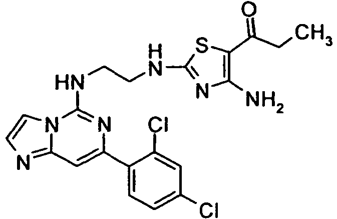 | LC/MS (Methode 3): Rₜ = 1.62 min MS (ESIpos): m/z = 456 (M+H)⁺. |
| **141** | 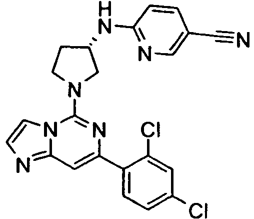 | LC/MS (Methode 3): Rₜ = 2.06 min MS (ESIpos): m/z = 450 (M+H)⁺. |
| **142** | 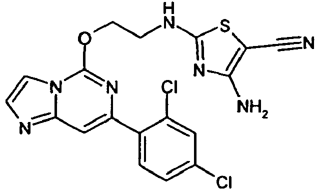 | LC/MS (Methode 8): Rₜ = 0.99 min MS (ESIpos): m/z = 446 (M+H)⁺. |

### Beispiel 143

N-[7-(2,4-Dichlorphenyl)imidazo[1,2-c]pyrimidin-5-yl]-N'-(5-nitro-1,3-thiazol-2-yl)ethan-1,2-diamin

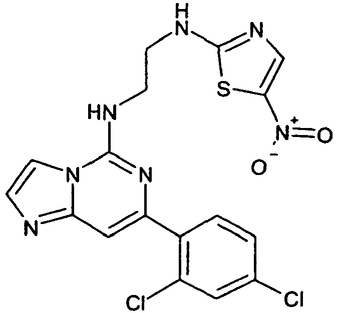

In 3 ml DMSO werden 100 mg (0.238 mmol) des Amins (Beispiel 8A) vorgelegt, mit 76 mg (0.357 mmol) 2-Brom-5-nitro-1,3-thiazol und 154 mg (1.19 mmol) DIEA versetzt und bei 130°C für 30 min in der Mikrowelle erhitzt. Man erhält nach Reinigung mittels präparativer HPLC 35 mg (33% d. Th.) des Produktes als Feststoff.

LCMS (Methode 9): R, = 1.70 min. (m/z = 450 (M+H)⁺)

¹H-NMR (400MHz, DMSO-d₆): δ = 9.42 (s, br, 1H), 8.22 (s, 1H), 8.15 (t, 1H), 7.97 (s, 1H), 7.66 (s, 1H), 7.62 (d, 2H), 7.47 (dd, 1H), 7.13 (s, 1H), 3.74-3.82 (m, 2H), 3.63-3.73 (m, 2H).

### Beispiel 144

2-Amino-6-[(2-{[7-(2,4-dichlorphenyl)-2-(trifluormethyl)imidazo[1,2-c]pyrimidin-5-yl]amino}-ethyl)amino]pyridin-3-carbonitril Trifluoracetat

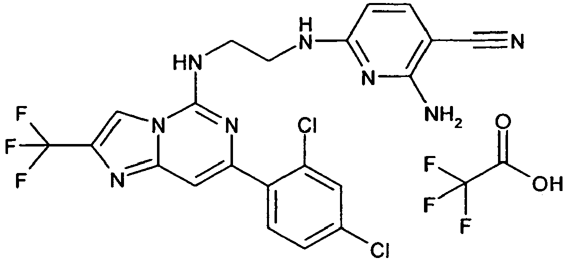

Analog der für Beispiel 7 beschriebenen Vorschrift wird ausgehend von 100 mg (0.25 mmol) 2-Amino-6-[(2-{[7-chlor-2-(trifluormethyl)imidazo[1,2-c]pyrimidin-5-yl]amino}ethyl)amino]-pyridin-3-carbonitril (Beispiel 62A) durch Kupplung mit 52.9 mg (0.277 mmol) (2,4-Dichlorphenyl)boronsäure und anschließende Reinigung durch präparative HPLC 47 mg (29% d. Th.) des Produktes als Feststoff erhalten.

LCMS (Methode 3): Rₜ = 2.66 min. (m/z = 507 (M+H)⁺)

¹H-NMR (400MHz, DMSO-d₆): δ = 8.61 (s, 1H), 8.26 (t, 1H), 7.74 (d, 1H), 7.63 (d, 1H), 7.52 (dd, 1H), 7.3 (s, br, 1 H), 7.27 (d, 1 H), 7.19 (s, 1 H), 6.29 (s, br, 2H), 5.76 (d, 1 H), 3.68 (dd, 2H), 3.52-3.62 (m, br, 2H).

### Beispiel 145

6-[(2-{[7-(2,4-Dichlorphenyl)-2-(morpholin-4-ylmethyl)imidazo[1,2-c]pyrimidin-5-yl]amino}-ethyl)amino]pyridin-3-carbonitril Trifluoracetat

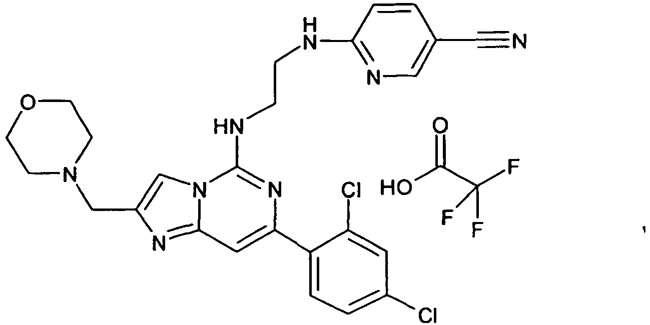

In 3 ml DMSO werden 122 mg (0.268 mmol) des Chlorpyrimidins Beispiel 95A vorgelegt, mit 112 mg (0.4 mmol) 6-[(2-Aminoethyl)amino]pyridine-3-carbonitril und 69 mg (0.54 mmol) DIEA versetzt und bei 150°C für 1.5 h in der Mikrowelle erhitzt. Man erhält nach Reinigung mittels präparativer HPLC 23 mg (13% d. Th.) des Produktes als Feststoff.

LCMS (Methode 9): Rₜ = 1.70 min. (m/z = 450 (M+H)⁺)

¹H-NMR (400MHz, DMSO-d₆): δ = 8.34 (d, 1H), 8.30 (t, 1H), 8.10 (s, 1H), 7.77 (s, br, 1H), 7.74 (d, 1H), 7.65 (d, 1H), 7.60 (dd, 1H), 7.53 (dd, 1H), 7.12 (s, 1H), 6.51 (d, 1H), 4.49 (s, 2H), 4.2-4.7 (m, 4H), 3.72-3.95 (m, 2H), 3.6-3.73 (m, 4H), 3.28 (m, 4H).

### Beispiel 146

6-{[2-({7-(2,4-Dichlorphenyl)-2-[(4-methylpiperazin-1-yl)methyl]imidazo[1,2-c]pyrimidin-5-yl}-amino)ethyl]amino}pyridin-3-carbonitril Trifluoracetat

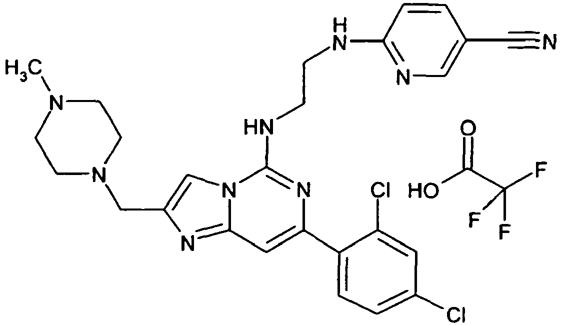

In 2 ml DMSO werden 40 mg (0.081 mmol) des Chlorpyrimidins Beispiel 96A vorgelegt, mit 34 mg (0.122 mmol) 6-[(2-Aminoethyl)amino]pyridine-3-carbonitril und 21 mg (0.16 mmol) DIEA versetzt und bei 150°C für 1 h in der Mikrowelle erhitzt. Man erhält nach Reinigung mittels präparativer HPLC 1.2 mg (2% d. Th.) des Produktes als Feststoff.

LCMS (Methode 8): Rₜ = 0.93 min. (m/z = 536 (M+H)⁺)

¹H-NMR (400MHz, DMSO-d₆): δ = 8.35 (d, 1H), 8.21 (s, br, 1H), 7.96 (s, 1H), 7.77 (s, br, 1H), 7.74 (d, 1H), 7.64 (d, 1H), 7.60 (dd, 1H), 7.53 (dd, 1H), 7.10 (s, 1H), 6.49 (d, 1H), 3.55-3.8 (m, 9H), 2.9-3.2 (m, 6H), 2.78 (m, 2H).

### Beispiel 147

N⁶-[2-({7-(2,4-Dichlorphenyl)-2-[(dimethylamino)methyl]imidazo[1,2-c]pyrimidin-5-yl}amino)-ethyl]-3-nitropyridin-2,6-diamin Trifluoracetat

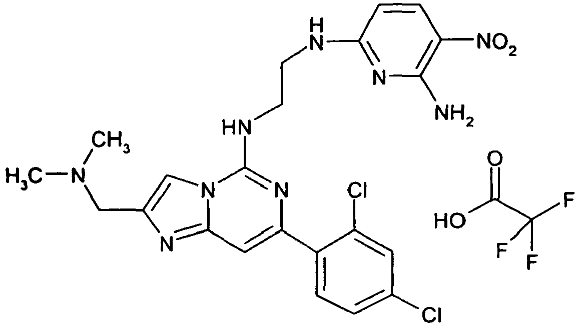

Analog der für Beispiel 7 beschriebenen Vorschrift wird ausgehend von 11 mg (0.027 mmol) N⁶-[2-({7-Chlor-2-[(dimethylamino)methyl]imidazo[1,2-c]pyrimidin-5-yl}amino)ethyl]-3-nitropyridin-2,6-diamin (Beispiel 100A) durch Kupplung mit 6 mg (0.032 mmol) (2,4-Dichlorphenyl)boronsäure und anschließende Reinigung durch präparative HPLC 1 mg (6% d. Th.) des Produktes erhalten.

LCMS (Methode 1): Rₜ = 1.43 min. (m/z = 516 (M+H)⁺)

¹H-NMR (400MHz, DMSO-d₆): δ = 10 (s, br, 1H), 8.22 (s, br, 1H), 8.10 (s, 1H), 8.06 (m, 1H), 7.89 (d, 1 H), 7.72 (s, 1H), 7.66 (d, 1H), 7.52-7.63 (m, 1H), 7.5 (d, 1H), 7.13 (s, 1H), 5.87 (d, 1H), 4.43 (s, 2H), 3.72 (d, 4H), 2.81 (s, 6H).

### Beispiel 148

2-Amino-6-[(2-{[7-(2,4-dichlorphenyl)imidazo[1,2-c]pyrimidin-5-yl]amino}ethyl)amino]pyridin-3-carbonitril

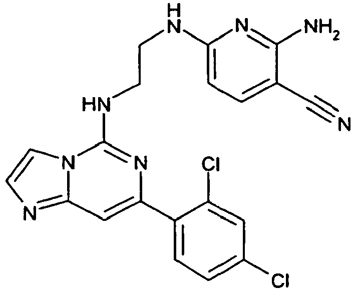

Analog der für Beispiel 5 beschriebenen Vorschrift wird ausgehend von 5-Chlor-7-(2,4-dichlorphenyl)imidazo[1,2-c]pyrimidin (Beispiel 6A) und 2-Amino-6-[(2-aminoethyl)amino]-pyridin-3-carbonitril Dihydrochlorid (Beispiel 111A) das gewünschte Produkt erhalten.

LCMS (Methode 3): Rₜ = 1.70 min. (m/z = 439 (M+H)⁺)

### Beispiel 149

1-{2-Amino-6-[(2-{[7-(2,4-dichlorphenyl)imidazo[1,2-c]pyrimidin-5-yl]amino}ethyl)amino]-pyridin-3-yl}-2,2,2-trifluorethanon Hydrochlorid

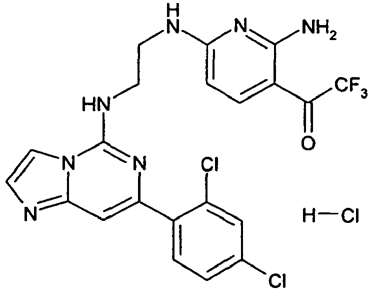

Analog der für Beispiel 5 beschriebenen Vorschrift wird ausgehend von 5-Chlor-7-(2,4-dichlorphenyl)imidazo[1,2-c]pyrimidin (Beispiel 6A) und 1-{2-Amino-6-[(2-aminoethyl)amino]-pyridin-3-yl)-2,2,2-trifluorethanon Hydrochlorid (Beispiel 115A) das gewünschte Produkt erhalten.

LCMS (Methode 8): Rₜ = 1.05 min. (m/z = 510 (M+H-HCl)⁺)

### Beispiel 150

1-{2-Amino-6-[(2-{[7-(2,4-dichlorphenyl)imidazo[1,2-c]pyrimidin-5-yl]amino}ethyl)amino]-pyridin-3-yl}ethanon Hydrochlorid 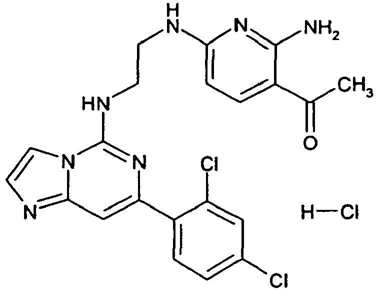 32 mg (0.01 mmol) tert-Butyl-{3-acetyl-6-[(2-([7-(2,4-dichlorphenyl)imidazo[1,2-c]pyrimidin-5-yl]amino}ethyl)amino]pyridin-2-yl}carbamat werden in Dioxan (2 ml) gelöst und 4M Chlorwasserstoff in Dioxan (2 ml) werden zugegeben. Nach 3 h Nachrühren bei RT wird das Lösemittel einrotiert, und der Rückstand wird mittels präparativer HPLC gereinigt. Man erhält 11 mg (39% d. Th.) des Produktes als Feststoff.

LCMS (Methode 8): Rₜ = 0.80 min. (m/z = 456 (M+H-HCl)⁺)

### Beispiel 151

Methyl-2-amino-6-[(2-{[7-(2,4-dichlorphenyl)imidazo[1,2-c]pyrimidin-5-yl]amino}ethyl)amino]-pyridin-3-carboxylat

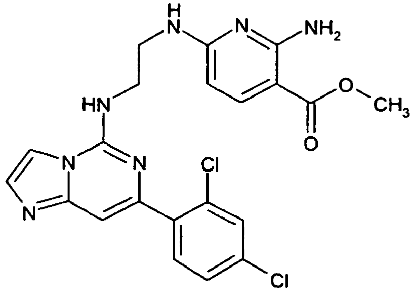

Analog der für Beispiel 5 beschriebenen Vorschrift wird ausgehend von 5-Chlor-7-(2,4-dichlorphenyl)imidazo[1,2-c]pyrimidin (Beispiel 6A) und Methyl-2-amino-6-[(2-aminoethyl)amino]pyridin-3-carboxylat (Beispiel 120A) das gewünschte Produkt erhalten.

LCMS (Methode 6): Rₜ = 1.10 min. (m/z = 472 (M+H)⁺)

### Beispiel 152

Ethyl-2-amino-6-[(2-{[7-(2,4-dichlorphenyl)imidazo[1,2-c]pyrimidin-5-yl]amino}ethyl)amino]-pyridin-3-carboxylat

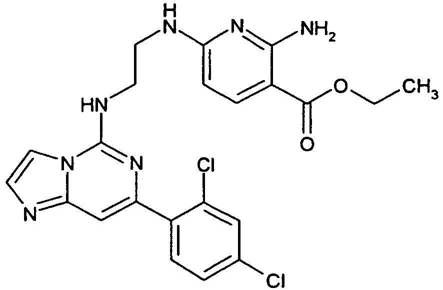

Analog der für Beispiel 5 beschriebenen Vorschrift wird ausgehend von 5-Chlor-7-(2,4-dichlorphenyl)imidazo[1,2-c]pyrimidin (Beispiel 6A) und Ethyl-2-amino-6-[(2-aminoethyl)amino]pyridin-3-carboxylat das gewünschte Produkt erhalten.

LCMS (Methode 3): Rₜ = 1.49 min. (m/z = 468 (M+H)⁺)

### Beispiel 153

6-[(2-{[3-Brom-7-(2,4-dichlorphenyl)imidazo[1,2-c]pyrimidin-5-yl]amino}ethyl)amino]pyridin-3-carbonitril

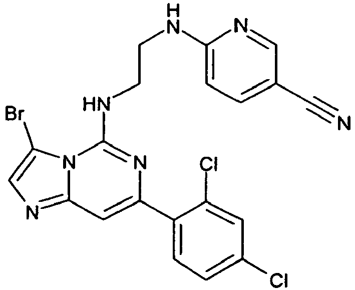

Analog der für Beispiel 5 beschriebenen Vorschrift wird ausgehend von 3-Brom-5-chlor-7-(2,4-dichlorphenyl)imidazo[1,2-c]pyrimidin (Beispiel 121A) und 6-[(2-Aminoethyl)amino]pyridin-3-carbonitril (Beispiel 13A) das gewünschte Produkt als Feststoff in 46% Ausbeute erhalten.

LCMS (Methode 3): Rₜ = 2.63 min. (m/z = 504 (M+H)⁺)

### Beispiel 154

6-[(2-{[3-Chlor-7-(2,4-dichlorphenyl)imidazo[1,2-c]pyrimidin-5-yl]amino}ethyl)amino]pyridin-3-carbonitril

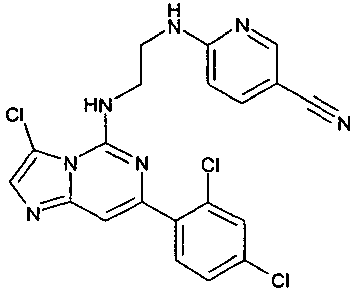

Analog der für Beispiel 5 beschriebenen Vorschrift wird ausgehend von 3,5-Dichlor-7-(2,4-dichlorphenyl)imidazo[1,2-c]pyrimidin (Beispiel 122A) und 6-[(2-Aminoethyl)amino]pyridin-3-carbonitril (Beispiel 13A) das gewünschte Produkt als Feststoff in 52% Ausbeute erhalten.

LCMS (Methode 6): Rₜ = 2.10 min. (m/z = 458 (M+H)⁺)

### Beispiel 155

6-[(2-{[3,8-Dibrom-7-(2,4-dichlorphenyl)imidazo[1,2-c]pyrimidin-5-yl]amino}ethyl)amino]-pyridin-3-carbonitril

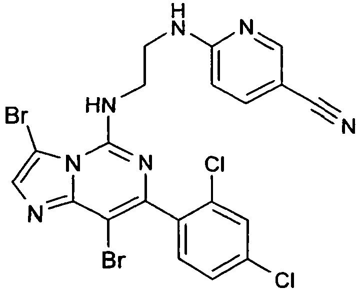

Analog der für Beispiel 5 beschriebenen Vorschrift wird ausgehend von 3,8-Dibrom-5-chlor-7-(2,4-dichlorphenyl)imidazo[1,2-c]pyrimidin (Beispiel 125A) und 2-Amino-6-[(2-aminoethyl)-amino]pyridin-3-carbonitril (Beispiel 13A) das gewünschte Produkt in 75% Ausbeute erhalten.

LCMS (Methode 8): Rₜ = 1.37 min. (m/z = 580 (M+H)⁺)

### Beispiel 156

6-[(2-{[3,8-Dichlor-7-(2,4-dichlorphenyl)imidazo[1,2-c]pyrimidin-5-yl]amino}ethyl)amino]-pyridin-3-carbonitril

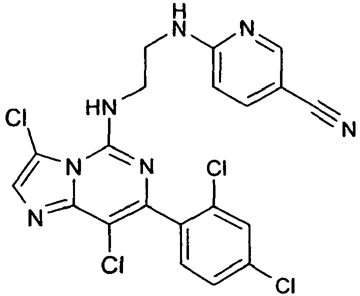

Analog der für Beispiel 5 beschriebenen Vorschrift wird ausgehend von 3,5,8-Trichlor-7-(2,4-dichlorphenyl)imidazo[1,2-c]pyrimidin (Beispiel 126A) und 2-Amino-6-[(2-aminoethyl)-amino]pyridin-3-carbonitril (Beispiel 13A) das gewünschte Produkt in 48% Ausbeute erhalten.

LCMS (Methode 3): Rₜ = 2.67 min. (m/z = 492 (M+H)⁺)

### Beispiel 157

6-{[2-({2-(Morpholin-4-ylmethyl)-7-[4-(trifluormethyl)phenyl][1,2,4]triazolo[1,5-c]pyrimidin-5-yl}amino)ethyl]amino}pyridin-3-carbonitril 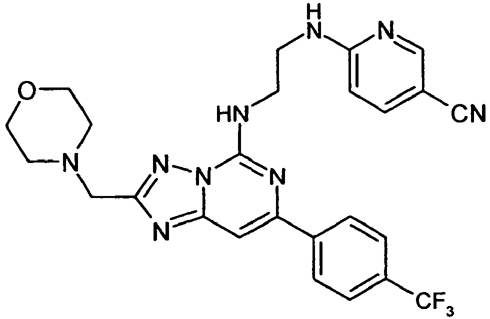 300 mg (0.75 mmol) 5-Chlor-2-(morpholin-4-ylmethyl)-7-[4-(trifluormethyl)phenyl][1,2,4]tria-zolo[1,5-c]pyrimidin (Beispiel 151A) und 180 mg (0.91 mmol) 6-[(2-Aminoethyl)amino]pyridin-3-carbonitril (Beispiel 13A) werden in DMSO (4 ml) vorgelegt, 0.79 ml (4.5 mmol) DIEA wird zugegeben, und die Reaktionslösung wird 30 min bei 140°C im Mikrowellenreaktor bestrahlt. Das Reaktionsgemisch wird mit Ethylacetat verdünnt, und die organische Phase wird mit gesättigter Natriumhydrogencarbonat-Lösung, Ammoniumchlorid-Lösung und gesättigter Natriumchlorid-Lösung gewaschen, getrocknet und eingeengt. Der Rückstand wird in Acetonitril (4 ml) aufgenommen, und der dabei ausgefallene Feststoff wird abfiltriert und getrocknet. Es werden 300 mg (76% d. Th.) des Produktes als ein Feststoff erhalten.

LCMS (Methode 8): Rₜ = 1.04 min. (m/z = 524 (M+H)⁺)

¹H-NMR (400MHz, DMSO-d₆): δ = 8.47 (t 1H), 8.43 (m, 1H), 8.19 (d, 2H), 7.77 (d, 2H), 7.63 (s, 1H), 7.73 (br m, 1H), 6.43 (br m, 1H), 3.85 (m, 2H), 3.74 (s, 2H), 3.67 (m, 2H), 3.61 (m, 4H), 3.28 (m, 4H).

### Beispiel 158

6-[(2-{[7-(2,4-Dichlorphenyl)-2-(morpholin-4-ylmethyl)[1,2,4]triazolo[1,5-c]pyrimidin-5-yl]-amino}ethyl)amino]pyridin-3-carbonitril 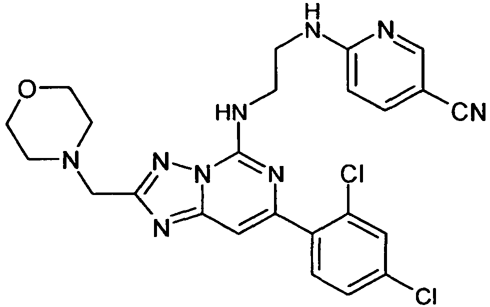 100 mg (0.25 mmol) 5-Chlor-7-(2,4-dichlorphenyl)-2-(morpholin-4-ylmethyl)[1,2,4]triazolo[1,5-c]pyrimidin (Beispiel 150A) und 60 mg (0.30 mmol) 6-[(2-Aminoethyl)amino]pyridin-3-carbonitril (Beispiel 13A) werden in DMSO (4 ml) vorgelegt, 0.26 ml (1.5 mmol) DIEA wird zugegeben, und die Reaktionslösung wird 45 min bei 140°C im Mikrowellenreaktor bestrahlt. Das Reaktionsgemisch wird mittels präparativer HPLC gereinigt. Es werden 62 mg (46% d. Th.) des Produktes als ein Feststoff erhalten.

LCMS (Methode 3): Rₜ = 1.72 min. (m/z = 524 (M+H)⁺)

### Beispiel 159

Ethyl-5-({2-[(5-cyanpyridin-2-yl)amino]ethyl}amino)-7-[4-(trifluormethyl)phenyl][1,2,4]tria-zolo[1,5-c]pyrimidin-2-carboxylat 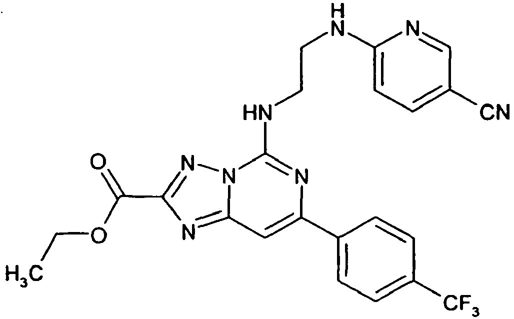 200 mg (0.52 mmol) Ethyl-5-chlor-7-(4-trifluormethylphenyl)[1,2,4]triazolo[1,5-c]pyrimidin-2-carboxylat (Beispiel 149A) und 123 mg (0.62 mmol) 6-[(2-Aminoethyl)amino]pyridin-3-carbonitril (Beispiel 13A) werden in DMSO (2 ml) vorgelegt, 0.54 ml (3.1 mmol) DIEA wird zugegeben, und die Reaktionslösung wird 30 min bei 90°C im Mikrowellenreaktor bestrahlt. Das Reaktionsgemisch wird mittels präparativer HPLC gereinigt. Es werden 98 mg (38% d. Th.) des Produktes als ein Feststoff erhalten.

LCMS (Methode 8): Rₜ = 1.31 min. (m/z = 497 (M+H)⁺)

¹H-NMR (400MHz, DMSO-d₆): δ = 8.79 (t, 1H), 8.45 (m, 1H), 8.20 (d, 2H), 7.86 (s, 1H), 7.79 (d, 2H), 7.77 (s, 1H), 7.50 (br m, 1H), 6.40 (br m, 1H), 4.43 (q, 2H), 3.86 (m, 2H), 3.67 (m, 2H), 1.37 (t, 3H).

### Beispiel 160

Ethyl-5-({2-[(6-amino-5-cyanpyridin-2-yl)amino]ethyl}amino)-7-(4-(trifluormethyl)phenyl] [1,2,4]triazolo[1,5-c]pyrimidin-2-carboxylat 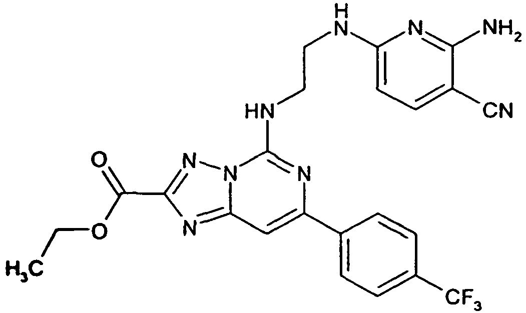 200 mg (0.52 mmol) Ethyl-5-chlor-7-(4-trifluormethylphenyl)[1,2,4]triazolo[1,5-c]pyrimidin-2-carboxylat (Beispiel 149A) und 133 mg (0.62 mmol) 2-Amino-6-[(2-aminoethyl)amino]pyridin-3-carbonitril Dihydrochlorid (Beispiel 111A) werden in DMSO (2 ml) vorgelegt, 0.54 ml (3.1 mmol) DIEA wird zugegeben, und die Reaktionslösung wird 30 min bei 90°C im Mikrowellenreaktor bestrahlt. Das Reaktionsgemisch wird mittels präparativer HPLC gereinigt. Es werden 98 mg (38% d. Th.) des Produktes als ein Feststoff erhalten.

LCMS (Methode 8): Rₜ = 1.30 min. (m/z = 512 (M+H)⁺)

¹H-NMR (400MHz, DMSO-d₆): δ = 8.73 (t, 1H), 8.24 (d, 2H), 7.81 (d, 2H), 7.77 (s, 1H), 7.32 (br m, 1H), 7.22 (br m, 1H), 6.31 (br s, 1H), 5.68 (br m, 1H), 4.44 (q, 2H), 3.85 (m, 2H), 3.58 (m, 2H), 1.37 (t, 3H).

Analog der für Beispiel 5 oder Beispiel 7 beschriebenen Vorschriften (oder explizit anders aufgeführt) werden folgende Produkte erhalten:

| **Beispiel** | **Struktur** | **Charakterisierung** |
|---|---|---|
| **161** | 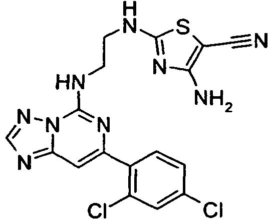 | LC/MS (Methode 6): Rₜ = 1.76 min MS (ESIpos): m/z = 446 (M+H)⁺. |
| **162** | 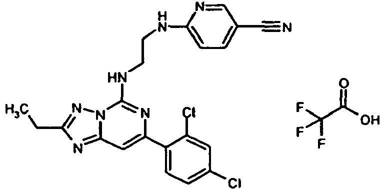 | LC/MS (Methode 6): Rₜ = 2.14 min MS (ESIpos): m/z = 453 (M+H)⁺. |
| **163** | 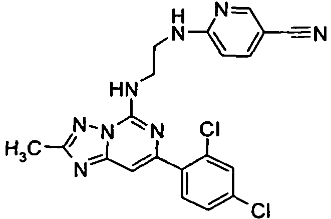 | LC/MS (Methode 8): Rₜ = 1.25 min MS (ESIpos): m/z = 439 (M+H)⁺. |
| **164** | 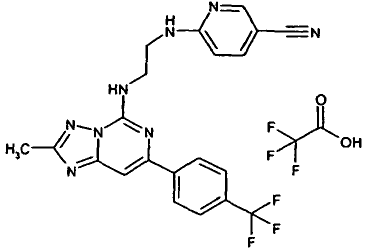 | LC/MS (Methode 3): Rₜ = 2.61 min MS (ESIpos): m/z = 439 (M+H)⁺. |
| **165** | 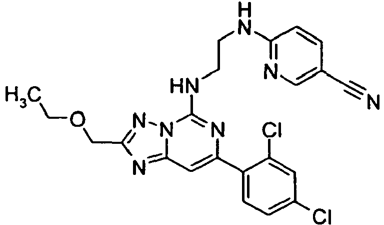 | LC/MS (Methode 8): Rₜ = 1.30 min MS (ESIpos): m/z = 483 (M+H)⁺. |
| **166** | 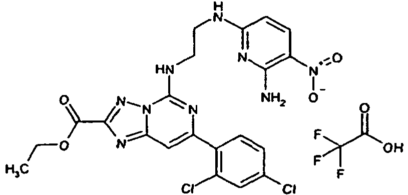 | LC/MS (Methode 8): Rₜ = 1.33 min MS (ESIpos): m/z = 532 (M+H)⁺. |
| **167** | 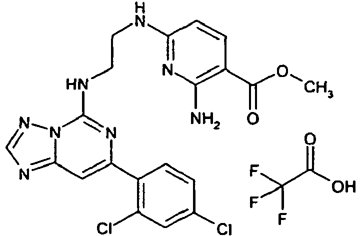 | LC/MS (Methode 3): Rₜ = 2.01 min MS (ESIpos): m/z = 473 (M+H)⁺. |
| **168** | 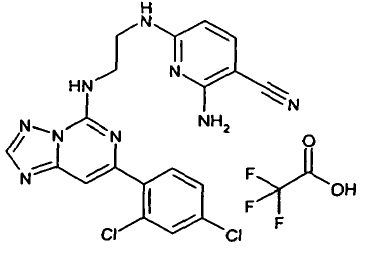 | LC/MS (Methode 8): Rₜ = 1.20 min MS (ESIpos): m/z = 440 (M+H)⁺. |
| **169** | 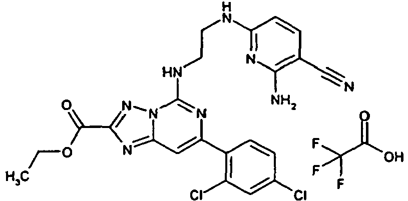 | LC/MS (Methode 6): Rₜ = 2.11 min MS (ESIpos): m/z = 512 (M+H)⁺. |
| **170** | 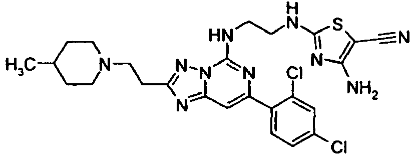 | LC/MS (Methode 3): Rₜ = 1.66 min MS (ESIpos): m/z = 571 (M+H)⁺. |
| **171** | 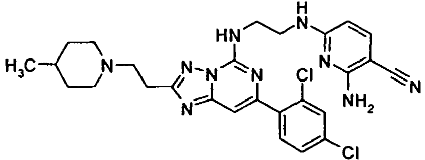 | LC/MS (Methode 3): Rₜ = 1.73 min MS (ESIpos): m/z = 565 (M+H)⁺. |
| **172** | 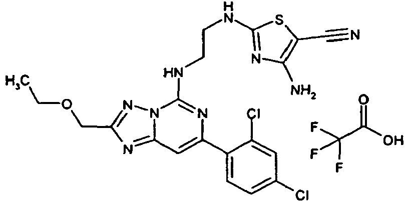 | LC/MS (Methode 8): Rₜ = 1.21 min MS (ESIpos): m/z = 504 (M+H)⁺. |
| **173** | 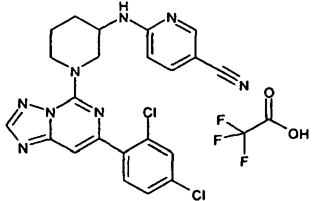 | LC/MS (Methode 8): Rₜ = 1.38 min MS (ESIpos): m/z = 465 (M+H)⁺. |
| **174** | 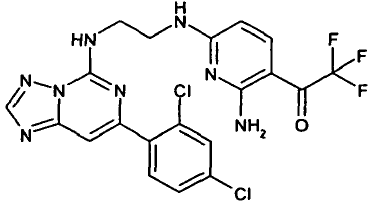 | LC/MS (Methode 9): Rₜ = 2.56 min MS (ESIpos): m/z = 511 (M+H)⁺. |
| **175** | 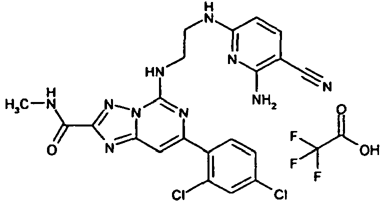 | LC/MS (Methode 6): Rₜ = 1.85 min MS (ESIpos): m/z = 497 (M+H)⁺. |
| **176** | 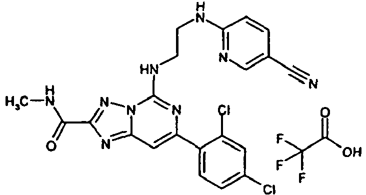 | LC/MS (Methode 6): Rₜ = 1.85 min MS (ESIpos): m/z = 482 (M+H)⁺. |
| **177** | 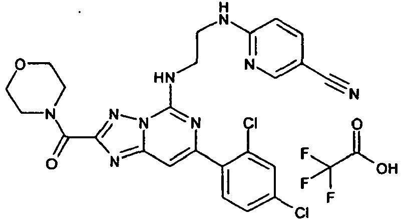 | LC/MS (Methode 9): Rₜ = 2.21 min MS (ESIpos): m/z = 538 (M+H)⁺. |
| **178** | 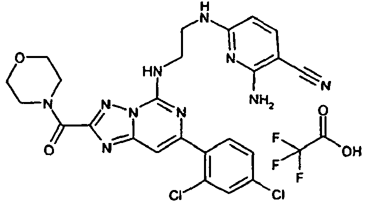 | LC/MS (Methode 8): Rₜ = 1.18 min MS (ESIpos): m/z = 553 (M+H)⁺. |
| **179** | 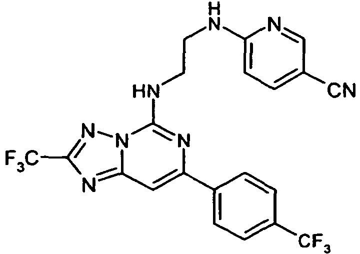 | LC/MS (Methode 8): Rₜ = 1.28 min MS (ESIpos): m/z = 453 (M+H)⁺. ¹H-NMR (400MHz, DMSO-d₆): δ = 8.59 (s, 1H), 8.43 (d, 2H), 7.93 (d, 2H), 2.59 (s, 3H). |
| **180** | 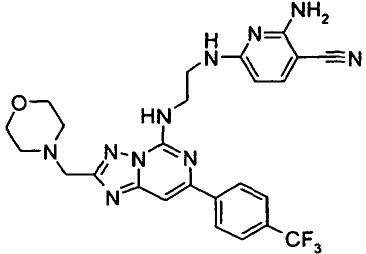 | LC/MS (Methode 8): Rₜ = 1.02 min MS (ESIpos): m/z = 539 (M+H)⁺. |
| **181** | 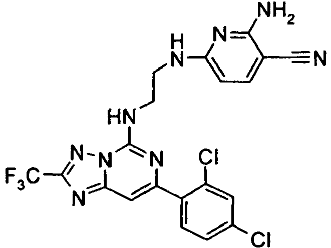 | LC/MS (Methode 3): Rₜ = 2.82 min MS (ESIpos): m/z = 508 (M+H)⁺. |
| **182** | 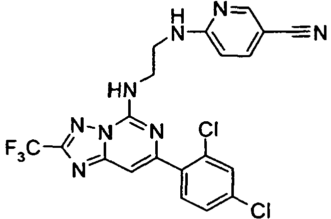 | LC/MS (Methode 8): Rₜ = 1.47 min MS (ESIpos): m/z = 493 (M+H)⁺. |
| **183** | 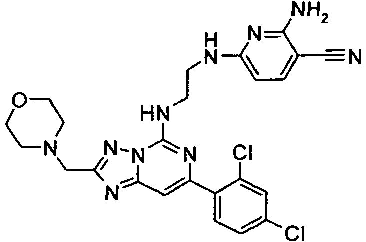 | LC/MS (Methode 3): Rₜ = 1.70 min MS (ESIpos): m/z = 539 (M+H)⁺. |
| **184** | 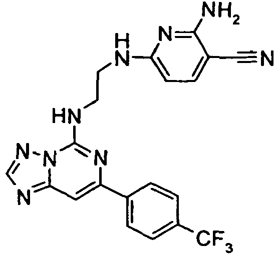 | LC/MS (Methode 3): Rₜ = 2.40 min MS (ESIpos): m/z = 440 (M+H)⁺. ¹H-NMR (400MHz, DMSO-d₆): δ = 8.55 (s, 1H), 8.51 (m, 1H), 8.26 (d, 2H), 7.80 (d, 2H), 7.74 (s, 1H), 7.35 (br m, 1H), 7.25 (br m, 1H), 6.35 (br m, 2H), 5.73 (br m, 1H), 3.84 (br m, 2H), 3.59 (br m, 2H). |
| **185** | 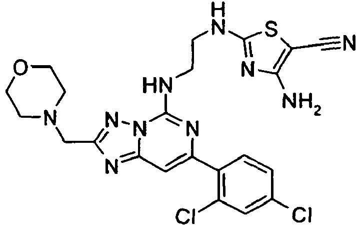 | LC/MS (Methode 6): Rₜ = 1.30 min MS (ESIpos): m/z = 545 (M+H)⁺. ¹H-NMR (400MHz, DMSO-d₆): δ = 8.50 (m, 2H), 7.75 (d, 1H), 7.63 (d, 1H), 7.53 (dd, 1H), 7.22 (s, 1H), 6.69 (s, 2H), 3.74 (s, 2H), 3.72 (m, 2H), 3.58 (m, 4H), 3.53 (m, 2H), 1.40 (s, 4H). |
| **186** | 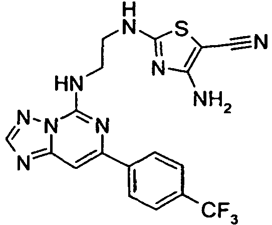 | LC/MS (Methode 6): Rₜ = 1.78 min MS (ESIpos): m/z = 446 (M+H)⁺. ¹H-NMR (400MHz, DMSO-d₆): δ = 8.57 (s, 1H), 8.50 (m, 2H), 8.27 (d, 2H), 7.81 (d, 2H), 7.77 (s, 1H), 3.86 (m, 2H), 3.58 (m, 2H). |
| **187** | 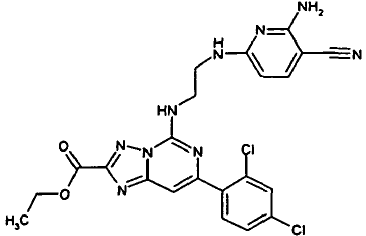 | LC/MS (Methode 8): Rₜ = 1.31 min MS (ESIpos): m/z = 512 (M+H)⁺. |
| **188** | 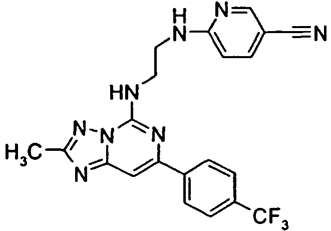 | LC/MS (Methode 8): Rₜ = 1.25 min MS (ESIpos): m/z = 439 (M+H)⁺. |
| **189** | 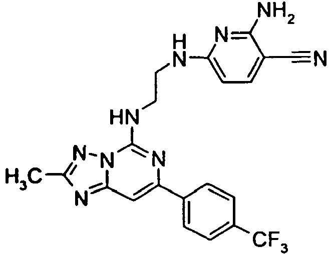 | LC/MS (Methode 8): Rₜ = 1.23 min MS (ESIpos): m/z = 454 (M+H)⁺. |
| **190** | 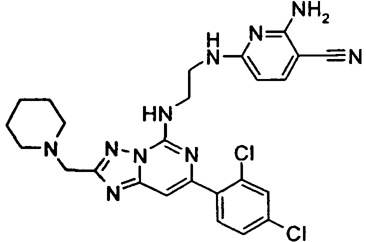 | LC/MS (Methode 8): Rₜ = 1.03 min MS (ESIpos): m/z = 537 (M+H)⁺. |
| **191** | 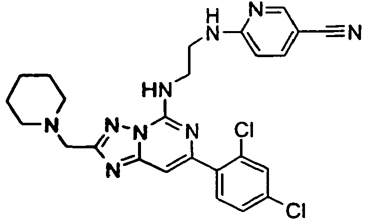 | LC/MS (Methode 9): Rₜ = 1.79 min MS (ESIpos): m/z = 522 (M+H)⁺. |
| **192** | 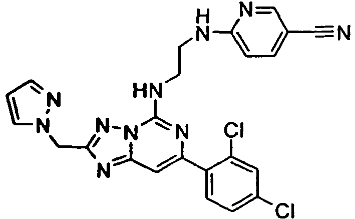 | LC/MS (Methode 8): Rₜ = 1.24 min MS (ESIpos): m/z = 505 (M+H)⁺. |
| **193** | 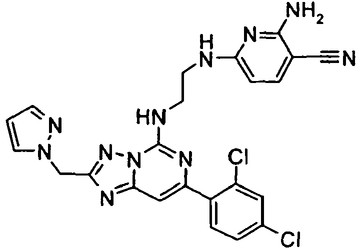 | LC/MS (Methode 3): Rₜ = 2.44 min MS (ESIpos): m/z = 520 (M+H)⁺. |
| **194** | 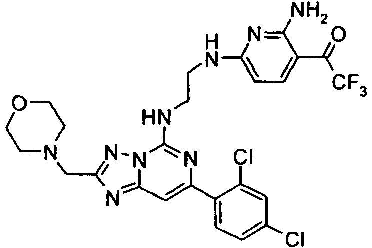 | LC/MS (Methode 6): Rₜ = 1.64 min MS (ESIpos): m/z = 610 (M+H)⁺. |
| **195** | 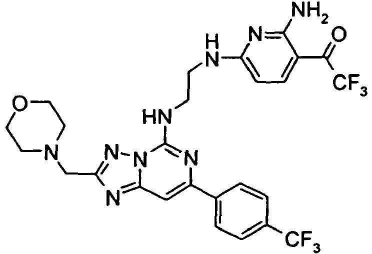 | LC/MS (Methode 6): Rₜ = 1.60 min MS (ESIpos): m/z = 610 (M+H)⁺. |
| **196** | 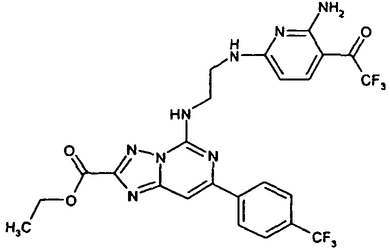 | LC/MS (Methode 6): Rₜ = 2.47 min MS (ESIpos): m/z = 583 (M+H)⁺. |
| **197** | 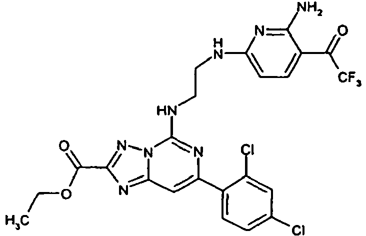 | LC/MS (Methode 6): Rₜ = 2.52 min MS (ESIpos): m/z = 583 (M+H)⁺. |
| **198** | 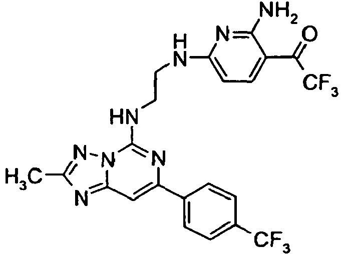 | LC/MS (Methode 6): Rₜ = 2.30 min MS (ESIpos): m/z = 525 (M+H)⁺. |
| **199** | 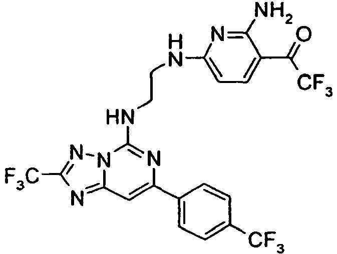 | LC/MS (Methode 6): Rₜ = 2.61 min MS (ESIpos): m/z = 579 (M+H)⁺. |
| **200** | 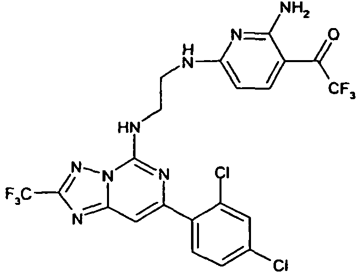 | LC/MS (Methode 6): Rₜ = 2.68 min MS (ESIpos): m/z = 579 (M+H)⁺. |
| **201** | 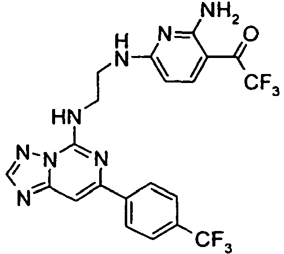 | LC/MS (Methode 6): Rₜ = 2.25 min MS (ESIpos): m/z = 511 (M+H)⁺. |
| **202** | 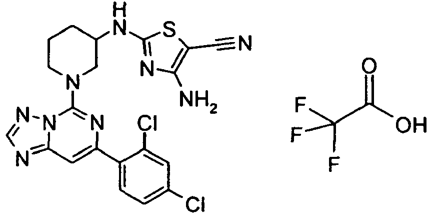 | LC/MS (Methode 8): Rₜ = 1.26 min MS (ESIpos): m/z = 486 (M+H)⁺. |
| **203** | 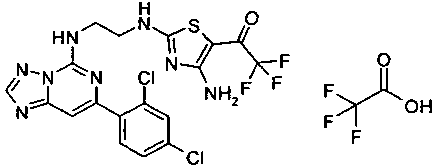 | LC/MS (Methode 3): Rₜ = 2.46 min MS (ESIpos): m/z = 517 (M+H)⁺. |
| **204** | 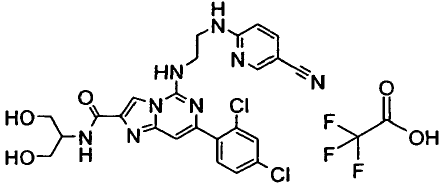 | LC/MS (Methode 8): Rₜ = 1.09 min MS (ESIpos): m/z = 541 (M+H)⁺. |
| **205** | 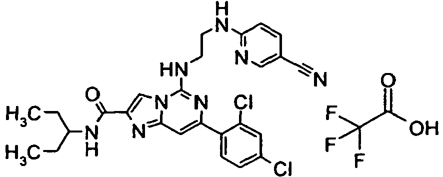 | LC/MS (Methode 8): Rₜ = 1.47 min MS (ESIpos): m/z = 537 (M+H)⁺. |

### B) Bewertung der physiologischen Wirksamkeit

Die Eignung der erfindungsgemäßen Verbindungen zur Behandlung von hämatologischen Erkrankungen kann in folgenden Assaysystemen gezeigt werden:

### In vitro Assay

Die inhibitorische Aktivität von Wirksubstanzen wird in einem biochemischen Assay bestimmt. Die dazu benötigten Bestandteile werden in einer schwarzen 384-Loch-Mikrotitterplatte mit transparentem Boden (Firma Greiner, Katalognummer 781092) gemischt. Benötigt werden dabei pro Loch der 384-Loch-Mikrotitterplatte 5 nM GSK3β (Firma Upstate, Katalognummer xy), 40 µM GSK3β-Substrat GSM (Sequenz H-RRRPASVPPSPSLSRHS-(pS)-HQRR, Firma Upstate, Katalognummer 2-533), 30 µM Nicotinsäureamid-Adenin-Dinucleotid NADH (Roche Diagnostics, Katalognummer 10107735), 50 µM Adenosin-triphoshat ATP (Firma Sigma, Katalognummer A7966), und 2 mM Phosphoenolpyruvat (Firma Roche, Katalognummer 128112). Der benötigte Reaktionspuffer, in dem die biochemische Reaktion abläuft, besteht aus 50 mM Trizma Hydrochlorid Tris- HCl pH: 7,5 (Firma Sigma, Katalognummer T3253), 5 mM Magnesiumchlorid MgCl₂ (Firma Sigma, Katalognummer M8266), 0,2mM DL-Dithiothreitol DTT (Firma Sigma, Katalognummer D9779), 2 mM Ethylendiaminethertetrasäure EDTA (Firma Sigma, Katalognummer E6758), 0.01% Triton X-100 (Firma Sigma, Katalognummer T8787) und 0.05% Bovines Serumalbumin BSA (Firma Sigma, Katalognummer B4287).

Wirksubstanzen werden in Dimethylsulfoxid DMSO (Firma Sigma, Katalognummer D8418) in einer Konzentration von 10 mM gelöst. Wirksubstanzen werden in Konzentrationsreihen von 10 µM, 1 µM, 0.1 µM, 0.01 µM, 0.001 µM, 0.0001 µM, 0.00001 µM, 0.000001 µM zu den Ansätzen der biochemischen Reaktion zugegeben. Als Kontrolle wird statt Substanz Dimethylsulfoxid in einer Endkonzentration von 0.1 % zugesetzt.

Die Reaktion wird für 2 Stunden bei 30°C inkubiert und anschließend die entstandene Fluoreszenz in einem Tecan Safire-XFLUOR4-Gerät, Version V4.50 (Serienummer 12901300283) unter den Spezifikationen: Messmodus - Fluoreszenz, von unten gemessen, Extinktionswellenlänge 340 nm, Emissionswellenlänge 465 nm, Spaltbreite Extinktion 5 nm, Spaltbreite Emission 5 nm, Verstärkermodus 120, Verzögerung 0 µs, Anzahl Lichtblitze pro Messung 3, und einer Integrationszeit von 40 µs gemessen.

Die Aktivität der GSK3β wird in Fluoreszenz-Einheiten ermittelt, wobei die Werte von nichtinhibierter Kinase gleich 100% und vollständig inhibierter Kinase gleich 0% gesetzt werden. Die Aktivität der Wirksubstanzen wird auf diese 0% und 100% verrechnet.

**Tabelle A**

| **Beispiel-Nr.** | **IC₅₀ [nM]** | | **Beispiel-Nr.** | **IC₅₀ [nM]** |
|---|---|---|---|---|
| **1** | 34 | | **117** | 94 |
| **4** | 5 | | **120** | 124 |
| **5** | 15 | | **128** | 3 |
| **6** | 44 | | **129** | 7 |
| **7** | 183 | | **131** | 6 |
| **8** | 178 | | **134** | 22 |
| **32** | 148 | | **137** | 5 |
| **36** | 15 | | **140** | 4 |
| **38** | 23 | | **145** | 62 |
| **39** | 23 | | **147** | 24 |
| **40** | 24 | | **148** | 12 |
| **53** | 81 | | **149** | 10 |
| **56** | 91 | | **150** | 11 |
| **57** | 93 | | **153** | 14 |
| **63** | 173 | | **154** | 19 |
| **66** | 196 | | **167** | 61 |
| **65** | 188 | | **180** | 50 |
| **77** | 17 | | **185** | 39 |
| **81** | 8 | | **186** | 27 |
| **93** | 72 | | **194** | 0.7 |
| **111** | 89 | | **202** | 5 |
| **112** | 54 | | **203** | 3 |
| **113** | 41 | | **Ent.B 123** | 22 |
| **115** | 5 | | | |

### CD34+-Proliefrationsassays zur Testung von GSK3β-Inhibitoren

Adulte hämatopoetische Stammzellen sind durch die spezifische Ausprägung von membranständigen Proteinen gekennzeichnet. Entsprechend ihrem Molekulargewicht sind diese Oberflächenmarker mit einer entsprechenden Nummer versehen. Zu dieser Klasse gehört auch das als CD34 bezeichnete Molekül, welches zur Identifizierung, Charakterisierung und Isolierung von adulten hämtopoetischen Stammzellen dient. Diese Stammzellen können dabei aus dem Knochenmark, dem peripheren Blut oder aus Nabelschnurblut isoliert werden. In in vitro-Kulturen sind diese Zellen begrenzt lebensfähig, können aber durch unterschiedlichste Zusätze zum Kulutrmedium zu Proliferation und Differenzierung angeregt werden. CD34-positive Zellen werden hier verwendet, um den Einfluss von Substanzen auf die Aktivität der Glykogen Synthase Kinase 3 zu testen. Zu diesem Zweck werden in einem ersten Schritt über differentielle Zentrifugationsschritte mononukleare Zellen aus Nabelschnurblut isoliert.

Dazu wird Nabelschnurblut 1:4 mit Phosphat-gepufferter Salzlösung verdünnt. 50 Milliliter Zentrifugationgefäße werden mit 17 Milliliter Ficoll (Dichte 1.077, Ficoll Paque Plus; Pharmacia, Katalognummer 17-1440-02) beschickt. Darauf werden 30 Milliliter des 1:4 verdünnten Nabelschnurblutes aufgeschichtet und anschließend für 30 Minuten bei 400 x g bei Raumtemperatur zentrifuigert. Die Bremsen der Zentrifuge sind dabei ausgeschaltet. Die mononukleären Zellen sammeln sich durch die Zentrifugation in der Interphase. Diese wird mit Hilfe einer 30 Milliliter-Pipette abgenommen und in ein neues 50 Milliliter Zentrifugationsgefäß überführt und das Volumen anschließend mit der Phosphat-gepufferten Salzlösung auf 30 ml aufgefüllt. Diese Zellen werden für 10 Minuten bei Raumtemperatur mit eingeschalteter Bremse bei 300 x g zentrifuigert. Der Überstand wird verworfen und das entstandene Zellpellet in 30 Milliliter Phosphat-gepufferter Salzlösung resuspendiert. Diese Zellen werden erneut für 15 Minuten bei 20 °C bei 200 x g und eingeschalteter Bremse zentrifugiert.

Zur Isolierung der CD34-positiven Zellen aus die angereicherten mononukleären Zellen in einer Konzentration von 1 X 10⁸ Zellen pro 300 Mikroliter MACS-Puffer (0.5% Endotoxin-freies bovines Serumalbumin in Phosphat-gepufferter Salzlösung) resuspendiert. Es erfolgt die Zugabe von 100 Mikrolitern FCR Blocking Reagenz (Miltenyi Biotec, Katalognummer 130-046-702) sowie 100 Mikrolitern an CD34 Micro Beads (Miltenyi Biotec, Katalognummer 130-046-702). Diese Suspension wird für 30 Minuten bei 4°C inkubiert. Anschließend werden die Zellen mit dem 20-fachem Volumen MACS Puffer verdünnen und 10 Minuten bei 300 x g zentrifugiert. Der Überstand wird verworfen und die Zellen in 500 Mikrolitern MACS Puffer resuspendiert. Die so behandelten Zellen werden auf einer LS-Säule (Miltenyi Biotec, katalognummer 130-042-401) aufgetragen und unter Verwendung eines Midi MACS Magneten (Miltenyi Biotec, Katalognummer 130-042-303) aufgereinigt.

Die Anzahl an CD34-positiven Zellen wird über das auszählen der Zellen unter Verwendung einer Neubauer-Kammer durchgeführt. Die Bestimmung der Reinheit der Zellen erfolgt nach Standardprotokollen unter Verwendung der Fluorescent Activated Cell Sorting -Methode (Becton Dickinson, BD FACS™ Sample Prep Assistant SPAII Upgrade Kit, Katalognummer 337642).

Zur Bestimmung des Einflusses einer Modulation der GSK3-Aktivität werden CD34-positive Zellen über 7 Tage in einer 96-Loch-Mikrotitterplatte bei 37°C und 5% Kohlendioxid inkubiert und anschließend die Proliferationsraten anhand der Zellzahlen bestimmt.

Zu diesem Zweck werden 5000 CD34-positive Zellen pro Loch einer 96-U-Boden-Loch-Mikrotitterplatte (Greiner Bio-One, Katalognummer 650 180) in 100 Mikroliter IMDM-Medium (Life Technology, Katalognummer 12440-046), 10% fetales Kälberserum (Life Technology, Katalognummer 10082-139) und 20 Nanogramm pro Milliliter Stem Cell Factor (R&D, Katalognummer 255-SC-010) aufgenommen. Zusätzlich werden die Zellen noch unterschiedlichen Konzentrationen an mit Dimethylsulfoxid (Sigma Aldrich, Katalognummer D5879-1L) gelösten Substanzen versetzt. Dabei werden jeweils 4 Löcher mit der angegebenen Zellzahl von 5000 CD34-positiven Zellen pro Loch mit 10 Mikromol, 4 Löcher mit 5 Mikromol, 4 Löcher mit 2.5 Mikromol, 4 Löcher mit 1.25 Mikromol, 4 Löcher mit 0.625 Mikromol, 4 Löcher mit 0.3125 Mikromol, 4 Löcher mit 0.156 Mikromol, 4 Löcher mit 0.078 Mikromol und als Kontrolle 4 Löcher mit 0.1% Dimethylsulfoxid als Endkonzentration versehen.

Diese so behandelten Zellen werden für 7 Tage in einem Zellkultur-Brutschrank bei 37°C und 5% Kohlendioxid inkubiert. Durch erneutes zählen der Zellen unter Verwendung einer Neubauer-Zählkammer wird die Proliferationsrate bestimmt, wobei die nur mit dem Stem Cell Factor versehenen Zellen als 100%-Wert gesetzt und alle anderen Werte auf diesen Wert bezogen sind.

### In-vivo Assay

Die Untersuchungen der in vivo-Wirkung der erfindungsmäßigen Verbindungen erfolgt unter Verwendung von 6 Wochen alten, 18 - 22 g schweren, männlichen C57BL/6-Mäusen (Charles River, Sulzfeld, Deutschland). Diese Tiere werden artgerecht mit 12-stündigen Licht- und Dunkelzyklen unter konstanten klimatischen Bedingungen gehalten und mit Wasser und Mausfutter *ad libitum* ernährt. Die Konzentrationen an verwendeten Chemotherapeutika werden den Tieren gemäß den Angaben der Hersteller mittels intra-peritonealen (i.p.) Injektionen im kaudalen Drittel des Bauches verabreicht. Gleichermaßen wird mit den erfindungsrelevanten Substanzen verfahren. Blutabnahmen erfolgen mit Hilfe von Pasteur-Pipetten aus dem retrobulbären Venenplexus. Die Bestimmung der Anzahl neutrophiler Granulozyten erfolgt vollautomatisch unter Verwendung von Durchflußzytometriesystemen.

### C) Ausführungsbeispiele für pharmazeutische Zusammensetzungen

Die erfindungsgemäßen Substanzen können folgendermaßen in pharmazeutische Zubereitungen überführt werden:

### Tablette:

### Zusammensetzung:

100 mg der Verbindung des Beispiels 1, 50 mg Lactose (Monohydrat), 50 mg Maisstärke, 10 mg Polyvinylpyrolidon (PVP 25) (Fa. BASF, Deutschland) und 2 mg Magnesiumstearat.

Tablettengewicht 212 mg. Durchmesser 8 mm, Wölbungsradius 12 mm.

### Herstellung:

Die Mischung aus der Verbindung des Beispiels 1, Lactose und Stärke wird mit einer 5%-igen Lösung (m/m) des PVPs in Wasser granuliert. Das Granulat wird nach dem Trocknen mit dem Magnesiumstearat für 5 min. gemischt. Diese Mischung wird mit einer üblichen Tablettenpresse verpresst (Format der Tablette siehe oben).

### Orale Suspension:

### Zusammensetzung:

1000 mg der Verbindung des Beispiels 1, 1000 mg Ethanol (96%), 400 mg Rhodigel (Xanthan gum) (Fa. FMC, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die Verbindung des Beispiels 1 wird der Suspension zugefügt. Unter Rühren erfolgt die Zugabe des Wassers. Bis zum Abschluss der Quellung des Rhodigels wird ca. 6 h gerührt.

### Intravenös applizierbare Lösung:

### Zusammensetzung:

1 mg der Verbindung von Beispiel 1, 15 g Polyethylenglykol 400 und 250 g Wasser für Injektionszwecke.

### Herstellung:

Die Verbindung von Beispiel 1 wird zusammen mit Polyethylenglykol 400 in dem Wasser unter Rühren gelöst. Die Lösung wird sterilfiltriert (Porendurchmesser 0.22 µm) und unter aseptischen Bedingungen in hitzesterilisierte Infusionsflaschen abgefüllt. Diese werden mit Infusionsstopfen und Bördelkappen verschlossen.

## Patentansprüche

1. Verbindung der Formel 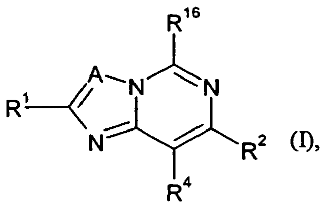 in welcher
A für N oder CR¹⁵ steht, wobei R¹⁵ für Wasserstoff, Brom oder Chlor steht,
R¹ für Wasserstoff, Hydroxy, Amino, Hydroxycarbonyl, Aminocarbonyl, Trifluormethyl, Trifluonmethoxy, Cyano, C₁-C₄-Alkyl, C₁-C₄-Alkoxy, C₁-C₆-Alkylamino, C₁-C₄-Alkylcarbonyl, C₁-C₄-Alkoxycarbonyl, C₁-C₆-Alkylaminocarbonyl, Dihydroxypropylaminocarbonyl, Dihydroxybutylaminocarbonyl, Dihydroxypentylaminocarbonyl, C₁-C₄-Alkylcarbonylamino, C₁-C₄-Alkylsulfonylamino, 5- oder 6-gliedriges Heterocyclyl-carbonyl, -CH₂R¹³ oder - CH₂CH₂R¹⁴ steht, wobei Alkoxy, Alkylamino, Alkylcarbonyl, Alkoxycarbonyl, Alkylaminocarbonyl, Alkylcarbonylamino und Alkylsulfonylamino substituiert sein können mit einem Substituenten, wobei der Substituent ausgewählt wird aus der Gruppe bestehend aus Hydroxy, Amino, Hydroxycarbonyl, Aminocarbonyl, C₁-C₄-Alkoxy, C₁-C₄-Alkylamino, C₁-C₄-Alkoxycarbonyl, C₁-C₄-Alkylaminocarbonyl, C₁-C₄-Alkylcarbonylamino und 5- oder 6-gliedriges Heterocyclyl, worin Heterocyclyl substituiert sein kann mit 1 bis 3 Substituenten, wobei die Substituenten unabhängig voneinander ausgewählt werden aus der Gruppe bestehend aus Halogen, Hydroxy, Oxo, C₁-C₄-Alkyl, C₁-C₄-Alkoxy, C₁-C₄-Alkylamino, C₁-C₄-Alkylcarbonyl, C₁-C₄-Alkoxycarbonyl und C₁-C₄-Alkylaminocarbonyl, und wobei Heterocyclylcarbonyl substituiert sein kann mit 1 bis 3 Substituenten, wobei die Substituenten unabhängig voneinander ausgewählt werden aus der Gruppe bestehend aus Halogen, Hydroxy, Oxo, C₁-C₄-Alkyl, C₁-C₄-Alkoxy, C₁-C₄-Alkylamino, C₁-C₄-Alkylcarbonyl, C₁-C₄-Alkoxycarbonyl und C₁-C₄-Alkylaminocarbonyl, und wobei
R¹³ für Hydroxy, Amino, Cyano, Hydroxycarbonyl, Aminocarbonyl, C₁-C₄-Alkoxy, C₁-C₄-Alkylainino, C₁-C₄-Alkoxycarbonyl, C₁-C₄-Alkylaminocarbonyl, C₁-C₄-Alkylcarbonylamino, 5- oder 6-gliedriges Heterocyclyl oder 5- oder 6-gliedriges Heteroaryl steht, worin Alkoxy, Alkylamino, Alkoxycarbonyl, Alkylaminocarbonyl und Alkylcarbonylamino substituiert sein können mit einem Substituenten, wobei der Substituent ausgewählt wird aus der Gruppe bestehend aus Hydroxy, Amino, Hydroxycarbonyl, Aminocarbonyl, C₁-C₄-Alkoxy, C₁-C₄-Alkylamino, C₁-C₄-Alkoxycarbonyl, C₁-C₄-Alkylaminocarbonyl und C₁-C₄-Alkylcarbonylamino, und - worin Heterocyclyl und Heteroaryl substituiert sein können mit 1 bis 3 Substituenten, wobei die Substituenten unabhängig voneinander ausgewählt werden aus der Gruppe bestehend aus Halogen, Hydroxy, Oxo, C₁-C₄-Alkyl, C₁-C₄-Alkoxy, C₁-C₄-Alkylamino, C₁-C₄-Alkylcarbonyl, C₁-C₄-Alkoxycarbonyl und C₁-C₄-Alkylaminocarbonyl, und wobei
R¹⁴ für Hydroxy, Amino, Cyano, Hydroxycarbonyl, Aminocarbonyl, C₁-C₄-Alkoxy, C₁-C₄-Alkylamino, C₁-C₄-Alkoxycarbonyl, C₁-C₄-Alkylaminocarbonyl, C₁-C₄-Alkylcarbonylamino, 5- oder 6-gliedriges Heterocyclyl oder 5- oder 6-gliedriges Heteroaryl steht, worin Alkoxy, Alkylamino, Alkoxycarbonyl, Alkylaminocarbonyl und Alkylcarbonylamino substituiert sein können mit einem Substituenten, wobei der Substituent ausgewählt wird aus der Gruppe bestehend aus Hydroxy, Amino, Hydroxycarbonyl, Aminocarbonyl, C₁-C₄-Alkoxy, C₁-C₄-Alkylamino, C₁-C₄-Alkoxycarbonyl, C₁-C₄-Alkylaminocarbonyl und C₁-C₄-Alkylcarbonylamino, und worin Heterocyclyl und Heteroaryl substituiert sein können mit 1 bis 3 Substituenten, wobei die Substituenten unabhängig voneinander ausgewählt werden aus der Gruppe bestehend aus Halogen, Hydroxy, Oxo, C₁-C₄-Alkyl, C₁-C₄-Alkoxy, C₁-C₄-Alkylamino, C₁-C₄-Alkylcarbonyl, C₁-C₄-Alkoxycarbonyl und C₁-C₄-Alkylaminocarbonyl,
R² für C₆-C₁₀-Aryl oder 5- bis 10-gliedriges Heteroaryl steht, wobei Aryl und Heteroaryl substituiert sein können mit 1 bis 3 Substituenten, wobei die Substituenten unabhängig voneinander ausgewählt werden aus der Gruppe bestehend aus Hydroxy, Hydroxymethyl, Amino, Halogen, Cyano, Trifluormethyl, Trifluormethoxy, Aminocarbonyl, C₁-C₄-Alkyl, C₁-C₄-Alkoxy, C₁-C₄-Alkoxymethyl, C₁-C₄-Alkylamino, C₁-C₄-Alkylaminomethyl, C₁-C₄-Alkylcarbonyl, C₁-C₄-Alkoxycarbonyl, C₁-C₄-Alkylaminocarbonyl, C₁-C₄-Alkylcarbonylamino, C₁-C₄-Alkylsulfonyl, C₁-C₄-Alkylsulfonylamino, C₁-C₄-Alkylaminosulfonyl, Phenyl, Benzyloxy, 5- oder 6-gliedrigem Heterocyclyl, 5-oder 6-gliedrigem Heterocyclylcarbonyl, 5- oder 6-gliedrigem Heterocyclylmethyl und 5- oder 6-gliedrigem Heteroaryl, worin Phenyl, Benzyloxy, Heterocyclyl, Heterocyclylcarbonyl, Heterocyclylmethyl und Heteroaryl substituiert sein können mit 1 bis 3 Substituenten, wobei die Substituenten unabhängig voneinander ausgewählt werden aus der Gruppe bestehend aus Halogen, Cyano, Trifluormethyl, Trifluormethoxy, Aminocarbonyl, C₁-C₄-Alkyl, C₁-C₄-Alkoxy, C₁-C₄-Alkylamino, C₁-C₄-Alkylcarbonyl, C₁-C₄-Alkoxycarbonyl, C₁-C₄-Alkylaminocarbonyl und C₁-C₄-Alkylcarbonylamino, oder zwei der Substituenten am Aryl zusammen mit den Kohlenstoffatomen, an die sie gebunden sind, ein 1,3-Dioxolan oder 1,4-Dioxan bilden,
R⁴ für Wasserstoff, Halogen, Cyano, Trifluormethyl, C₁-C₃-Alkyl, Methylthio oder Cyclopropyl steht,
R¹⁶ für eine Gruppe der Formel 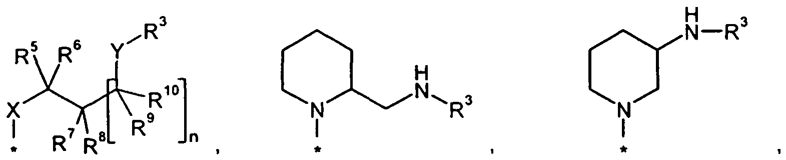 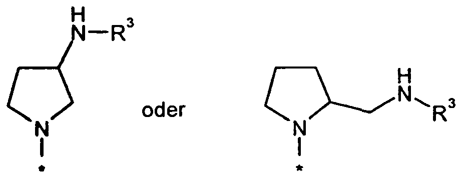 steht, wobei
* die Anknüpfstelle an den Heterocyclus bedeutet,
n für die Zahl 0 oder 1 steht,
X für NR¹¹, S oder O steht, wobei R¹¹ für Wasserstoff, C₁-C₃-Alkyl oder Cyclopropyl steht,
Y für NR¹², S oder O steht, wobei R¹² für Wasserstoff, C₁-C₃-Alkyl oder Cyclopropyl steht,
R³ für 2-Pyridyl, Pyrimid-2-yl, 2-Aminopyrimid-4-yl, 2-Cyclopropyl-aminopyrimid-4-yl, 2-Methylaminopyrimid-4-yl, 2-Ethylaminopyrimid-4-yl, 1,3-Thiazol-2-yl, 1,3-Thiazol-4-yl oder 1,3-Thiazol-5-yl steht, wobei 2-Pyridyl, Pyrimid-2-yl, 2-Aminopyrimid-4-yl, 1,3-Thiazol-2-yl, 1,3-Thiazol-4-yl und 1,3-Thiazol-5-yl substituiert sind mit 1 bis 3 Substituenten, wobei die Substituenten unabhängig voneinander ausgewählt werden aus der Gruppe bestehend aus Halogen, Cyano, Nitro, Amino, Trifluormethyl, Trifluormethoxy, Aminocarbonyl, Trifluormethylcarbonyl, C₁-C₄-Alkyl, C₁-C₄-Alkoxy, C₁-C₄-Alkylamino, C₁-C₄-Alkylcarbonyl, C₁-C₄-Alkoxycarbonyl, C₁-C₄-Alkylaminocarbonyl und C₃-C₆-Cycloalkylcarbonyl, worin Alkyl, Alkoxy, Alkylamino, Alkylcarbonyl, Alkoxycarbonyl, Alkylaminocarbonyl und Cycloalkylcarbonyl substituiert sein können mit einem Substituenten, wobei der Substituent ausgewählt wird aus der Gruppe bestehend aus Halogen, Cyano, Hydroxy, Amino, Trifluormethyl und C₃-C₆-Cycloalkyl, oder
R³ für eine Gruppe der Formel 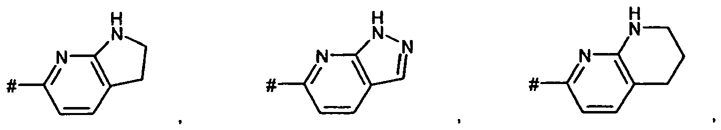 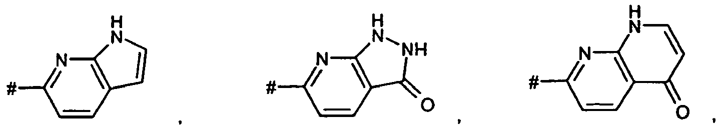 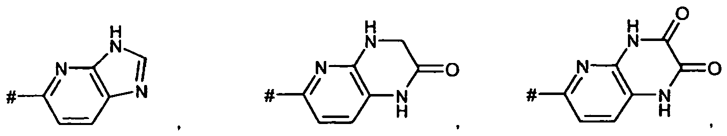 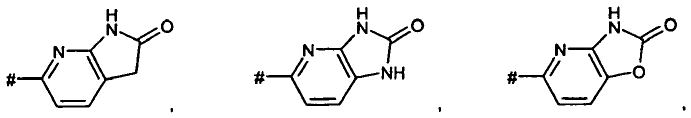 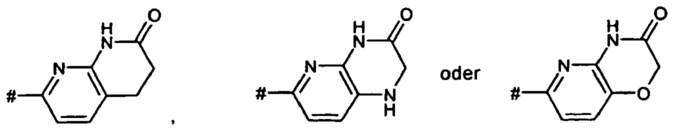 steht, wobei # die Anknüpfstelle an Y bedeutet,
R⁵ für Wasserstoff, C₁-C₃-Alkyl oder Cyclopropyl steht,
R⁶ für Wasserstoff oder C₁-C₃-Alkyl steht,
R⁷ für Wasserstoff, C₁-C₃-Alkyl oder Cyclopropyl steht,
R⁸ für Wasserstoff oder C₁-C₃-Alkyl steht,
R⁹ für Wasserstoff, C₁-C₃-Alkyl oder Cyclopropyl steht,
R¹⁰ für Wasserstoff oder C₁-C₃-Alkyl steht,
oder eines ihrer Salze, ihrer Solvate oder der Solvate ihrer Salze.

2. Verbindung nach Anspruch 1, **dadurch gekennzeichnet, dass**
A für N oder CR¹⁵ steht, wobei R¹⁵ für Wasserstoff steht,
R¹ für Wasserstoff, Trifluormethyl, Cyano, C₁-C₄-Alkyl, C₁-C₆-Alkylaminocarbonyl, Dihydroxypropylaminocarbonyl, Dihydroxybutylaminocarbonyl, Dihydroxypentylaminocarbonyl, Pyrrolidinylcarbonyl, Piperidinylcarbonyl, Morpholinylcarbonyl, Piperazinylcarbonyl, -CH₂R¹³ oder -CH₂CH₂R¹⁴ steht, wobei Alkylaminocarbonyl substituiert sein kann mit einem Substituenten, wobei der Substituent ausgewählt wird aus der Gruppe bestehend aus Hydroxy, Amino, Hydroxycarbonyl, Aminocarbonyl, C₁-C₄-Alkoxy, C₁-C₄-Alkylamino, Pyrrolidinyl, Piperidinyl, Morpholinyl und Piperazinyl, worin Pyrrolidinyl, Piperidinyl, Morpholinyl und Piperazinyl substituiert sein können mit 1 bis 3 Substituenten, wobei die Substituenten unabhängig voneinander ausgewählt werden aus der Gruppe bestehend aus Halogen, Hydroxy, Oxo, C₁-C₄-Alkyl, C₁-C₄-Alkoxy und C₁-C₄-Alkylamino, und wobei Pyrrolidinylcarbonyl, Piperidinylcarbonyl, Morpholinylcarbonyl und Piperazinyl-carbonyl substituiert sein können mit 1 bis 3 Substituenten, wobei die Substituenten unabhängig voneinander ausgewählt werden aus der Gruppe bestehend aus Halogen, Hydroxy, Oxo, C₁-C₄-Alkyl, C₁-C₄-Alkoxy und C₁-C₄-Alkylamino, und wobei
R¹³ für Hydroxycarbonyl, C₁-C₄-Alkoxy, C₁-C₄-Alkylamino, C₁-C₄-Alkylaminocarbonyl, Pyrrolidinyl, Piperidinyl, Morpholinyl, Piperazinyl, Pyrazolyl, Imidazolyl, Triazolyl oder Pyridyl steht, worin Alkoxy, Alkylamino und Alkylaminocarbonyl substituiert sein können mit einem Substituenten, wobei der Substituent ausgewählt wird aus der Gruppe bestehend aus Hydroxy, Amino, Hydroxycarbonyl, Aminocarbonyl, C₁-C₄-Alkoxy, C₁-C₄-Alkylamino, C₁-C₄-Alkoxycarbonyl, C₁-C₄-Alkylaminocarbonyl und C₁-C₄-Alkylcarbonylamino, und worin Pyrrolidinyl, Piperidinyl, Morpholinyl, Piperazinyl, Pyrazolyl, Imidazolyl, Triazolyl und Pyridyl substituiert sein können mit 1 bis 3 Substituenten, wobei die Substituenten unabhängig voneinander ausgewählt werden aus der Gruppe bestehend aus Halogen, Hydroxy, Oxo, C₁-C₄-Alkyl, C₁-C₄-Alkoxy und C₁-C₄-Alkylamino, und wobei
R¹⁴ für Hydroxycarbonyl, C₁-C₄-Alkoxy, C₁-C₄-Alkylamino, C₁-C₄-Alkylaminocarbonyl, Pyrrolidinyl, Piperidinyl, Morpholinyl, Piperazinyl, Pyrazolyl, Imidazolyl, Triazolyl oder Pyridyl steht, worin Alkoxy, Alkylamino und Alkylaminocarbonyl substituiert sein können mit einem Substituenten, wobei der Substituent ausgewählt wird aus der Gruppe bestehend aus Hydroxy, Amino, Hydroxycarbonyl, Aminocarbonyl, C₁-C₄-Alkoxy, C₁-C₄-Alkylamino, C₁-C₄-Alkoxycarbonyl, C₁-C₄-Alkylaminocarbonyl und C₁-C₄-Alkylcarbonylamino, und worin Pyrrolidinyl, Piperidinyl, Morpholinyl, Piperazinyl, Pyrazolyl, Imidazolyl, Triazolyl und Pyridyl substituiert sein können mit 1 bis 3 Substituenten, wobei die Substituenten unabhängig voneinander ausgewählt werden aus der Gruppe bestehend aus Halogen, Hydroxy, Oxo, C₁-C₄-Alkyl, C₁-C₄-Alkoxy und C₁-C₄-Alkylamino,
R² für Phenyl steht, wobei Phenyl substituiert sein kann mit 1 bis 3 Substituenten, wobei die Substituenten unabhängig voneinander ausgewählt werden aus der Gruppe bestehend aus Hydroxy, Halogen, Cyano, Trifluormethyl, Trifluormethoxy, Aminocarbonyl, C₁-C₄-Alkyl, C₁-C₄-Alkoxy, C₁-C₄-Alkoxymethyl, C₁-C₄-Alkylaminomethyl, C₁-C₄-Alkylcarbonyl, C₁-C₄-Alkoxycarbonyl, C₁-C₄-Alkylaminocarbonyl, C₁-C₄-Alkylcarbonylamino, C₁-C₄-Alkyl-sulfonyl, C₁-C₄-Alkylsulfonylamino, C₁-C₄-Alkylaminosulfonyl, Phenyl, Pyrrolidinyl, Piperidinyl, Morpholinyl; Piperazinyl, Pyrrolidinylmethyl, Piperidinylmethyl, Morpholinylmethyl und Piperazinylmethyl, worin Phenyl, Pyrrolidinyl, Piperidinyl, Morpholinyl, Piperazinyl, Pyrrolidinylmethyl, Piperidinylmethyl, Morpholinylmethyl und Piperazinylmethyl substituiert sein können mit 1 bis 3 Substituenten, wobei die Substituenten unabhängig voneinander ausgewählt werden aus der Gruppe bestehend aus Halogen, Cyano, Trifluormethyl, Trifluormethoxy und C₁-C₄-Alkyl,
R⁴ für Wasserstoff oder Chlor steht,
R¹⁶ für eine Gruppe der Formel 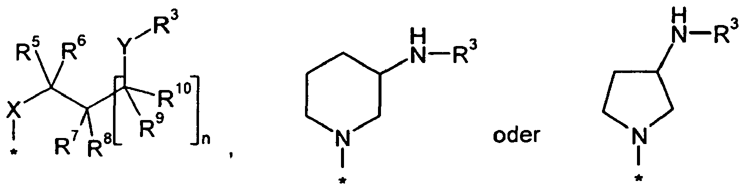 steht, wobei
* die Anknüpfstelle an den Heterocyclus bedeutet,
n für die Zahl 0 steht,
X für NR¹¹, S oder O steht, wobei R¹¹ für Wasserstoff oder Methyl steht,
Y für NR¹², S oder O steht, wobei R¹² für Wasserstoff oder Methyl steht,
R³ für 2-Pyridyl, Pyrimid-2-yl, 2-Aminopyrimid-4-yl, 2-Cyclopropylamino-pyrimid-4-yl, 2-Methylaminopyrimid-4-yl, 2-Ethylaminopyrimid-4-yl, 1,3-Thiazol-2-yl, 1,3-Thiazol-4-yl oder 1,3-Thiazol-5-yl steht, wobei 2-Pyridyl, Pyrimid-2-yl, 2-Aminopyrimid-4-yl, 1,3-Thiazol-2-yl, 1,3-Thiazol-4-yl und 1,3-Thiazol-5-yl substituiert sind mit 1 bis 3 Substituenten, wobei die Substituenten unabhängig voneinander ausgewählt werden aus der Gruppe bestehend aus Halogen, Cyano, Nitro, Amino, Trifluormethyl, Trifluormethoxy, Aminocarbonyl, Trifluormethylcarbonyl, C₁-C₄-Alkylamino, C₁-C₄-Alkylcarbonyl, C₁-C₄-Alkoxycarbonyl, C₁-C₄-Alkylaminocarbonyl und C₃-C₆-Cycloalkylcarbonyl, worin Alkylamino, Alkylcarbonyl, Alkoxycarbonyl, Alkylaminocarbonyl und Cycloalkylcarbonyl substituiert sein können mit einem Substituenten, wobei der Substituent ausgewählt wird aus der Gruppe bestehend aus Halogen, Cyano, Hydroxy, Amino, Trifluormethyl und C₃-C₆-Cycloalkyl,
R⁵ für Wasserstoff oder Methyl steht,
R⁶ für Wasserstoff steht,
R⁷ für Wasserstoff oder Methyl steht,
R⁸ für Wasserstoff steht,
R⁹ für Wasserstoff steht,
R¹⁰ für Wasserstoff steht,
oder eines ihrer Salze, ihrer Solvate oder der Solvate ihrer Salze.

3. Verbindung nach einem der Ansprüche 1 oder 2, **dadurch gekennzeichnet, dass**
A für N oder CR¹⁵ steht, wobei R¹⁵ für Wasserstoff steht,
R¹ für Wasserstoff, Methyl oder -CH₂R¹³ steht, wobei R¹³ für Morpholinyl steht,
R² für Phenyl steht, wobei Phenyl substituiert sein kann mit 1 bis 3 Substituenten, wobei die Substituenten unabhängig voneinander ausgewählt werden aus der Gruppe bestehend aus Chlor, Fluor, Trifluormethyl, Trifluormethoxy und Methyl,
R⁴ für Wasserstoff steht,
R¹⁶ für eine Gruppe der Formel 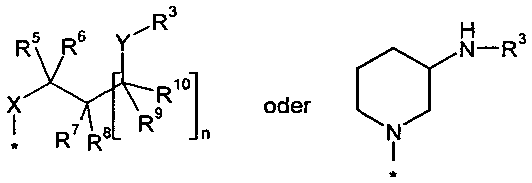 steht, wobei
* die Anknüpfstelle an den Heterocyclus bedeutet,
n für die Zahl 0 steht,
X für NR¹¹ steht, wobei R¹¹ für Wasserstoff steht,
Y für NR¹² steht, wobei R¹² für Wasserstoff steht,
R³ für 2-Pyridyl oder 1,3-Thiazol-2-yl steht, wobei 2-Pyridyl und 1,3-Thiazol-2-yl substituiert sind mit 1 bis 2 Substituenten, wobei die Substituenten unabhängig voneinander ausgewählt werden aus der Gruppe bestehend aus Cyano, Nitro, Amino, Trifluormethylcarbonyl und Methylcarbonyl,
R⁵ für Wasserstoff oder Methyl steht,
R⁶ für Wasserstoff steht,
R⁷ für Wasserstoff oder Methyl steht,
R⁸ für Wasserstoff steht,
R⁹ für Wasserstoff steht,
R¹⁰ für Wasserstoff steht,
oder eines ihrer Salze, ihrer Solvate oder der Solvate ihrer Salze.

4. Verfahren zur Herstellung einer Verbindung der Formel (I) oder eines ihrer Salze, ihrer Solvate oder der Solvate ihrer Salze nach Anspruch 1, **dadurch gekennzeichnet, dass**
[A] eine Verbindung der Formel 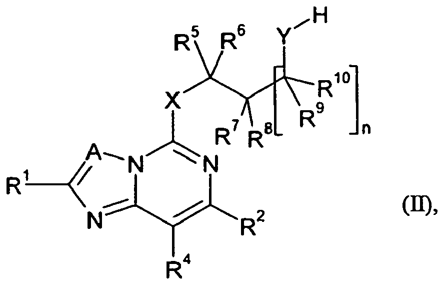 in welcher
A, n, X, Y, R¹, R², R⁴, R⁵, R⁶, R⁷, R⁸, R⁹ und R¹⁰ die in Anspruch 1 angegebene Bedeutung haben,
mit einer Verbindung der Formel
R³—X¹ , (III)
in welcher
R³ die in Anspruch 1 angegebene Bedeutung hat, und
X¹ für Halogen, bevorzugt Chlor oder Fluor, steht, umgesetzt wird,
oder
[B] eine Verbindung der Formel 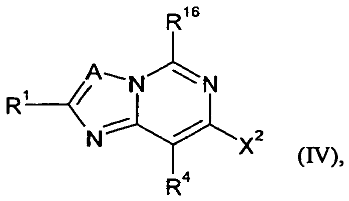 in welcher
A, R¹, R⁴ und R¹⁶ die in Anspruch 1 angegebene Bedeutung haben, und
X² für Iod, Brom, Chlor oder Trifluormethansulfonyl, bevorzugt Iod oder Brom, steht, mit einer Verbindung der Formel
Q-R² , (V)
in welcher
R² die in Anspruch 1 angegebene Bedeutung hat, und
Q für -B(OH)₂, einen Boronsäure-Ester, bevorzugt Boronsäurepinakolester, oder -BF₃⁻K⁺ steht,
unter Suzuki-Kupplungsbedingungen umgesetzt wird,
oder
[C] eine Verbindung der Formel 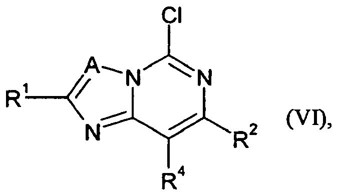 in welcher
A, R¹, R² und R⁴ die in Anspruch 1 angegebene Bedeutung haben,
mit einer Verbindung der Formel
H—R¹⁶ , (IX)
in welcher
R¹⁶ die in Anspruch 1 angegebene Bedeutung hat, umgesetzt wird.

5. Verbindung nach einem der Ansprüche 1 bis 3 zur Behandlung und/oder Prophylaxe von Krankheiten.

6. Verwendung einer Verbindung nach einem der Ansprüche 1 bis 3 zur Herstellung eines Arzneimittels zur Behandlung und/oder Prophylaxe von Krankheiten.

7. Verwendung einer Verbindung nach einem der Ansprüche 1 bis 3 zur Herstellung eines Arzneimittels zur Behandlung und/oder Prophylaxe von hämatologischen Erkrankungen.

8. Arzneimittel enthaltend eine Verbindung nach einem der Ansprüche 1 bis 3 in Kombination mit einem inerten, nichttoxischen, pharmazeutisch geeigneten Hilfsstoff.

9. Arzneimittel nach Anspruch 8 zur Behandlung und/oder Prophylaxe von hämatologischen Erkrankungen.

## Claims

1. Compound of the formula 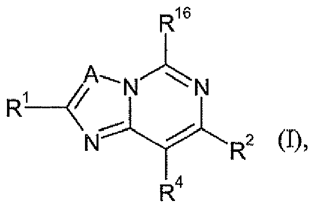 in which
A is N or CR¹⁵, where R¹⁵ is hydrogen, bromine or chlorine,
R¹ is hydrogen, hydroxy, amino, hydroxycarbonyl, aminocarbonyl, trifluoromethyl, trifluoromethoxy, cyano, C₁-C₄-alkyl, C₁-C₄-alkoxy, C₁-C₆-alkylamino, C₁-C₄-alkylcarbonyl, C₁-C₄-alkoxycarbonyl, C₁-C₆-alkylaminocarbonyl, dihydroxypropylaminocarbonyl, dihydroxybutylaminocarbonyl, dihydroxypentylaminocarbonyl, C₁-C₄-alkylcarbonylamino, C₁-C₄-alkylsulphonylamino, 5- or 6-membered heterocyclylcarbonyl, -CH₂R¹³ or -CH₂CH₂R¹⁴, where alkoxy, alkylamino, alkylcarbonyl, alkoxycarbonyl, alkylaminocarbonyl, alkylcarbonylamino and alkylsulphonylamino may be substituted by a substituent, where the substituent is selected from the group consisting of hydroxy, amino, hydroxycarbonyl, aminocarbonyl, C₁-C₄-alkoxy, C₁-C₄-alkylamino, C₁-C₄-alkoxycarbonyl, C₁-C₄-alkylaminocarbonyl, C₁-C₄-alkylcarbonylamino and 5- or 6-membered heterocyclyl, in which heterocyclyl may be substituted by 1 to 3 substituents, where the substituents are selected independently of one another from the group consisting of halogen, hydroxy, oxo, C₁-C₄-alkyl, C₁-C₄-alkoxy, C₁-C₄-alkylamino, C₁-C₄-alkylcarbonyl, C₁-C₄-alkoxycarbonyl and C₁-C₄-alkylaminocarbonyl,
and where heterocyclylcarbonyl may be substituted by 1 to 3 substituents, where the substituents are selected independently of one another from the group consisting of halogen, hydroxy, oxo, C₁-C₄-alkyl, C₁-C₄-alkoxy, C₁-C₄-alkylamino, C₁-C₄-alkylcarbonyl, C₁-C₄-alkoxycarbonyl and C₁-C₄-alkylaminocarbonyl,
and where R¹³ is hydroxy, amino, cyano, hydroxycarbonyl, aminocarbonyl, C₁-C₄-alkoxy, C₁-C₄-alkylamino, C₁-C₄-alkoxycarbonyl, C₁-C₄-alkylaminocarbonyl, C₁-C₄-alkylcarbonylamino, 5- or 6-membered heterocyclyl or 5- or 6-membered heteroaryl, in which alkoxy, alkylamino, alkoxycarbonyl, alkylaminocarbonyl and alkylcarbonylamino may be substituted by a substituent, where the substituent is selected from the group consisting of hydroxy, amino, hydroxycarbonyl, aminocarbonyl, C₁-C₄-alkoxy, C₁-C₄-alkylamino, C₁-C₄-alkoxycarbonyl, C₁-C₄-alkylaminocarbonyl and C₁-C₄-alkylcarbonylamino, and in which heterocyclyl and heteroaryl may be substituted by 1 to 3 substituents, where the substituents are selected independently of one another from the group consisting of halogen, hydroxy, oxo, C₁-C₄- alkyl, C₁-C₄-alkoxy, C₁-C₄-alkylamino, C₁-C₄-alkylcarbonyl, C₁-C₄-alkoxycarbonyl and C₁-C₄-alkylaminocarbonyl,
and where R¹⁴ is hydroxy, amino, cyano, hydroxycarbonyl, aminocarbonyl, C₁-C₄-alkoxy, C₁-C₄-alkylamino, C₁-C₄-alkoxycarbonyl, C₁-C₄-alkylaminocarbonyl, C₁-C₄-alkylcarbonylamino, 5- or 6-membered heterocyclyl or 5- or 6-membered heteroaryl, in which alkoxy, alkylamino, alkoxycarbonyl, alkylaminocarbonyl and alkylcarbonylamino may be substituted by a substituent, where the substituent is selected from the group consisting of hydroxy, amino, hydroxycarbonyl, aminocarbonyl, C₁-C₄-alkoxy, C₁-C₄-alkylamino, C₁-C₄-alkoxycarbonyl, C₁-C₄-alkylaminocarbonyl and C₁-C₄-alkylcarbonylamino, and in which heterocyclyl and heteroaryl may be substituted by 1 to 3 substituents, where the substituents are selected independently of one another from the group consisting of halogen, hydroxy, oxo, C₁-C₄-alkyl, C₁-C₄-alkoxy, C₁-C₄-alkylamino, C₁-C₄-alkylcarbonyl, C₁-C₄-alkoxycarbonyl and C₁-C₄-alkylaminocarbonyl,
R² is C₆-C₁₀-aryl or 5- to 10-membered heteroaryl, where aryl and heteroaryl may be substituted by 1 to 3 substituents, where the substituents are selected independently of one another from the group consisting of hydroxy, hydroxymethyl, amino,, halogen, cyano, trifluoromethyl, trifluoromethoxy, aminocarbonyl, C₁-C₄-alkyl, C₁-C₄-alkoxy, C₁-C₄-alkoxymethyl, C₁-C₄-alkylamino, C₁-C₄-alkylaminomethyl, C₁-C₄-alkylcarbonyl, C₁-C₄-alkoxycarbonyl, C₁-C₄-alkylaminocarbonyl, C₁-C₄-alkylcarbonylamino, C₁-C₄-alkylsulphonyl, C₁-C₄-alkylsulphonylamino, C₁-C₄-alkylaminosulphonyl, phenyl, benzyloxy, 5- or 6-membered heterocyclyl, 5- or 6-membered heterocyclylcarbonyl, 5- or 6-membered heterocyclylmethyl and 5- or 6-membered heteroaryl, in which phenyl, benzyloxy, heterocyclyl, heterocyclylcarbonyl, heterocyclylmethyl and heteroaryl may be substituted by 1 to 3 substituents, where the substituents are selected independently of one another from the group consisting of halogen, cyano, trifluoromethyl, trifluoromethoxy, aminocarbonyl, C₁-C₄-alkyl, C₁-C₄-alkoxy, C₁-C₄-alkylamino, C₁-C₄-alkylcarbonyl, C₁-C₄-alkoxycarbonyl, C₁-C₄-alkylaminocarbonyl and C₁-C₄-alkylcarbonylamino,
or two of the substituents on the aryl form together with the carbon atoms to which they are bonded a 1,3-dioxolane or 1,4-dioxane,
R⁴ is hydrogen, halogen, cyano, trifluoromethyl, C₁-C₃-alkyl, methylthio or cyclopropyl,
R¹⁶ is a group of the formula 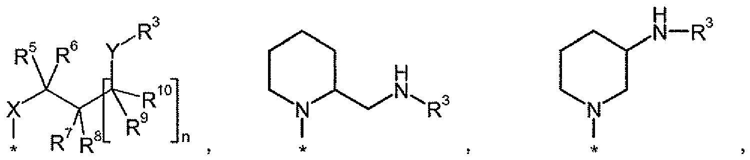 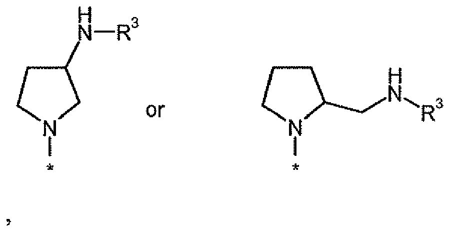 where
* is the point of attachment to the heterocycle,* is the point of attachment to the heterocycle,
n is the number 0 or 1,
X is NR¹¹, S or O, where
R¹¹ is hydrogen, C₁-C₃-alkyl or cyclopropyl,
Y is NR¹², S or O, where
R¹² is hydrogen, C₁-C₃-alkyl or cyclopropyl,
R³ is 2-pyridyl, pyrimid-2-yl, 2-aminopyrimid-4-yl, 2-cyclopropylamino-pyrimid-4-yl, 2-methylaminopyrimid-4-yl, 2-ethylaminopyrimid-4- yl, 1,3-thiazol-2-yl, 1,3-thiazol-4-yl or 1,3-thiazol-5-yl, where 2-pyridyl, pyrimid-2-yl, 2-aminopyrimid-4-yl, 1,3-thiazol-2-yl, 1,3-thiazol-4-yl and 1,3-thiazol-5-yl are substituted by 1 to 3 substituents, where the substituents are selected independently of one another from the group consisting of halogen, cyano, nitro, amino, trifluoromethyl, trifluoromethoxy, aminocarbonyl, trifluoromethylcarbonyl, C₁-C₄-alkyl, C₁-C₄-alkoxy, C₁-C₄-alkylamino, C₁-C₄-alkylcarbonyl, C₁-C₄-alkoxycarbonyl, C₁-C₄-alkylaminocarbonyl and C₃-C₆-cycloalkylcarbonyl, in which alkyl, alkoxy, alkylamino, alkylcarbonyl, alkoxycarbonyl, alkylaminocarbonyl and cycloalkylcarbonyl may be substituted by a substituent, where the substituent is selected from the group consisting of halogen, cyano, hydroxy, amino, trifluoromethyl and C₃-C₆-cycloalkyl,
or R³ is a group of the formula 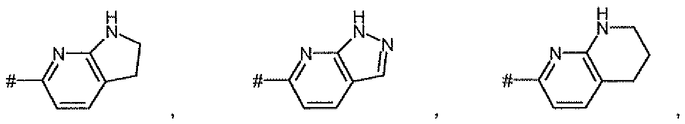 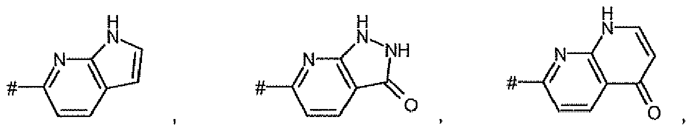 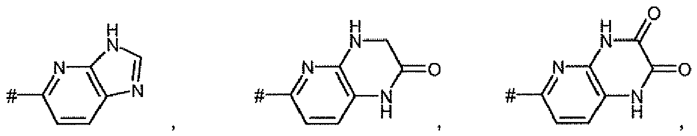 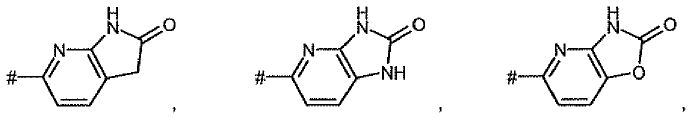 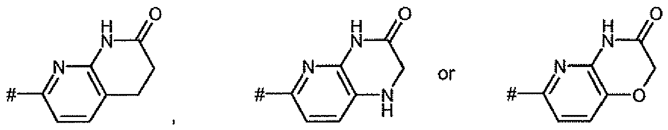 where # is the point of attachment to Y,
R⁵ is hydrogen, C₁-C₃-alkyl or cyclopropyl,
R⁶ is hydrogen or C₁-C₃-alkyl,
R⁷ is hydrogen, C₁-C₃-alkyl or cyclopropyl,
R⁸ is hydrogen or C₁-C₃-alkyl,
R⁹ is hydrogen, C₁-C₃-alkyl or cyclopropyl,
R¹⁰ is hydrogen or C₁-C₃-alkyl,
or one of the salts thereof, the solvates thereof or the solvates of the salts thereof.

2. Compound according to Claim 1, **characterized in that**
A is N or CR¹⁵, where
R¹⁵ is hydrogen,
R¹ is hydrogen, trifluoromethyl, cyano, C₁-C₄-alkyl, C₁-C₆-alkylaminocarbonyl, dihydroxypropylaminocarbonyl, dihydroxybutylaminocarbonyl, dihydroxypentylaminocarbonyl, pyrrolidinylcarbonyl, piperidinylcarbonyl, morpholinylcarbonyl, piperazinylcarbonyl, -CH₂R¹³ or -CH₂CH₂R¹⁴, where alkylaminocarbonyl may be substituted by a substituent, where the substituent is selected from the group consisting of hydroxy, amino, hydroxycarbonyl, aminocarbonyl, C₁-C₄-alkoxy, C₁-C₄-alkylamino, pyrrolidinyl, piperidinyl, morpholinyl and piperazinyl, in which pyrrolidinyl, piperidinyl, morpholinyl and piperazinyl may be substituted by 1 to 3 substituents, where the substituents are selected independently of one another from the group consisting of halogen, hydroxy, oxo, C₁-C₄-alkyl, C₁-C₄-alkoxy and C₁-C₄-alkylamino,
and where pyrrolidinylcarbonyl, piperidinylcarbonyl, morpholinylcarbonyl and piperazinyl-carbonyl may be substituted by 1 to 3 substituents, where the substituents are selected independently of one another from the group consisting of halogen, hydroxy, oxo, C₁-C₄-alkyl, C₁-C₄-alkoxy and C₁-C₄-alkylamino,
and where R¹³ is hydroxycarbonyl, C₁-C₄-alkoxy, C₁-C₄-alkylamino, C₁-C₄-alkylaminocarbonyl, pyrrolidinyl, piperidinyl, morpholinyl, piperazinyl, pyrazolyl, imidazolyl, triazolyl or pyridyl, In which alkoxy, alkylamino and alkylaminocarbonyl may be substituted by a substituent, where the substituent is selected from the group consisting of hydroxy, amino, hydroxycarbonyl, aminocarbonyl, C₁-C₄-alkoxy, C₁-C₄-alkylamino, C₁-C₄-alkoxycarbonyl, C₁-C₄-alkylaminocarbonyl and C₁-C₄-alkylcarbonylamino, and in which pyrrolidinyl, piperidinyl, morpholinyl, piperazinyl, pyrazolyl, imidazolyl, triazolyl and pyridyl may be substituted by 1 to 3 substituents, where the substituents are selected independently of one another from the group consisting of halogen, hydroxy, oxo, C₁-C₄-alkyl, C₁-C₄-alkoxy and C₁-C₄-alkylamino,
and where R¹⁴ is hydroxycarbonyl, C₁-C₄-alkoxy, C₁-C₄-alkylamino, C₁-C₄-alkylaminocarbonyl, pyrrolidinyl, piperidinyl, morpholinyl, piperazinyl, pyrazolyl, imidazolyl, triazolyl or pyridyl, in which alkoxy, alkylamino and alkylaminocarbonyl may be substituted by a substituent, where the substituent is selected from the group consisting of hydroxy, amino, hydroxycarbonyl, aminocarbonyl, C₁-C₄-alkoxy, C₁-C₄-alkylamino, C₁-C₄-alkoxycarbonyl, C₁-C₄-alkylaminocarbonyl and C₁-C₄-alkylcarbonylamino, and in which pyrrolidinyl, piperidinyl, morpholinyl, piperazinyl, pyrazolyl, imidazolyl, triazolyl and pyridyl may be substituted by 1 to 3 substituents, where the substituents are selected independently of one another from the group consisting of halogen, hydroxy, oxo, C₁-C₄-alkyl, C₁-C₄-alkoxy and C₁-C₄-alkylamino,
R² is phenyl, where phenyl may be substituted by 1 to 3 substituents, where the substituents are selected independently of one another from the group consisting of hydroxy, halogen, cyano, trifluoromethyl, trifluoromethoxy, aminocarbonyl, C₁-C₄-alkyl, C₁-C₄-alkoxy, C₁-C₄-alkoxymethyl, C₁-C₄-alkylaminomethyl, C₁-C₄-alkylcarbonyl, C₁-C₄- alkoxycarbonyl, C₁-C₄-alkylaminocarbonyl, C₁-C₄-alkylcarbonylamino, C₁-C₄-alkylsulphonyl, C₁-C₄-alkylsulphonylamino, C₁-C₄-alkylaminosulphonyl, phenyl, pyrrolidinyl, piperidinyl, morpholinyl, piperazinyl, pyrrolidinylmethyl, piperidinylmethyl, morpholinylmethyl and piperazinylmethyl, in which phenyl, pyrrolidinyl, piperidinyl, morpholinyl, piperazinyl, pyrrolidinylmethyl, piperidinylmethyl, morpholinylmethyl and piperazinylmethyl may be substituted by 1 to 3 substituents, where the substituents are selected independently of one another from the group consisting of halogen, cyano, trifluoromethyl, trifluoromethoxy and C₁-C₄-alkyl,
R⁴ is hydrogen or chlorine,
R¹⁶ is a group of the formula 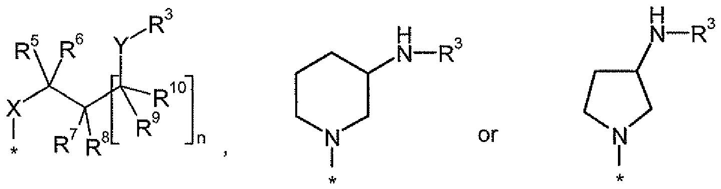 where
* is the point of attachment to the heterocycle,
n is the number 0,
X is NR¹¹, S or O, where R¹¹ is hydrogen or methyl,
Y is NR¹², S or O, where R¹² is hydrogen or methyl,
R³ is 2-pyridyl, pyrimid-2-yl, 2-aminopyrimid-4-yl, 2-cyclopropylaminopyrimid-4-yl, 2-methylaminopyrimid-4-yl, 2-ethylaminopyrimid-4-yl, 1,3-thiazol-2-yl, 1,3-thiazol-4-yl or 1,3-thiazol-5-yl, where 2-pyridyl, pyrimid-2-yl, 2-aminopyrimid-4-yl, 1,3-thiazol-2-yl, 1,3-thiazol-4-yl and 1,3-thiazol-5-yl are substituted by 1 to 3 substituents, where the substituents are selected independently of one another from the group consisting of halogen, cyano, nitro, amino, trifluoromethyl, trifluoromethoxy, aminocarbonyl, trifluoromethylcarbonyl, C₁-C₄-alkylamino, C₁-C₄-alkylcarbonyl, C₁-C₄-alkoxycarbonyl, C₁-C₄-alkylaminocarbonyl and C₃-C₆-cycloalkylcarbonyl, in which alkylamino, alkylcarbonyl, alkoxycarbonyl, alkylaminocarbonyl and cycloalkylcarbonyl may be substituted by a substituent, where the substituent is selected from the group consisting of halogen, cyano, hydroxy, amino, trifluoromethyl and C₃-C₆-cycloalkyl,
R⁵ is hydrogen or methyl,
R⁶ is hydrogen,
R⁷ is hydrogen or methyl,
R⁸ is hydrogen,
R⁹ is hydrogen,
R¹⁰ is hydrogen,
or one of the salts thereof, the solvates thereof or the solvates of the salts thereof.

3. Compound according to either of Claims 1 or 2, **characterized in that**
A is N or CR¹⁵, where
R¹⁵ is hydrogen,
R¹ is hydrogen, methyl or -CH₂R¹³, where
R¹³ is morpholinyl,
R² is phenyl, where phenyl may be substituted by 1 to 3 substituents, where the substituents are selected independently of one another from the group consisting of chlorine, fluorine, trifluoromethyl, trifluoromethoxy and methyl,
R⁴ is hydrogen,
R¹⁶ is a group of the formula 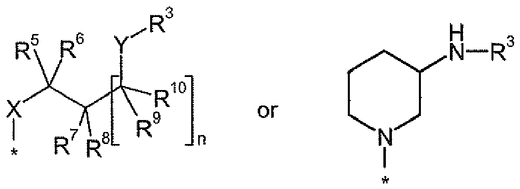 where
* is the point of attachment to the heterocycle,
n is the number 0,
X is NR¹¹, where R¹¹ is hydrogen,
Y is NR¹², where R¹² is hydrogen,
R³ is 2-pyridyl or 1,3-thiazol-2-yl, where 2-pyridyl and 1,3-thiazol-2-yl are substituted by 1 to 2 substituents, where the substituents are selected independently of one another from the group consisting of cyano, nitro, amino, trifluoromethylcarbonyl and methylcarbonyl,
R⁵is hydrogen or methyl,
R⁶is hydrogen,
R⁷is hydrogen or methyl,
R⁸is hydrogen,
R⁹is hydrogen,
R¹⁰is hydrogen,
or one of the salts thereof, the solvates thereof or the solvates of the salts thereof.

4. Process for preparing a compound of the formula (I) or one of its salts, its solvates or solvates of its salts according to Claim 1, **characterized in that**
[A] a compound of the formula 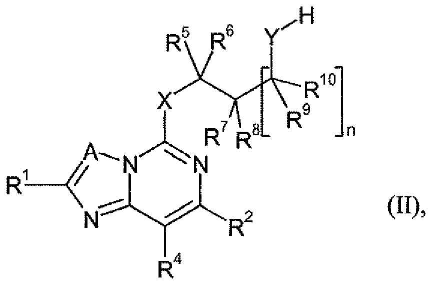 in which
A, n, X, Y, R¹, R², R⁴, R⁵, R⁶, R⁷, R⁸, R⁹ and R¹⁰ have the meaning indicated in Claim 1, is reacted
with a compound of the formula
R³-X¹ , (III)
in which
R³ has the meaning indicated in Claim 1, and
X¹ is halogen, preferably chlorine or fluorine,
or
[B] a compound of the formula 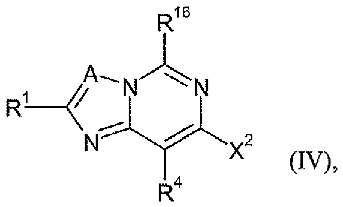 in which
A, R¹, R⁴ and R¹⁶ have the meaning indicated in Claim 1, and
X² is iodine, bromine, chlorine or trifluoromethanesulphonyl, preferably iodine or bromine,
is reacted with a compound of the formula
Q-R² , (V)
in which
R² has the meaning indicated in Claim 1, and
Q is -B(OH)₂, a boronic acid ester, preferably boronic acid pinacol ester, or -BF₃⁻K⁺,
under Suzuki coupling conditions,
or
[C] a compound of the formula 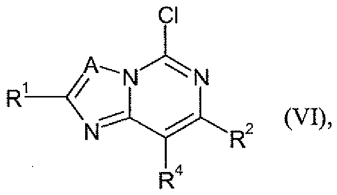 in which
A, R¹, R² and R⁴ have the meaning indicated in Claim 1, is reacted with a compound of the formula
H-R¹⁶ , (IX)
in which
R¹⁶ has the meaning indicated in Claim 1.

5. Compound according to any of Claims 1 to 3 for the treatment and/or prophylaxis of diseases.

6. Use of a compound according to any of Claims 1 to 3 for the manufacture of a medicament for the treatment and/or prophylaxis of diseases.

7. Use of a compound according to any of Claims 1 to 3 for the manufacture of a medicament for the treatment and/or prophylaxis of haematological disorders.

8. Medicament comprising a compound according to any of Claims 1 to 3 in combination with an inert, non-toxic, pharmaceutically suitable excipient.

9. Medicament according to Claim 8 for the treatment and/or prophylaxis of haematological disorders.

## Revendications

1. Composé de formule 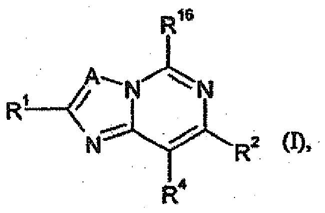 dans laquelle
A représente N ou CR¹⁵, R¹⁵ représentant hydrogène, brome ou chlore,
R¹ représente hydrogène, hydroxy, amino, hydroxycarbonyle, aminocarbonyle, trifluorométhyle, trifluorométhoxy, cyano, alkyle en C₁-C₄, alcoxy en C₁-C₄, alkylamino, en C₁-C₆, alkylcarbonyle en C₁-C₄, alcoxycarbonyle en C₁-C₄, alkylaminocarbonyle en C₁-C₆, dihydroxypropylaminocarbonyle, dihydroxybutylaminocarbonyle, dihydroxypentylaminocarbonyle, alkylcarbonylamino en C₁-C₄, alkylsulfonylamino en C₁-C₄, hétérocyclylcarbonyle à 5 ou 6 éléments, -CH₂R¹³ ou -CH₂CH₂R¹⁴, alcoxy, alkylamino, alkylcarbonyl, alcoxycarbonyle, alkylaminocarbonyle, alkylcarbonylamino et alkylsulfonylamino pouvant être substitués avec un substituant, le substituant étant choisi dans le groupe constitué par hydroxy, amino, hydroxycarbonyle, aminocarbonyle, alcoxy en C₁-C₄, alkylamino, en C₁-C₄, alcoxycarbonyle en C₁-C₄, alkylaminocarbonyle en C₁-C₄, alkylcarbonylamino en C₁-C₄ et hétérocyclyle à 5 ou 6 éléments,
hétérocyclyle pouvant être substitué avec 1 à 3 substituant, les substituants étant choisis indépendamment les uns des autres dans le groupe constitué par halogène, hydroxy, oxo, alkyle en C₁-C₄, alcoxy en C₁-C₄, alkylamino, en C₁-C₄, alkylcarbonyl, en C₁-C₄, alcoxycarbonyle en C₁-C₄ et alkylaminocarbonyle en C₁-C₄,
et
hétérocyclylcarbonyle pouvant être substitué avec 1 à 3 substituants, les substituants étant choisis indépendamment les uns des autres dans le groupe constitué par halogène, hydroxy, oxo, alkyle en C₁-C₄, alcoxy en C₁-C₄, alkylamino, en C₁-C₄, alkylcarbonyle en C₁-C₄, alcoxycarbonyle en C₁-C₄ et alkylaminocarbonyle en C₁-C₄,
et
R¹³ représentant hydroxy, amino, cyano, hydroxycarbonyle, aminocarbonyle, alcoxy en C₁-C₄, alkylamino, en C₁-C₄, alcoxycarbonyle en C₁-C₄, alkylaminocarbonyle en C₁-C₄, alkylcarbonylamino en C₁-C₄, hétérocyclyle à 5 ou 6 éléments ou hétéroaryle à 5 ou 6 éléments,
alcoxy, alkylamino, alcoxycarbonyle, alkylaminocarbonyle et alkylcarbonylamino pouvant être substitués avec un substituant, le substituant étant choisi dans le groupe constitué par hydroxy, amino, hydroxycarbonyle, aminocarbonyle, alcoxy en C₁-C₄, alkylamino, en C₁-C₄, alcoxycarbonyle en C₁-C₄, alkylaminocarbonyle en C₁-C₄ et alkylcarbonylamino en C₁-C₄,
et
hétérocyclyle et hétéroaryle pouvant être substitués avec 1 à 3 substituants, les substituants étant choisis indépendamment les uns des autres dans le groupe constitué par halogène, hydroxy, oxo, alkyle en C₁-C₄, alcoxy en C₁-C₄, alkylamino, en C₁-C₄, alkylcarbonyle en C₁-C₄, alcoxycarbonyle en C₁-C₄ et alkylaminocarbonyle en C₁-C₄,
et
R¹⁴ représentant hydroxy, amino, cyano, hydroxycarbonyle, aminocarbonyle, alcoxy en C₁-C₄, alkylamino en C₁-C₄, alcoxycarbonyle en C₁-C₄, alkylaminocarbonyle en C₁-C₄, alkylcarbonylamino en C₁-C₄, hétérocyclyle à 5 ou 6 éléments ou hétéroaryle à 5 ou 6 éléments,
alcoxy, alkylamino, alcoxycarbonyle, alkylaminocarbonyle et alkylcarbonylamino pouvant être substitués avec un substituant, le substituant étant choisi dans le groupe constitué par hydroxy, amino, hydroxycarbonyle, aminocarbonyle, alcoxy en C₁-C₄, alkylamino en C₁-C₄, alcoxycarbonyle en C₁-C₄, alkylaminocarbonyle en C₁-C₄ et alkylcarbonylamino en C₁-C₄,
et
hétérocyclyle et hétéroaryle pouvant être substitués avec 1 à 3 substituants, les substituants étant choisis indépendamment les uns des autres dans le groupe constitué par halogène, hydroxy, oxo, alkyle en C₁-C₄, alcoxy en C₁-C₄, alkylamino en C₁-C₄, alkylcarbonyle en C₁-C₄, alcoxycarbonyle en C₁-C₄ et alkylaminocarbonyle en C₁-C₄,
R² représente aryle en C₆-C₁₀ ou hétéroaryle de 5 à 10 éléments,
aryle et hétéroaryle pouvant être substitués avec 1 à 3 substituants, les substituants étant choisis indépendamment les uns des autres dans le groupe constitué par hydroxy, hydroxyméthyle, amino, halogène, cyano, trifluorométhyle, trifluorométhoxy, aminocarbonyle, alkyle en C₁-C₄, alcoxy en C₁-C₄, alcoxyméthyle en C₁-C₄, alkylamino en C₁-C₄, alkylaminométhyle en C₁-C₄, alkylcarbonyle en C₁-C₄, alcoxycarbonyle en C₁-C₄, alkylaminocarbonyle en C₁-C₄, alkylcarbonylamino en C₁-C₄, alkylsulfonyle en C₁-C₄, alkylsulfonylamino en C₁-C₄, alkylaminosulfonyle en C₁-C₄, phényle, benzyloxy, hétérocyclyle à 5 ou 6 éléments, hétérocyclylcarbonyle à 5 ou 6 éléments, hétérocyclylméthyle à 5 ou 6 éléments et hétéroaryle à 5 ou 6 éléments,
phényle, benzyloxy, hétérocyclyle, hétérocyclylcarbonyle, hétérocyclylméthyle et hétéroaryle pouvant être substitués avec 1 à 3 substituants, les substituants étant choisis indépendamment les uns des autres dans le groupe constitué par halogène, cyano, trifluorométhyle, trifluorométhoxy, aminocarbonyle, alkyle en C₁-C₄, alcoxy en C₁-C₄, alkylamino, en C₁-C₄, alkylcarbonyle, en C₁-C₄, alcoxycarbonyle en C₁-C₄, alkylaminocarbonyle en C₁-C₄ et alkylcarbonylamino en C₁-C₄,
ou
deux des substituants sur l'aryle forment avec les atomes de carbone auxquels ils sont reliés, un 1,3-dioxolane ou 1,4-dioxane,
R⁴ représente hydrogène, halogène, cyano, trifluorométhyle, alkyle en C₁-C₃, méthylthio ou cyclopropyle,
R¹⁶ représente un groupe de formule 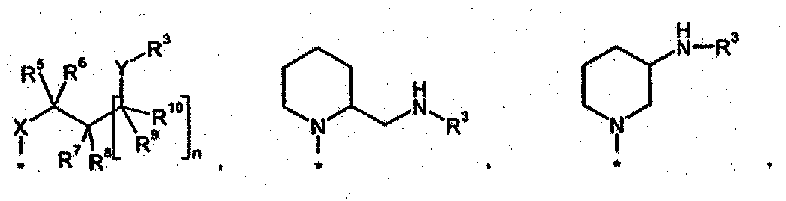 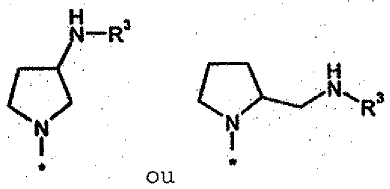 dans lesquelles
* signifie l'emplacement de liaison à l'hétérocycle,
n représente le nombre 0 ou 1,
X représente NR¹¹, S ou O, R¹¹ représentant hydrogène, alkyle en C₁-C₃ ou cyclopropyle,
Y représente NR¹², S ou O, R¹² représentant hydrogène, alkyle en C₁-C₃ ou cyclopropyle,
R³ représente 2-pyridyle, pyrimid-2-yle, 2-aminopyrimid-4-yle, 2-cyclopropylaminopyrimid-4-yle, 2-méthylaminopyrimid-4-yle, 2-éthylaminopyrimid-4-yle, 1,3-thiazol-2-yle, 1,3-thiazol-4-yle ou 1,3-thiazol-5-yle,
2-pyridyle, pyrimid-2-yle, 2-aminopyrimid-4-yle, 1,3-thiazol-2-yle, 1,3-thiazol-4-yle et 1,3-thiazol-5-yle étant substitués avec 1 à 3 substituants, les substituants étant choisis indépendamment les uns des autres dans le groupe constitué par halogène, cyano, nitro, amino, trifluorométhyle, trifluorométhoxy, aminocarbonyle, trifluorométhylcarbonyle, alkyle en C₁-C₄, alcoxy en C₁-C₄, alkylamino en C₁-C₄, alkylcarbonyle en C₁-C₄, alcoxycarbonyle en C₁-C₄, alkylaminocarbonyle en C₁-C₄ et cycloalkylcarbonyle en C₃-C₆,
alkyle, alcoxy, alkylamino, alkylcarbonyle, alcoxycarbonyle, alkylaminocarbonyle et cycloalkylcarbonyle pouvant être substitués avec un substituant, le substituant étant choisi dans le groupe constitué par halogène, cyano, hydroxy, amino, trifluorométhyle et cycloalkyle en C₃-C₆,
ou
R³ représente un groupe de formule 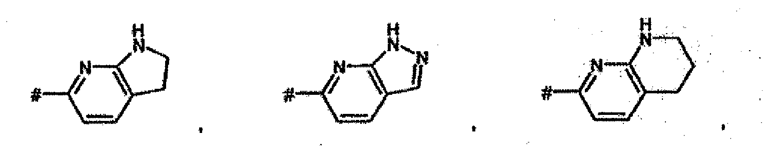 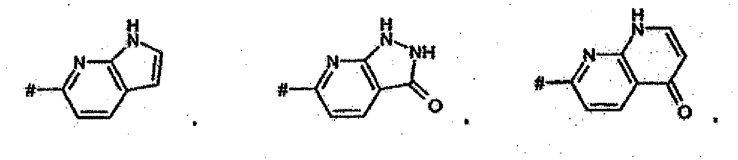 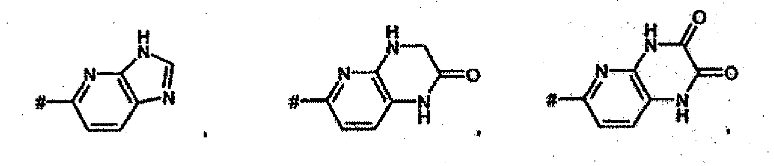 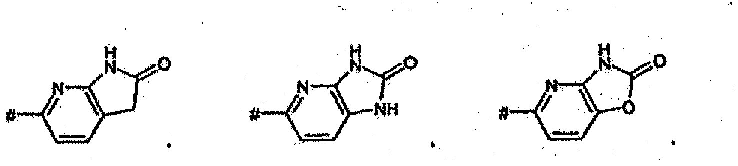 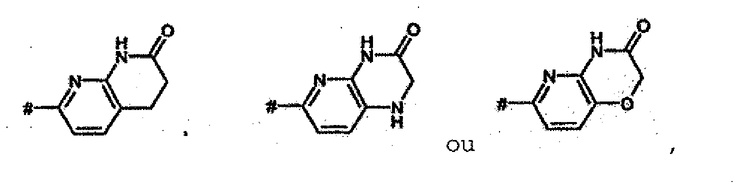
dans lesquelles # signifie l'emplacement de liaison à Y,
R⁵ représente hydrogène, alkyle en C₁-C₃ ou cyclopropyle,
R⁶ représente hydrogène ou alkyle en C₁-C₃,
R⁷ représente hydrogène, alkyle en C₁-C₃ ou cyclopropyle,
R⁸ représente hydrogène ou alkyle en C₁-C₃,
R⁹ représente hydrogène, alkyle en C₁-C₃ ou cyclopropyle,
R¹⁰ représente hydrogène ou alkyle en C₁-C₃,
ou un de ses sels, de ses solvates ou des solvates de ses sels.

2. Composé selon la revendication 1, **caractérisé en ce que**
A représente N ou CR¹⁵, R¹⁵ représentant hydrogène, R¹ représente hydrogène, trifluorométhyle, cyano, alkyle en C₁-C₄, alkylaminocarbonyle en C₁-C₆, dihydroxypropylaminocarbonyle, dihydroxybutylaminocarbonyle, dihydroxypentylaminocarbonyle, pyrrolidinylcarbonyle, pipéridinylcarbonyle, morpholinylcarbonyle, pipérazinylcarbonyle, -CH₂R¹³ ou -CH₂CH₂R¹⁴,
alkylaminocarbonyle pouvant être substitué avec un substituant, le substituant étant choisi dans le groupe constitué par hydroxy, amino, hydroxycarbonyle, aminocarbonyle, alcoxy en C₁-C₄, alkylamino en C₁-C₄, pyrrolidinyle, pipéridinyle, morpholinyle et pipérazinyle,
pyrrolidinyle, pipéridinyle, morpholinyle et pipérazinyle pouvant être substitués avec 1 à 3 substituants, les substituants étant choisis indépendamment les uns des autres dans le groupe constitué par halogène, hydroxy, oxo, alkyle en C₁-C₄, alcoxy en C₁-C₄ et alkylamino, en C₁-C₄,
et
pyrrolidinylcarbonyle, pipéridinylcarbonyle, morpholinylcarbonyle et pipérazinylcarbonyle pouvant être substitués avec 1 à 3 substituants, les substituants étant choisis indépendamment les uns des autres dans le groupe constitué par halogène, hydroxy, oxo, alkyle en C₁-C₄, alcoxy en C₁-C₄ et alkylamino en C₁-C₄,
et
R¹³ représentant hydroxycarbonyle, alcoxy en C₁-C₄, alkylamino en C₁-C₄, alkylaminocarbonyle en C₁-C₄, pyrrolidinyle, pipéridinyle, morpholinyle, pipérazinyle, pyrazolyle, imidazolyle, triazolyle ou pyridyle,
alcoxy, alkylamino, et alkylaminocarbonyle pouvant être substitués avec un substituant, le substituant étant choisi dans le groupe constitué par hydroxy, amino, hydroxycarbonyle, aminocarbonyle, alcoxy en C₁-C₄, alkylamino en C₁-C₄, alcoxycarbonyle en C₁-C₄, alkylaminocarbonyle en C₁-C₄ et alkylcarbonylamino en C₁-C₄,
et
pyrrolidinyle, pipéridinyle, morpholinyle, pipérazinyle, pyrazolyle, imidazolyle, triazolyle et pyridyle pouvant être substitués avec 1 à 3 substituant, les substituants étant choisis indépendamment les uns des autres dans le groupe constitué par halogène, hydroxy, oxo, alkyle en C₁-C₄, alcoxy en C₁-C₄ et alkylamino en C₁-C₄,
et
R¹⁴ représentant hydroxycarbonyle, alcoxy en C₁-C₄, alkylamino en C₁-C₄, alkylaminocarbonyle en C₁-C₄, pyrrolidinyle, pipéridinyle, morpholinyle, pipérazinyle, pyrazolyle, imidazolyle, triazolyle ou pyridyle,
alcoxy, alkylamino et alkylaminocarbonyle pouvant être substitués avec un substituant, le substituant étant choisi dans le groupe constitué par hydroxy, amino, hydroxycarbonyle, aminocarbonyle, alcoxy en C₁-C₄, alkylamino, en C₁-C₄, alcoxycarbonyle en C₁-C₄, alkylaminocarbonyle en C₁-C₄ et alkylcarbonylamino en C₁-C₄,
et
pyrrolidinyle, pipéridinyle, morpholinyle, pipérazinyle, pyrazolyle, imidazolyle, triazolyle et pyridyle pouvant être substitués avec 1 à 3 substituants, les substituants étant choisis indépendamment les uns des autres dans le groupe constitué par halogène, hydroxy, oxo, alkyle en C₁-C₄, alcoxy en C₁-C₄ et alkylamino en C₁-C₄,
R² représente phényle,
phényle pouvant être substitué avec 1 à 3 substituants, les substituants étant choisis indépendamment les uns des autres dans le groupe constitué par hydroxy, halogène, cyano, trifluorométhyle, trifluorométhoxy, aminocarbonyle, alkyle en C₁-C₄, alcoxy en C₁-C₄, alcoxyméthyle en C₁-C₄, alkylaminométhyle en C₁-C₄, alkylcarbonyle en C₁-C₄, alcoxycarbonyle en C₁-C₄, alkylaminocarbonyle en C₁-C₄, alkylcarbonylamino en C₁-C₄, alkylsulfonyle en C₁-C₄, alkylsulfonylamino en C₁-C₄, alkylaminosulfonyle en C₁-C₄, phényle, pyrrolidinyle, pipéridinyle, morpholinyle, pipérazinyle, pyrrolidinylméthyle, pipéridinylméthyle, morpholinylméthyle et pipérazinylméthyle,
phényle, pyrrolidinyle, pipéridinyle, morpholinyle, pipérazinyle, pyrrolidinylméthyle, pipéridinylméthyle, morpholinylméthyle et pipérazinylméthyle pouvant être substitués avec 1 à 3 substituants, les substituants étant choisis indépendamment les uns des autres dans le groupe constitué par halogène, cyano, trifluorométhyle, trifluorométhoxy et alkyle en C₁-C₄,
R⁴ représente hydrogène ou chlore,
R¹⁶ représente un groupe de formule 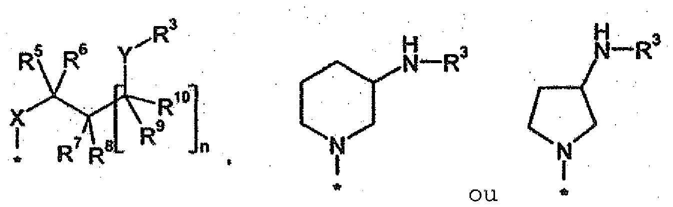
dans lesquelles
* signifie l'emplacement de liaison à l'hétérocycle,
n représente le nombre 0,
X représente NR¹¹, S ou O, R¹¹ représentant hydrogène ou méthyle,
Y représente NR¹², S ou O, R¹² représentant hydrogène ou méthyle,
R³ représente 2-pyridyle, pyrimid-2-yle, 2-aminopyrimid-4-yle, 2-cyclopropylaminopyrimid-4-yle, 2-méthylaminopyrimid-4-yle, 2-éthylaminopyrimid-4-yle, 1,3-thiazol-2-yle, 1,3-thiazol-4-yle ou 1,3-thiazol-5-yle,
2-pyridyle, pyrimid-2-yle, 2-aminopyrimid-4-yle, 1,3-thiazol-2-yle, 1,3-thiazol-4-yle et 1,3-thiazol-5-yle étant substitués avec 1 à 3 substituant, les substituants étant choisis indépendamment les uns des autres dans le groupe constitué par halogène, cyano, nitro, amino, trifluorométhyle, trifluorométhoxy, aminocarbonyle, trifluorométhylcarbonyle, alkylamino en C₁-C₄, alkylcarbonyl, en C₁-C₄, alcoxycarbonyle en C₁-C₄, alkylaminocarbonyle en C₁-C₄ et cycloalkylcarbonyle en C₃-C₆,
alkylamino, alkylcarbonyle, alcoxycarbonyle, alkylaminocarbonyle et cycloalkylcarbonyle pouvant être substitués avec un substituant, le substituant étant choisi dans le groupe constitué par halogène, cyano, hydroxy, amino, trifluorométhyle et cycloalkyle en C₃-C₆,
R⁵ représente hydrogène ou méthyle,
R⁶ représente hydrogène,
R⁷ représente hydrogène ou méthyle,
R⁸ représente hydrogène,
R⁹ représente hydrogène,
R¹⁰ représente hydrogène,
ou un de ses sels, de ses solvates ou des solvates de ses sels.

3. Composé selon l'une quelconque des revendications 1 ou 2, **caractérisé en ce que**
A représente N ou CR¹⁵, R¹⁵ représentant hydrogène,
R¹ représente hydrogène, méthyle ou -CH₂R¹³, R¹³ représentant morpholinyle,
R² représente phényle, phényle pouvant être substitué avec 1 à 3 substituants, les substituants étant choisis indépendamment les uns des autres dans le groupe constitué par chlore, fluor, trifluorométhyle, trifluorométhoxy et méthyle,
R⁴ représente hydrogène,
R¹⁶ représente un groupe de formule 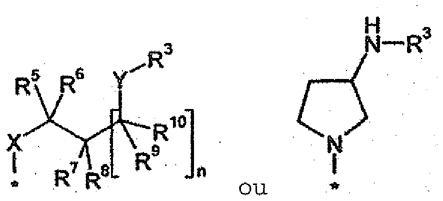
dans lesquelles
* signifie l'emplacement de liaison à l'hétérocycle,
n représente le nombre 0,
X représente NR¹¹, R¹¹ représentant hydrogène,
Y représente NR¹², R¹² représentant hydrogène,
R³ représente 2-pyridyle ou 1,3-thiazol-2-yle, 2-pyridyle et 1,3-thiazol-2-yle étant substitués avec 1 à 2 substituants, les substituants étant choisis indépendamment les uns des autres dans le groupe constitué par cyano, nitro, amino, trifluorométhylcarbonyle et méthylcarbonyle,
R⁵ représente hydrogène ou méthyle,
R⁶ représente hydrogène,
R⁷ représente hydrogène ou méthyle,
R⁸ représente hydrogène,
R⁹ représente hydrogène,
R¹⁰ représente hydrogène,
ou un de ses sels, de ses solvates ou des solvates de ses sels.

4. Procédé de fabrication d'un composé de formule (I) ou d'un de ses sels, de ses solvates ou des solvates de ses sels selon la revendication 1, **caractérisé en ce que**
[A] un composé de formule 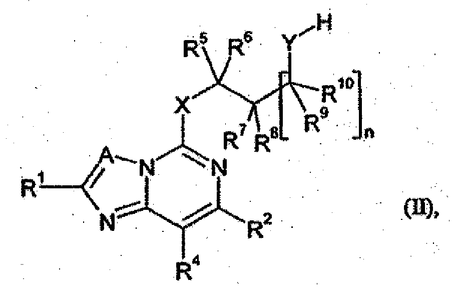 dans laquelle
A, n, X, Y, R¹, R², R⁴, R⁵, R⁶, R⁷, R⁸, R⁹ et R¹⁰ ont la signification donnée dans la revendication 1, est mis en réaction avec un composé de formule
R³-X¹ , (III)
dans laquelle
R³ a la signification donnée dans la revendication 1, et
X¹ représente halogène, de préférence chlore ou fluor,
ou
[B] un composé de formule 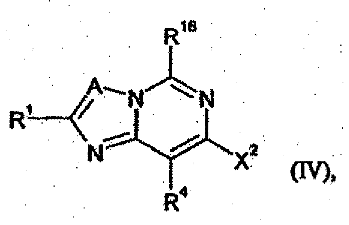 dans laquelle
A, R¹, R⁴ et R¹⁶ ont la signification donnée dans la revendication 1, et
X² représente iode, brome, chlore ou trifluorométhanesulfonyle, de préférence iode ou brome, est mis en réaction avec un composé de formule
Q-R² , (V)
dans laquelle
R² a la signification donnée dans la revendication 1, et Q représente -B(OH)₂, un ester de l'acide boronique, de préférence l'ester pinacolique de l'acide boronique, ou -BF₃⁻K⁺,
dans les conditions de couplage de Suzuki,
ou
[C] un composé de formule 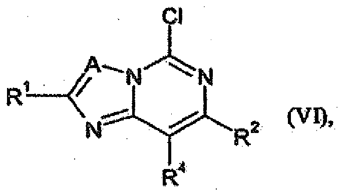 dans laquelle
A, R¹, R² et R⁴ ont la signification donnée dans la revendication 1,
est mis en réaction avec un composé de formule
H-R¹⁶ , (IX)
dans laquelle
R¹⁶ a la signification donnée dans la revendication 1.

5. Composé selon l'une quelconque des revendications 1 à 3 pour le traitement et/ou la prophylaxie de maladies.

6. Utilisation d'un composé selon l'une quelconque des revendications 1 à 3 pour la fabrication d'un médicament pour le traitement et/ou la prophylaxie de maladies.

7. Utilisation d'un composé selon l'une quelconque des revendications 1 à 3 pour la fabrication d'un médicament pour le traitement et/ou la prophylaxie de maladies hématologiques.

8. Médicament contentant un composé selon l'une quelconque des revendications 1 à 3 en combinaison avec un adjuvant inerte, non toxique et pharmaceutiquement approprié.

9. Médicament selon la revendication 8 pour le traitement et/ou la prophylaxie de maladies hématologiques.
